# EUROPEAN PATENT APPLICATION

(11) **EP 1 792 995 A2**
(43) Date of publication of application: **06.06.2007**
(21) Application number: 06024788.9
(22) Date of filing: 08.05.2001
(51) Int. Cl.: C12N 15/85, A61K 48/00, C07K 19/00, C07K 16/12, A61K 39/118, A61K 39/40

(54) **Chlamydia secretory locus orf and uses thereof**

(30) Priority: 08.05.2000 US 202672 P; 30.05.2000 US 207852; 16.06.2000 US 211801; 16.06.2000 US 212044; 16.06.2000 US 211797; 16.06.2000 US 211796; 16.06.2000 US 211798; 26.09.2000 US 235335; 26.09.2000 US 235361; 26.09.2000 US 235398
(62) Divisional of application: 01931274.3
(71) Applicant: Sanofi Pasteur Limited, Toronto, Ontario M2R 3T4 (CA)
(72) Inventor: Murdin, Andrew D., Richmond Hill Ontario L4B 3C2 (CA); Oomen, Raymond P., Aurora Ontario L4G 2L6 (CA); Wang, Joe, Toronto Ontario M2H 2E8 (CA); Dunn, Pamela, Woodbridge Ontario L4L 3Z1 (CA)
(74) Representative: Desaix, Anne

(57) **Abstract**

The present invention provides nucleic acids, proteins and vectors for a method of nucleic acid, including DNA, immunization of a host, including humans, against disease caused by infection by a strain of *Chlamydia,* specifically *C*. *pneumoniae*. The method employs a vector containing a nucleotide sequence encoding a polypeptide of a strain of *Chlamydia pneumoniae* operably linked to a promoter to effect expression of the gene product in the host. The polypeptides are derived from *C*. *pneumoniae* and are selected from an ATP-binding cassette protein, a secretory locus ORF, an endopeptidase, a protease, a metalloprotease, CLP protease ATPase, a CLP protease subunit, a translycolase/transpeptidase, a CLPc protease and thioredoxin.

### Annex to the application documents - subsequently filed sequences listing

SEQUENCE LISTING

<110> Aventis Pasteur Limited

<120> Chlamydia antigens and corresponding DNA fragments and uses thereof

<130> 77813-123D

<140> EP06024788.9
<141> 2001-05-08

<150> US 60/194,475
<151> 2000-04-04

<160> 74

<170> PatentIn Ver. 2.0

<210> 1
<211> 1787
<212> DNA
<213> Chlamydia pneumoniae

<220>
<221> CDS
<222> (101)..(1684)

<400> 1
aatctcattc ccccatcgac taaatccacc acggactccg acctcccatg tcttcaatcc 60

atatgaacgt aatattaagt agcaaattga gtactatata atg aag atg cat agg 115
Met Lys Met His Arg
1               5

ctt aaa cct acc tta aaa agt ctg atc cct aat ctt ctt ttc tta ttg 163
Leu Lys Pro Thr Leu Lys Ser Leu Ile Pro Asn Leu Leu Phe Leu Leu
                10                  15                  20

ctc act ctt tca agc tgc tca aag caa aaa caa gaa ccc tta gga aaa 211
Leu Thr Leu Ser Ser Cys Ser Lys Gln Lys Gln Glu Pro Leu Gly Lys
            25                  30                  35

cat ctc gtt att gcg atg agc cat gat ctc gcc gac cta gat cct cgc 259
His Leu Val Ile Ala Met Ser His Asp Leu Ala Asp Leu Asp Pro Arg
        40                  45                  50

aat gcc tat tta agc aga gat gct tcc cta gca aaa gcc ctc tat gaa 307
Asn Ala Tyr Leu Ser Arg Asp Ala Ser Leu Ala Lys Ala Leu Tyr Glu
    55                  60                  65

gga ctg aca aga gaa act gat caa gga atc gca ctg gct ctt gca gaa 355
Gly Leu Thr Arg Glu Thr Asp Gln Gly Ile Ala Leu Ala Leu Ala Glu
70                  75                  80                  85

agt tat acc ctg tca aaa gat cat aag gtc tat acc ttt aaa ctc aga 403
Ser Tyr Thr Leu Ser Lys Asp His Lys Val Tyr Thr Phe Lys Leu Arg
                90                  95                  100

cct tct gtg tgg agc gat ggc act cca ctc act gct tat gac ttt gaa 451
Pro Ser Val Trp Ser Asp Gly Thr Pro Leu Thr Ala Tyr Asp Phe Glu
            105                 110                 115

aaa tct ata aaa caa ctg tac ttc gaa gaa ttt tca cct tcc ata cat 499
Lys Ser Ile Lys Gln Leu Tyr Phe Glu Glu Phe Ser Pro Ser Ile His
        120                 125                 130

act tta ctc ggc gtg att aaa aat tct tcg gca atc cac aat gct caa 547
Thr Leu Leu Gly Val Ile Lys Asn Ser Ser Ala Ile His Asn Ala Gln

135                 140                 145

aaa tct ctg gaa act ctt ggg ata cag gca aaa gat gat ctt act ttg     595
Lys Ser Leu Glu Thr Leu Gly Ile Gln Ala Lys Asp Asp Leu Thr Leu
150                 155                 160                 165

gtg att acc cta gag caa cct ttc cca tac ttt ctc aca ctt atc gct     643
Val Ile Thr Leu Glu Gln Pro Phe Pro Tyr Phe Leu Thr Leu Ile Ala
                170                 175                 180

cgc ccc gta ttc tcc cct gtt cat cac acc ctt agg gaa tcc tat aag     691
Arg Pro Val Phe Ser Pro Val His His Thr Leu Arg Glu Ser Tyr Lys
            185                 190                 195

aaa gga aca ccc cca tcc aca tac atc tcc aat ggg ccc ttt gtc tta     739
Lys Gly Thr Pro Pro Ser Thr Tyr Ile Ser Asn Gly Pro Phe Val Leu
        200                 205                 210

aaa aaa cat gaa cac caa aac tac tta att tta gaa aaa aat cct cac     787
Lys Lys His Glu His Gln Asn Tyr Leu Ile Leu Glu Lys Asn Pro His
    215                 220                 225

tac tat gat cat gaa tca gta aag tta gac cga gtc acc tta aaa att     835
Tyr Tyr Asp His Glu Ser Val Lys Leu Asp Arg Val Thr Leu Lys Ile
230                 235                 240                 245

atc cca gac gcc tcc aca gcc acg aaa ctt ttc aaa agt aaa tct ata     883
Ile Pro Asp Ala Ser Thr Ala Thr Lys Leu Phe Lys Ser Lys Ser Ile
                250                 255                 260

gat tgg att ggc tca cct tgg agc gct ccg ata tct aac gaa gac caa     931
Asp Trp Ile Gly Ser Pro Trp Ser Ala Pro Ile Ser Asn Glu Asp Gln
            265                 270                 275

aaa gtt ctc tcc caa gaa aag att ctt acc tat tct gtt tca agc acc     979
Lys Val Leu Ser Gln Glu Lys Ile Leu Thr Tyr Ser Val Ser Ser Thr
        280                 285                 290

acc ctt ctt atc tat aac ctg caa aaa cct cta ata caa aat aaa gcc     1027
Thr Leu Leu Ile Tyr Asn Leu Gln Lys Pro Leu Ile Gln Asn Lys Ala
    295                 300                 305

ctc agg aaa gcc att gct cat gct att gat aga aaa tct atc tta aga     1075
Leu Arg Lys Ala Ile Ala His Ala Ile Asp Arg Lys Ser Ile Leu Arg
310                 315                 320                 325

ctc gtg cct tca gga caa gaa gct gta act cta gtt ccc cca aat ctt     1123
Leu Val Pro Ser Gly Gln Glu Ala Val Thr Leu Val Pro Pro Asn Leu
                330                 335                 340

tca caa ctc aat ctt caa aaa gag atc tca aca gaa gaa cga caa aca     1171
Ser Gln Leu Asn Leu Gln Lys Glu Ile Ser Thr Glu Glu Arg Gln Thr
            345                 350                 355

aaa gcc aga gca tat ttt caa gaa gct aaa gaa aca ctt tct gaa aaa     1219
Lys Ala Arg Ala Tyr Phe Gln Glu Ala Lys Glu Thr Leu Ser Glu Lys
        360                 365                 370

gaa ctc gca gaa ctc agc atc ctc tat cct ata gat tcc tcg aat tcc     1267
Glu Leu Ala Glu Leu Ser Ile Leu Tyr Pro Ile Asp Ser Ser Asn Ser
    375                 380                 385

tcc atc ata gct caa gaa atc caa aga caa ctt aaa gat acc tta gga     1315
Ser Ile Ile Ala Gln Glu Ile Gln Arg Gln Leu Lys Asp Thr Leu Gly
390                 395                 400                 405

ttg aaa atc aaa atc caa ggc atg gag tac cac tgc ttt tta aag aaa     1363
Leu Lys Ile Lys Ile Gln Gly Met Glu Tyr His Cys Phe Leu Lys Lys

410                 415                 420

cgt cgt caa gga gat ttc ttc ata gcg aca gga gga tgg att gcg gaa    1411
Arg Arg Gln Gly Asp Phe Phe Ile Ala Thr Gly Gly Trp Ile Ala Glu
            425                 430                 435

tac gta agc ccc gta gcc ttc cta tct att cta ggc aac ccc aga gac    1459
Tyr Val Ser Pro Val Ala Phe Leu Ser Ile Leu Gly Asn Pro Arg Asp
        440                 445                 450

ctc aca caa tgg aga aac agt gat tac gaa aag act tta gag aaa ctc    1507
Leu Thr Gln Trp Arg Asn Ser Asp Tyr Glu Lys Thr Leu Glu Lys Leu
    455                 460                 465

tat ctc cct cat gcc tac aaa gag aat tta aaa cgc gca gaa atg ata    1555
Tyr Leu Pro His Ala Tyr Lys Glu Asn Leu Lys Arg Ala Glu Met Ile
470                 475                 480                 485

ata gaa gaa gaa acc ccg att atc ccc ctg tat cac ggc aaa tat att    1603
Ile Glu Glu Glu Thr Pro Ile Ile Pro Leu Tyr His Gly Lys Tyr Ile
                490                 495                 500

tac gct ata cat cct aaa atc cag aat aca ttc gga tct ctt cta ggc    1651
Tyr Ala Ile His Pro Lys Ile Gln Asn Thr Phe Gly Ser Leu Leu Gly
            505                 510                 515

cac aca gat ctc aaa aat atc gat atc tta agt tagatccgaa atggaaaaat 1704
His Thr Asp Leu Lys Asn Ile Asp Ile Leu Ser
        520                 525

taaaaatttt atagacaatc ttgaaaagag aattaaaaat ttttaattta aattatagtt 1764

gcaattgaaa acgcccctaa gaa                                          1787

<210> 2
<211> 528
<212> PRT
<213> Chlamydia pneumoniae

<220>
<221> SITE
<222> (188)...(204)
<223> B-cell epitope

<220>
<221> SITE
<222> (345)...(355)
<223> B-cell epitope

<220>
<221> SITE
<222> (434)...(442)
<223> T-cell epitope

<400> 2
Met Lys Met His Arg Leu Lys Pro Thr Leu Lys Ser Leu Ile Pro Asn
 1               5                  10                  15

Leu Leu Phe Leu Leu Leu Thr Leu Ser Ser Cys Ser Lys Gln Lys Gln
            20                  25                  30

Glu Pro Leu Gly Lys His Leu Val Ile Ala Met Ser His Asp Leu Ala
        35                  40                  45

Asp Leu Asp Pro Arg Asn Ala Tyr Leu Ser Arg Asp Ala Ser Leu Ala
    50                  55                  60

Lys Ala Leu Tyr Glu Gly Leu Thr Arg Glu Thr Asp Gln Gly Ile Ala
65                  70                  75                  80

Leu Ala Leu Ala Glu Ser Tyr Thr Leu Ser Lys Asp His Lys Val Tyr
                85                  90                  95

Thr Phe Lys Leu Arg Pro Ser Val Trp Ser Asp Gly Thr Pro Leu Thr
            100                 105                 110

Ala Tyr Asp Phe Glu Lys Ser Ile Lys Gln Leu Tyr Phe Glu Glu Phe
        115                 120                 125

Ser Pro Ser Ile His Thr Leu Leu Gly Val Ile Lys Asn Ser Ser Ala
    130                 135                 140

Ile His Asn Ala Gln Lys Ser Leu Glu Thr Leu Gly Ile Gln Ala Lys
145                 150                 155                 160

Asp Asp Leu Thr Leu Val Ile Thr Leu Glu Gln Pro Phe Pro Tyr Phe
                165                 170                 175

Leu Thr Leu Ile Ala Arg Pro Val Phe Ser Pro Val His His Thr Leu
            180                 185                 190

Arg Glu Ser Tyr Lys Lys Gly Thr Pro Pro Ser Thr Tyr Ile Ser Asn
        195                 200                 205

Gly Pro Phe Val Leu Lys Lys His Glu His Gln Asn Tyr Leu Ile Leu
    210                 215                 220

Glu Lys Asn Pro His Tyr Tyr Asp His Glu Ser Val Lys Leu Asp Arg
225                 230                 235                 240

Val Thr Leu Lys Ile Ile Pro Asp Ala Ser Thr Ala Thr Lys Leu Phe
                245                 250                 255

Lys Ser Lys Ser Ile Asp Trp Ile Gly Ser Pro Trp Ser Ala Pro Ile
            260                 265                 270

Ser Asn Glu Asp Gln Lys Val Leu Ser Gln Glu Lys Ile Leu Thr Tyr
        275                 280                 285

Ser Val Ser Ser Thr Thr Leu Leu Ile Tyr Asn Leu Gln Lys Pro Leu
    290                 295                 300

Ile Gln Asn Lys Ala Leu Arg Lys Ala Ile Ala His Ala Ile Asp Arg
305                 310                 315                 320

Lys Ser Ile Leu Arg Leu Val Pro Ser Gly Gln Glu Ala Val Thr Leu
                325                 330                 335

Val Pro Pro Asn Leu Ser Gln Leu Asn Leu Gln Lys Glu Ile Ser Thr
            340                 345                 350

Glu Glu Arg Gln Thr Lys Ala Arg Ala Tyr Phe Gln Glu Ala Lys Glu
        355                 360                 365

Thr Leu Ser Glu Lys Glu Leu Ala Glu Leu Ser Ile Leu Tyr Pro Ile
    370                 375                 380

Asp Ser Ser Asn Ser Ser Ile Ile Ala Gln Glu Ile Gln Arg Gln Leu
385                 390                 395                 400

Lys Asp Thr Leu Gly Leu Lys Ile Lys Ile Gln Gly Met Glu Tyr His
                405                 410                 415

Cys Phe Leu Lys Lys Arg Arg Gln Gly Asp Phe Phe Ile Ala Thr Gly
            420                 425                 430

Gly Trp Ile Ala Glu Tyr Val Ser Pro Val Ala Phe Leu Ser Ile Leu
        435                 440                 445

Gly Asn Pro Arg Asp Leu Thr Gln Trp Arg Asn Ser Asp Tyr Glu Lys
    450                 455                 460

Thr Leu Glu Lys Leu Tyr Leu Pro His Ala Tyr Lys Glu Asn Leu Lys
465                 470                 475                 480

Arg Ala Glu Met Ile Ile Glu Glu Glu Thr Pro Ile Ile Pro Leu Tyr
                485                 490                 495

His Gly Lys Tyr Ile Tyr Ala Ile His Pro Lys Ile Gln Asn Thr Phe
            500                 505                 510

Gly Ser Leu Leu Gly His Thr Asp Leu Lys Asn Ile Asp Ile Leu Ser
        515                 520                 525

<210> 3
<211> 1226
<212> DNA
<213> Chlamydia pneumoniae

<220>
<221> CDS
<222> (101)..(1123)

<400> 3
ttccagagaa atcctgatcc tgaaaaactt cctgaaacaa ttgctttaac tataacacgg 60

gaacctaaag catatcctcc aaggacgtta acataccaat ttg cgg ttg gga aat    115
                                           Leu Arg Leu Gly Asn
                                           1               5

aag cct atg caa cct ttt atc ttt act tta ctg tgc ttg aca tct ttg    163
Lys Pro Met Gln Pro Phe Ile Phe Thr Leu Leu Cys Leu Thr Ser Leu
                10                  15                  20

gtt tct tta gtc gcc ttt gat gct gcg aat gct cgt aaa cgt tgt gcc    211
Val Ser Leu Val Ala Phe Asp Ala Ala Asn Ala Arg Lys Arg Cys Ala
            25                  30                  35

tgt gct caa act ata gaa cgt gga gag aac ttc ttt tcc ata aaa cgc    259
Cys Ala Gln Thr Ile Glu Arg Gly Glu Asn Phe Phe Ser Ile Lys Arg
        40                  45                  50

tct gct tgt gct gaa atc gaa tat caa gaa aaa tct cgc cac gcc tca    307
Ser Ala Cys Ala Glu Ile Glu Tyr Gln Glu Lys Ser Arg His Ala Ser
    55                  60                  65

gca att gaa aga atc tca aaa gat aaa ggc aaa gtc act cca aag cag    355
Ala Ile Glu Arg Ile Ser Lys Asp Lys Gly Lys Val Thr Pro Lys Gln
70                  75                  80                  85

att gcg aaa gta gct act aag aaa aag caa aga tac cgt tta ttg cag    403
Ile Ala Lys Val Ala Thr Lys Lys Lys Gln Arg Tyr Arg Leu Leu Gln
                90                  95                  100

gtt cct ttt tca agg cct ccg aat aac tca agg tat aac ctc tat gct    451
Val Pro Phe Ser Arg Pro Pro Asn Asn Ser Arg Tyr Asn Leu Tyr Ala
            105                 110                 115

ttg ctt agt gaa cct ccc gaa tgc tat agc gat aca gca tca tgg tat    499
Leu Leu Ser Glu Pro Pro Glu Cys Tyr Ser Asp Thr Ala Ser Trp Tyr
        120                 125                 130

gct att ttt att cgg tta ctt cga cgt gct tat gta gac acg gga aat    547

Ala Ile Phe Ile Arg Leu Leu Arg Arg Ala Tyr Val Asp Thr Gly Asn
    135                 140                 145

gta cct cct gga tct gag tat gcc atc gct aat gct ttg ata agt aac     595
Val Pro Pro Gly Ser Glu Tyr Ala Ile Ala Asn Ala Leu Ile Ser Asn
150                 155                 160                 165

aaa caa gag att tta gag agg gga gcg cag ctt gga ccc gat gtt att     643
Lys Gln Glu Ile Leu Glu Arg Gly Ala Gln Leu Gly Pro Asp Val Ile
                170                 175                 180

gaa act cta aca ttg cct gag gaa caa gcc gag att ttt tat aaa atg     691
Glu Thr Leu Thr Leu Pro Glu Glu Gln Ala Glu Ile Phe Tyr Lys Met
            185                 190                 195

ctc aaa ggg tcg tca aac tct cag tcg cta ctg aat ttt ctg cat tat     739
Leu Lys Gly Ser Ser Asn Ser Gln Ser Leu Leu Asn Phe Leu His Tyr
        200                 205                 210

gaa gag aaa agc tta ggc cac tgt aag cta aat ctg atc ttc atg gat     787
Glu Glu Lys Ser Leu Gly His Cys Lys Leu Asn Leu Ile Phe Met Asp
    215                 220                 225

ccc cta ctg tta gaa gct gtt cta gat cat ccc gat gct tat agg gaa     835
Pro Leu Leu Leu Glu Ala Val Leu Asp His Pro Asp Ala Tyr Arg Glu
230                 235                 240                 245

acg tcg ctc ctg cgc gat ggc att tgg gaa gcg gtg aag cgt caa gaa     883
Thr Ser Leu Leu Arg Asp Gly Ile Trp Glu Ala Val Lys Arg Gln Glu
                250                 255                 260

cat gcc atc caa gaa cat ggc cag gca gct gct ttg gag ctt ttt aaa     931
His Ala Ile Gln Glu His Gly Gln Ala Ala Ala Leu Glu Leu Phe Lys
            265                 270                 275

aca cgc acc gac ttc cgc ctg gag ctg cga gat aag atg cag tta ctt     979
Thr Arg Thr Asp Phe Arg Leu Glu Leu Arg Asp Lys Met Gln Leu Leu
        280                 285                 290

cta agt cga tac gat ttg ctc ccc tta tta aat aaa aaa atg ttc gac     1027
Leu Ser Arg Tyr Asp Leu Leu Pro Leu Leu Asn Lys Lys Met Phe Asp
    295                 300                 305

tac acc tta gga agt gcc gga gat tac tta ttt ttg gta gac cca gat     1075
Tyr Thr Leu Gly Ser Ala Gly Asp Tyr Leu Phe Leu Val Asp Pro Asp
310                 315                 320                 325

act aag gca att tct cga tgt cgc tgc cct tca aag agt att aaa tta     1123
Thr Lys Ala Ile Ser Arg Cys Arg Cys Pro Ser Lys Ser Ile Lys Leu
                330                 335                 340

taatttaatt ttaatattta ttttaaatag ttttttttga taattgtctt aataagtact 1183

ataaaaaata tttctatagg taggaccatg gcagacgaga ccc                   1226


<210> 4
<211> 341
<212> PRT
<213> Chlamydia pneumoniae

<400> 4
Leu Arg Leu Gly Asn Lys Pro Met Gln Pro Phe Ile Phe Thr Leu Leu
1               5                   10                  15

Cys Leu Thr Ser Leu Val Ser Leu Val Ala Phe Asp Ala Ala Asn Ala
            20                  25                  30

Arg Lys Arg Cys Ala Cys Ala Gln Thr Ile Glu Arg Gly Glu Asn Phe
        35                  40                  45

Phe Ser Ile Lys Arg Ser Ala Cys Ala Glu Ile Glu Tyr Gln Glu Lys
    50                  55                  60

Ser Arg His Ala Ser Ala Ile Glu Arg Ile Ser Lys Asp Lys Gly Lys
65                  70                  75                  80

Val Thr Pro Lys Gln Ile Ala Lys Val Ala Thr Lys Lys Lys Gln Arg
                85                  90                  95

Tyr Arg Leu Leu Gln Val Pro Phe Ser Arg Pro Pro Asn Asn Ser Arg
            100                 105                 110

Tyr Asn Leu Tyr Ala Leu Leu Ser Glu Pro Pro Glu Cys Tyr Ser Asp
        115                 120                 125

Thr Ala Ser Trp Tyr Ala Ile Phe Ile Arg Leu Leu Arg Arg Ala Tyr
    130                 135                 140

Val Asp Thr Gly Asn Val Pro Pro Gly Ser Glu Tyr Ala Ile Ala Asn
145                 150                 155                 160

Ala Leu Ile Ser Asn Lys Gln Glu Ile Leu Glu Arg Gly Ala Gln Leu
                165                 170                 175

Gly Pro Asp Val Ile Glu Thr Leu Thr Leu Pro Glu Glu Gln Ala Glu
            180                 185                 190

Ile Phe Tyr Lys Met Leu Lys Gly Ser Ser Asn Ser Gln Ser Leu Leu
        195                 200                 205

Asn Phe Leu His Tyr Glu Glu Lys Ser Leu Gly His Cys Lys Leu Asn
    210                 215                 220

Leu Ile Phe Met Asp Pro Leu Leu Leu Glu Ala Val Leu Asp His Pro
225                 230                 235                 240

Asp Ala Tyr Arg Glu Thr Ser Leu Leu Arg Asp Gly Ile Trp Glu Ala
                245                 250                 255

Val Lys Arg Gln Glu His Ala Ile Gln Glu His Gly Gln Ala Ala Ala
            260                 265                 270

Leu Glu Leu Phe Lys Thr Arg Thr Asp Phe Arg Leu Glu Leu Arg Asp
        275                 280                 285

Lys Met Gln Leu Leu Leu Ser Arg Tyr Asp Leu Leu Pro Leu Leu Asn
    290                 295                 300

Lys Lys Met Phe Asp Tyr Thr Leu Gly Ser Ala Gly Asp Tyr Leu Phe
305                 310                 315                 320

Leu Val Asp Pro Asp Thr Lys Ala Ile Ser Arg Cys Arg Cys Pro Ser
                325                 330                 335

Lys Ser Ile Lys Leu
            340

<210> 5
<211> 1235
<212> DNA
<213> Chlamydia pneumoniae

<220>
<221> CDS
<222> (101)..(1132)

<400> 5
gttacttttt ttttcataaa aaccccatgt aacttttact tgctcatatt gagaagtccc 60

ccatactata aaaggcaacg ttttcttttc ttggtttttt atg ctc acc cta ggc 115
Met Leu Thr Leu Gly
1               5

ttg gaa agt tct tgc gat gag act gcc tgc gct ata gtt aat gag gat 163
Leu Glu Ser Ser Cys Asp Glu Thr Ala Cys Ala Ile Val Asn Glu Asp
                10                  15                  20

aag cag ata tta gca aat att att gcc tct caa gat atc cat gca tcc 211
Lys Gln Ile Leu Ala Asn Ile Ile Ala Ser Gln Asp Ile His Ala Ser
            25                  30                  35

tat ggc gga gtc gtt cct gaa ctt gct tca aga gca cat ctc cat atc 259
Tyr Gly Gly Val Val Pro Glu Leu Ala Ser Arg Ala His Leu His Ile
        40                  45                  50

ttc cca caa gtg ata aat aaa gct cta caa cag gcc aac tta ttg atc 307
Phe Pro Gln Val Ile Asn Lys Ala Leu Gln Gln Ala Asn Leu Leu Ile
    55                  60                  65

gaa gat atg gat ctg att gca gta acg caa act cca ggg ttg ata ggt 355
Glu Asp Met Asp Leu Ile Ala Val Thr Gln Thr Pro Gly Leu Ile Gly
70                  75                  80                  85

tct cta tca gta gga gtg cat ttt ggt aaa ggc att gcc ata gga gca 403
Ser Leu Ser Val Gly Val His Phe Gly Lys Gly Ile Ala Ile Gly Ala
                90                  95                  100

aaa aaa tcc ttg att gga gtc aat cac gtc gaa gct cat ctc tat gct 451
Lys Lys Ser Leu Ile Gly Val Asn His Val Glu Ala His Leu Tyr Ala
            105                 110                 115

gcc tat atg gca gcg caa aac gtg caa ttc cct gct tta ggt ctt gtg 499
Ala Tyr Met Ala Ala Gln Asn Val Gln Phe Pro Ala Leu Gly Leu Val
        120                 125                 130

gtc tct gga gct cat acc gca gcg ttt ttt ata gaa aat cct aca tcc 547
Val Ser Gly Ala His Thr Ala Ala Phe Phe Ile Glu Asn Pro Thr Ser
    135                 140                 145

tat aaa ctc ata gga aaa act cga gat gat gct ata gga gaa act ttt 595
Tyr Lys Leu Ile Gly Lys Thr Arg Asp Asp Ala Ile Gly Glu Thr Phe
150                 155                 160                 165

gat aaa gta gga cgc ttt cta gga tta cca tac cct gca ggc cca tta 643
Asp Lys Val Gly Arg Phe Leu Gly Leu Pro Tyr Pro Ala Gly Pro Leu
                170                 175                 180

att gaa aaa ctc gct tta gaa ggc tct gag gac agt tat cct ttt agt 691
Ile Glu Lys Leu Ala Leu Glu Gly Ser Glu Asp Ser Tyr Pro Phe Ser
            185                 190                 195

cca gct aaa gtc cca aac tat gac ttt tca ttc agc ggt ctt aaa aca 739
Pro Ala Lys Val Pro Asn Tyr Asp Phe Ser Phe Ser Gly Leu Lys Thr
        200                 205                 210

gct gtt ctc tac gca atc aaa gga aat aat agt agc ccc cgc tct cct 787
Ala Val Leu Tyr Ala Ile Lys Gly Asn Asn Ser Ser Pro Arg Ser Pro
    215                 220                 225

gct cca gag ata tct tta gaa aaa caa aga gat atc gct gct tca ttt 835
Ala Pro Glu Ile Ser Leu Glu Lys Gln Arg Asp Ile Ala Ala Ser Phe
230                 235                 240                 245

caa aaa gcg gcc tgc act act att gca caa aaa ctt ccc act att ata    883
Gln Lys Ala Ala Cys Thr Thr Ile Ala Gln Lys Leu Pro Thr Ile Ile
                250                 255                 260

aaa gaa ttt tcg tgc cga tct ata ctt att gga ggt ggc gta gcc att    931
Lys Glu Phe Ser Cys Arg Ser Ile Leu Ile Gly Gly Gly Val Ala Ile
            265                 270                 275

aat gaa tac ttt aga tcc gca ata caa act gcg tgt aat cta cct gta    979
Asn Glu Tyr Phe Arg Ser Ala Ile Gln Thr Ala Cys Asn Leu Pro Val
        280                 285                 290

tac ttc ccc cct gct aaa cta tgc tca gat aat gct gct atg att gca    1027
Tyr Phe Pro Pro Ala Lys Leu Cys Ser Asp Asn Ala Ala Met Ile Ala
    295                 300                 305

ggt cta ggg gga gaa aat ttt caa aaa aac tct agt att ccg gaa att    1075
Gly Leu Gly Gly Glu Asn Phe Gln Lys Asn Ser Ser Ile Pro Glu Ile
310                 315                 320                 325

cgt ata tgc gca aga tat cag tgg gaa tct gta tca cca ttc tcc tta    1123
Arg Ile Cys Ala Arg Tyr Gln Trp Glu Ser Val Ser Pro Phe Ser Leu
                330                 335                 340

gcc tct ccg tagtcctcca aggctgcaag gagtccagtc actcctctac            1172
Ala Ser Pro

atctcgggga gaactcgcta ttaatataag agatgaaccc cgttctttag atccaagaca 1232

agt                                                                1235


<210> 6
<211> 344
<212> PRT
<213> Chlamydia pneumoniae

<220>
<221> SITE
<222> (220)...(231)
<223> B-cell epitope

<220>
<221> SITE
<222> (313)...(321)
<223> B-cell epitope

<220>
<221> SITE
<222> (67)...(76)
<223> T-cell epitope

<220>
<221> SITE
<222> (66)...(75)
<223> T-cell epitope

<400> 6
Met Leu Thr Leu Gly Leu Glu Ser Ser Cys Asp Glu Thr Ala Cys Ala
  1               5                  10                  15

Ile Val Asn Glu Asp Lys Gln Ile Leu Ala Asn Ile Ile Ala Ser Gln
             20                  25                  30

Asp Ile His Ala Ser Tyr Gly Gly Val Val Pro Glu Leu Ala Ser Arg
         35                  40                  45

Ala His Leu His Ile Phe Pro Gln Val Ile Asn Lys Ala Leu Gln Gln

50                  55                  60

Ala Asn Leu Leu Ile Glu Asp Met Asp Leu Ile Ala Val Thr Gln Thr
65                  70                  75                  80

Pro Gly Leu Ile Gly Ser Leu Ser Val Gly Val His Phe Gly Lys Gly
                85                  90                  95

Ile Ala Ile Gly Ala Lys Lys Ser Leu Ile Gly Val Asn His Val Glu
            100                 105                 110

Ala His Leu Tyr Ala Ala Tyr Met Ala Ala Gln Asn Val Gln Phe Pro
        115                 120                 125

Ala Leu Gly Leu Val Val Ser Gly Ala His Thr Ala Ala Phe Phe Ile
    130                 135                 140

Glu Asn Pro Thr Ser Tyr Lys Leu Ile Gly Lys Thr Arg Asp Asp Ala
145                 150                 155                 160

Ile Gly Glu Thr Phe Asp Lys Val Gly Arg Phe Leu Gly Leu Pro Tyr
                165                 170                 175

Pro Ala Gly Pro Leu Ile Glu Lys Leu Ala Leu Glu Gly Ser Glu Asp
            180                 185                 190

Ser Tyr Pro Phe Ser Pro Ala Lys Val Pro Asn Tyr Asp Phe Ser Phe
        195                 200                 205

Ser Gly Leu Lys Thr Ala Val Leu Tyr Ala Ile Lys Gly Asn Asn Ser
    210                 215                 220

Ser Pro Arg Ser Pro Ala Pro Glu Ile Ser Leu Glu Lys Gln Arg Asp
225                 230                 235                 240

Ile Ala Ala Ser Phe Gln Lys Ala Ala Cys Thr Thr Ile Ala Gln Lys
                245                 250                 255

Leu Pro Thr Ile Ile Lys Glu Phe Ser Cys Arg Ser Ile Leu Ile Gly
            260                 265                 270

Gly Gly Val Ala Ile Asn Glu Tyr Phe Arg Ser Ala Ile Gln Thr Ala
        275                 280                 285

Cys Asn Leu Pro Val Tyr Phe Pro Pro Ala Lys Leu Cys Ser Asp Asn
    290                 295                 300

Ala Ala Met Ile Ala Gly Leu Gly Gly Glu Asn Phe Gln Lys Asn Ser
305                 310                 315                 320

Ser Ile Pro Glu Ile Arg Ile Cys Ala Arg Tyr Gln Trp Glu Ser Val
                325                 330                 335

Ser Pro Phe Ser Leu Ala Ser Pro
            340

<210> 7
<211> 2060
<212> DNA
<213> Chlamydia pneumoniae

<220>
<221> CDS
<222> (101)..(1957)

<400> 7
   gattttgtgt attttttcag ataatgtttt taaaaaaatg ttttaaaacc ctaaaatcct 60
   acctccttgt aaccattctc ggtagaaaag agaggtattt atg aaa aaa ggg aaa 115
   Met Lys Lys Gly Lys
   1 5
   tta gga gcc ata gtt ttt ggc ctt cta ttt aca agt agt gtt gct ggt 163
   Leu Gly Ala Ile Val Phe Gly Leu Leu Phe Thr Ser Ser Val Ala Gly
   10 15 20
   ttt tct aag gat ttg act aaa gac aac gct tat caa gat tta aat gtc 211
   Phe Ser Lys Asp Leu Thr Lys Asp Asn Ala Tyr Gln Asp Leu Asn Val
   25 30 35
   ata gag cat tta ata tcg tta aaa tat gct cct tta cca tgg aag gaa 259
   Ile Glu His Leu Ile Ser Leu Lys Tyr Ala Pro Leu Pro Trp Lys Glu
   40 45 50
   cta tta ttt ggt tgg gat tta tct cag caa aca cag caa gct cgc ttg 307
   Leu Leu Phe Gly Trp Asp Leu Ser Gln Gln Thr Gln Gln Ala Arg Leu
   55 60 65
   caa ctg gtc tta gaa gaa aaa cca aca acc aac tac tgc cag aag gta 355
   Gln Leu Val Leu Glu Glu Lys Pro Thr Thr Asn Tyr Cys Gln Lys Val
   70 75 80 85
   ctc tct aac tac gtg aga tca tta aac gat tat cat gca ggg att acg 403
   Leu Ser Asn Tyr Val Arg Ser Leu Asn Asp Tyr His Ala Gly Ile Thr
   90 95 100
   ttt tat cgt act gaa agt gcg tat atc cct tac gta ttg aag tta agt 451
   Phe Tyr Arg Thr Glu Ser Ala Tyr Ile Pro Tyr Val Leu Lys Leu Ser
   105 110 115
   gaa gat ggt cat gtc ttt gta gtc gac gta cag act agc caa ggg gat 499
   Glu Asp Gly His Val Phe Val Val Asp Val Gln Thr Ser Gln Gly Asp
   120 125 130
   att tac tta ggg gat gaa atc ctt gaa gta gat gga atg ggg att cgt 547
   Ile Tyr Leu Gly Asp Glu Ile Leu Glu Val Asp Gly Met Gly Ile Arg
   135 140 145
   gag gct atc gaa agc ctt cgc ttt gga cga ggg agt gcc aca gac tat 595
   Glu Ala Ile Glu Ser Leu Arg Phe Gly Arg Gly Ser Ala Thr Asp Tyr
   150 155 160 165
   tct gct gca gtt cgt tcc ttg aca tcg cgt tcc gcc gct ttt gga gat 643
   Ser Ala Ala Val Arg Ser Leu Thr Ser Arg Ser Ala Ala Phe Gly Asp
   170 175 180
   gcg gtt cct tca gga att gcc atg ttg aaa ctt cgc cga ccc agt ggt 691
   Ala Val Pro Ser Gly Ile Ala Met Leu Lys Leu Arg Arg Pro Ser Gly
   185 190 195
   ttg atc cgt tcg aca ccg gtc cgt tgg cgt tat act cca gag cat atc 739
   Leu Ile Arg Ser Thr Pro Val Arg Trp Arg Tyr Thr Pro Glu His Ile
   200 205 210
   gga gat ttt tct tta gtt gct cct ttg att cct gaa cat aaa cct caa 787
   Gly Asp Phe Ser Leu Val Ala Pro Leu Ile Pro Glu His Lys Pro Gln
   215 220 225
   tta cct aca caa agt tgt gtg cta ttc cgt tcc ggg gta aat tca cag 835
   Leu Pro Thr Gln Ser Cys Val Leu Phe Arg Ser Gly Val Asn Ser Gln
   230 235 240 245
   tct tct agt agc tct tta ttc agt tcc tac atg gtg cct tat ttc tgg 883
   Ser Ser Ser Ser Ser Leu Phe Ser Ser Tyr Met Val Pro Tyr Phe Trp
   250 255 260
   gaa gaa ttg cgg gtt caa aat aag cag cgt ttt gac agt aat cac cat     931
   Glu Glu Leu Arg Val Gln Asn Lys Gln Arg Phe Asp Ser Asn His His
               265                 270                 275
   ata ggg agc cgt aat gga ttt tta cct acg ttt ggt cct att ctt tgg     979
   Ile Gly Ser Arg Asn Gly Phe Leu Pro Thr Phe Gly Pro Ile Leu Trp
           280                 285                 290
   gaa caa gac aag ggg ccc tat cgt tcc tat atc ttt aaa gca aaa gat     1027
   Glu Gln Asp Lys Gly Pro Tyr Arg Ser Tyr Ile Phe Lys Ala Lys Asp
       295                 300                 305
   tct cag ggc aat ccc cat cgc ata gga ttt tta aga att tct tct tat     1075
   Ser Gln Gly Asn Pro His Arg Ile Gly Phe Leu Arg Ile Ser Ser Tyr
   310                 315                 320                 325
   gtt tgg act gat tta gaa gga ctt gaa gag gat cat aag gat agt cct     1123
   Val Trp Thr Asp Leu Glu Gly Leu Glu Glu Asp His Lys Asp Ser Pro
                   330                 335                 340
   tgg gag ctc ttt gga gag atc atc gat cat ttg gaa aaa gag act gat     1171
   Trp Glu Leu Phe Gly Glu Ile Ile Asp His Leu Glu Lys Glu Thr Asp
               345                 350                 355
   gct ttg att att gat cag acc cat aat cct gga ggc agt gtt ttc tat     1219
   Ala Leu Ile Ile Asp Gln Thr His Asn Pro Gly Gly Ser Val Phe Tyr
           360                 365                 370
   ctc tat tcg tta cta tct atg tta aca gat cat cct tta gat act cct     1267
   Leu Tyr Ser Leu Leu Ser Met Leu Thr Asp His Pro Leu Asp Thr Pro
       375                 380                 385
   aaa cat aga atg att ttc act cag gat gaa gtc agc tcg gct ttg cac     1315
   Lys His Arg Met Ile Phe Thr Gln Asp Glu Val Ser Ser Ala Leu His
   390                 395                 400                 405
   tgg caa gat cta cta gaa gat gtc ttc aca gat gag cag gca gtt gcc     1363
   Trp Gln Asp Leu Leu Glu Asp Val Phe Thr Asp Glu Gln Ala Val Ala
                   410                 415                 420
   gtg cta ggg gaa act atg gaa gga tat tgc atg gat atg cat gct gta     1411
   Val Leu Gly Glu Thr Met Glu Gly Tyr Cys Met Asp Met His Ala Val
               425                 430                 435
   gcc tct ctt caa aac ttc tct cag agt gtc ctt tct tcc tgg gtt tca     1459
   Ala Ser Leu Gln Asn Phe Ser Gln Ser Val Leu Ser Ser Trp Val Ser
           440                 445                 450
   ggt gat att aac ctt tca aaa cct atg cct ttg cta gga ttt gca cag     1507
   Gly Asp Ile Asn Leu Ser Lys Pro Met Pro Leu Leu Gly Phe Ala Gln
       455                 460                 465
   gtt cga cct cat cct aaa cat caa tat act aaa cct ttg ttt atg ttg     1555
   Val Arg Pro His Pro Lys His Gln Tyr Thr Lys Pro Leu Phe Met Leu
   470                 475                 480                 485
   ata gac gag gat gac ttc tct tgt gga gat tta gcg cct gca att ttg     1603
   Ile Asp Glu Asp Asp Phe Ser Cys Gly Asp Leu Ala Pro Ala Ile Leu
                   490                 495                 500
   aag gat aat ggc cgc gct act ctc att gga aag cca aca gca gga gct     1651
   Lys Asp Asn Gly Arg Ala Thr Leu Ile Gly Lys Pro Thr Ala Gly Ala
               505                 510                 515
   gga ggt ttt gta ttc caa gtc act ttc cct aac cgt tct gga att aaa     1699
   Gly Gly Phe Val Phe Gln Val Thr Phe Pro Asn Arg Ser Gly Ile Lys
           520                 525                 530
   ggt ctt tct tta aca gga tct tta gct gtt agg aaa gat ggt gag ttt 1747
   Gly Leu Ser Leu Thr Gly Ser Leu Ala Val Arg Lys Asp Gly Glu Phe
   535 540 545
   att gaa aac tta gga gtg gct cct cat att gat tta gga ttt acc tcc 1795
   Ile Glu Asn Leu Gly Val Ala Pro His Ile Asp Leu Gly Phe Thr Ser
   550 555 560 565
   agg gat ttg caa act tcc agg ttt act gat tac gtt gag gca gtg aaa 1843
   Arg Asp Leu Gln Thr Ser Arg Phe Thr Asp Tyr Val Glu Ala Val Lys
   570 575 580
   act ata gtt tta act tct ttg tct gag aac gct aag aag agt gaa gag 1891
   Thr Ile Val Leu Thr Ser Leu Ser Glu Asn Ala Lys Lys Ser Glu Glu
   585 590 595
   cag act tct ccg caa gag acg cct gaa gtt att cga gtc tct tat ccc 1939
   Gln Thr Ser Pro Gln Glu Thr Pro Glu Val Ile Arg Val Ser Tyr Pro
   600 605 610
   aca acg act tct gct tcg taaacgggac gtaatagaat aatttttatt 1987
   Thr Thr Thr Ser Ala Ser
   615
   attgctttaa tatgcgcgct tccaatataa gcattgtgaa gcgcgtttca tatgtctttt 2047
   atctttaggt aat 2060

<210> 8
<211> 619
<212> PRT
<213> Chlamydia pneumoniae

<220>
<221> SITE
<222> (328)...(343)
<223> B-cell epitope

<220>
<221> SITE
<222> (589)...(606)
<223> B-cell epitope

<220>
<221> SITE
<222> (135)...(144)
<223> T-cell epitope

<220>
<221> SITE
<222> (373)...(382)
<223> T-cell epitope

<400> 8
Met Lys Lys Gly Lys Leu Gly Ala Ile Val Phe Gly Leu Leu Phe Thr
1               5                   10                  15

Ser Ser Val Ala Gly Phe Ser Lys Asp Leu Thr Lys Asp Asn Ala Tyr
            20                  25                  30

Gln Asp Leu Asn Val Ile Glu His Leu Ile Ser Leu Lys Tyr Ala Pro
        35                  40                  45

Leu Pro Trp Lys Glu Leu Leu Phe Gly Trp Asp Leu Ser Gln Gln Thr
    50                  55                  60

Gln Gln Ala Arg Leu Gln Leu Val Leu Glu Glu Lys Pro Thr Thr Asn

65                  70                  75                  80

Tyr Cys Gln Lys Val Leu Ser Asn Tyr Val Arg Ser Leu Asn Asp Tyr
                85                  90                  95

His Ala Gly Ile Thr Phe Tyr Arg Thr Glu Ser Ala Tyr Ile Pro Tyr
            100                 105                 110

Val Leu Lys Leu Ser Glu Asp Gly His Val Phe Val Val Asp Val Gln
        115                 120                 125

Thr Ser Gln Gly Asp Ile Tyr Leu Gly Asp Glu Ile Leu Glu Val Asp
    130                 135                 140

Gly Met Gly Ile Arg Glu Ala Ile Glu Ser Leu Arg Phe Gly Arg Gly
145                 150                 155                 160

Ser Ala Thr Asp Tyr Ser Ala Ala Val Arg Ser Leu Thr Ser Arg Ser
                165                 170                 175

Ala Ala Phe Gly Asp Ala Val Pro Ser Gly Ile Ala Met Leu Lys Leu
            180                 185                 190

Arg Arg Pro Ser Gly Leu Ile Arg Ser Thr Pro Val Arg Trp Arg Tyr
        195                 200                 205

Thr Pro Glu His Ile Gly Asp Phe Ser Leu Val Ala Pro Leu Ile Pro
    210                 215                 220

Glu His Lys Pro Gln Leu Pro Thr Gln Ser Cys Val Leu Phe Arg Ser
225                 230                 235                 240

Gly Val Asn Ser Gln Ser Ser Ser Ser Ser Leu Phe Ser Ser Tyr Met
                245                 250                 255

Val Pro Tyr Phe Trp Glu Glu Leu Arg Val Gln Asn Lys Gln Arg Phe
            260                 265                 270

Asp Ser Asn His His Ile Gly Ser Arg Asn Gly Phe Leu Pro Thr Phe
        275                 280                 285

Gly Pro Ile Leu Trp Glu Gln Asp Lys Gly Pro Tyr Arg Ser Tyr Ile
    290                 295                 300

Phe Lys Ala Lys Asp Ser Gln Gly Asn Pro His Arg Ile Gly Phe Leu
305                 310                 315                 320

Arg Ile Ser Ser Tyr Val Trp Thr Asp Leu Glu Gly Leu Glu Glu Asp
                325                 330                 335

His Lys Asp Ser Pro Trp Glu Leu Phe Gly Glu Ile Ile Asp His Leu
            340                 345                 350

Glu Lys Glu Thr Asp Ala Leu Ile Ile Asp Gln Thr His Asn Pro Gly
        355                 360                 365

Gly Ser Val Phe Tyr Leu Tyr Ser Leu Leu Ser Met Leu Thr Asp His
    370                 375                 380

Pro Leu Asp Thr Pro Lys His Arg Met Ile Phe Thr Gln Asp Glu Val
385                 390                 395                 400

Ser Ser Ala Leu His Trp Gln Asp Leu Leu Glu Asp Val Phe Thr Asp
                405                 410                 415

Glu Gln Ala Val Ala Val Leu Gly Glu Thr Met Glu Gly Tyr Cys Met
            420                 425                 430

Asp Met His Ala Val Ala Ser Leu Gln Asn Phe Ser Gln Ser Val Leu
        435                 440                 445

Ser Ser Trp Val Ser Gly Asp Ile Asn Leu Ser Lys Pro Met Pro Leu
    450                 455                 460

Leu Gly Phe Ala Gln Val Arg Pro His Pro Lys His Gln Tyr Thr Lys
465                 470                 475                 480

Pro Leu Phe Met Leu Ile Asp Glu Asp Asp Phe Ser Cys Gly Asp Leu
                485                 490                 495

Ala Pro Ala Ile Leu Lys Asp Asn Gly Arg Ala Thr Leu Ile Gly Lys
            500                 505                 510

Pro Thr Ala Gly Ala Gly Gly Phe Val Phe Gln Val Thr Phe Pro Asn
        515                 520                 525

Arg Ser Gly Ile Lys Gly Leu Ser Leu Thr Gly Ser Leu Ala Val Arg
    530                 535                 540

Lys Asp Gly Glu Phe Ile Glu Asn Leu Gly Val Ala Pro His Ile Asp
545                 550                 555                 560

Leu Gly Phe Thr Ser Arg Asp Leu Gln Thr Ser Arg Phe Thr Asp Tyr
                565                 570                 575

Val Glu Ala Val Lys Thr Ile Val Leu Thr Ser Leu Ser Glu Asn Ala
            580                 585                 590

Lys Lys Ser Glu Glu Gln Thr Ser Pro Gln Glu Thr Pro Glu Val Ile
        595                 600                 605

Arg Val Ser Tyr Pro Thr Thr Thr Ser Ala Ser
    610                 615


<210> 9
<211> 1133
<212> DNA
<213> Chlamydia pneumoniae

<220>
<221> CDS
<222> (101)..(1030)

<400> 9
gacgtaatag aataattttt attattgctt taatatgcgc gcttccaata taagcattgt 60

gaagcgcgtt tcatatgtct tttatcttta ggtaattatt atg aga aaa ctt att    115
                                            Met Arg Lys Leu Ile
                                            1               5

tta tgc aat cct aga gga ttt tgc tct gga gtt gtg cgc gct att caa    163
Leu Cys Asn Pro Arg Gly Phe Cys Ser Gly Val Val Arg Ala Ile Gln
                10                  15                  20

gtt gta gag gtt gct tta gaa aag tgg gga gct cct atc tat gta aaa    211
Val Val Glu Val Ala Leu Glu Lys Trp Gly Ala Pro Ile Tyr Val Lys
            25                  30                  35

cat gag att gtt cac aat cgc cat gtt gtt aat gct tta cga gcc aag    259
His Glu Ile Val His Asn Arg His Val Val Asn Ala Leu Arg Ala Lys
        40                  45                  50

gga gcg atc ttt gtt gaa gaa ctt gtt gat gtt cct gaa ggt gag aga    307
Gly Ala Ile Phe Val Glu Glu Leu Val Asp Val Pro Glu Gly Glu Arg
    55                  60                  65

gtc att tat tca gct cat gga att cct cct tca gtt aga gct gaa gca 355
Val Ile Tyr Ser Ala His Gly Ile Pro Pro Ser Val Arg Ala Glu Ala
70                  75                  80                  85

aaa gcc cgt aag ctt att gat att gat gct acc tgt ggt ttg gtt act 403
Lys Ala Arg Lys Leu Ile Asp Ile Asp Ala Thr Cys Gly Leu Val Thr
                90                  95                  100

aag gtg cat tct gct gcg aag tta tac gca agt aaa gga tac aaa atc 451
Lys Val His Ser Ala Ala Lys Leu Tyr Ala Ser Lys Gly Tyr Lys Ile
            105                 110                 115

ata ctg atc ggc cat aag aag cac gtt gag gtg att ggt att gtt gga 499
Ile Leu Ile Gly His Lys Lys His Val Glu Val Ile Gly Ile Val Gly
        120                 125                 130

gaa gtt cct gaa cac att act gtt gtc gag aag gtt gct gac gtc gag 547
Glu Val Pro Glu His Ile Thr Val Val Glu Lys Val Ala Asp Val Glu
    135                 140                 145

gcc tta cct ttt agt tct gat aca cct tta ttt tat att act caa acg 595
Ala Leu Pro Phe Ser Ser Asp Thr Pro Leu Phe Tyr Ile Thr Gln Thr
150                 155                 160                 165

acg ttg agt ttg gat gat gtt cag gag atc tca tcg gct ttg cta aag 643
Thr Leu Ser Leu Asp Asp Val Gln Glu Ile Ser Ser Ala Leu Leu Lys
                170                 175                 180

cga tat ccc tct atc att act ctg cct agt tct tcg att tgt tat gca 691
Arg Tyr Pro Ser Ile Ile Thr Leu Pro Ser Ser Ser Ile Cys Tyr Ala
            185                 190                 195

acc acg aac cgt caa aaa gca ttg cgt tct gtt tta tct cgc gtg aat 739
Thr Thr Asn Arg Gln Lys Ala Leu Arg Ser Val Leu Ser Arg Val Asn
        200                 205                 210

tac gtc tat gtg gtt gga gat gtc aac agc tcg aat tcc aat cgt ctt 787
Tyr Val Tyr Val Val Gly Asp Val Asn Ser Ser Asn Ser Asn Arg Leu
    215                 220                 225

cgc gaa gtg gct ttg aga agg gga gtt ccc gct gat ttg atc aac aat 835
Arg Glu Val Ala Leu Arg Arg Gly Val Pro Ala Asp Leu Ile Asn Asn
230                 235                 240                 245

ccc gag gat att gat acg aac atc gta aat cat tct gga gat ata gca 883
Pro Glu Asp Ile Asp Thr Asn Ile Val Asn His Ser Gly Asp Ile Ala
                250                 255                 260

atg act gca gga gcc tca act ccc gaa gac gta gtt caa gct tgc att 931
Met Thr Ala Gly Ala Ser Thr Pro Glu Asp Val Val Gln Ala Cys Ile
            265                 270                 275

cga aag cta tca tca ctt atc cct ggt tta caa gtg gaa aat gat ata 979
Arg Lys Leu Ser Ser Leu Ile Pro Gly Leu Gln Val Glu Asn Asp Ile
        280                 285                 290

ttt gct gta gag gat gtc gta ttt caa tta cca aaa gaa ctc cgt tgt 1027
Phe Ala Val Glu Asp Val Val Phe Gln Leu Pro Lys Glu Leu Arg Cys
    295                 300                 305

tct taggtcttta ggcttacttg ccaagttttt ctcgagattg ctttatagag 1080
Ser
310

tcttcttctc gttcagagag ggtatttacc tttttagttc tctgtatttg aaa 1133

<210> 10
<211> 310
<212> PRT
<213> Chlamydia pneumoniae

<220>
<221> SITE
<222> (198)...(205)
<223> B-cell epitope

<220>
<221> SITE
<222> (221)...(231)
<223> B-cell epitope

<220>
<221> SITE
<222> (207)...(216)
<223> T-cell epitope

<220>
<221> SITE
<222> (279)...(288)
<223> T-cell epitope

<220>
<221> SITE
<222> (118)...(127)
<223> T-cell epitope

<400> 10
Met Arg Lys Leu Ile Leu Cys Asn Pro Arg Gly Phe Cys Ser Gly Val
1               5                   10                  15

Val Arg Ala Ile Gln Val Val Glu Val Ala Leu Glu Lys Trp Gly Ala
            20                  25                  30

Pro Ile Tyr Val Lys His Glu Ile Val His Asn Arg His Val Val Asn
        35                  40                  45

Ala Leu Arg Ala Lys Gly Ala Ile Phe Val Glu Glu Leu Val Asp Val
    50                  55                  60

Pro Glu Gly Glu Arg Val Ile Tyr Ser Ala His Gly Ile Pro Pro Ser
65                  70                  75                  80

Val Arg Ala Glu Ala Lys Ala Arg Lys Leu Ile Asp Ile Asp Ala Thr
                85                  90                  95

Cys Gly Leu Val Thr Lys Val His Ser Ala Ala Lys Leu Tyr Ala Ser
            100                 105                 110

Lys Gly Tyr Lys Ile Ile Leu Ile Gly His Lys Lys His Val Glu Val
        115                 120                 125

Ile Gly Ile Val Gly Glu Val Pro Glu His Ile Thr Val Val Glu Lys
    130                 135                 140

Val Ala Asp Val Glu Ala Leu Pro Phe Ser Ser Asp Thr Pro Leu Phe
145                 150                 155                 160

Tyr Ile Thr Gln Thr Thr Leu Ser Leu Asp Asp Val Gln Glu Ile Ser
                165                 170                 175

Ser Ala Leu Leu Lys Arg Tyr Pro Ser Ile Ile Thr Leu Pro Ser Ser
            180                 185                 190

Ser Ile Cys Tyr Ala Thr Thr Asn Arg Gln Lys Ala Leu Arg Ser Val

195                 200                 205

Leu Ser Arg Val Asn Tyr Val Tyr Val Val Gly Asp Val Asn Ser Ser
    210                 215                 220

Asn Ser Asn Arg Leu Arg Glu Val Ala Leu Arg Arg Gly Val Pro Ala
225                 230                 235                 240

Asp Leu Ile Asn Asn Pro Glu Asp Ile Asp Thr Asn Ile Val Asn His
                245                 250                 255

Ser Gly Asp Ile Ala Met Thr Ala Gly Ala Ser Thr Pro Glu Asp Val
            260                 265                 270

Val Gln Ala Cys Ile Arg Lys Leu Ser Ser Leu Ile Pro Gly Leu Gln
        275                 280                 285

Val Glu Asn Asp Ile Phe Ala Val Glu Asp Val Val Phe Gln Leu Pro
    290                 295                 300

Lys Glu Leu Arg Cys Ser
305                 310

<210> 11
<211> 1466
<212> DNA
<213> Chlamydia pneumoniae

<220>
<221> CDS
<222> (101)..(1363)

<400> 11
catgggagcc gaggaagcca tctcctacgg acttattgat aaggtggtaa cttctgcgaa 60

agaaactaat aaggatacaa gtagcactta gagagaacat atg aat aaa aaa aat    115
                                           Met Asn Lys Lys Asn
                                           1               5

cta act att tgt tca ttt tgc ggt cgg tct gaa aaa gat gta gag aaa    163
Leu Thr Ile Cys Ser Phe Cys Gly Arg Ser Glu Lys Asp Val Glu Lys
                10                  15                  20

ctg att gct ggg cct tcg gta tac att tgt gac tac tgc atc aaa tta    211
Leu Ile Ala Gly Pro Ser Val Tyr Ile Cys Asp Tyr Cys Ile Lys Leu
            25                  30                  35

tgc tct gga att tta gat aag aaa ccc tcc tct aca ata tcc tca gct    259
Cys Ser Gly Ile Leu Asp Lys Lys Pro Ser Ser Thr Ile Ser Ser Ala
        40                  45                  50

cca gtt tct gaa aca cct tca cag cct tct gat ctc agg gtg ctt acc    307
Pro Val Ser Glu Thr Pro Ser Gln Pro Ser Asp Leu Arg Val Leu Thr
    55                  60                  65

cct aag gaa atc aaa aag cat att gat gaa tat gtc att ggt cag gaa    355
Pro Lys Glu Ile Lys Lys His Ile Asp Glu Tyr Val Ile Gly Gln Glu
70                  75                  80                  85

aga gct aaa aag aca atc gct gtt gct gtt tat aat cac tat aaa cgt    403
Arg Ala Lys Lys Thr Ile Ala Val Ala Val Tyr Asn His Tyr Lys Arg
                90                  95                  100

ata cgt gct cta cta cat aac aaa cag gta agc tac ggg aaa tct aac    451
Ile Arg Ala Leu Leu His Asn Lys Gln Val Ser Tyr Gly Lys Ser Asn
            105                 110                 115

gtg ctt ctc cta ggc cct aca gga tct gga aaa aca tta att gca aaa    499

Val Leu Leu Leu Gly Pro Thr Gly Ser Gly Lys Thr Leu Ile Ala Lys
        120                 125                 130

aca ttg gca aaa att tta gat gtt ccc ttc acc ata gcc gac gca acg     547
Thr Leu Ala Lys Ile Leu Asp Val Pro Phe Thr Ile Ala Asp Ala Thr
    135                 140                 145

acc cta acg gaa gca ggt tat gtc ggt gaa gat gta gag aac att gtc     595
Thr Leu Thr Glu Ala Gly Tyr Val Gly Glu Asp Val Glu Asn Ile Val
150                 155                 160                 165

tta cgt tta tta caa gct gct gat tac gat gtc gcc cgt gca gaa cga     643
Leu Arg Leu Leu Gln Ala Ala Asp Tyr Asp Val Ala Arg Ala Glu Arg
                170                 175                 180

ggc att atc tat atc gat gaa atc gat aaa att gga agg aca aca gca     691
Gly Ile Ile Tyr Ile Asp Glu Ile Asp Lys Ile Gly Arg Thr Thr Ala
            185                 190                 195

aac gtc tcc att act aga gat gtt tct ggc gaa ggg gtt caa caa gca     739
Asn Val Ser Ile Thr Arg Asp Val Ser Gly Glu Gly Val Gln Gln Ala
        200                 205                 210

ttg tta aaa atc gtt gaa gga acc aca gca aac gtt cct cct aaa gga     787
Leu Leu Lys Ile Val Glu Gly Thr Thr Ala Asn Val Pro Pro Lys Gly
    215                 220                 225

gga cgt aag cat cct aac caa gag tat atc cga gtc aat acg gaa aat     835
Gly Arg Lys His Pro Asn Gln Glu Tyr Ile Arg Val Asn Thr Glu Asn
230                 235                 240                 245

atc tta ttt atc gta ggc gga gcc ttc gtc aac cta gat aag att atc     883
Ile Leu Phe Ile Val Gly Gly Ala Phe Val Asn Leu Asp Lys Ile Ile
                250                 255                 260

gca aag cga ttg ggg aaa act acc ata ggg ttt tct gat gat caa gca     931
Ala Lys Arg Leu Gly Lys Thr Thr Ile Gly Phe Ser Asp Asp Gln Ala
            265                 270                 275

gac ctc tct caa aaa acc aga gac cat cta ctt gct aaa gtt gaa acc     979
Asp Leu Ser Gln Lys Thr Arg Asp His Leu Leu Ala Lys Val Glu Thr
        280                 285                 290

gaa gac ctg att gcc ttc gga atg atc cct gaa ttt gtc gga aga ttc     1027
Glu Asp Leu Ile Ala Phe Gly Met Ile Pro Glu Phe Val Gly Arg Phe
    295                 300                 305

aac tgc att gta aac tgt gaa gag ctt tct ttg gat gag ctt gta gcc     1075
Asn Cys Ile Val Asn Cys Glu Glu Leu Ser Leu Asp Glu Leu Val Ala
310                 315                 320                 325

atc ctt aca gaa cct aca aat gcg att gtg aaa caa tat atg gag cta     1123
Ile Leu Thr Glu Pro Thr Asn Ala Ile Val Lys Gln Tyr Met Glu Leu
                330                 335                 340

ttc gca gaa gaa aac gtc aag tta gtc ttc aaa aaa gaa gcc cta tat     1171
Phe Ala Glu Glu Asn Val Lys Leu Val Phe Lys Lys Glu Ala Leu Tyr
            345                 350                 355

gct ata gca aaa aaa gcc aag caa gca aaa act gga gct cgt gct cta     1219
Ala Ile Ala Lys Lys Ala Lys Gln Ala Lys Thr Gly Ala Arg Ala Leu
        360                 365                 370

ggg atg atc cta gaa aat ctc ctt aga gac ctt atg ttt gaa att cct     1267
Gly Met Ile Leu Glu Asn Leu Leu Arg Asp Leu Met Phe Glu Ile Pro
    375                 380                 385

tca gat cct aca gta gaa gct att cat atc caa gaa gac act atc gca     1315

Ser Asp Pro Thr Val Glu Ala Ile His Ile Gln Glu Asp Thr Ile Ala
390                 395                 400                 405

gaa aat aaa gcg cca ata att atc aga agg acc cca gaa gct atc gct    1363
Glu Asn Lys Ala Pro Ile Ile Ile Arg Arg Thr Pro Glu Ala Ile Ala
                410                 415                 420

tagctctttt tagttcctat tttaggggtg tcatgacaac aattgccata gaagctgcaa 1423

aaaaagttct tatcaaacta cgtaatgcag gatatcaggc ata                   1466

<210> 12
<211> 421
<212> PRT
<213> Chlamydia pneumoniae

<220>
<221> SITE
<222> (226)...(239)
<223> B-cell epitope

<220>
<221> SITE
<222> (273)...(286)
<223> B-cell epitope

<220>
<221> SITE
<222> (137)...(146)
<223> T-cell epitope

<220>
<221> SITE
<222> (168)...(177)
<223> T-cell epitope

<400> 12
Met Asn Lys Lys Asn Leu Thr Ile Cys Ser Phe Cys Gly Arg Ser Glu
  1               5                  10                  15

Lys Asp Val Glu Lys Leu Ile Ala Gly Pro Ser Val Tyr Ile Cys Asp
             20                  25                  30

Tyr Cys Ile Lys Leu Cys Ser Gly Ile Leu Asp Lys Lys Pro Ser Ser
         35                  40                  45

Thr Ile Ser Ser Ala Pro Val Ser Glu Thr Pro Ser Gln Pro Ser Asp
     50                  55                  60

Leu Arg Val Leu Thr Pro Lys Glu Ile Lys Lys His Ile Asp Glu Tyr
 65                  70                  75                  80

Val Ile Gly Gln Glu Arg Ala Lys Lys Thr Ile Ala Val Ala Val Tyr
                 85                  90                  95

Asn His Tyr Lys Arg Ile Arg Ala Leu Leu His Asn Lys Gln Val Ser
            100                 105                 110

Tyr Gly Lys Ser Asn Val Leu Leu Leu Gly Pro Thr Gly Ser Gly Lys
        115                 120                 125

Thr Leu Ile Ala Lys Thr Leu Ala Lys Ile Leu Asp Val Pro Phe Thr
    130                 135                 140

Ile Ala Asp Ala Thr Thr Leu Thr Glu Ala Gly Tyr Val Gly Glu Asp
145                 150                 155                 160

Val Glu Asn Ile Val Leu Arg Leu Leu Gln Ala Ala Asp Tyr Asp Val
                165                 170                 175

Ala Arg Ala Glu Arg Gly Ile Ile Tyr Ile Asp Glu Ile Asp Lys Ile
            180                 185                 190

Gly Arg Thr Thr Ala Asn Val Ser Ile Thr Arg Asp Val Ser Gly Glu
        195                 200                 205

Gly Val Gln Gln Ala Leu Leu Lys Ile Val Glu Gly Thr Thr Ala Asn
    210                 215                 220

Val Pro Pro Lys Gly Gly Arg Lys His Pro Asn Gln Glu Tyr Ile Arg
225                 230                 235                 240

Val Asn Thr Glu Asn Ile Leu Phe Ile Val Gly Gly Ala Phe Val Asn
                245                 250                 255

Leu Asp Lys Ile Ile Ala Lys Arg Leu Gly Lys Thr Thr Ile Gly Phe
            260                 265                 270

Ser Asp Asp Gln Ala Asp Leu Ser Gln Lys Thr Arg Asp His Leu Leu
        275                 280                 285

Ala Lys Val Glu Thr Glu Asp Leu Ile Ala Phe Gly Met Ile Pro Glu
    290                 295                 300

Phe Val Gly Arg Phe Asn Cys Ile Val Asn Cys Glu Glu Leu Ser Leu
305                 310                 315                 320

Asp Glu Leu Val Ala Ile Leu Thr Glu Pro Thr Asn Ala Ile Val Lys
                325                 330                 335

Gln Tyr Met Glu Leu Phe Ala Glu Glu Asn Val Lys Leu Val Phe Lys
            340                 345                 350

Lys Glu Ala Leu Tyr Ala Ile Ala Lys Lys Ala Lys Gln Ala Lys Thr
        355                 360                 365

Gly Ala Arg Ala Leu Gly Met Ile Leu Glu Asn Leu Leu Arg Asp Leu
    370                 375                 380

Met Phe Glu Ile Pro Ser Asp Pro Thr Val Glu Ala Ile His Ile Gln
385                 390                 395                 400

Glu Asp Thr Ile Ala Glu Asn Lys Ala Pro Ile Ile Ile Arg Arg Thr
                405                 410                 415

Pro Glu Ala Ile Ala
            420

<210> 13
<211> 812
<212> DNA
<213> Chlamydia pneumoniae

<220>
<221> CDS
<222> (101)..(709)

<400> 13
tgacgtagac agcctaaaaa gtcttagcta cgttcctagg gtcatttcgt gatcgggaac 60

gtatggacac aactgaaaat tatttgatga ggaaacgcaa atg aca ctg gta ccc   115
                                          Met Thr Leu Val Pro
                                          1               5

tat gtt gtc gag gat acg ggc cgt ggt gaa agg gcc atg gat att tac   163

Tyr Val Val Glu Asp Thr Gly Arg Gly Glu Arg Ala Met Asp Ile Tyr
                10                  15                  20

tcc cgt ctt ctg aaa gat cgt att gta atg atc ggt cag gaa atc acg     211
Ser Arg Leu Leu Lys Asp Arg Ile Val Met Ile Gly Gln Glu Ile Thr
            25                  30                  35

gag ccc ctc gca aac aca gta att gcc cag ctc ctt ttc ctc atg tcc     259
Glu Pro Leu Ala Asn Thr Val Ile Ala Gln Leu Leu Phe Leu Met Ser
        40                  45                  50

gaa gat cct aaa aag gat att caa att ttc atc aat tcc cca ggc ggc     307
Glu Asp Pro Lys Lys Asp Ile Gln Ile Phe Ile Asn Ser Pro Gly Gly
    55                  60                  65

tac atc acc gct gga ctg gca atc tat gat acc att cgc ttt tta ggt     355
Tyr Ile Thr Ala Gly Leu Ala Ile Tyr Asp Thr Ile Arg Phe Leu Gly
70                  75                  80                  85

tgt gat gta aat acc tac tgc atc ggt caa gct gca tcc atg gga gcc     403
Cys Asp Val Asn Thr Tyr Cys Ile Gly Gln Ala Ala Ser Met Gly Ala
                90                  95                  100

ctc tta tta tcc gca gga act aaa gga aag cgt cac gct ctt ccc cat     451
Leu Leu Leu Ser Ala Gly Thr Lys Gly Lys Arg His Ala Leu Pro His
            105                 110                 115

agc cgt atg atg atc cac caa cct tct gga ggc att atc gga aca tcc     499
Ser Arg Met Met Ile His Gln Pro Ser Gly Gly Ile Ile Gly Thr Ser
        120                 125                 130

gca gac atc caa ctc caa gca gct gaa att cta aca cta aaa aaa cac     547
Ala Asp Ile Gln Leu Gln Ala Ala Glu Ile Leu Thr Leu Lys Lys His
    135                 140                 145

ctt gcc aat atc ctc tct gaa tgc aca gga caa cct gta gaa aaa att     595
Leu Ala Asn Ile Leu Ser Glu Cys Thr Gly Gln Pro Val Glu Lys Ile
150                 155                 160                 165

ata gaa gat tct gaa cga gat ttc ttc atg gga gcc gag gaa gcc atc     643
Ile Glu Asp Ser Glu Arg Asp Phe Phe Met Gly Ala Glu Glu Ala Ile
                170                 175                 180

tcc tac gga ctt att gat aag gtg gta act tct gcg aaa gaa act aat     691
Ser Tyr Gly Leu Ile Asp Lys Val Val Thr Ser Ala Lys Glu Thr Asn
            185                 190                 195

aag gat aca agt agc act tagagagaac atatgaataa aaaaaatcta            739
Lys Asp Thr Ser Ser Thr
        200

actatttgtt cattttgcgg tcggtctgaa aaagatgtag agaaactgat tgctgggcct 799

tcggtataca ttt                                                    812


<210> 14
<211> 203
<212> PRT
<213> Chlamydia pneumoniae

<220>
<221> SITE
<222> (107)...(116)
<223> B-cell epitope

<220>
<221> SITE

<222> (193)...(203)
<223> B-cell epitope

<220>
<221> SITE
<222> (76)...(85)
<223> T-cell epitope


<400> 14
Met Thr Leu Val Pro Tyr Val Val Glu Asp Thr Gly Arg Gly Glu Arg
1               5                   10                  15

Ala Met Asp Ile Tyr Ser Arg Leu Leu Lys Asp Arg Ile Val Met Ile
            20                  25                  30

Gly Gln Glu Ile Thr Glu Pro Leu Ala Asn Thr Val Ile Ala Gln Leu
        35                  40                  45

Leu Phe Leu Met Ser Glu Asp Pro Lys Lys Asp Ile Gln Ile Phe Ile
    50                  55                  60

Asn Ser Pro Gly Gly Tyr Ile Thr Ala Gly Leu Ala Ile Tyr Asp Thr
65                  70                  75                  80

Ile Arg Phe Leu Gly Cys Asp Val Asn Thr Tyr Cys Ile Gly Gln Ala
                85                  90                  95

Ala Ser Met Gly Ala Leu Leu Leu Ser Ala Gly Thr Lys Gly Lys Arg
            100                 105                 110

His Ala Leu Pro His Ser Arg Met Met Ile His Gln Pro Ser Gly Gly
        115                 120                 125

Ile Ile Gly Thr Ser Ala Asp Ile Gln Leu Gln Ala Ala Glu Ile Leu
    130                 135                 140

Thr Leu Lys Lys His Leu Ala Asn Ile Leu Ser Glu Cys Thr Gly Gln
145                 150                 155                 160

Pro Val Glu Lys Ile Ile Glu Asp Ser Glu Arg Asp Phe Phe Met Gly
                165                 170                 175

Ala Glu Glu Ala Ile Ser Tyr Gly Leu Ile Asp Lys Val Val Thr Ser
            180                 185                 190

Ala Lys Glu Thr Asn Lys Asp Thr Ser Ser Thr
        195                 200


<210> 15
<211> 2162
<212> DNA
<213> Chlamydia pneumoniae

<220>
<221> CDS
<222> (101)..(2059)

<400> 15
gataaaatag aaagacctga tcatttgatg gaaatagcag ctcttcccga ataccaatat 60

ttggaatatc cctcagaaga aagtatcagt cttttatcct atg agc tac cgt aaa    115
                                          Met Ser Tyr Arg Lys
                                          1               5

cgt tcg act cta att gtt cta gga gtg ttt gct ctt tat gct ctt cta    163
Arg Ser Thr Leu Ile Val Leu Gly Val Phe Ala Leu Tyr Ala Leu Leu

                10                  15                  20

gta ttg cgt tat tat aaa att caa att tgt gaa gga gac cac tgg gcc 211
Val Leu Arg Tyr Tyr Lys Ile Gln Ile Cys Glu Gly Asp His Trp Ala
            25                  30                  35

gca gaa gct ctc ggg caa cac gaa ttt tgt gtc cgt gat cct ttt cga 259
Ala Glu Ala Leu Gly Gln His Glu Phe Cys Val Arg Asp Pro Phe Arg
        40                  45                  50

agg ggc acc ttt ttt gct aac acg aca gta cgt aag gga gac aaa gac 307
Arg Gly Thr Phe Phe Ala Asn Thr Thr Val Arg Lys Gly Asp Lys Asp
    55                  60                  65

ctt cag cag cct ttc gct gtc gat att aca aaa ttt cac ctt tgt gca 355
Leu Gln Gln Pro Phe Ala Val Asp Ile Thr Lys Phe His Leu Cys Ala
70                  75                  80                  85

gat cct tta gct att ccc gaa tgt cat cgt gat gag atc atc caa ggg 403
Asp Pro Leu Ala Ile Pro Glu Cys His Arg Asp Glu Ile Ile Gln Gly
                90                  95                  100

att ctc caa ttt att gag ggg cag acc tac gac gac ctc tcc cta aag 451
Ile Leu Gln Phe Ile Glu Gly Gln Thr Tyr Asp Asp Leu Ser Leu Lys
            105                 110                 115

tta gat aag aaa tct cgg tat tgt aag ctg tat cct tta tta gat gtt 499
Leu Asp Lys Lys Ser Arg Tyr Cys Lys Leu Tyr Pro Leu Leu Asp Val
        120                 125                 130

tct gtc cat gac cgg cta tcc ctt tgg tgg aaa gga tat gca aca aag 547
Ser Val His Asp Arg Leu Ser Leu Trp Trp Lys Gly Tyr Ala Thr Lys
    135                 140                 145

cat cgc tta cca aca aac gcc cta ttt ttt att acg gac tac caa cgc 595
His Arg Leu Pro Thr Asn Ala Leu Phe Phe Ile Thr Asp Tyr Gln Arg
150                 155                 160                 165

tcg tat cct ttt ggg aag ctc ctt gga caa gtt ctc cat acc tta aga 643
Ser Tyr Pro Phe Gly Lys Leu Leu Gly Gln Val Leu His Thr Leu Arg
                170                 175                 180

gaa att aag gat gag aaa aca gga aaa gcc ttt ccc aca ggc ggg atg 691
Glu Ile Lys Asp Glu Lys Thr Gly Lys Ala Phe Pro Thr Gly Gly Met
            185                 190                 195

gag gcg tac ttt aat cat att ctg gaa ggg gac gtt gga gag aga aag 739
Glu Ala Tyr Phe Asn His Ile Leu Glu Gly Asp Val Gly Glu Arg Lys
        200                 205                 210

ctg ttg cgt tct cct ttg aac cgt tta gat acg aat cgt gtt atc aaa 787
Leu Leu Arg Ser Pro Leu Asn Arg Leu Asp Thr Asn Arg Val Ile Lys
    215                 220                 225

ctg cct aaa gat ggc tct gat atc tac ctt acg atc aat cct gtg atc 835
Leu Pro Lys Asp Gly Ser Asp Ile Tyr Leu Thr Ile Asn Pro Val Ile
230                 235                 240                 245

cag acc att gca gag gaa gaa ctc gaa cgg ggc gtg cta gaa gct aaa 883
Gln Thr Ile Ala Glu Glu Glu Leu Glu Arg Gly Val Leu Glu Ala Lys
                250                 255                 260

gcc cag ggg ggt agg ctc att cta atg aac tcc caa aca gga gag att 931
Ala Gln Gly Gly Arg Leu Ile Leu Met Asn Ser Gln Thr Gly Glu Ile
            265                 270                 275

ctt gca ctg gct caa tat ccg ttt ttc gat ccc aca aat tat aag gaa 979
Leu Ala Leu Ala Gln Tyr Pro Phe Phe Asp Pro Thr Asn Tyr Lys Glu

280                 285                 290

tac ttc aat aac aaa gag cgc atc gaa cat acg aag gta tct ttt gtg     1027
Tyr Phe Asn Asn Lys Glu Arg Ile Glu His Thr Lys Val Ser Phe Val
    295                 300                 305

agc gat gtt ttt gaa ccc ggg tcg atc atg aaa cct ttg act gtg gcg     1075
Ser Asp Val Phe Glu Pro Gly Ser Ile Met Lys Pro Leu Thr Val Ala
310                 315                 320                 325

att gct tta caa gct aac gaa gag gct agc tta aaa tcg cag aaa aag     1123
Ile Ala Leu Gln Ala Asn Glu Glu Ala Ser Leu Lys Ser Gln Lys Lys
                330                 335                 340

att ttt gat cct gaa gaa cct atc gat gtg acc agg aca ctc ttc cct     1171
Ile Phe Asp Pro Glu Glu Pro Ile Asp Val Thr Arg Thr Leu Phe Pro
            345                 350                 355

gga cga aaa gga tct ccg ctt aag gat att tct aga aac tct caa ttg     1219
Gly Arg Lys Gly Ser Pro Leu Lys Asp Ile Ser Arg Asn Ser Gln Leu
        360                 365                 370

aat atg tac atg gct atc cag aaa tct tcg aat gtc tat gta gct cag     1267
Asn Met Tyr Met Ala Ile Gln Lys Ser Ser Asn Val Tyr Val Ala Gln
    375                 380                 385

ctg gct gac cgc atc ata caa tct tta gga gtg gcc tgg tac caa cag     1315
Leu Ala Asp Arg Ile Ile Gln Ser Leu Gly Val Ala Trp Tyr Gln Gln
390                 395                 400                 405

aag ttg cta gct ctg gga ttt gga aga aaa aca ggg atc gag ctt ccc     1363
Lys Leu Leu Ala Leu Gly Phe Gly Arg Lys Thr Gly Ile Glu Leu Pro
                410                 415                 420

agt gag gcc tct ggt ttg gtg cct tct ccc cat cgt ttc cat att aat     1411
Ser Glu Ala Ser Gly Leu Val Pro Ser Pro His Arg Phe His Ile Asn
            425                 430                 435

ggt tcc ctg gaa tgg tcc tta tct act cca tat tct ttg gct atg gga     1459
Gly Ser Leu Glu Trp Ser Leu Ser Thr Pro Tyr Ser Leu Ala Met Gly
        440                 445                 450

tat aat att ttg gca aca ggg ata caa atg gtt caa gcc tac gct atc     1507
Tyr Asn Ile Leu Ala Thr Gly Ile Gln Met Val Gln Ala Tyr Ala Ile
    455                 460                 465

ctt gca aac gga ggt tat gcc gtc cgg ccc act tta gta aaa aag atc     1555
Leu Ala Asn Gly Gly Tyr Ala Val Arg Pro Thr Leu Val Lys Lys Ile
470                 475                 480                 485

gtc tct gct tca gga gag gaa tat cat ctt cct act aaa gag aag aca     1603
Val Ser Ala Ser Gly Glu Glu Tyr His Leu Pro Thr Lys Glu Lys Thr
                490                 495                 500

cga ctc ttt tca gaa gaa att act aga gaa gtt gtt cgt gcc atg cgt     1651
Arg Leu Phe Ser Glu Glu Ile Thr Arg Glu Val Val Arg Ala Met Arg
            505                 510                 515

ttt aca acg tta ccc gga ggt tcg gga ttt cga gcc tct cct aag cat     1699
Phe Thr Thr Leu Pro Gly Gly Ser Gly Phe Arg Ala Ser Pro Lys His
        520                 525                 530

cac tct agt gct ggg aaa aca gga act aca gaa aag atg att cat gga     1747
His Ser Ser Ala Gly Lys Thr Gly Thr Thr Glu Lys Met Ile His Gly
    535                 540                 545

aaa tat gat aaa cgc cgt cat att gct tct ttt ata ggt ttt act ccc     1795
Lys Tyr Asp Lys Arg Arg His Ile Ala Ser Phe Ile Gly Phe Thr Pro

550                 555                 560                 565

gta gag agc tcg gag gga aat ttc cca cct tta gtg atg ctc gtc tcc     1843
Val Glu Ser Ser Glu Gly Asn Phe Pro Pro Leu Val Met Leu Val Ser
                570                 575                 580

ata gat gat cct gaa tat ggt ttg cga gcc gac ggc acg aaa aat tat     1891
Ile Asp Asp Pro Glu Tyr Gly Leu Arg Ala Asp Gly Thr Lys Asn Tyr
            585                 590                 595

atg ggg ggg cgt tgt gcg gca ccc att ttt tct agg gtt gct gac cgc     1939
Met Gly Gly Arg Cys Ala Ala Pro Ile Phe Ser Arg Val Ala Asp Arg
        600                 605                 610

aca ctc ctc tat tta ggg att ctt cca gac aag aag cta aga aat tgc     1987
Thr Leu Leu Tyr Leu Gly Ile Leu Pro Asp Lys Lys Leu Arg Asn Cys
    615                 620                 625

gac gaa gaa gct gct gca tta aag cgt ctc tat gaa gaa tgg aat cgt     2035
Asp Glu Glu Ala Ala Ala Leu Lys Arg Leu Tyr Glu Glu Trp Asn Arg
630                 635                 640                 645

tct ccg aaa caa ggg gga acg agg tgaggatctc tatttccatc ttgctataga    2089
Ser Pro Lys Gln Gly Gly Thr Arg
                650

cttttaccgt tgagcaaaga ctctctatca gagagcccgt ctcctcttta tcctctatga 2149

gtagtttatg tta                                                      2162


<210> 16
<211> 653
<212> PRT
<213> Chlamydia pneumoniae

<220>
<221> SITE
<222> (287)...(306)
<223> B-cell epitope

<220>
<221> SITE
<222> (637)...(653)
<223> B-cell epitope

<220>
<221> SITE
<222> (40)...(48)
<223> T-cell epitope

<220>
<221> SITE
<222> (456)...(465)
<223> T-cell epitope

<400> 16
   Met Ser Tyr Arg Lys Arg Ser Thr Leu Ile Val Leu Gly Val Phe Ala
     1               5                  10                  15
   Leu Tyr Ala Leu Leu Val Leu Arg Tyr Tyr Lys Ile Gln Ile Cys Glu
                20                  25                  30
   Gly Asp His Trp Ala Ala Glu Ala Leu Gly Gln His Glu Phe Cys Val
            35                  40                  45
   Arg Asp Pro Phe Arg Arg Gly Thr Phe Phe Ala Asn Thr Thr Val Arg
        50                  55                  60
   Lys Gly Asp Lys Asp Leu Gln Gln Pro Phe Ala Val Asp Ile Thr Lys
   65                  70                  75                  80
   Phe His Leu Cys Ala Asp Pro Leu Ala Ile Pro Glu Cys His Arg Asp
                   85                  90                  95
   Glu Ile Ile Gln Gly Ile Leu Gln Phe Ile Glu Gly Gln Thr Tyr Asp
               100                 105                 110
   Asp Leu Ser Leu Lys Leu Asp Lys Lys Ser Arg Tyr Cys Lys Leu Tyr
           115                 120                 125
   Pro Leu Leu Asp Val Ser Val His Asp Arg Leu Ser Leu Trp Trp Lys
       130                 135                 140
   Gly Tyr Ala Thr Lys His Arg Leu Pro Thr Asn Ala Leu Phe Phe Ile
   145                 150                 155                 160
   Thr Asp Tyr Gln Arg Ser Tyr Pro Phe Gly Lys Leu Leu Gly Gln Val
                   165                 170                 175
   Leu His Thr Leu Arg Glu Ile Lys Asp Glu Lys Thr Gly Lys Ala Phe
               180                 185                 190
   Pro Thr Gly Gly Met Glu Ala Tyr Phe Asn His Ile Leu Glu Gly Asp
           195                 200                 205
   Val Gly Glu Arg Lys Leu Leu Arg Ser Pro Leu Asn Arg Leu Asp Thr
       210                 215                 220
   Asn Arg Val Ile Lys Leu Pro Lys Asp Gly Ser Asp Ile Tyr Leu Thr
   225                 230                 235                 240
   Ile Asn Pro Val Ile Gln Thr Ile Ala Glu Glu Glu Leu Glu Arg Gly
                   245                 250                 255
   Val Leu Glu Ala Lys Ala Gln Gly Gly Arg Leu Ile Leu Met Asn Ser
               260                 265                 270
   Gln Thr Gly Glu Ile Leu Ala Leu Ala Gln Tyr Pro Phe Phe Asp Pro
           275                 280                 285
   Thr Asn Tyr Lys Glu Tyr Phe Asn Asn Lys Glu Arg Ile Glu His Thr
       290                 295                 300
   Lys Val Ser Phe Val Ser Asp Val Phe Glu Pro Gly Ser Ile Met Lys
   305                 310                 315                 320
   Pro Leu Thr Val Ala Ile Ala Leu Gln Ala Asn Glu Glu Ala Ser Leu
                   325                 330                 335
   Lys Ser Gln Lys Lys Ile Phe Asp Pro Glu Glu Pro Ile Asp Val Thr
               340                 345                 350
   Arg Thr Leu Phe Pro Gly Arg Lys Gly Ser Pro Leu Lys Asp Ile Ser
           355                 360                 365
   Arg Asn Ser Gln Leu Asn Met Tyr Met Ala Ile Gln Lys Ser Ser Asn
       370                 375                 380
   Val Tyr Val Ala Gln Leu Ala Asp Arg Ile Ile Gln Ser Leu Gly Val
   385                 390                 395                 400
   Ala Trp Tyr Gln Gln Lys Leu Leu Ala Leu Gly Phe Gly Arg Lys Thr
                   405                 410                 415
   Gly Ile Glu Leu Pro Ser Glu Ala Ser Gly Leu Val Pro Ser Pro His
   420                     425                     430
   Arg Phe His Ile Asn Gly Ser Leu Glu Trp Ser Leu Ser Thr Pro Tyr
           435                     440                     445
   Ser Leu Ala Met Gly Tyr Asn Ile Leu Ala Thr Gly Ile Gln Met Val
       450                     455                     460
   Gln Ala Tyr Ala Ile Leu Ala Asn Gly Gly Tyr Ala Val Arg Pro Thr
   465                     470                     475                 480
   Leu Val Lys Lys Ile Val Ser Ala Ser Gly Glu Glu Tyr His Leu Pro
                   485                     490                     495
   Thr Lys Glu Lys Thr Arg Leu Phe Ser Glu Glu Ile Thr Arg Glu Val
               500                     505                     510
   Val Arg Ala Met Arg Phe Thr Thr Leu Pro Gly Gly Ser Gly Phe Arg
           515                     520                     525
   Ala Ser Pro Lys His His Ser Ser Ala Gly Lys Thr Gly Thr Thr Glu
       530                     535                     540
   Lys Met Ile His Gly Lys Tyr Asp Lys Arg Arg His Ile Ala Ser Phe
   545                     550                     555                 560
   Ile Gly Phe Thr Pro Val Glu Ser Ser Glu Gly Asn Phe Pro Pro Leu
                   565                     570                     575
   Val Met Leu Val Ser Ile Asp Asp Pro Glu Tyr Gly Leu Arg Ala Asp
               580                     585                     590
   Gly Thr Lys Asn Tyr Met Gly Gly Arg Cys Ala Ala Pro Ile Phe Ser
           595                     600                     605
   Arg Val Ala Asp Arg Thr Leu Leu Tyr Leu Gly Ile Leu Pro Asp Lys
       610                     615                     620
   Lys Leu Arg Asn Cys Asp Glu Glu Ala Ala Ala Leu Lys Arg Leu Tyr
   625                     630                     635                 640
   Glu Glu Trp Asn Arg Ser Pro Lys Gln Gly Gly Thr Arg
                   645                     650

<210> 17
<211> 2738
<212> DNA
<213> Chlamydia pneumoniae

<220>
<221> CDS
<222> (101)..(2635)

<400> 17
gaattttacc aaatttgctg gtttagagcg aagagttgca tcattatttt aaatttcgta 60

tatgcttaag gaaagttcta cccctgtctt ttaggttttt atg ttt gag aag ttc    115
                                            Met Phe Glu Lys Phe
                                            1               5

act aat aga gca aaa caa gtc att aaa ctg gcg aaa aag gag gct cag    163
Thr Asn Arg Ala Lys Gln Val Ile Lys Leu Ala Lys Lys Glu Ala Gln
                10                  15                  20

cgt tta aat cat aac tac ctg ggt act gag cac atc ctg ctt ggt ctt    211
Arg Leu Asn His Asn Tyr Leu Gly Thr Glu His Ile Leu Leu Gly Leu
            25                  30                  35

ctc aaa ctt ggt caa ggg gta gct gtt aat gta tta cgc aac ctc ggt     259
Leu Lys Leu Gly Gln Gly Val Ala Val Asn Val Leu Arg Asn Leu Gly
        40                  45                  50

ata gat ttt gat acg gca cgg caa gag gtg gaa cgc ctg att ggt tat     307
Ile Asp Phe Asp Thr Ala Arg Gln Glu Val Glu Arg Leu Ile Gly Tyr
    55                  60                  65

ggt cca gaa att caa gtc tac gga gac cct gcc ctt aca gga aga gta     355
Gly Pro Glu Ile Gln Val Tyr Gly Asp Pro Ala Leu Thr Gly Arg Val
70                  75                  80                  85

aaa aaa tct ttt gaa tca gca aat gaa gag gcc agc ctt tta gag cac     403
Lys Lys Ser Phe Glu Ser Ala Asn Glu Glu Ala Ser Leu Leu Glu His
                90                  95                  100

aat tat gtc ggg acg gag cat tta ctc tta ggg atc cta cat caa tca     451
Asn Tyr Val Gly Thr Glu His Leu Leu Leu Gly Ile Leu His Gln Ser
            105                 110                 115

gat agt gtc gct ctt cag gta tta gaa aac tta cat atc gat cca aga     499
Asp Ser Val Ala Leu Gln Val Leu Glu Asn Leu His Ile Asp Pro Arg
        120                 125                 130

gag gtt cgt aag gaa att ctt aga gaa tta gag acc ttc aat cta caa     547
Glu Val Arg Lys Glu Ile Leu Arg Glu Leu Glu Thr Phe Asn Leu Gln
    135                 140                 145

ctt cct cct tcg tcg tcg tct tct tcc tca tcc tct cga agc aac cct     595
Leu Pro Pro Ser Ser Ser Ser Ser Ser Ser Ser Ser Arg Ser Asn Pro
150                 155                 160                 165

tca tct tca aaa tct cct tta ggt cat agc tta ggt tct gac aaa aac     643
Ser Ser Ser Lys Ser Pro Leu Gly His Ser Leu Gly Ser Asp Lys Asn
                170                 175                 180

gaa aag ctt tct gct ctg aaa gca tat ggt tat gat tta acg gag atg     691
Glu Lys Leu Ser Ala Leu Lys Ala Tyr Gly Tyr Asp Leu Thr Glu Met
            185                 190                 195

gtc cga gag tct aag ctc gat cct gtc att ggt cgt tct tca gaa gtc     739
Val Arg Glu Ser Lys Leu Asp Pro Val Ile Gly Arg Ser Ser Glu Val
        200                 205                 210

gaa cgg ttg att ttg att ctt tgc cga aga aga aaa aac aat cct gta     787
Glu Arg Leu Ile Leu Ile Leu Cys Arg Arg Arg Lys Asn Asn Pro Val
    215                 220                 225

ctt att gga gaa gct gga gtt ggt aag act gca att gtt gag ggt ctg     835
Leu Ile Gly Glu Ala Gly Val Gly Lys Thr Ala Ile Val Glu Gly Leu
230                 235                 240                 245

gct caa aaa atc att ctg aat gag gtt cct gat gcc tta cgg aaa aag     883
Ala Gln Lys Ile Ile Leu Asn Glu Val Pro Asp Ala Leu Arg Lys Lys
                250                 255                 260

cga ctg att act cta gat cta gca tta atg att gct gga aca aaa tat     931
Arg Leu Ile Thr Leu Asp Leu Ala Leu Met Ile Ala Gly Thr Lys Tyr
            265                 270                 275

cga ggg caa ttt gag gaa cgg atc aaa gct gtc atg gat gaa gtt cgc     979
Arg Gly Gln Phe Glu Glu Arg Ile Lys Ala Val Met Asp Glu Val Arg
        280                 285                 290

aag cat gga aac atc ttg ctc ttc att gac gag ctc cac acg att gta     1027
Lys His Gly Asn Ile Leu Leu Phe Ile Asp Glu Leu His Thr Ile Val
    295                 300                 305

gga gca gga gca gct gaa ggt gct atc gat gct tca aac att tta aaa 1075
Gly Ala Gly Ala Ala Glu Gly Ala Ile Asp Ala Ser Asn Ile Leu Lys
310                 315                 320                 325

cct gcg tta gcg cga ggt gaa att cag tgt att gga gca act acg ata 1123
Pro Ala Leu Ala Arg Gly Glu Ile Gln Cys Ile Gly Ala Thr Thr Ile
                330                 335                 340

gat gag tat cgc aag cac ata gaa aaa gac gca gct tta gaa cgt cgt 1171
Asp Glu Tyr Arg Lys His Ile Glu Lys Asp Ala Ala Leu Glu Arg Arg
            345                 350                 355

ttc caa aaa atc gtg gtt cac cct cct agt gta gat gag act att gag 1219
Phe Gln Lys Ile Val Val His Pro Pro Ser Val Asp Glu Thr Ile Glu
        360                 365                 370

att tta cgt ggc ctc aag aaa aag tat gaa gaa cat cac aat gtc ttc 1267
Ile Leu Arg Gly Leu Lys Lys Lys Tyr Glu Glu His His Asn Val Phe
    375                 380                 385

att act gaa gaa gct tta aaa gca gct gcg act ctt tct gat caa tat 1315
Ile Thr Glu Glu Ala Leu Lys Ala Ala Ala Thr Leu Ser Asp Gln Tyr
390                 395                 400                 405

gtt cat gga cgt ttc ctc cct gat aaa gca ata gat ctt tta gat gaa 1363
Val His Gly Arg Phe Leu Pro Asp Lys Ala Ile Asp Leu Leu Asp Glu
                410                 415                 420

gct ggg gct cgt gtc cgt gtg aat aca atg ggt cag cct aca gat tta 1411
Ala Gly Ala Arg Val Arg Val Asn Thr Met Gly Gln Pro Thr Asp Leu
            425                 430                 435

atg aag cta gag gct gaa atc gaa aat aca aaa ttg gcc aaa gag cag 1459
Met Lys Leu Glu Ala Glu Ile Glu Asn Thr Lys Leu Ala Lys Glu Gln
        440                 445                 450

gcc att gga act caa gaa tac gaa aaa gct gca ggt tta cgt gat gaa 1507
Ala Ile Gly Thr Gln Glu Tyr Glu Lys Ala Ala Gly Leu Arg Asp Glu
    455                 460                 465

gag aaa aaa ctt cgc gaa cgt ctg caa agt atg aaa cag gaa tgg gaa 1555
Glu Lys Lys Leu Arg Glu Arg Leu Gln Ser Met Lys Gln Glu Trp Glu
470                 475                 480                 485

aat cat aaa gaa gag cac caa gtt cct gta gat gaa gaa gca gtc gct 1603
Asn His Lys Glu Glu His Gln Val Pro Val Asp Glu Glu Ala Val Ala
                490                 495                 500

cag gta gtt tct cta caa aca gga att ccc tca gca agg ctc aca gaa 1651
Gln Val Val Ser Leu Gln Thr Gly Ile Pro Ser Ala Arg Leu Thr Glu
            505                 510                 515

gct gaa agt gag aag ctt ctg aag tta gaa gac acg tta aga aga aaa 1699
Ala Glu Ser Glu Lys Leu Leu Lys Leu Glu Asp Thr Leu Arg Arg Lys
        520                 525                 530

gtc att ggt caa aat gat gcc gtt acc agc att tgc cgt gcc atc cga 1747
Val Ile Gly Gln Asn Asp Ala Val Thr Ser Ile Cys Arg Ala Ile Arg
    535                 540                 545

cgt tct cga aca ggg atc aaa gat cct aac cga cct acg ggc tcc ttc 1795
Arg Ser Arg Thr Gly Ile Lys Asp Pro Asn Arg Pro Thr Gly Ser Phe
550                 555                 560                 565

cta ttc ctt ggg cct acc ggt gta ggg aaa agc ctg ctc gcc caa caa 1843
Leu Phe Leu Gly Pro Thr Gly Val Gly Lys Ser Leu Leu Ala Gln Gln
                570                 575                 580

att gct ata gag atg ttc ggt ggt gaa gac gct ctg att cag gta gac 1891
Ile Ala Ile Glu Met Phe Gly Gly Glu Asp Ala Leu Ile Gln Val Asp
            585                 590                 595

atg tca gag tac atg gag aaa ttt gct gct acc aag atg atg gga tca 1939
Met Ser Glu Tyr Met Glu Lys Phe Ala Ala Thr Lys Met Met Gly Ser
        600                 605                 610

cct cca gga tat gta ggt cat gaa gaa ggg ggc cac ctt acg gaa cag 1987
Pro Pro Gly Tyr Val Gly His Glu Glu Gly Gly His Leu Thr Glu Gln
    615                 620                 625

gta cgt cgc cgt cct tac tgc gtt gtt ctc ttt gat gag ata gaa aag 2035
Val Arg Arg Arg Pro Tyr Cys Val Val Leu Phe Asp Glu Ile Glu Lys
630                 635                 640                 645

gca cac cca gac att atg gac ctg atg ttg caa att tta gag caa gga 2083
Ala His Pro Asp Ile Met Asp Leu Met Leu Gln Ile Leu Glu Gln Gly
                650                 655                 660

cgt ctt act gat tct ttt ggt cgc aaa gtg gat ttc cgt cat gcc att 2131
Arg Leu Thr Asp Ser Phe Gly Arg Lys Val Asp Phe Arg His Ala Ile
            665                 670                 675

att atc atg acc tcc aat ttg gga gct gat ctc att cgt aaa agc gga 2179
Ile Ile Met Thr Ser Asn Leu Gly Ala Asp Leu Ile Arg Lys Ser Gly
        680                 685                 690

gaa att ggt ttt ggc ttg aag tcc cat atg gac tat aag gtc atc caa 2227
Glu Ile Gly Phe Gly Leu Lys Ser His Met Asp Tyr Lys Val Ile Gln
    695                 700                 705

gag aaa atc gaa cat gct atg aag aaa cac tta aag cct gag ttc att 2275
Glu Lys Ile Glu His Ala Met Lys Lys His Leu Lys Pro Glu Phe Ile
710                 715                 720                 725

aac cgt ttg gat gaa agt gtg att ttc cgt ccc ctc gag aaa gaa tct 2323
Asn Arg Leu Asp Glu Ser Val Ile Phe Arg Pro Leu Glu Lys Glu Ser
                730                 735                 740

cta tcg gag atc atc cat tta gag atc aac aaa ctg gac tcg aga ctg 2371
Leu Ser Glu Ile Ile His Leu Glu Ile Asn Lys Leu Asp Ser Arg Leu
            745                 750                 755

aaa aac tac caa atg gct ttg aac atc cca gac tct gtg att tcc ttc 2419
Lys Asn Tyr Gln Met Ala Leu Asn Ile Pro Asp Ser Val Ile Ser Phe
        760                 765                 770

cta gta acg aag ggg cat tct cca gaa atg gga gca cgt cct cta cgc 2467
Leu Val Thr Lys Gly His Ser Pro Glu Met Gly Ala Arg Pro Leu Arg
    775                 780                 785

cgt gtc att gag cag tac ctt gaa gat cct cta gcg gag ctc ttg ctt 2515
Arg Val Ile Glu Gln Tyr Leu Glu Asp Pro Leu Ala Glu Leu Leu Leu
790                 795                 800                 805

aaa gag tcc tgc cgt caa gaa gct cgc aag cta cga gca acc ttg gtt 2563
Lys Glu Ser Cys Arg Gln Glu Ala Arg Lys Leu Arg Ala Thr Leu Val
                810                 815                 820

gaa aat cgc gtt gcc ttt gaa agg gaa gaa gag gag cag gaa gct gct 2611
Glu Asn Arg Val Ala Phe Glu Arg Glu Glu Glu Glu Gln Glu Ala Ala
            825                 830                 835

ctc cct agc cct cac ttg gaa tca taggaacgtc gataactcca ctaccaaggc 2665
Leu Pro Ser Pro His Leu Glu Ser
        840                 845

aggtatctcc ttgataaaac gctattgttt gtcctggagt taccgccttg acgggttgtg 2725

aaaatcgcac ctt 2738

<210> 18
<211> 845
<212> PRT
<213> Chlamydia pneumoniae

<220>
<221> SITE
<222> (467)...(492)
<223> B-cell epitope

<220>
<221> SITE
<222> (548)...(563)
<223> B-cell epitope

<220>
<221> SITE
<222> (565)...(574)
<223> T-cell epitope

<220>
<221> SITE
<222> (410)...(418)
<223> T-cell epitope

<400> 18
Met Phe Glu Lys Phe Thr Asn Arg Ala Lys Gln Val Ile Lys Leu Ala
1               5                   10                  15

Lys Lys Glu Ala Gln Arg Leu Asn His Asn Tyr Leu Gly Thr Glu His
            20                  25                  30

Ile Leu Leu Gly Leu Leu Lys Leu Gly Gln Gly Val Ala Val Asn Val
        35                  40                  45

Leu Arg Asn Leu Gly Ile Asp Phe Asp Thr Ala Arg Gln Glu Val Glu
    50                  55                  60

Arg Leu Ile Gly Tyr Gly Pro Glu Ile Gln Val Tyr Gly Asp Pro Ala
65                  70                  75                  80

Leu Thr Gly Arg Val Lys Lys Ser Phe Glu Ser Ala Asn Glu Glu Ala
                85                  90                  95

Ser Leu Leu Glu His Asn Tyr Val Gly Thr Glu His Leu Leu Leu Gly
            100                 105                 110

Ile Leu His Gln Ser Asp Ser Val Ala Leu Gln Val Leu Glu Asn Leu
        115                 120                 125

His Ile Asp Pro Arg Glu Val Arg Lys Glu Ile Leu Arg Glu Leu Glu
    130                 135                 140

Thr Phe Asn Leu Gln Leu Pro Pro Ser Ser Ser Ser Ser Ser Ser Ser
145                 150                 155                 160

Ser Arg Ser Asn Pro Ser Ser Ser Lys Ser Pro Leu Gly His Ser Leu
                165                 170                 175

Gly Ser Asp Lys Asn Glu Lys Leu Ser Ala Leu Lys Ala Tyr Gly Tyr
            180                 185                 190

Asp Leu Thr Glu Met Val Arg Glu Ser Lys Leu Asp Pro Val Ile Gly

195                 200                 205

Arg Ser Ser Glu Val Glu Arg Leu Ile Leu Ile Leu Cys Arg Arg Arg
    210                 215                 220

Lys Asn Asn Pro Val Leu Ile Gly Glu Ala Gly Val Gly Lys Thr Ala
225                 230                 235                 240

Ile Val Glu Gly Leu Ala Gln Lys Ile Ile Leu Asn Glu Val Pro Asp
                245                 250                 255

Ala Leu Arg Lys Lys Arg Leu Ile Thr Leu Asp Leu Ala Leu Met Ile
            260                 265                 270

Ala Gly Thr Lys Tyr Arg Gly Gln Phe Glu Glu Arg Ile Lys Ala Val
        275                 280                 285

Met Asp Glu Val Arg Lys His Gly Asn Ile Leu Leu Phe Ile Asp Glu
    290                 295                 300

Leu His Thr Ile Val Gly Ala Gly Ala Ala Glu Gly Ala Ile Asp Ala
305                 310                 315                 320

Ser Asn Ile Leu Lys Pro Ala Leu Ala Arg Gly Glu Ile Gln Cys Ile
                325                 330                 335

Gly Ala Thr Thr Ile Asp Glu Tyr Arg Lys His Ile Glu Lys Asp Ala
            340                 345                 350

Ala Leu Glu Arg Arg Phe Gln Lys Ile Val Val His Pro Pro Ser Val
        355                 360                 365

Asp Glu Thr Ile Glu Ile Leu Arg Gly Leu Lys Lys Lys Tyr Glu Glu
    370                 375                 380

His His Asn Val Phe Ile Thr Glu Glu Ala Leu Lys Ala Ala Ala Thr
385                 390                 395                 400

Leu Ser Asp Gln Tyr Val His Gly Arg Phe Leu Pro Asp Lys Ala Ile
                405                 410                 415

Asp Leu Leu Asp Glu Ala Gly Ala Arg Val Arg Val Asn Thr Met Gly
            420                 425                 430

Gln Pro Thr Asp Leu Met Lys Leu Glu Ala Glu Ile Glu Asn Thr Lys
        435                 440                 445

Leu Ala Lys Glu Gln Ala Ile Gly Thr Gln Glu Tyr Glu Lys Ala Ala
    450                 455                 460

Gly Leu Arg Asp Glu Glu Lys Lys Leu Arg Glu Arg Leu Gln Ser Met
465                 470                 475                 480

Lys Gln Glu Trp Glu Asn His Lys Glu Glu His Gln Val Pro Val Asp
                485                 490                 495

Glu Glu Ala Val Ala Gln Val Val Ser Leu Gln Thr Gly Ile Pro Ser
            500                 505                 510

Ala Arg Leu Thr Glu Ala Glu Ser Glu Lys Leu Leu Lys Leu Glu Asp
        515                 520                 525

Thr Leu Arg Arg Lys Val Ile Gly Gln Asn Asp Ala Val Thr Ser Ile
    530                 535                 540

Cys Arg Ala Ile Arg Arg Ser Arg Thr Gly Ile Lys Asp Pro Asn Arg
545                 550                 555                 560

Pro Thr Gly Ser Phe Leu Phe Leu Gly Pro Thr Gly Val Gly Lys Ser
                565                 570                 575

Leu Leu Ala Gln Gln Ile Ala Ile Glu Met Phe Gly Gly Glu Asp Ala
            580                 585                 590

Leu Ile Gln Val Asp Met Ser Glu Tyr Met Glu Lys Phe Ala Ala Thr
        595                 600                 605

Lys Met Met Gly Ser Pro Pro Gly Tyr Val Gly His Glu Glu Gly Gly
    610                 615                 620

His Leu Thr Glu Gln Val Arg Arg Arg Pro Tyr Cys Val Val Leu Phe
625                 630                 635                 640

Asp Glu Ile Glu Lys Ala His Pro Asp Ile Met Asp Leu Met Leu Gln
                645                 650                 655

Ile Leu Glu Gln Gly Arg Leu Thr Asp Ser Phe Gly Arg Lys Val Asp
            660                 665                 670

Phe Arg His Ala Ile Ile Ile Met Thr Ser Asn Leu Gly Ala Asp Leu
        675                 680                 685

Ile Arg Lys Ser Gly Glu Ile Gly Phe Gly Leu Lys Ser His Met Asp
    690                 695                 700

Tyr Lys Val Ile Gln Glu Lys Ile Glu His Ala Met Lys Lys His Leu
705                 710                 715                 720

Lys Pro Glu Phe Ile Asn Arg Leu Asp Glu Ser Val Ile Phe Arg Pro
                725                 730                 735

Leu Glu Lys Glu Ser Leu Ser Glu Ile Ile His Leu Glu Ile Asn Lys
            740                 745                 750

Leu Asp Ser Arg Leu Lys Asn Tyr Gln Met Ala Leu Asn Ile Pro Asp
        755                 760                 765

Ser Val Ile Ser Phe Leu Val Thr Lys Gly His Ser Pro Glu Met Gly
    770                 775                 780

Ala Arg Pro Leu Arg Arg Val Ile Glu Gln Tyr Leu Glu Asp Pro Leu
785                 790                 795                 800

Ala Glu Leu Leu Leu Lys Glu Ser Cys Arg Gln Glu Ala Arg Lys Leu
                805                 810                 815

Arg Ala Thr Leu Val Glu Asn Arg Val Ala Phe Glu Arg Glu Glu Glu
            820                 825                 830

Glu Gln Glu Ala Ala Leu Pro Ser Pro His Leu Glu Ser
        835                 840                 845

<210> 19
<211> 509
<212> DNA
<213> Chlamydia pneumoniae

<220>
<221> CDS
<222> (101)..(406)

<400> 19
gattcaggtt ctagtgagct tatgctcatg gaagttcaag tcttcttagc tgcaagaaaa 60

taacagggac agtaattcga tttttcgaga agggaaactt atg gta aag atc ata   115
                                           Met Val Lys Ile Ile

1                 5

tca agt gaa aat ttt gac tct ttt att gca tcg ggg ctc gtt ctc gtt     163
Ser Ser Glu Asn Phe Asp Ser Phe Ile Ala Ser Gly Leu Val Leu Val
                10                  15                  20

gat ttc ttt gca gaa tgg tgt ggc ccc tgt cgg atg ctc act cct atc     211
Asp Phe Phe Ala Glu Trp Cys Gly Pro Cys Arg Met Leu Thr Pro Ile
            25                  30                  35

tta gaa aat ctt gct gcg gaa ctt cct cat gtc act att gga aaa atc     259
Leu Glu Asn Leu Ala Ala Glu Leu Pro His Val Thr Ile Gly Lys Ile
        40                  45                  50

aat ata gat gag aac agc aag cct gca gaa acg tac gaa gtc agc tct     307
Asn Ile Asp Glu Asn Ser Lys Pro Ala Glu Thr Tyr Glu Val Ser Ser
    55                  60                  65

att cct acg ctt att ctt ttt aag gat ggg aac gag gtg gct cgg gtc     355
Ile Pro Thr Leu Ile Leu Phe Lys Asp Gly Asn Glu Val Ala Arg Val
70                  75                  80                  85

gta ggt ctt aag gat aaa gaa ttc cta acc aat ctt atc aat aag cac     403
Val Gly Leu Lys Asp Lys Glu Phe Leu Thr Asn Leu Ile Asn Lys His
                90                  95                  100

gct taaaaagacg ctgcaatatt aaaccgtagg attcttttgc aatgctacgg          456
Ala

ttttctgcct taccacttca tataaaacga tccctacact ggtagctaaa ttt          509


<210> 20
<211> 102
<212> PRT
<213> Chlamydia pneumoniae

<220>
<221> SITE
<222> (54)...(66)
<223> B-cell epitope

<220>
<221> SITE
<222> (40)...(48)
<223> T-cell epitope

<220>
<221> SITE
<222> (74)...(83)
<223> T-cell epitope

<400> 20
Met Val Lys Ile Ile Ser Ser Glu Asn Phe Asp Ser Phe Ile Ala Ser
1               5                   10                  15

Gly Leu Val Leu Val Asp Phe Phe Ala Glu Trp Cys Gly Pro Cys Arg
            20                  25                  30

Met Leu Thr Pro Ile Leu Glu Asn Leu Ala Ala Glu Leu Pro His Val
        35                  40                  45

Thr Ile Gly Lys Ile Asn Ile Asp Glu Asn Ser Lys Pro Ala Glu Thr
    50                  55                  60

Tyr Glu Val Ser Ser Ile Pro Thr Leu Ile Leu Phe Lys Asp Gly Asn
65                  70                  75                  80

Glu Val Ala Arg Val Val Gly Leu Lys Asp Lys Glu Phe Leu Thr Asn
                85                  90                  95

Leu Ile Asn Lys His Ala
            100

<210> 21
<211> 43
<212> DNA
<213> Artificial Sequence

<220>
<223> 5' PCR primer

<400> 21
ataagaatgc ggccgccacc atgaagatgc ataggcttaa acc          43

<210> 22
<211> 36
<212> DNA
<213> Artificial Sequence

<220>
<223> 3' PCR primer

<400> 22
gcgccggatc ccacttaaga tatcgatatt tttgag          36

<210> 23
<211> 45
<212> DNA
<213> Artificial Sequence

<220>
<223> 5' PCR primer

<400> 23
ataagaatgc ggccgccacc atgcggttgg gaaataagcc tatgc          45

<210> 24
<211> 35
<212> DNA
<213> Artificial Sequence

<220>
<223> 3' PCR primer

<400> 24
gcgccggtac cgtaatttaa tactctttga agggc          35

<210> 25
<211> 49
<212> DNA
<213> Artificial Sequence

<220>
<223> 5' PCR primer

<400> 25
ataagaatgc ggccgccacc atgctcaccc taggcttgga aagttcttg          49

<210> 26
<211> 36

<212> DNA
<213> Artificial Sequence

<220>
<223> 3' PCR primer

<400> 26
gctttggagg atccccggag aggctaagga gaatgg 36

<210> 27
<211> 44
<212> DNA
<213> Artificial Sequence

<220>
<223> 5' PCR primer

<400> 27
ataagaatgc ggccgccacc atgaaaaaag ggaaattagg agcc 44

<210> 28
<211> 33
<212> DNA
<213> Artificial Sequence

<220>
<223> 3' PCR primer

<400> 28
gcgccggatc cccgaagcag aagtcgttgt ggg 33

<210> 29
<211> 48
<212> DNA
<213> Artificial Sequence

<220>
<223> 5' PCR primer

<400> 29
ataagaatgc ggccgccacc atgagaaaac ttattttatg caatccta 48

<210> 30
<211> 33
<212> DNA
<213> Artificial Sequence

<220>
<223> 3' PCR primer

<400> 30
gcgccggatc ccagaacaac ggagttcttt tgg 33

<210> 31
<211> 46
<212> DNA
<213> Artificial Sequence

<220>
<223> 5' PCR primer

<400> 31
ataagaatgc ggccgccacc atgaataaaa aaaatctaac tatttg 46

<210> 32
<211> 32
<212> DNA
<213> Artificial Sequence

<220>
<223> 3' PCR primer

<400> 32
gcgccggatc ccagcgatag cttctggggt cc                                    32


<210> 33
<211> 42
<212> DNA
<213> Artificial Sequence

<220>
<223> 5' PCR primer

<400> 33
ataagaatgc ggccgccacc atgacactgg taccctatgt tg                          42


<210> 34
<211> 35
<212> DNA
<213> Artificial Sequence

<220>
<223> 3' PCR primer

<400> 34
gcgccggatc ccagtgctac ttgtatcctt attag                                  35

<210> 35
<211> 45
<212> DNA
<213> Artificial Sequence

<220>
<223> 5' PCR primer

<400> 35
ataagaatgc ggccgccacc atgagctacc gtaaacgttc gactc                       45


<210> 36
<211> 35
<212> DNA
<213> Artificial Sequence

<220>
<223> 3' PCR primer

<400> 36
gcgccggatc cccctcgttc ccccttgttt cggag                                  35

<210> 37
<211> 46
<212> DNA
<213> Artificial Sequence

<220>
<223> 5' PCR primer

<400> 37

ataagaatgc ggccgccacc atgtttgaga agttcactaa tagagc 46

<210> 38
<211> 34
<212> DNA
<213> Artificial Sequence

<220>
<223> 3' PCR primer

<400> 38
gcgccggtac cgtgattcca agtgagggct aggg 34

<210> 39
<211> 42
<212> DNA
<213> Artificial Sequence

<220>
<223> 5' PCR primer

<400> 39
ataagaatgc ggccgccacc atggtaaaga tcatatcaag tg 42

<210> 40
<211> 30
<212> DNA
<213> Artificial Sequence

<220>
<223> 3' PCR primer

<400> 40
gcgccggatc ccagcgtgct tattgataag 30

<210> 41
<211> 17
<212> PRT
<213> Artificial Sequence

<220>
<223> B-cell epitope of ATP-binding cassette protein

<400> 41
Val His His Thr Leu Arg Glu Ser Tyr Lys Lys Gly Thr Pro Pro
1               5                   10                  15

Ser Thr

<210> 42
<211> 16

<212> PRT
<213> Artificial Sequence

<220>
<223> B-cell epitope of ATP-binding cassette protein

<400> 42
Asn Leu Gln Lys Glu Ile Ser Thr Glu Glu Arg Gln Thr Lys Ala
1               5                   10                  15

Arg

<210> 43
<211> 9
<212> PRT
<213> Artificial Sequence

<220>
<223> T-cell epitope of ATP-binding cassette protein

<400> 43
Trp Ile Ala Glu Tyr Val Ser Pro Val
1               5

<210> 44
<211> 12
<212> PRT
<213> Artificial Sequence

<220>
<223> B-cell epitope of endopeptidase protein

<400> 44
Lys Gly Asn Asn Ser Ser Pro Arg Ser Pro Ala Pro
1               5                   10

<210> 45
<211> 9
<212> PRT
<213> Artificial Sequence

<220>
<223> B-cell epitope of endopeptidase protein

<400> 45
Gly Glu Asn Phe Gln Lys Asn Ser Ser
1               5

<210> 46
<211> 9
<212> PRT
<213> Artificial Sequence

<220>
<223> T-cell epitope of endopeptidase protein

<400> 46
Leu Leu Ile Glu Asp Met Asp Leu Ile
1               5

<210> 47
<211> 9
<212> PRT
<213> Artificial Sequence

<220>
<223> T-cell epitope of endopeptidase protein

<400> 47
Asn Leu Leu Ile Glu Asp Met Asp Leu
1               5

<210> 48
<211> 16
<212> PRT
<213> Artificial Sequence

<220>
<223> B-cell epitope of protease protein

<400> 48
Thr Asp Leu Glu Gly Leu Glu Glu Asp His Lys Asp Ser Pro Trp
1               5                   10                  15

Glu

<210> 49
<211> 18
<212> PRT
<213> Artificial Sequence

<220>
<223> B-cell epitope of protease protein

<400> 49
Ser Glu Asn Ala Lys Lys Ser Glu Glu Gln Thr Ser Pro Gln Glu
1               5                   10                  15

Thr Pro Glu

<210> 50
<211> 9
<212> PRT
<213> Artificial Sequence

<220>
<223> T-cell epitope of protease protein

<400> 50
Tyr Leu Gly Asp Glu Ile Leu Glu Val
1               5

<210> 51
<211> 9
<212> PRT
<213> Artificial Sequence

<220>
<223> T-cell epitope of protease protein

<400> 51
Tyr Leu Tyr Ser Leu Leu Ser Met Leu
1               5

<210> 52
<211> 8
<212> PRT
<213> Artificial Sequence

<220>
<223> B-cell epitope of metalloprotease protein

<400> 52
Thr Thr Asn Arg Gln Lys Ala Leu
1               5

<210> 53
<211> 11
<212> PRT
<213> Artificial Sequence

<220>
<223> B-cell epitope of metalloprotease protein

<400> 53
Val Asn Ser Ser Asn Ser Asn Arg Leu Arg Glu

1                 5                   10

<210> 54
<211> 9
<212> PRT
<213> Artificial Sequence

<220>
<223> T-cell epitope of metalloprotease protein

<400> 54
Ser Val Leu Ser Arg Val Asn Tyr Val
1               5

<210> 55
<211> 9
<212> PRT
<213> Artificial Sequence

<220>
<223> T-cell epitope of metalloprotease protein

<400> 55
Lys Leu Ser Ser Leu Ile Pro Gly Leu
1               5

<210> 56
<211> 9
<212> PRT
<213> Artificial Sequence

<220>
<223> T-cell epitope of metalloprotease protein

<400> 56
Ile Leu Ile Gly His Lys Lys His Val
1               5

<210> 57
<211> 14
<212> PRT
<213> Artificial Sequence

<220>
<223> B-cell epitope of CLP protease ATPase protein

<400> 57
Pro Pro Lys Gly Gly Arg Lys His Pro Asn Gln Glu Tyr Ile
                5                   10

<210> 58
<211> 14
<212> PRT
<213> Artificial Sequence

<220>
<223> B-cell epitope of CLP protease ATPase protein

<400> 58
Ser Asp Asp Gln Ala Asp Leu Ser Gln Lys Thr Arg Asp His
1               5                   10

<210> 59
<211> 9
<212> PRT
<213> Artificial Sequence

<220>

<223> T-cell epitope of CLP protease ATPase protein

<400> 59
Lys Ile Leu Asp Val Pro Phe Thr Ile
1               5

<210> 60
<211> 9
<212> PRT
<213> Artificial Sequence

<220>
<223> T-cell epitope of CLP protease ATPase protein

<400> 60
Leu Leu Gln Ala Ala Asp Tyr Asp Val
1               5

<210> 61
<211> 9
<212> PRT
<213> Artificial Sequence

<220>
<223> B-cell epitope of CLP protease subunit protein

<400> 61
Gly Thr Lys Gly Lys Arg His Ala Leu
1               5

<210> 62
<211> 11
<212> PRT
<213> Artificial Sequence

<220>
<223> B-cell epitope of CLP protease subunit protein

<400> 62
Ala Lys Glu Thr Asn Lys Asp Thr Ser Ser Thr
1               5                   10

<210> 63
<211> 9
<212> PRT
<213> Artificial Sequence

<220>
<223> T-cell epitope of CLP protease subunit protein

<400> 63
Ala Ile Tyr Asp Thr Ile Arg Phe Leu
1               5

<210> 64
<211> 19
<212> PRT
<213> Artificial Sequence

<220>
<223> B-cell epitope of translycolase/transpeptidase protein

<400> 64
Asp Pro Thr Asn Tyr Lys Glu Tyr Phe Asn Asn Lys Glu Arg Ile
1               5                   10                  15

Glu His Thr Lys

<210> 65
<211> 17
<212> PRT
<213> Artificial Sequence

<220>
<223> B-cell epitope of translycolase/transpeptidase protein

<400> 65
Lys Arg Leu Tyr Glu Glu Trp Asn Arg Ser Pro Lys Gln Gly Gly
1               5                   10                  15

Thr Arg


<210> 66
<211> 9
<212> PRT
<213> Artificial Sequence

<220>
<223> T-cell epitope of translycolase/transpeptidase protein

<400> 66
Ala Leu Gly Gln His Glu Phe Cys Val
1               5

<210> 67
<211> 9
<212> PRT
<213> Artificial Sequence

<220>
<223> T-cell epitope of translycolase/transpeptidase protein

<400> 67
Ile Leu Ala Thr Gly Ile Gln Met Val
1               5

<210> 68
<211> 26
<212> PRT
<213> Artificial Sequence

<220>
<223> B-cell epitope of CLPc protease protein

<400> 68
Arg Asp Glu Glu Lys Lys Leu Arg Glu Arg Leu Gln Ser Met Lys
1               5                   10                  15

Gln Glu Trp Glu Asn His Lys Glu Glu His Gln
                20                  25


<210> 69
<211> 16
<212> PRT
<213> Artificial Sequence

<220>
<223> B-cell epitope of CLPc protease protein

<400> 69
Ile Arg Arg Ser Arg Thr Gly Ile Lys Asp Pro Asn Arg Pro Thr
1               5                   10                  15

Gly

<210> 70
<211> 9
<212> PRT
<213> Artificial Sequence

<220>
<223> T-cell epitope of CLPc protease protein

<400> 70
Phe Leu Phe Leu Gly Pro Thr Gly Val
1               5

<210> 71
<211> 9
<212> PRT
<213> Artificial Sequence

<220>
<223> T-cell epitope of CLPc protease protein

<400> 71
Phe Leu Pro Asp Lys Ala Ile Asp Leu
1               5

<210> 72
<211> 13
<212> PRT
<213> Artificial Sequence

<220>
<223> B-cell epitope of thioredoxin

<400> 72
Asn Ile Asp Glu Asn Ser Lys Pro Ala Glu Thr Tyr Glu
1               5                   10

<210> 73
<211> 9
<212> PRT
<213> Artificial Sequence

<220>
<223> B-cell epitope of thioredoxin

<400> 73
Asn Leu Ala Ala Glu Leu Pro His Val
1               5

<210> 74
<211> 9
<212> PRT
<213> Artificial Sequence

<220>
<223> T-cell epitope of thioredoxin

<400> 74
Ile Leu Phe Lys Asp Gly Asn Glu Val
1               5

SEQUENCE LISTING

<110> Sanofi Pasteur Limited

<120> Chlamydia secretory locus orf and uses thereof

<130> B5530A-AD/KP

<140> New European Divisional Patent Application
<141> 2001-05-08

<150> US 60/194,475
<151> 2000-04-04

<160> 74

<170> PatentIn Ver. 2.0

<210> 1
<211> 1787
<212> DNA
<213> Chlamydia pneumoniae

<220>
<221> CDS
<222> (101)..(1684)

<400> 1
aatctcattc ccccatcgac taaatccacc acggactccg acctcccatg tcttcaatcc 60

atatgaacgt aatattaagt agcaaattga gtactatata atg aag atg cat agg 115
Met Lys Met His Arg
1               5

ctt aaa cct acc tta aaa agt ctg atc cct aat ctt ctt ttc tta ttg 163
Leu Lys Pro Thr Leu Lys Ser Leu Ile Pro Asn Leu Leu Phe Leu Leu
                10                  15                  20

ctc act ctt tca agc tgc tca aag caa aaa caa gaa ccc tta gga aaa 211
Leu Thr Leu Ser Ser Cys Ser Lys Gln Lys Gln Glu Pro Leu Gly Lys
            25                  30                  35

cat ctc gtt att gcg atg agc cat gat ctc gcc gac cta gat cct cgc 259
His Leu Val Ile Ala Met Ser His Asp Leu Ala Asp Leu Asp Pro Arg
        40                  45                  50

aat gcc tat tta agc aga gat gct tcc cta gca aaa gcc ctc tat gaa 307
Asn Ala Tyr Leu Ser Arg Asp Ala Ser Leu Ala Lys Ala Leu Tyr Glu
    55                  60                  65

gga ctg aca aga gaa act gat caa gga atc gca ctg gct ctt gca gaa 355
Gly Leu Thr Arg Glu Thr Asp Gln Gly Ile Ala Leu Ala Leu Ala Glu
70                  75                  80                  85

agt tat acc ctg tca aaa gat cat aag gtc tat acc ttt aaa ctc aga 403
Ser Tyr Thr Leu Ser Lys Asp His Lys Val Tyr Thr Phe Lys Leu Arg
                90                  95                  100

cct tct gtg tgg agc gat ggc act cca ctc act gct tat gac ttt gaa 451
Pro Ser Val Trp Ser Asp Gly Thr Pro Leu Thr Ala Tyr Asp Phe Glu
            105                 110                 115

aaa tct ata aaa caa ctg tac ttc gaa gaa ttt tca cct tcc ata cat 499
Lys Ser Ile Lys Gln Leu Tyr Phe Glu Glu Phe Ser Pro Ser Ile His
        120                 125                 130

act tta ctc ggc gtg att aaa aat tct tcg gca atc cac aat gct caa 547
Thr Leu Leu Gly Val Ile Lys Asn Ser Ser Ala Ile His Asn Ala Gln

135                 140                 145

aaa tct ctg gaa act ctt ggg ata cag gca aaa gat gat ctt act ttg     595
Lys Ser Leu Glu Thr Leu Gly Ile Gln Ala Lys Asp Asp Leu Thr Leu
150                 155                 160                 165

gtg att acc cta gag caa cct ttc cca tac ttt ctc aca ctt atc gct     643
Val Ile Thr Leu Glu Gln Pro Phe Pro Tyr Phe Leu Thr Leu Ile Ala
                170                 175                 180

cgc ccc gta ttc tcc cct gtt cat cac acc ctt agg gaa tcc tat aag     691
Arg Pro Val Phe Ser Pro Val His His Thr Leu Arg Glu Ser Tyr Lys
            185                 190                 195

aaa gga aca ccc cca tcc aca tac atc tcc aat ggg ccc ttt gtc tta     739
Lys Gly Thr Pro Pro Ser Thr Tyr Ile Ser Asn Gly Pro Phe Val Leu
        200                 205                 210

aaa aaa cat gaa cac caa aac tac tta att tta gaa aaa aat cct cac     787
Lys Lys His Glu His Gln Asn Tyr Leu Ile Leu Glu Lys Asn Pro His
    215                 220                 225

tac tat gat cat gaa tca gta aag tta gac cga gtc acc tta aaa att     835
Tyr Tyr Asp His Glu Ser Val Lys Leu Asp Arg Val Thr Leu Lys Ile
230                 235                 240                 245

atc cca gac gcc tcc aca gcc acg aaa ctt ttc aaa agt aaa tct ata     883
Ile Pro Asp Ala Ser Thr Ala Thr Lys Leu Phe Lys Ser Lys Ser Ile
                250                 255                 260

gat tgg att ggc tca cct tgg agc gct ccg ata tct aac gaa gac caa     931
Asp Trp Ile Gly Ser Pro Trp Ser Ala Pro Ile Ser Asn Glu Asp Gln
            265                 270                 275

aaa gtt ctc tcc caa gaa aag att ctt acc tat tct gtt tca agc acc     979
Lys Val Leu Ser Gln Glu Lys Ile Leu Thr Tyr Ser Val Ser Ser Thr
        280                 285                 290

acc ctt ctt atc tat aac ctg caa aaa cct cta ata caa aat aaa gcc     1027
Thr Leu Leu Ile Tyr Asn Leu Gln Lys Pro Leu Ile Gln Asn Lys Ala
    295                 300                 305

ctc agg aaa gcc att gct cat gct att gat aga aaa tct atc tta aga     1075
Leu Arg Lys Ala Ile Ala His Ala Ile Asp Arg Lys Ser Ile Leu Arg
310                 315                 320                 325

ctc gtg cct tca gga caa gaa gct gta act cta gtt ccc cca aat ctt     1123
Leu Val Pro Ser Gly Gln Glu Ala Val Thr Leu Val Pro Pro Asn Leu
                330                 335                 340

tca caa ctc aat ctt caa aaa gag atc tca aca gaa gaa cga caa aca     1171
Ser Gln Leu Asn Leu Gln Lys Glu Ile Ser Thr Glu Glu Arg Gln Thr
            345                 350                 355

aaa gcc aga gca tat ttt caa gaa gct aaa gaa aca ctt tct gaa aaa     1219
Lys Ala Arg Ala Tyr Phe Gln Glu Ala Lys Glu Thr Leu Ser Glu Lys
        360                 365                 370

gaa ctc gca gaa ctc agc atc ctc tat cct ata gat tcc tcg aat tcc     1267
Glu Leu Ala Glu Leu Ser Ile Leu Tyr Pro Ile Asp Ser Ser Asn Ser
    375                 380                 385

tcc atc ata gct caa gaa atc caa aga caa ctt aaa gat acc tta gga     1315
Ser Ile Ile Ala Gln Glu Ile Gln Arg Gln Leu Lys Asp Thr Leu Gly
390                 395                 400                 405

ttg aaa atc aaa atc caa ggc atg gag tac cac tgc ttt tta aag aaa     1363
Leu Lys Ile Lys Ile Gln Gly Met Glu Tyr His Cys Phe Leu Lys Lys

                410                 415                 420

cgt cgt caa gga gat ttc ttc ata gcg aca gga gga tgg att gcg gaa 1411
Arg Arg Gln Gly Asp Phe Phe Ile Ala Thr Gly Gly Trp Ile Ala Glu
            425                 430                 435

tac gta agc ccc gta gcc ttc cta tct att cta ggc aac ccc aga gac 1459
Tyr Val Ser Pro Val Ala Phe Leu Ser Ile Leu Gly Asn Pro Arg Asp
        440                 445                 450

ctc aca caa tgg aga aac agt gat tac gaa aag act tta gag aaa ctc 1507
Leu Thr Gln Trp Arg Asn Ser Asp Tyr Glu Lys Thr Leu Glu Lys Leu
    455                 460                 465

tat ctc cct cat gcc tac aaa gag aat tta aaa cgc gca gaa atg ata 1555
Tyr Leu Pro His Ala Tyr Lys Glu Asn Leu Lys Arg Ala Glu Met Ile
470                 475                 480                 485

ata gaa gaa gaa acc ccg att atc ccc ctg tat cac ggc aaa tat att 1603
Ile Glu Glu Glu Thr Pro Ile Ile Pro Leu Tyr His Gly Lys Tyr Ile
                490                 495                 500

tac gct ata cat cct aaa atc cag aat aca ttc gga tct ctt cta ggc 1651
Tyr Ala Ile His Pro Lys Ile Gln Asn Thr Phe Gly Ser Leu Leu Gly
            505                 510                 515

cac aca gat ctc aaa aat atc gat atc tta agt tagatccgaa atggaaaaat 1704
His Thr Asp Leu Lys Asn Ile Asp Ile Leu Ser
        520                 525

taaaaatttt atagacaatc ttgaaaagag aattaaaaat ttttaattta aattatagtt 1764

gcaattgaaa acgcccctaa gaa 1787

<210> 2
<211> 528
<212> PRT
<213> Chlamydia pneumoniae

<220>
<221> SITE
<222> (188)...(61)
<223> B-cell epitope

<220>
<221> SITE
<222> (345)...(355)
<223> B-cell epitope

<220>
<221> SITE
<222> (434)...(442)
<223> T-cell epitope

<400> 2
Met Lys Met His Arg Leu Lys Pro Thr Leu Lys Ser Leu Ile Pro Asn
1               5                   10                  15

Leu Leu Phe Leu Leu Leu Thr Leu Ser Ser Cys Ser Lys Gln Lys Gln
            20                  25                  30

Glu Pro Leu Gly Lys His Leu Val Ile Ala Met Ser His Asp Leu Ala
        35                  40                  45

Asp Leu Asp Pro Arg Asn Ala Tyr Leu Ser Arg Asp Ala Ser Leu Ala
    50                  55                  60

Lys Ala Leu Tyr Glu Gly Leu Thr Arg Glu Thr Asp Gln Gly Ile Ala
65                  70                  75                  80

Leu Ala Leu Ala Glu Ser Tyr Thr Leu Ser Lys Asp His Lys Val Tyr
                85                  90                  95

Thr Phe Lys Leu Arg Pro Ser Val Trp Ser Asp Gly Thr Pro Leu Thr
            100                 105                 110

Ala Tyr Asp Phe Glu Lys Ser Ile Lys Gln Leu Tyr Phe Glu Glu Phe
        115                 120                 125

Ser Pro Ser Ile His Thr Leu Leu Gly Val Ile Lys Asn Ser Ser Ala
    130                 135                 140

Ile His Asn Ala Gln Lys Ser Leu Glu Thr Leu Gly Ile Gln Ala Lys
145                 150                 155                 160

Asp Asp Leu Thr Leu Val Ile Thr Leu Glu Gln Pro Phe Pro Tyr Phe
                165                 170                 175

Leu Thr Leu Ile Ala Arg Pro Val Phe Ser Pro Val His His Thr Leu
            180                 185                 190

Arg Glu Ser Tyr Lys Lys Gly Thr Pro Pro Ser Thr Tyr Ile Ser Asn
        195                 200                 205

Gly Pro Phe Val Leu Lys Lys His Glu His Gln Asn Tyr Leu Ile Leu
    210                 215                 220

Glu Lys Asn Pro His Tyr Tyr Asp His Glu Ser Val Lys Leu Asp Arg
225                 230                 235                 240

Val Thr Leu Lys Ile Ile Pro Asp Ala Ser Thr Ala Thr Lys Leu Phe
                245                 250                 255

Lys Ser Lys Ser Ile Asp Trp Ile Gly Ser Pro Trp Ser Ala Pro Ile
            260                 265                 270

Ser Asn Glu Asp Gln Lys Val Leu Ser Gln Glu Lys Ile Leu Thr Tyr
        275                 280                 285

Ser Val Ser Ser Thr Thr Leu Leu Ile Tyr Asn Leu Gln Lys Pro Leu
    290                 295                 300

Ile Gln Asn Lys Ala Leu Arg Lys Ala Ile Ala His Ala Ile Asp Arg
305                 310                 315                 320

Lys Ser Ile Leu Arg Leu Val Pro Ser Gly Gln Glu Ala Val Thr Leu
                325                 330                 335

Val Pro Pro Asn Leu Ser Gln Leu Asn Leu Gln Lys Glu Ile Ser Thr
            340                 345                 350

Glu Glu Arg Gln Thr Lys Ala Arg Ala Tyr Phe Gln Glu Ala Lys Glu
        355                 360                 365

Thr Leu Ser Glu Lys Glu Leu Ala Glu Leu Ser Ile Leu Tyr Pro Ile
    370                 375                 380

Asp Ser Ser Asn Ser Ser Ile Ile Ala Gln Glu Ile Gln Arg Gln Leu
385                 390                 395                 400

Lys Asp Thr Leu Gly Leu Lys Ile Lys Ile Gln Gly Met Glu Tyr His
                405                 410                 415

Cys Phe Leu Lys Lys Arg Arg Gln Gly Asp Phe Phe Ile Ala Thr Gly
            420                 425                 430

Gly Trp Ile Ala Glu Tyr Val Ser Pro Val Ala Phe Leu Ser Ile Leu
        435                 440                 445

Gly Asn Pro Arg Asp Leu Thr Gln Trp Arg Asn Ser Asp Tyr Glu Lys
    450                 455                 460

Thr Leu Glu Lys Leu Tyr Leu Pro His Ala Tyr Lys Glu Asn Leu Lys
465                 470                 475                 480

Arg Ala Glu Met Ile Ile Glu Glu Glu Thr Pro Ile Ile Pro Leu Tyr
                485                 490                 495

His Gly Lys Tyr Ile Tyr Ala Ile His Pro Lys Ile Gln Asn Thr Phe
            500                 505                 510

Gly Ser Leu Leu Gly His Thr Asp Leu Lys Asn Ile Asp Ile Leu Ser
        515                 520                 525

<210> 3
<211> 1226
<212> DNA
<213> Chlamydia pneumoniae

<220>
<221> CDS
<222> (101)..(1123)

<400> 3
ttccagagaa atcctgatcc tgaaaaactt cctgaaacaa ttgctttaac tataacacgg 60

gaacctaaag catatcctcc aaggacgtta acataccaat ttg cgg ttg gga aat    115
                                           Leu Arg Leu Gly Asn
                                           1               5

aag cct atg caa cct ttt atc ttt act tta ctg tgc ttg aca tct ttg    163
Lys Pro Met Gln Pro Phe Ile Phe Thr Leu Leu Cys Leu Thr Ser Leu
                10                  15                  20

gtt tct tta gtc gcc ttt gat gct gcg aat gct cgt aaa cgt tgt gcc    211
Val Ser Leu Val Ala Phe Asp Ala Ala Asn Ala Arg Lys Arg Cys Ala
            25                  30                  35

tgt gct caa act ata gaa cgt gga gag aac ttc ttt tcc ata aaa cgc    259
Cys Ala Gln Thr Ile Glu Arg Gly Glu Asn Phe Phe Ser Ile Lys Arg
        40                  45                  50

tct gct tgt gct gaa atc gaa tat caa gaa aaa tct cgc cac gcc tca    307
Ser Ala Cys Ala Glu Ile Glu Tyr Gln Glu Lys Ser Arg His Ala Ser
    55                  60                  65

gca att gaa aga atc tca aaa gat aaa ggc aaa gtc act cca aag cag    355
Ala Ile Glu Arg Ile Ser Lys Asp Lys Gly Lys Val Thr Pro Lys Gln
70                  75                  80                  85

att gcg aaa gta gct act aag aaa aag caa aga tac cgt tta ttg cag    403
Ile Ala Lys Val Ala Thr Lys Lys Lys Gln Arg Tyr Arg Leu Leu Gln
                90                  95                  100

gtt cct ttt tca agg cct ccg aat aac tca agg tat aac ctc tat gct    451
Val Pro Phe Ser Arg Pro Pro Asn Asn Ser Arg Tyr Asn Leu Tyr Ala
            105                 110                 115

ttg ctt agt gaa cct ccc gaa tgc tat agc gat aca gca tca tgg tat    499
Leu Leu Ser Glu Pro Pro Glu Cys Tyr Ser Asp Thr Ala Ser Trp Tyr
        120                 125                 130

gct att ttt att cgg tta ctt cga cgt gct tat gta gac acg gga aat    547

Ala Ile Phe Ile Arg Leu Leu Arg Arg Ala Tyr Val Asp Thr Gly Asn
    135                 140                 145

gta cct cct gga tct gag tat gcc atc gct aat gct ttg ata agt aac     595
Val Pro Pro Gly Ser Glu Tyr Ala Ile Ala Asn Ala Leu Ile Ser Asn
150                 155                 160                 165

aaa caa gag att tta gag agg gga gcg cag ctt gga ccc gat gtt att     643
Lys Gln Glu Ile Leu Glu Arg Gly Ala Gln Leu Gly Pro Asp Val Ile
                170                 175                 180

gaa act cta aca ttg cct gag gaa caa gcc gag att ttt tat aaa atg     691
Glu Thr Leu Thr Leu Pro Glu Glu Gln Ala Glu Ile Phe Tyr Lys Met
            185                 190                 195

ctc aaa ggg tcg tca aac tct cag tcg cta ctg aat ttt ctg cat tat     739
Leu Lys Gly Ser Ser Asn Ser Gln Ser Leu Leu Asn Phe Leu His Tyr
        200                 205                 210

gaa gag aaa agc tta ggc cac tgt aag cta aat ctg atc ttc atg gat     787
Glu Glu Lys Ser Leu Gly His Cys Lys Leu Asn Leu Ile Phe Met Asp
    215                 220                 225

ccc cta ctg tta gaa gct gtt cta gat cat ccc gat gct tat agg gaa     835
Pro Leu Leu Leu Glu Ala Val Leu Asp His Pro Asp Ala Tyr Arg Glu
230                 235                 240                 245

acg tcg ctc ctg cgc gat ggc att tgg gaa gcg gtg aag cgt caa gaa     883
Thr Ser Leu Leu Arg Asp Gly Ile Trp Glu Ala Val Lys Arg Gln Glu
                250                 255                 260

cat gcc atc caa gaa cat ggc cag gca gct gct ttg gag ctt ttt aaa     931
His Ala Ile Gln Glu His Gly Gln Ala Ala Ala Leu Glu Leu Phe Lys
            265                 270                 275

aca cgc acc gac ttc cgc ctg gag ctg cga gat aag atg cag tta ctt     979
Thr Arg Thr Asp Phe Arg Leu Glu Leu Arg Asp Lys Met Gln Leu Leu
        280                 285                 290

cta agt cga tac gat ttg ctc ccc tta tta aat aaa aaa atg ttc gac     1027
Leu Ser Arg Tyr Asp Leu Leu Pro Leu Leu Asn Lys Lys Met Phe Asp
    295                 300                 305

tac acc tta gga agt gcc gga gat tac tta ttt ttg gta gac cca gat     1075
Tyr Thr Leu Gly Ser Ala Gly Asp Tyr Leu Phe Leu Val Asp Pro Asp
310                 315                 320                 325

act aag gca att tct cga tgt cgc tgc cct tca aag agt att aaa tta     1123
Thr Lys Ala Ile Ser Arg Cys Arg Cys Pro Ser Lys Ser Ile Lys Leu
                330                 335                 340

taatttaatt ttaatattta ttttaaatag ttttttttga taattgtctt aataagtact 1183

ataaaaaata tttctatagg taggaccatg gcagacgaga ccc                  1226

<210> 4
<211> 341
<212> PRT
<213> Chlamydia pneumoniae

<400> 4
Leu Arg Leu Gly Asn Lys Pro Met Gln Pro Phe Ile Phe Thr Leu Leu
1               5                   10                  15

Cys Leu Thr Ser Leu Val Ser Leu Val Ala Phe Asp Ala Ala Asn Ala
            20                  25                  30

Arg Lys Arg Cys Ala Cys Ala Gln Thr Ile Glu Arg Gly Glu Asn Phe
        35                  40                  45

Phe Ser Ile Lys Arg Ser Ala Cys Ala Glu Ile Glu Tyr Gln Glu Lys
    50                  55                  60

Ser Arg His Ala Ser Ala Ile Glu Arg Ile Ser Lys Asp Lys Gly Lys
65                  70                  75                  80

Val Thr Pro Lys Gln Ile Ala Lys Val Ala Thr Lys Lys Lys Gln Arg
                85                  90                  95

Tyr Arg Leu Leu Gln Val Pro Phe Ser Arg Pro Pro Asn Asn Ser Arg
            100                 105                 110

Tyr Asn Leu Tyr Ala Leu Leu Ser Glu Pro Pro Glu Cys Tyr Ser Asp
        115                 120                 125

Thr Ala Ser Trp Tyr Ala Ile Phe Ile Arg Leu Leu Arg Arg Ala Tyr
    130                 135                 140

Val Asp Thr Gly Asn Val Pro Pro Gly Ser Glu Tyr Ala Ile Ala Asn
145                 150                 155                 160

Ala Leu Ile Ser Asn Lys Gln Glu Ile Leu Glu Arg Gly Ala Gln Leu
                165                 170                 175

Gly Pro Asp Val Ile Glu Thr Leu Thr Leu Pro Glu Glu Gln Ala Glu
            180                 185                 190

Ile Phe Tyr Lys Met Leu Lys Gly Ser Ser Asn Ser Gln Ser Leu Leu
        195                 200                 205

Asn Phe Leu His Tyr Glu Glu Lys Ser Leu Gly His Cys Lys Leu Asn
    210                 215                 220

Leu Ile Phe Met Asp Pro Leu Leu Leu Glu Ala Val Leu Asp His Pro
225                 230                 235                 240

Asp Ala Tyr Arg Glu Thr Ser Leu Leu Arg Asp Gly Ile Trp Glu Ala
                245                 250                 255

Val Lys Arg Gln Glu His Ala Ile Gln Glu His Gly Gln Ala Ala Ala
            260                 265                 270

Leu Glu Leu Phe Lys Thr Arg Thr Asp Phe Arg Leu Glu Leu Arg Asp
        275                 280                 285

Lys Met Gln Leu Leu Leu Ser Arg Tyr Asp Leu Leu Pro Leu Leu Asn
    290                 295                 300

Lys Lys Met Phe Asp Tyr Thr Leu Gly Ser Ala Gly Asp Tyr Leu Phe
305                 310                 315                 320

Leu Val Asp Pro Asp Thr Lys Ala Ile Ser Arg Cys Arg Cys Pro Ser
                325                 330                 335

Lys Ser Ile Lys Leu
            340

<210> 5
<211> 1235
<212> DNA
<213> Chlamydia pneumoniae

<220>
<221> CDS
<222> (101)..(1132)

<400> 5
gttacttttt ttttcataaa aaccccatgt aacttttact tgctcatatt gagaagtccc 60

ccatactata aaaggcaacg ttttcttttc ttggtttttt atg ctc acc cta ggc 115
Met Leu Thr Leu Gly
1               5

ttg gaa agt tct tgc gat gag act gcc tgc gct ata gtt aat gag gat 163
Leu Glu Ser Ser Cys Asp Glu Thr Ala Cys Ala Ile Val Asn Glu Asp
                10                  15                  20

aag cag ata tta gca aat att att gcc tct caa gat atc cat gca tcc 211
Lys Gln Ile Leu Ala Asn Ile Ile Ala Ser Gln Asp Ile His Ala Ser
            25                  30                  35

tat ggc gga gtc gtt cct gaa ctt gct tca aga gca cat ctc cat atc 259
Tyr Gly Gly Val Val Pro Glu Leu Ala Ser Arg Ala His Leu His Ile
        40                  45                  50

ttc cca caa gtg ata aat aaa gct cta caa cag gcc aac tta ttg atc 307
Phe Pro Gln Val Ile Asn Lys Ala Leu Gln Gln Ala Asn Leu Leu Ile
    55                  60                  65

gaa gat atg gat ctg att gca gta acg caa act cca ggg ttg ata ggt 355
Glu Asp Met Asp Leu Ile Ala Val Thr Gln Thr Pro Gly Leu Ile Gly
70                  75                  80                  85

tct cta tca gta gga gtg cat ttt ggt aaa ggc att gcc ata gga gca 403
Ser Leu Ser Val Gly Val His Phe Gly Lys Gly Ile Ala Ile Gly Ala
                90                  95                  100

aaa aaa tcc ttg att gga gtc aat cac gtc gaa gct cat ctc tat gct 451
Lys Lys Ser Leu Ile Gly Val Asn His Val Glu Ala His Leu Tyr Ala
            105                 110                 115

gcc tat atg gca gcg caa aac gtg caa ttc cct gct tta ggt ctt gtg 499
Ala Tyr Met Ala Ala Gln Asn Val Gln Phe Pro Ala Leu Gly Leu Val
        120                 125                 130

gtc tct gga gct cat acc gca gcg ttt ttt ata gaa aat cct aca tcc 547
Val Ser Gly Ala His Thr Ala Ala Phe Phe Ile Glu Asn Pro Thr Ser
    135                 140                 145

tat aaa ctc ata gga aaa act cga gat gat gct ata gga gaa act ttt 595
Tyr Lys Leu Ile Gly Lys Thr Arg Asp Asp Ala Ile Gly Glu Thr Phe
150                 155                 160                 165

gat aaa gta gga cgc ttt cta gga tta cca tac cct gca ggc cca tta 643
Asp Lys Val Gly Arg Phe Leu Gly Leu Pro Tyr Pro Ala Gly Pro Leu
                170                 175                 180

att gaa aaa ctc gct tta gaa ggc tct gag gac agt tat cct ttt agt 691
Ile Glu Lys Leu Ala Leu Glu Gly Ser Glu Asp Ser Tyr Pro Phe Ser
            185                 190                 195

cca gct aaa gtc cca aac tat gac ttt tca ttc agc ggt ctt aaa aca 739
Pro Ala Lys Val Pro Asn Tyr Asp Phe Ser Phe Ser Gly Leu Lys Thr
        200                 205                 210

gct gtt ctc tac gca atc aaa gga aat aat agt agc ccc cgc tct cct 787
Ala Val Leu Tyr Ala Ile Lys Gly Asn Asn Ser Ser Pro Arg Ser Pro
    215                 220                 225

gct cca gag ata tct tta gaa aaa caa aga gat atc gct gct tca ttt 835
Ala Pro Glu Ile Ser Leu Glu Lys Gln Arg Asp Ile Ala Ala Ser Phe
230                 235                 240                 245

caa aaa gcg gcc tgc act act att gca caa aaa ctt ccc act att ata     883
Gln Lys Ala Ala Cys Thr Thr Ile Ala Gln Lys Leu Pro Thr Ile Ile
                250                 255                 260

aaa gaa ttt tcg tgc cga tct ata ctt att gga ggt ggc gta gcc att     931
Lys Glu Phe Ser Cys Arg Ser Ile Leu Ile Gly Gly Gly Val Ala Ile
            265                 270                 275

aat gaa tac ttt aga tcc gca ata caa act gcg tgt aat cta cct gta     979
Asn Glu Tyr Phe Arg Ser Ala Ile Gln Thr Ala Cys Asn Leu Pro Val
        280                 285                 290

tac ttc ccc cct gct aaa cta tgc tca gat aat gct gct atg att gca     1027
Tyr Phe Pro Pro Ala Lys Leu Cys Ser Asp Asn Ala Ala Met Ile Ala
    295                 300                 305

ggt cta ggg gga gaa aat ttt caa aaa aac tct agt att ccg gaa att     1075
Gly Leu Gly Gly Glu Asn Phe Gln Lys Asn Ser Ser Ile Pro Glu Ile
310                 315                 320                 325

cgt ata tgc gca aga tat cag tgg gaa tct gta tca cca ttc tcc tta     1123
Arg Ile Cys Ala Arg Tyr Gln Trp Glu Ser Val Ser Pro Phe Ser Leu
                330                 335                 340

gcc tct ccg tagtcctcca aggctgcaag gagtccagtc actcctctac             1172
Ala Ser Pro

atctcgggga gaactcgcta ttaatataag agatgaaccc cgttctttag atccaagaca 1232

agt                                                                 1235


<210> 6
<211> 344
<212> PRT
<213> Chlamydia pneumoniae

<220>
<221> SITE
<222> (220)...(231)
<223> B-cell epitope

<220>
<221> SITE
<222> (313)...(321)
<223> B-cell epitope

<220>
<221> SITE
<222> (67)...(76)
<223> T-cell epitope

<220>
<221> SITE
<222> (66)...(75)
<223> T-cell epitope

<400> 6
Met Leu Thr Leu Gly Leu Glu Ser Ser Cys Asp Glu Thr Ala Cys Ala
  1               5                  10                  15

Ile Val Asn Glu Asp Lys Gln Ile Leu Ala Asn Ile Ile Ala Ser Gln
             20                  25                  30

Asp Ile His Ala Ser Tyr Gly Gly Val Val Pro Glu Leu Ala Ser Arg
         35                  40                  45

Ala His Leu His Ile Phe Pro Gln Val Ile Asn Lys Ala Leu Gln Gln

50                  55                  60

Ala Asn Leu Leu Ile Glu Asp Met Asp Leu Ile Ala Val Thr Gln Thr
65                  70                  75                  80

Pro Gly Leu Ile Gly Ser Leu Ser Val Gly Val His Phe Gly Lys Gly
                85                  90                  95

Ile Ala Ile Gly Ala Lys Lys Ser Leu Ile Gly Val Asn His Val Glu
            100                 105                 110

Ala His Leu Tyr Ala Ala Tyr Met Ala Ala Gln Asn Val Gln Phe Pro
        115                 120                 125

Ala Leu Gly Leu Val Val Ser Gly Ala His Thr Ala Ala Phe Phe Ile
    130                 135                 140

Glu Asn Pro Thr Ser Tyr Lys Leu Ile Gly Lys Thr Arg Asp Asp Ala
145                 150                 155                 160

Ile Gly Glu Thr Phe Asp Lys Val Gly Arg Phe Leu Gly Leu Pro Tyr
                165                 170                 175

Pro Ala Gly Pro Leu Ile Glu Lys Leu Ala Leu Glu Gly Ser Glu Asp
            180                 185                 190

Ser Tyr Pro Phe Ser Pro Ala Lys Val Pro Asn Tyr Asp Phe Ser Phe
        195                 200                 205

Ser Gly Leu Lys Thr Ala Val Leu Tyr Ala Ile Lys Gly Asn Asn Ser
    210                 215                 220

Ser Pro Arg Ser Pro Ala Pro Glu Ile Ser Leu Glu Lys Gln Arg Asp
225                 230                 235                 240

Ile Ala Ala Ser Phe Gln Lys Ala Ala Cys Thr Thr Ile Ala Gln Lys
                245                 250                 255

Leu Pro Thr Ile Ile Lys Glu Phe Ser Cys Arg Ser Ile Leu Ile Gly
            260                 265                 270

Gly Gly Val Ala Ile Asn Glu Tyr Phe Arg Ser Ala Ile Gln Thr Ala
        275                 280                 285

Cys Asn Leu Pro Val Tyr Phe Pro Pro Ala Lys Leu Cys Ser Asp Asn
    290                 295                 300

Ala Ala Met Ile Ala Gly Leu Gly Gly Glu Asn Phe Gln Lys Asn Ser
305                 310                 315                 320

Ser Ile Pro Glu Ile Arg Ile Cys Ala Arg Tyr Gln Trp Glu Ser Val
                325                 330                 335

Ser Pro Phe Ser Leu Ala Ser Pro
            340

<210> 7
<211> 2060
<212> DNA
<213> Chlamydia pneumoniae

<220>
<221> CDS
<222> (101)..(1957)

<400> 7
gattttgtgt attttttcag ataatgtttt taaaaaaatg ttttaaaacc ctaaaatcct 60

acctccttgt aaccattctc ggtagaaaag agaggtattt atg aaa aaa ggg aaa 115
Met Lys Lys Gly Lys
1               5

tta gga gcc ata gtt ttt ggc ctt cta ttt aca agt agt gtt gct ggt 163
Leu Gly Ala Ile Val Phe Gly Leu Leu Phe Thr Ser Ser Val Ala Gly
                10                  15                  20

ttt tct aag gat ttg act aaa gac aac gct tat caa gat tta aat gtc 211
Phe Ser Lys Asp Leu Thr Lys Asp Asn Ala Tyr Gln Asp Leu Asn Val
            25                  30                  35

ata gag cat tta ata tcg tta aaa tat gct cct tta cca tgg aag gaa 259
Ile Glu His Leu Ile Ser Leu Lys Tyr Ala Pro Leu Pro Trp Lys Glu
        40                  45                  50

cta tta ttt ggt tgg gat tta tct cag caa aca cag caa gct cgc ttg 307
Leu Leu Phe Gly Trp Asp Leu Ser Gln Gln Thr Gln Gln Ala Arg Leu
    55                  60                  65

caa ctg gtc tta gaa gaa aaa cca aca acc aac tac tgc cag aag gta 355
Gln Leu Val Leu Glu Glu Lys Pro Thr Thr Asn Tyr Cys Gln Lys Val
70                  75                  80                  85

ctc tct aac tac gtg aga tca tta aac gat tat cat gca ggg att acg 403
Leu Ser Asn Tyr Val Arg Ser Leu Asn Asp Tyr His Ala Gly Ile Thr
                90                  95                  100

ttt tat cgt act gaa agt gcg tat atc cct tac gta ttg aag tta agt 451
Phe Tyr Arg Thr Glu Ser Ala Tyr Ile Pro Tyr Val Leu Lys Leu Ser
            105                 110                 115

gaa gat ggt cat gtc ttt gta gtc gac gta cag act agc caa ggg gat 499
Glu Asp Gly His Val Phe Val Val Asp Val Gln Thr Ser Gln Gly Asp
        120                 125                 130

att tac tta ggg gat gaa atc ctt gaa gta gat gga atg ggg att cgt 547
Ile Tyr Leu Gly Asp Glu Ile Leu Glu Val Asp Gly Met Gly Ile Arg
    135                 140                 145

gag gct atc gaa agc ctt cgc ttt gga cga ggg agt gcc aca gac tat 595
Glu Ala Ile Glu Ser Leu Arg Phe Gly Arg Gly Ser Ala Thr Asp Tyr
150                 155                 160                 165

tct gct gca gtt cgt tcc ttg aca tcg cgt tcc gcc gct ttt gga gat 643
Ser Ala Ala Val Arg Ser Leu Thr Ser Arg Ser Ala Ala Phe Gly Asp
                170                 175                 180

gcg gtt cct tca gga att gcc atg ttg aaa ctt cgc cga ccc agt ggt 691
Ala Val Pro Ser Gly Ile Ala Met Leu Lys Leu Arg Arg Pro Ser Gly
            185                 190                 195

ttg atc cgt tcg aca ccg gtc cgt tgg cgt tat act cca gag cat atc 739
Leu Ile Arg Ser Thr Pro Val Arg Trp Arg Tyr Thr Pro Glu His Ile
        200                 205                 210

gga gat ttt tct tta gtt gct cct ttg att cct gaa cat aaa cct caa 787
Gly Asp Phe Ser Leu Val Ala Pro Leu Ile Pro Glu His Lys Pro Gln
    215                 220                 225

tta cct aca caa agt tgt gtg cta ttc cgt tcc ggg gta aat tca cag 835
Leu Pro Thr Gln Ser Cys Val Leu Phe Arg Ser Gly Val Asn Ser Gln
230                 235                 240                 245

tct tct agt agc tct tta ttc agt tcc tac atg gtg cct tat ttc tgg 883
Ser Ser Ser Ser Ser Leu Phe Ser Ser Tyr Met Val Pro Tyr Phe Trp
                250                 255                 260

gaa gaa ttg cgg gtt caa aat aag cag cgt ttt gac agt aat cac cat     931
Glu Glu Leu Arg Val Gln Asn Lys Gln Arg Phe Asp Ser Asn His His
            265                 270                 275

ata ggg agc cgt aat gga ttt tta cct acg ttt ggt cct att ctt tgg     979
Ile Gly Ser Arg Asn Gly Phe Leu Pro Thr Phe Gly Pro Ile Leu Trp
        280                 285                 290

gaa caa gac aag ggg ccc tat cgt tcc tat atc ttt aaa gca aaa gat     1027
Glu Gln Asp Lys Gly Pro Tyr Arg Ser Tyr Ile Phe Lys Ala Lys Asp
    295                 300                 305

tct cag ggc aat ccc cat cgc ata gga ttt tta aga att tct tct tat     1075
Ser Gln Gly Asn Pro His Arg Ile Gly Phe Leu Arg Ile Ser Ser Tyr
310                 315                 320                 325

gtt tgg act gat tta gaa gga ctt gaa gag gat cat aag gat agt cct     1123
Val Trp Thr Asp Leu Glu Gly Leu Glu Glu Asp His Lys Asp Ser Pro
                330                 335                 340

tgg gag ctc ttt gga gag atc atc gat cat ttg gaa aaa gag act gat     1171
Trp Glu Leu Phe Gly Glu Ile Ile Asp His Leu Glu Lys Glu Thr Asp
            345                 350                 355

gct ttg att att gat cag acc cat aat cct gga ggc agt gtt ttc tat     1219
Ala Leu Ile Ile Asp Gln Thr His Asn Pro Gly Gly Ser Val Phe Tyr
        360                 365                 370

ctc tat tcg tta cta tct atg tta aca gat cat cct tta gat act cct     1267
Leu Tyr Ser Leu Leu Ser Met Leu Thr Asp His Pro Leu Asp Thr Pro
    375                 380                 385

aaa cat aga atg att ttc act cag gat gaa gtc agc tcg gct ttg cac     1315
Lys His Arg Met Ile Phe Thr Gln Asp Glu Val Ser Ser Ala Leu His
390                 395                 400                 405

tgg caa gat cta cta gaa gat gtc ttc aca gat gag cag gca gtt gcc     1363
Trp Gln Asp Leu Leu Glu Asp Val Phe Thr Asp Glu Gln Ala Val Ala
                410                 415                 420

gtg cta ggg gaa act atg gaa gga tat tgc atg gat atg cat gct gta     1411
Val Leu Gly Glu Thr Met Glu Gly Tyr Cys Met Asp Met His Ala Val
            425                 430                 435

gcc tct ctt caa aac ttc tct cag agt gtc ctt tct tcc tgg gtt tca     1459
Ala Ser Leu Gln Asn Phe Ser Gln Ser Val Leu Ser Ser Trp Val Ser
        440                 445                 450

ggt gat att aac ctt tca aaa cct atg cct ttg cta gga ttt gca cag     1507
Gly Asp Ile Asn Leu Ser Lys Pro Met Pro Leu Leu Gly Phe Ala Gln
    455                 460                 465

gtt cga cct cat cct aaa cat caa tat act aaa cct ttg ttt atg ttg     1555
Val Arg Pro His Pro Lys His Gln Tyr Thr Lys Pro Leu Phe Met Leu
470                 475                 480                 485

ata gac gag gat gac ttc tct tgt gga gat tta gcg cct gca att ttg     1603
Ile Asp Glu Asp Asp Phe Ser Cys Gly Asp Leu Ala Pro Ala Ile Leu
                490                 495                 500

aag gat aat ggc cgc gct act ctc att gga aag cca aca gca gga gct     1651
Lys Asp Asn Gly Arg Ala Thr Leu Ile Gly Lys Pro Thr Ala Gly Ala
            505                 510                 515

gga ggt ttt gta ttc caa gtc act ttc cct aac cgt tct gga att aaa     1699
Gly Gly Phe Val Phe Gln Val Thr Phe Pro Asn Arg Ser Gly Ile Lys
        520                 525                 530

ggt ctt tct tta aca gga tct tta gct gtt agg aaa gat ggt gag ttt 1747
Gly Leu Ser Leu Thr Gly Ser Leu Ala Val Arg Lys Asp Gly Glu Phe
    535                 540                 545

att gaa aac tta gga gtg gct cct cat att gat tta gga ttt acc tcc 1795
Ile Glu Asn Leu Gly Val Ala Pro His Ile Asp Leu Gly Phe Thr Ser
550                 555                 560                 565

agg gat ttg caa act tcc agg ttt act gat tac gtt gag gca gtg aaa 1843
Arg Asp Leu Gln Thr Ser Arg Phe Thr Asp Tyr Val Glu Ala Val Lys
                570                 575                 580

act ata gtt tta act tct ttg tct gag aac gct aag aag agt gaa gag 1891
Thr Ile Val Leu Thr Ser Leu Ser Glu Asn Ala Lys Lys Ser Glu Glu
            585                 590                 595

cag act tct ccg caa gag acg cct gaa gtt att cga gtc tct tat ccc 1939
Gln Thr Ser Pro Gln Glu Thr Pro Glu Val Ile Arg Val Ser Tyr Pro
        600                 605                 610

aca acg act tct gct tcg taaacgggac gtaatagaat aatttttatt 1987
Thr Thr Thr Ser Ala Ser
    615

attgctttaa tatgcgcgct tccaatataa gcattgtgaa gcgcgtttca tatgtctttt 2047

atctttaggt aat 2060

<210> 8
<211> 619
<212> PRT
<213> Chlamydia pneumoniae

<220>
<221> SITE
<222> (328)...(343)
<223> B-cell epitope

<220>
<221> SITE
<222> (589)...(606)
<223> B-cell epitope

<220>
<221> SITE
<222> (135)...(144)
<223> T-cell epitope

<220>
<221> SITE
<222> (373)...(382)
<223> T-cell epitope

<400> 8
Met Lys Lys Gly Lys Leu Gly Ala Ile Val Phe Gly Leu Leu Phe Thr
1               5                   10                  15

Ser Ser Val Ala Gly Phe Ser Lys Asp Leu Thr Lys Asp Asn Ala Tyr
            20                  25                  30

Gln Asp Leu Asn Val Ile Glu His Leu Ile Ser Leu Lys Tyr Ala Pro
        35                  40                  45

Leu Pro Trp Lys Glu Leu Leu Phe Gly Trp Asp Leu Ser Gln Gln Thr
    50                  55                  60

Gln Gln Ala Arg Leu Gln Leu Val Leu Glu Glu Lys Pro Thr Thr Asn

65                  70                  75                  80

Tyr Cys Gln Lys Val Leu Ser Asn Tyr Val Arg Ser Leu Asn Asp Tyr
                85                  90                  95

His Ala Gly Ile Thr Phe Tyr Arg Thr Glu Ser Ala Tyr Ile Pro Tyr
            100                 105                 110

Val Leu Lys Leu Ser Glu Asp Gly His Val Phe Val Val Asp Val Gln
        115                 120                 125

Thr Ser Gln Gly Asp Ile Tyr Leu Gly Asp Glu Ile Leu Glu Val Asp
    130                 135                 140

Gly Met Gly Ile Arg Glu Ala Ile Glu Ser Leu Arg Phe Gly Arg Gly
145                 150                 155                 160

Ser Ala Thr Asp Tyr Ser Ala Ala Val Arg Ser Leu Thr Ser Arg Ser
                165                 170                 175

Ala Ala Phe Gly Asp Ala Val Pro Ser Gly Ile Ala Met Leu Lys Leu
            180                 185                 190

Arg Arg Pro Ser Gly Leu Ile Arg Ser Thr Pro Val Arg Trp Arg Tyr
        195                 200                 205

Thr Pro Glu His Ile Gly Asp Phe Ser Leu Val Ala Pro Leu Ile Pro
    210                 215                 220

Glu His Lys Pro Gln Leu Pro Thr Gln Ser Cys Val Leu Phe Arg Ser
225                 230                 235                 240

Gly Val Asn Ser Gln Ser Ser Ser Ser Ser Leu Phe Ser Ser Tyr Met
                245                 250                 255

Val Pro Tyr Phe Trp Glu Glu Leu Arg Val Gln Asn Lys Gln Arg Phe
            260                 265                 270

Asp Ser Asn His His Ile Gly Ser Arg Asn Gly Phe Leu Pro Thr Phe
        275                 280                 285

Gly Pro Ile Leu Trp Glu Gln Asp Lys Gly Pro Tyr Arg Ser Tyr Ile
    290                 295                 300

Phe Lys Ala Lys Asp Ser Gln Gly Asn Pro His Arg Ile Gly Phe Leu
305                 310                 315                 320

Arg Ile Ser Ser Tyr Val Trp Thr Asp Leu Glu Gly Leu Glu Glu Asp
                325                 330                 335

His Lys Asp Ser Pro Trp Glu Leu Phe Gly Glu Ile Ile Asp His Leu
            340                 345                 350

Glu Lys Glu Thr Asp Ala Leu Ile Ile Asp Gln Thr His Asn Pro Gly
        355                 360                 365

Gly Ser Val Phe Tyr Leu Tyr Ser Leu Leu Ser Met Leu Thr Asp His
    370                 375                 380

Pro Leu Asp Thr Pro Lys His Arg Met Ile Phe Thr Gln Asp Glu Val
385                 390                 395                 400

Ser Ser Ala Leu His Trp Gln Asp Leu Leu Glu Asp Val Phe Thr Asp
                405                 410                 415

Glu Gln Ala Val Ala Val Leu Gly Glu Thr Met Glu Gly Tyr Cys Met
            420                 425                 430

Asp Met His Ala Val Ala Ser Leu Gln Asn Phe Ser Gln Ser Val Leu
        435                 440                 445

Ser Ser Trp Val Ser Gly Asp Ile Asn Leu Ser Lys Pro Met Pro Leu
    450                 455                 460

Leu Gly Phe Ala Gln Val Arg Pro His Pro Lys His Gln Tyr Thr Lys
465                 470                 475                 480

Pro Leu Phe Met Leu Ile Asp Glu Asp Asp Phe Ser Cys Gly Asp Leu
                485                 490                 495

Ala Pro Ala Ile Leu Lys Asp Asn Gly Arg Ala Thr Leu Ile Gly Lys
            500                 505                 510

Pro Thr Ala Gly Ala Gly Gly Phe Val Phe Gln Val Thr Phe Pro Asn
        515                 520                 525

Arg Ser Gly Ile Lys Gly Leu Ser Leu Thr Gly Ser Leu Ala Val Arg
    530                 535                 540

Lys Asp Gly Glu Phe Ile Glu Asn Leu Gly Val Ala Pro His Ile Asp
545                 550                 555                 560

Leu Gly Phe Thr Ser Arg Asp Leu Gln Thr Ser Arg Phe Thr Asp Tyr
                565                 570                 575

Val Glu Ala Val Lys Thr Ile Val Leu Thr Ser Leu Ser Glu Asn Ala
            580                 585                 590

Lys Lys Ser Glu Glu Gln Thr Ser Pro Gln Glu Thr Pro Glu Val Ile
        595                 600                 605

Arg Val Ser Tyr Pro Thr Thr Thr Ser Ala Ser
    610                 615


<210> 9
<211> 1133
<212> DNA
<213> Chlamydia pneumoniae

<220>
<221> CDS
<222> (101)..(1030)

<400> 9
gacgtaatag aataattttt attattgctt taatatgcgc gcttccaata taagcattgt 60

gaagcgcgtt tcatatgtct tttatcttta ggtaattatt atg aga aaa ctt att    115
                                            Met Arg Lys Leu Ile
                                            1               5

tta tgc aat cct aga gga ttt tgc tct gga gtt gtg cgc gct att caa    163
Leu Cys Asn Pro Arg Gly Phe Cys Ser Gly Val Val Arg Ala Ile Gln
                10                  15                  20

gtt gta gag gtt gct tta gaa aag tgg gga gct cct atc tat gta aaa    211
Val Val Glu Val Ala Leu Glu Lys Trp Gly Ala Pro Ile Tyr Val Lys
            25                  30                  35

cat gag att gtt cac aat cgc cat gtt gtt aat gct tta cga gcc aag    259
His Glu Ile Val His Asn Arg His Val Val Asn Ala Leu Arg Ala Lys
        40                  45                  50

gga gcg atc ttt gtt gaa gaa ctt gtt gat gtt cct gaa ggt gag aga    307
Gly Ala Ile Phe Val Glu Glu Leu Val Asp Val Pro Glu Gly Glu Arg
    55                  60                  65

gtc att tat tca gct cat gga att cct cct tca gtt aga gct gaa gca     355
Val Ile Tyr Ser Ala His Gly Ile Pro Pro Ser Val Arg Ala Glu Ala
70                  75                  80                  85

aaa gcc cgt aag ctt att gat att gat gct acc tgt ggt ttg gtt act     403
Lys Ala Arg Lys Leu Ile Asp Ile Asp Ala Thr Cys Gly Leu Val Thr
                90                  95                  100

aag gtg cat tct gct gcg aag tta tac gca agt aaa gga tac aaa atc     451
Lys Val His Ser Ala Ala Lys Leu Tyr Ala Ser Lys Gly Tyr Lys Ile
            105                 110                 115

ata ctg atc ggc cat aag aag cac gtt gag gtg att ggt att gtt gga     499
Ile Leu Ile Gly His Lys Lys His Val Glu Val Ile Gly Ile Val Gly
        120                 125                 130

gaa gtt cct gaa cac att act gtt gtc gag aag gtt gct gac gtc gag     547
Glu Val Pro Glu His Ile Thr Val Val Glu Lys Val Ala Asp Val Glu
    135                 140                 145

gcc tta cct ttt agt tct gat aca cct tta ttt tat att act caa acg     595
Ala Leu Pro Phe Ser Ser Asp Thr Pro Leu Phe Tyr Ile Thr Gln Thr
150                 155                 160                 165

acg ttg agt ttg gat gat gtt cag gag atc tca tcg gct ttg cta aag     643
Thr Leu Ser Leu Asp Asp Val Gln Glu Ile Ser Ser Ala Leu Leu Lys
                170                 175                 180

cga tat ccc tct atc att act ctg cct agt tct tcg att tgt tat gca     691
Arg Tyr Pro Ser Ile Ile Thr Leu Pro Ser Ser Ser Ile Cys Tyr Ala
            185                 190                 195

acc acg aac cgt caa aaa gca ttg cgt tct gtt tta tct cgc gtg aat     739
Thr Thr Asn Arg Gln Lys Ala Leu Arg Ser Val Leu Ser Arg Val Asn
        200                 205                 210

tac gtc tat gtg gtt gga gat gtc aac agc tcg aat tcc aat cgt ctt     787
Tyr Val Tyr Val Val Gly Asp Val Asn Ser Ser Asn Ser Asn Arg Leu
    215                 220                 225

cgc gaa gtg gct ttg aga agg gga gtt ccc gct gat ttg atc aac aat     835
Arg Glu Val Ala Leu Arg Arg Gly Val Pro Ala Asp Leu Ile Asn Asn
230                 235                 240                 245

ccc gag gat att gat acg aac atc gta aat cat tct gga gat ata gca     883
Pro Glu Asp Ile Asp Thr Asn Ile Val Asn His Ser Gly Asp Ile Ala
                250                 255                 260

atg act gca gga gcc tca act ccc gaa gac gta gtt caa gct tgc att     931
Met Thr Ala Gly Ala Ser Thr Pro Glu Asp Val Val Gln Ala Cys Ile
            265                 270                 275

cga aag cta tca tca ctt atc cct ggt tta caa gtg gaa aat gat ata     979
Arg Lys Leu Ser Ser Leu Ile Pro Gly Leu Gln Val Glu Asn Asp Ile
        280                 285                 290

ttt gct gta gag gat gtc gta ttt caa tta cca aaa gaa ctc cgt tgt     1027
Phe Ala Val Glu Asp Val Val Phe Gln Leu Pro Lys Glu Leu Arg Cys
    295                 300                 305

tct taggtcttta ggcttacttg ccaagttttt ctcgagattg ctttatagag          1080
Ser
310

tcttcttctc gttcagagag ggtatttacc tttttagttc tctgtatttg aaa          1133

<210> 10
<211> 310
<212> PRT
<213> Chlamydia pneumoniae

<220>
<221> SITE
<222> (198)...(205)
<223> B-cell epitope

<220>
<221> SITE
<222> (221)...(231)
<223> B-cell epitope

<220>
<221> SITE
<222> (207)...(216)
<223> T-cell epitope

<220>
<221> SITE
<222> (279)...(288)
<223> T-cell epitope

<220>
<221> SITE
<222> (118)...(127)
<223> T-cell epitope

<400> 10
Met Arg Lys Leu Ile Leu Cys Asn Pro Arg Gly Phe Cys Ser Gly Val
1               5                   10                  15

Val Arg Ala Ile Gln Val Val Glu Val Ala Leu Glu Lys Trp Gly Ala
            20                  25                  30

Pro Ile Tyr Val Lys His Glu Ile Val His Asn Arg His Val Val Asn
        35                  40                  45

Ala Leu Arg Ala Lys Gly Ala Ile Phe Val Glu Glu Leu Val Asp Val
    50                  55                  60

Pro Glu Gly Glu Arg Val Ile Tyr Ser Ala His Gly Ile Pro Pro Ser
65                  70                  75                  80

Val Arg Ala Glu Ala Lys Ala Arg Lys Leu Ile Asp Ile Asp Ala Thr
                85                  90                  95

Cys Gly Leu Val Thr Lys Val His Ser Ala Ala Lys Leu Tyr Ala Ser
            100                 105                 110

Lys Gly Tyr Lys Ile Ile Leu Ile Gly His Lys Lys His Val Glu Val
        115                 120                 125

Ile Gly Ile Val Gly Glu Val Pro Glu His Ile Thr Val Val Glu Lys
    130                 135                 140

Val Ala Asp Val Glu Ala Leu Pro Phe Ser Ser Asp Thr Pro Leu Phe
145                 150                 155                 160

Tyr Ile Thr Gln Thr Thr Leu Ser Leu Asp Asp Val Gln Glu Ile Ser
                165                 170                 175

Ser Ala Leu Leu Lys Arg Tyr Pro Ser Ile Ile Thr Leu Pro Ser Ser
            180                 185                 190

Ser Ile Cys Tyr Ala Thr Thr Asn Arg Gln Lys Ala Leu Arg Ser Val

195                 200                 205

Leu Ser Arg Val Asn Tyr Val Tyr Val Val Gly Asp Val Asn Ser Ser
    210                 215                 220

Asn Ser Asn Arg Leu Arg Glu Val Ala Leu Arg Arg Gly Val Pro Ala
225                 230                 235                 240

Asp Leu Ile Asn Asn Pro Glu Asp Ile Asp Thr Asn Ile Val Asn His
                245                 250                 255

Ser Gly Asp Ile Ala Met Thr Ala Gly Ala Ser Thr Pro Glu Asp Val
            260                 265                 270

Val Gln Ala Cys Ile Arg Lys Leu Ser Ser Leu Ile Pro Gly Leu Gln
        275                 280                 285

Val Glu Asn Asp Ile Phe Ala Val Glu Asp Val Val Phe Gln Leu Pro
    290                 295                 300

Lys Glu Leu Arg Cys Ser
305                 310

<210> 11
<211> 1466
<212> DNA
<213> Chlamydia pneumoniae

<220>
<221> CDS
<222> (101)..(1363)

<400> 11
catgggagcc gaggaagcca tctcctacgg acttattgat aaggtggtaa cttctgcgaa 60

agaaactaat aaggatacaa gtagcactta gagagaacat atg aat aaa aaa aat    115
                                           Met Asn Lys Lys Asn
                                           1               5

cta act att tgt tca ttt tgc ggt cgg tct gaa aaa gat gta gag aaa    163
Leu Thr Ile Cys Ser Phe Cys Gly Arg Ser Glu Lys Asp Val Glu Lys
                10                  15                  20

ctg att gct ggg cct tcg gta tac att tgt gac tac tgc atc aaa tta    211
Leu Ile Ala Gly Pro Ser Val Tyr Ile Cys Asp Tyr Cys Ile Lys Leu
            25                  30                  35

tgc tct gga att tta gat aag aaa ccc tcc tct aca ata tcc tca gct    259
Cys Ser Gly Ile Leu Asp Lys Lys Pro Ser Ser Thr Ile Ser Ser Ala
        40                  45                  50

cca gtt tct gaa aca cct tca cag cct tct gat ctc agg gtg ctt acc    307
Pro Val Ser Glu Thr Pro Ser Gln Pro Ser Asp Leu Arg Val Leu Thr
    55                  60                  65

cct aag gaa atc aaa aag cat att gat gaa tat gtc att ggt cag gaa    355
Pro Lys Glu Ile Lys Lys His Ile Asp Glu Tyr Val Ile Gly Gln Glu
70                  75                  80                  85

aga gct aaa aag aca atc gct gtt gct gtt tat aat cac tat aaa cgt    403
Arg Ala Lys Lys Thr Ile Ala Val Ala Val Tyr Asn His Tyr Lys Arg
                90                  95                  100

ata cgt gct cta cta cat aac aaa cag gta agc tac ggg aaa tct aac    451
Ile Arg Ala Leu Leu His Asn Lys Gln Val Ser Tyr Gly Lys Ser Asn
            105                 110                 115

gtg ctt ctc cta ggc cct aca gga tct gga aaa aca tta att gca aaa    499

Val Leu Leu Leu Gly Pro Thr Gly Ser Gly Lys Thr Leu Ile Ala Lys
        120                 125                 130

aca ttg gca aaa att tta gat gtt ccc ttc acc ata gcc gac gca acg     547
Thr Leu Ala Lys Ile Leu Asp Val Pro Phe Thr Ile Ala Asp Ala Thr
    135                 140                 145

acc cta acg gaa gca ggt tat gtc ggt gaa gat gta gag aac att gtc     595
Thr Leu Thr Glu Ala Gly Tyr Val Gly Glu Asp Val Glu Asn Ile Val
150                 155                 160                 165

tta cgt tta tta caa gct gct gat tac gat gtc gcc cgt gca gaa cga     643
Leu Arg Leu Leu Gln Ala Ala Asp Tyr Asp Val Ala Arg Ala Glu Arg
                170                 175                 180

ggc att atc tat atc gat gaa atc gat aaa att gga agg aca aca gca     691
Gly Ile Ile Tyr Ile Asp Glu Ile Asp Lys Ile Gly Arg Thr Thr Ala
            185                 190                 195

aac gtc tcc att act aga gat gtt tct ggc gaa ggg gtt caa caa gca     739
Asn Val Ser Ile Thr Arg Asp Val Ser Gly Glu Gly Val Gln Gln Ala
        200                 205                 210

ttg tta aaa atc gtt gaa gga acc aca gca aac gtt cct cct aaa gga     787
Leu Leu Lys Ile Val Glu Gly Thr Thr Ala Asn Val Pro Pro Lys Gly
    215                 220                 225

gga cgt aag cat cct aac caa gag tat atc cga gtc aat acg gaa aat     835
Gly Arg Lys His Pro Asn Gln Glu Tyr Ile Arg Val Asn Thr Glu Asn
230                 235                 240                 245

atc tta ttt atc gta ggc gga gcc ttc gtc aac cta gat aag att atc     883
Ile Leu Phe Ile Val Gly Gly Ala Phe Val Asn Leu Asp Lys Ile Ile
                250                 255                 260

gca aag cga ttg ggg aaa act acc ata ggg ttt tct gat gat caa gca     931
Ala Lys Arg Leu Gly Lys Thr Thr Ile Gly Phe Ser Asp Asp Gln Ala
            265                 270                 275

gac ctc tct caa aaa acc aga gac cat cta ctt gct aaa gtt gaa acc     979
Asp Leu Ser Gln Lys Thr Arg Asp His Leu Leu Ala Lys Val Glu Thr
        280                 285                 290

gaa gac ctg att gcc ttc gga atg atc cct gaa ttt gtc gga aga ttc     1027
Glu Asp Leu Ile Ala Phe Gly Met Ile Pro Glu Phe Val Gly Arg Phe
    295                 300                 305

aac tgc att gta aac tgt gaa gag ctt tct ttg gat gag ctt gta gcc     1075
Asn Cys Ile Val Asn Cys Glu Glu Leu Ser Leu Asp Glu Leu Val Ala
310                 315                 320                 325

atc ctt aca gaa cct aca aat gcg att gtg aaa caa tat atg gag cta     1123
Ile Leu Thr Glu Pro Thr Asn Ala Ile Val Lys Gln Tyr Met Glu Leu
                330                 335                 340

ttc gca gaa gaa aac gtc aag tta gtc ttc aaa aaa gaa gcc cta tat     1171
Phe Ala Glu Glu Asn Val Lys Leu Val Phe Lys Lys Glu Ala Leu Tyr
            345                 350                 355

gct ata gca aaa aaa gcc aag caa gca aaa act gga gct cgt gct cta     1219
Ala Ile Ala Lys Lys Ala Lys Gln Ala Lys Thr Gly Ala Arg Ala Leu
        360                 365                 370

ggg atg atc cta gaa aat ctc ctt aga gac ctt atg ttt gaa att cct     1267
Gly Met Ile Leu Glu Asn Leu Leu Arg Asp Leu Met Phe Glu Ile Pro
    375                 380                 385

tca gat cct aca gta gaa gct att cat atc caa gaa gac act atc gca     1315

Ser Asp Pro Thr Val Glu Ala Ile His Ile Gln Glu Asp Thr Ile Ala
390                 395                 400                 405

gaa aat aaa gcg cca ata att atc aga agg acc cca gaa gct atc gct     1363
Glu Asn Lys Ala Pro Ile Ile Ile Arg Arg Thr Pro Glu Ala Ile Ala
                410                 415                 420

tagctctttt tagttcctat tttaggggtg tcatgacaac aattgccata gaagctgcaa 1423

aaaaagttct tatcaaacta cgtaatgcag gatatcaggc ata                  1466

<210> 12
<211> 421
<212> PRT
<213> Chlamydia pneumoniae

<220>
<221> SITE
<222> (226)...(239)
<223> B-cell epitope

<220>
<221> SITE
<222> (273)...(286)
<223> B-cell epitope

<220>
<221> SITE
<222> (137)...(146)
<223> T-cell epitope

<220>
<221> SITE
<222> (168)...(177)
<223> T-cell epitope

<400> 12
Met Asn Lys Lys Asn Leu Thr Ile Cys Ser Phe Cys Gly Arg Ser Glu
  1               5                  10                  15

Lys Asp Val Glu Lys Leu Ile Ala Gly Pro Ser Val Tyr Ile Cys Asp
             20                  25                  30

Tyr Cys Ile Lys Leu Cys Ser Gly Ile Leu Asp Lys Lys Pro Ser Ser
         35                  40                  45

Thr Ile Ser Ser Ala Pro Val Ser Glu Thr Pro Ser Gln Pro Ser Asp
     50                  55                  60

Leu Arg Val Leu Thr Pro Lys Glu Ile Lys Lys His Ile Asp Glu Tyr
 65                  70                  75                  80

Val Ile Gly Gln Glu Arg Ala Lys Lys Thr Ile Ala Val Ala Val Tyr
                 85                  90                  95

Asn His Tyr Lys Arg Ile Arg Ala Leu Leu His Asn Lys Gln Val Ser
            100                 105                 110

Tyr Gly Lys Ser Asn Val Leu Leu Leu Gly Pro Thr Gly Ser Gly Lys
        115                 120                 125

Thr Leu Ile Ala Lys Thr Leu Ala Lys Ile Leu Asp Val Pro Phe Thr
    130                 135                 140

Ile Ala Asp Ala Thr Thr Leu Thr Glu Ala Gly Tyr Val Gly Glu Asp
145                 150                 155                 160

Val Glu Asn Ile Val Leu Arg Leu Leu Gln Ala Ala Asp Tyr Asp Val
                165                 170                 175

Ala Arg Ala Glu Arg Gly Ile Ile Tyr Ile Asp Glu Ile Asp Lys Ile
            180                 185                 190

Gly Arg Thr Thr Ala Asn Val Ser Ile Thr Arg Asp Val Ser Gly Glu
        195                 200                 205

Gly Val Gln Gln Ala Leu Leu Lys Ile Val Glu Gly Thr Thr Ala Asn
    210                 215                 220

Val Pro Pro Lys Gly Gly Arg Lys His Pro Asn Gln Glu Tyr Ile Arg
225                 230                 235                 240

Val Asn Thr Glu Asn Ile Leu Phe Ile Val Gly Gly Ala Phe Val Asn
                245                 250                 255

Leu Asp Lys Ile Ile Ala Lys Arg Leu Gly Lys Thr Thr Ile Gly Phe
            260                 265                 270

Ser Asp Asp Gln Ala Asp Leu Ser Gln Lys Thr Arg Asp His Leu Leu
        275                 280                 285

Ala Lys Val Glu Thr Glu Asp Leu Ile Ala Phe Gly Met Ile Pro Glu
    290                 295                 300

Phe Val Gly Arg Phe Asn Cys Ile Val Asn Cys Glu Glu Leu Ser Leu
305                 310                 315                 320

Asp Glu Leu Val Ala Ile Leu Thr Glu Pro Thr Asn Ala Ile Val Lys
                325                 330                 335

Gln Tyr Met Glu Leu Phe Ala Glu Glu Asn Val Lys Leu Val Phe Lys
            340                 345                 350

Lys Glu Ala Leu Tyr Ala Ile Ala Lys Lys Ala Lys Gln Ala Lys Thr
        355                 360                 365

Gly Ala Arg Ala Leu Gly Met Ile Leu Glu Asn Leu Leu Arg Asp Leu
    370                 375                 380

Met Phe Glu Ile Pro Ser Asp Pro Thr Val Glu Ala Ile His Ile Gln
385                 390                 395                 400

Glu Asp Thr Ile Ala Glu Asn Lys Ala Pro Ile Ile Ile Arg Arg Thr
                405                 410                 415

Pro Glu Ala Ile Ala
            420

<210> 13
<211> 812
<212> DNA
<213> Chlamydia pneumoniae

<220>
<221> CDS
<222> (101)..(709)

<400> 13
tgacgtagac agcctaaaaa gtcttagcta cgttcctagg gtcatttcgt gatcgggaac 60

gtatggacac aactgaaaat tatttgatga ggaaacgcaa atg aca ctg gta ccc    115
                                          Met Thr Leu Val Pro
                                          1               5

tat gtt gtc gag gat acg ggc cgt ggt gaa agg gcc atg gat att tac    163

Tyr Val Val Glu Asp Thr Gly Arg Gly Glu Arg Ala Met Asp Ile Tyr
                10                  15                  20

tcc cgt ctt ctg aaa gat cgt att gta atg atc ggt cag gaa atc acg     211
Ser Arg Leu Leu Lys Asp Arg Ile Val Met Ile Gly Gln Glu Ile Thr
            25                  30                  35

gag ccc ctc gca aac aca gta att gcc cag ctc ctt ttc ctc atg tcc     259
Glu Pro Leu Ala Asn Thr Val Ile Ala Gln Leu Leu Phe Leu Met Ser
        40                  45                  50

gaa gat cct aaa aag gat att caa att ttc atc aat tcc cca ggc ggc     307
Glu Asp Pro Lys Lys Asp Ile Gln Ile Phe Ile Asn Ser Pro Gly Gly
    55                  60                  65

tac atc acc gct gga ctg gca atc tat gat acc att cgc ttt tta ggt     355
Tyr Ile Thr Ala Gly Leu Ala Ile Tyr Asp Thr Ile Arg Phe Leu Gly
70                  75                  80                  85

tgt gat gta aat acc tac tgc atc ggt caa gct gca tcc atg gga gcc     403
Cys Asp Val Asn Thr Tyr Cys Ile Gly Gln Ala Ala Ser Met Gly Ala
                90                  95                  100

ctc tta tta tcc gca gga act aaa gga aag cgt cac gct ctt ccc cat     451
Leu Leu Leu Ser Ala Gly Thr Lys Gly Lys Arg His Ala Leu Pro His
            105                 110                 115

agc cgt atg atg atc cac caa cct tct gga ggc att atc gga aca tcc     499
Ser Arg Met Met Ile His Gln Pro Ser Gly Gly Ile Ile Gly Thr Ser
        120                 125                 130

gca gac atc caa ctc caa gca gct gaa att cta aca cta aaa aaa cac     547
Ala Asp Ile Gln Leu Gln Ala Ala Glu Ile Leu Thr Leu Lys Lys His
    135                 140                 145

ctt gcc aat atc ctc tct gaa tgc aca gga caa cct gta gaa aaa att     595
Leu Ala Asn Ile Leu Ser Glu Cys Thr Gly Gln Pro Val Glu Lys Ile
150                 155                 160                 165

ata gaa gat tct gaa cga gat ttc ttc atg gga gcc gag gaa gcc atc     643
Ile Glu Asp Ser Glu Arg Asp Phe Phe Met Gly Ala Glu Glu Ala Ile
                170                 175                 180

tcc tac gga ctt att gat aag gtg gta act tct gcg aaa gaa act aat     691
Ser Tyr Gly Leu Ile Asp Lys Val Val Thr Ser Ala Lys Glu Thr Asn
            185                 190                 195

aag gat aca agt agc act tagagagaac atatgaataa aaaaaatcta            739
Lys Asp Thr Ser Ser Thr
        200

actatttgtt cattttgcgg tcggtctgaa aaagatgtag agaaactgat tgctgggcct 799

tcggtataca ttt                                                      812

<210> 14
<211> 203
<212> PRT
<213> Chlamydia pneumoniae

<220>
<221> SITE
<222> (107)...(116)
<223> B-cell epitope

<220>
<221> SITE

<222> (193)...(203)
<223> B-cell epitope

<220>
<221> SITE
<222> (76)...(85)
<223> T-cell epitope


<400> 14
Met Thr Leu Val Pro Tyr Val Val Glu Asp Thr Gly Arg Gly Glu Arg
1               5                   10                  15

Ala Met Asp Ile Tyr Ser Arg Leu Leu Lys Asp Arg Ile Val Met Ile
            20                  25                  30

Gly Gln Glu Ile Thr Glu Pro Leu Ala Asn Thr Val Ile Ala Gln Leu
        35                  40                  45

Leu Phe Leu Met Ser Glu Asp Pro Lys Lys Asp Ile Gln Ile Phe Ile
    50                  55                  60

Asn Ser Pro Gly Gly Tyr Ile Thr Ala Gly Leu Ala Ile Tyr Asp Thr
65                  70                  75                  80

Ile Arg Phe Leu Gly Cys Asp Val Asn Thr Tyr Cys Ile Gly Gln Ala
                85                  90                  95

Ala Ser Met Gly Ala Leu Leu Leu Ser Ala Gly Thr Lys Gly Lys Arg
            100                 105                 110

His Ala Leu Pro His Ser Arg Met Met Ile His Gln Pro Ser Gly Gly
        115                 120                 125

Ile Ile Gly Thr Ser Ala Asp Ile Gln Leu Gln Ala Ala Glu Ile Leu
    130                 135                 140

Thr Leu Lys Lys His Leu Ala Asn Ile Leu Ser Glu Cys Thr Gly Gln
145                 150                 155                 160

Pro Val Glu Lys Ile Ile Glu Asp Ser Glu Arg Asp Phe Phe Met Gly
                165                 170                 175

Ala Glu Glu Ala Ile Ser Tyr Gly Leu Ile Asp Lys Val Val Thr Ser
            180                 185                 190

Ala Lys Glu Thr Asn Lys Asp Thr Ser Ser Thr
        195                 200


<210> 15
<211> 2162
<212> DNA
<213> Chlamydia pneumoniae

<220>
<221> CDS
<222> (101)..(2059)

<400> 15
gataaaatag aaagacctga tcatttgatg gaaatagcag ctcttcccga ataccaatat 60

ttggaatatc cctcagaaga aagtatcagt cttttatcct atg agc tac cgt aaa   115
                                          Met Ser Tyr Arg Lys
                                          1               5

cgt tcg act cta att gtt cta gga gtg ttt gct ctt tat gct ctt cta   163
Arg Ser Thr Leu Ile Val Leu Gly Val Phe Ala Leu Tyr Ala Leu Leu

10                  15                  20

gta ttg cgt tat tat aaa att caa att tgt gaa gga gac cac tgg gcc     211
Val Leu Arg Tyr Tyr Lys Ile Gln Ile Cys Glu Gly Asp His Trp Ala
            25                  30                  35

gca gaa gct ctc ggg caa cac gaa ttt tgt gtc cgt gat cct ttt cga     259
Ala Glu Ala Leu Gly Gln His Glu Phe Cys Val Arg Asp Pro Phe Arg
        40                  45                  50

agg ggc acc ttt ttt gct aac acg aca gta cgt aag gga gac aaa gac     307
Arg Gly Thr Phe Phe Ala Asn Thr Thr Val Arg Lys Gly Asp Lys Asp
    55                  60                  65

ctt cag cag cct ttc gct gtc gat att aca aaa ttt cac ctt tgt gca     355
Leu Gln Gln Pro Phe Ala Val Asp Ile Thr Lys Phe His Leu Cys Ala
70                  75                  80                  85

gat cct tta gct att ccc gaa tgt cat cgt gat gag atc atc caa ggg     403
Asp Pro Leu Ala Ile Pro Glu Cys His Arg Asp Glu Ile Ile Gln Gly
                90                  95                  100

att ctc caa ttt att gag ggg cag acc tac gac gac ctc tcc cta aag     451
Ile Leu Gln Phe Ile Glu Gly Gln Thr Tyr Asp Asp Leu Ser Leu Lys
            105                 110                 115

tta gat aag aaa tct cgg tat tgt aag ctg tat cct tta tta gat gtt     499
Leu Asp Lys Lys Ser Arg Tyr Cys Lys Leu Tyr Pro Leu Leu Asp Val
        120                 125                 130

tct gtc cat gac cgg cta tcc ctt tgg tgg aaa gga tat gca aca aag     547
Ser Val His Asp Arg Leu Ser Leu Trp Trp Lys Gly Tyr Ala Thr Lys
    135                 140                 145

cat cgc tta cca aca aac gcc cta ttt ttt att acg gac tac caa cgc     595
His Arg Leu Pro Thr Asn Ala Leu Phe Phe Ile Thr Asp Tyr Gln Arg
150                 155                 160                 165

tcg tat cct ttt ggg aag ctc ctt gga caa gtt ctc cat acc tta aga     643
Ser Tyr Pro Phe Gly Lys Leu Leu Gly Gln Val Leu His Thr Leu Arg
                170                 175                 180

gaa att aag gat gag aaa aca gga aaa gcc ttt ccc aca ggc ggg atg     691
Glu Ile Lys Asp Glu Lys Thr Gly Lys Ala Phe Pro Thr Gly Gly Met
            185                 190                 195

gag gcg tac ttt aat cat att ctg gaa ggg gac gtt gga gag aga aag     739
Glu Ala Tyr Phe Asn His Ile Leu Glu Gly Asp Val Gly Glu Arg Lys
        200                 205                 210

ctg ttg cgt tct cct ttg aac cgt tta gat acg aat cgt gtt atc aaa     787
Leu Leu Arg Ser Pro Leu Asn Arg Leu Asp Thr Asn Arg Val Ile Lys
    215                 220                 225

ctg cct aaa gat ggc tct gat atc tac ctt acg atc aat cct gtg atc     835
Leu Pro Lys Asp Gly Ser Asp Ile Tyr Leu Thr Ile Asn Pro Val Ile
230                 235                 240                 245

cag acc att gca gag gaa gaa ctc gaa cgg ggc gtg cta gaa gct aaa     883
Gln Thr Ile Ala Glu Glu Glu Leu Glu Arg Gly Val Leu Glu Ala Lys
                250                 255                 260

gcc cag ggg ggt agg ctc att cta atg aac tcc caa aca gga gag att     931
Ala Gln Gly Gly Arg Leu Ile Leu Met Asn Ser Gln Thr Gly Glu Ile
            265                 270                 275

ctt gca ctg gct caa tat ccg ttt ttc gat ccc aca aat tat aag gaa     979
Leu Ala Leu Ala Gln Tyr Pro Phe Phe Asp Pro Thr Asn Tyr Lys Glu

280                 285                 290

tac ttc aat aac aaa gag cgc atc gaa cat acg aag gta tct ttt gtg     1027
Tyr Phe Asn Asn Lys Glu Arg Ile Glu His Thr Lys Val Ser Phe Val
    295                 300                 305

agc gat gtt ttt gaa ccc ggg tcg atc atg aaa cct ttg act gtg gcg     1075
Ser Asp Val Phe Glu Pro Gly Ser Ile Met Lys Pro Leu Thr Val Ala
310                 315                 320                 325

att gct tta caa gct aac gaa gag gct agc tta aaa tcg cag aaa aag     1123
Ile Ala Leu Gln Ala Asn Glu Glu Ala Ser Leu Lys Ser Gln Lys Lys
                330                 335                 340

att ttt gat cct gaa gaa cct atc gat gtg acc agg aca ctc ttc cct     1171
Ile Phe Asp Pro Glu Glu Pro Ile Asp Val Thr Arg Thr Leu Phe Pro
            345                 350                 355

gga cga aaa gga tct ccg ctt aag gat att tct aga aac tct caa ttg     1219
Gly Arg Lys Gly Ser Pro Leu Lys Asp Ile Ser Arg Asn Ser Gln Leu
        360                 365                 370

aat atg tac atg gct atc cag aaa tct tcg aat gtc tat gta gct cag     1267
Asn Met Tyr Met Ala Ile Gln Lys Ser Ser Asn Val Tyr Val Ala Gln
    375                 380                 385

ctg gct gac cgc atc ata caa tct tta gga gtg gcc tgg tac caa cag     1315
Leu Ala Asp Arg Ile Ile Gln Ser Leu Gly Val Ala Trp Tyr Gln Gln
390                 395                 400                 405

aag ttg cta gct ctg gga ttt gga aga aaa aca ggg atc gag ctt ccc     1363
Lys Leu Leu Ala Leu Gly Phe Gly Arg Lys Thr Gly Ile Glu Leu Pro
                410                 415                 420

agt gag gcc tct ggt ttg gtg cct tct ccc cat cgt ttc cat att aat     1411
Ser Glu Ala Ser Gly Leu Val Pro Ser Pro His Arg Phe His Ile Asn
            425                 430                 435

ggt tcc ctg gaa tgg tcc tta tct act cca tat tct ttg gct atg gga     1459
Gly Ser Leu Glu Trp Ser Leu Ser Thr Pro Tyr Ser Leu Ala Met Gly
        440                 445                 450

tat aat att ttg gca aca ggg ata caa atg gtt caa gcc tac gct atc     1507
Tyr Asn Ile Leu Ala Thr Gly Ile Gln Met Val Gln Ala Tyr Ala Ile
    455                 460                 465

ctt gca aac gga ggt tat gcc gtc cgg ccc act tta gta aaa aag atc     1555
Leu Ala Asn Gly Gly Tyr Ala Val Arg Pro Thr Leu Val Lys Lys Ile
470                 475                 480                 485

gtc tct gct tca gga gag gaa tat cat ctt cct act aaa gag aag aca     1603
Val Ser Ala Ser Gly Glu Glu Tyr His Leu Pro Thr Lys Glu Lys Thr
                490                 495                 500

cga ctc ttt tca gaa gaa att act aga gaa gtt gtt cgt gcc atg cgt     1651
Arg Leu Phe Ser Glu Glu Ile Thr Arg Glu Val Val Arg Ala Met Arg
            505                 510                 515

ttt aca acg tta ccc gga ggt tcg gga ttt cga gcc tct cct aag cat     1699
Phe Thr Thr Leu Pro Gly Gly Ser Gly Phe Arg Ala Ser Pro Lys His
        520                 525                 530

cac tct agt gct ggg aaa aca gga act aca gaa aag atg att cat gga     1747
His Ser Ser Ala Gly Lys Thr Gly Thr Thr Glu Lys Met Ile His Gly
    535                 540                 545

aaa tat gat aaa cgc cgt cat att gct tct ttt ata ggt ttt act ccc     1795
Lys Tyr Asp Lys Arg Arg His Ile Ala Ser Phe Ile Gly Phe Thr Pro

550                 555                 560                 565

gta gag agc tcg gag gga aat ttc cca cct tta gtg atg ctc gtc tcc     1843
Val Glu Ser Ser Glu Gly Asn Phe Pro Pro Leu Val Met Leu Val Ser
                570                 575                 580

ata gat gat cct gaa tat ggt ttg cga gcc gac ggc acg aaa aat tat     1891
Ile Asp Asp Pro Glu Tyr Gly Leu Arg Ala Asp Gly Thr Lys Asn Tyr
            585                 590                 595

atg ggg ggg cgt tgt gcg gca ccc att ttt tct agg gtt gct gac cgc     1939
Met Gly Gly Arg Cys Ala Ala Pro Ile Phe Ser Arg Val Ala Asp Arg
        600                 605                 610

aca ctc ctc tat tta ggg att ctt cca gac aag aag cta aga aat tgc     1987
Thr Leu Leu Tyr Leu Gly Ile Leu Pro Asp Lys Lys Leu Arg Asn Cys
    615                 620                 625

gac gaa gaa gct gct gca tta aag cgt ctc tat gaa gaa tgg aat cgt     2035
Asp Glu Glu Ala Ala Ala Leu Lys Arg Leu Tyr Glu Glu Trp Asn Arg
630                 635                 640                 645

tct ccg aaa caa ggg gga acg agg tgaggatctc tatttccatc ttgctataga    2089
Ser Pro Lys Gln Gly Gly Thr Arg
                650

cttttaccgt tgagcaaaga ctctctatca gagagcccgt ctcctcttta tcctctatga 2149

gtagtttatg tta                                                      2162

<210> 16
<211> 653
<212> PRT
<213> Chlamydia pneumoniae

<220>
<221> SITE
<222> (287)...(306)
<223> B-cell epitope

<220>
<221> SITE
<222> (637)...(653)
<223> B-cell epitope

<220>
<221> SITE
<222> (40)...(48)
<223> T-cell epitope

<220>
<221> SITE
<222> (456)...(465)
<223> T-cell epitope

<400> 16
Met Ser Tyr Arg Lys Arg Ser Thr Leu Ile Val Leu Gly Val Phe Ala
  1               5                  10                  15

Leu Tyr Ala Leu Leu Val Leu Arg Tyr Tyr Lys Ile Gln Ile Cys Glu
             20                  25                  30

Gly Asp His Trp Ala Ala Glu Ala Leu Gly Gln His Glu Phe Cys Val
         35                  40                  45

Arg Asp Pro Phe Arg Arg Gly Thr Phe Phe Ala Asn Thr Thr Val Arg
     50                  55                  60

Lys Gly Asp Lys Asp Leu Gln Gln Pro Phe Ala Val Asp Ile Thr Lys
65                  70                  75                  80

Phe His Leu Cys Ala Asp Pro Leu Ala Ile Pro Glu Cys His Arg Asp
                85                  90                  95

Glu Ile Ile Gln Gly Ile Leu Gln Phe Ile Glu Gly Gln Thr Tyr Asp
            100                 105                 110

Asp Leu Ser Leu Lys Leu Asp Lys Lys Ser Arg Tyr Cys Lys Leu Tyr
        115                 120                 125

Pro Leu Leu Asp Val Ser Val His Asp Arg Leu Ser Leu Trp Trp Lys
    130                 135                 140

Gly Tyr Ala Thr Lys His Arg Leu Pro Thr Asn Ala Leu Phe Phe Ile
145                 150                 155                 160

Thr Asp Tyr Gln Arg Ser Tyr Pro Phe Gly Lys Leu Leu Gly Gln Val
                165                 170                 175

Leu His Thr Leu Arg Glu Ile Lys Asp Glu Lys Thr Gly Lys Ala Phe
            180                 185                 190

Pro Thr Gly Gly Met Glu Ala Tyr Phe Asn His Ile Leu Glu Gly Asp
        195                 200                 205

Val Gly Glu Arg Lys Leu Leu Arg Ser Pro Leu Asn Arg Leu Asp Thr
    210                 215                 220

Asn Arg Val Ile Lys Leu Pro Lys Asp Gly Ser Asp Ile Tyr Leu Thr
225                 230                 235                 240

Ile Asn Pro Val Ile Gln Thr Ile Ala Glu Glu Glu Leu Glu Arg Gly
                245                 250                 255

Val Leu Glu Ala Lys Ala Gln Gly Gly Arg Leu Ile Leu Met Asn Ser
            260                 265                 270

Gln Thr Gly Glu Ile Leu Ala Leu Ala Gln Tyr Pro Phe Phe Asp Pro
        275                 280                 285

Thr Asn Tyr Lys Glu Tyr Phe Asn Asn Lys Glu Arg Ile Glu His Thr
    290                 295                 300

Lys Val Ser Phe Val Ser Asp Val Phe Glu Pro Gly Ser Ile Met Lys
305                 310                 315                 320

Pro Leu Thr Val Ala Ile Ala Leu Gln Ala Asn Glu Glu Ala Ser Leu
                325                 330                 335

Lys Ser Gln Lys Lys Ile Phe Asp Pro Glu Glu Pro Ile Asp Val Thr
            340                 345                 350

Arg Thr Leu Phe Pro Gly Arg Lys Gly Ser Pro Leu Lys Asp Ile Ser
        355                 360                 365

Arg Asn Ser Gln Leu Asn Met Tyr Met Ala Ile Gln Lys Ser Ser Asn
    370                 375                 380

Val Tyr Val Ala Gln Leu Ala Asp Arg Ile Ile Gln Ser Leu Gly Val
385                 390                 395                 400

Ala Trp Tyr Gln Gln Lys Leu Leu Ala Leu Gly Phe Gly Arg Lys Thr
                405                 410                 415

Gly Ile Glu Leu Pro Ser Glu Ala Ser Gly Leu Val Pro Ser Pro His

420                         425                         430

Arg Phe His Ile Asn Gly Ser Leu Glu Trp Ser Leu Ser Thr Pro Tyr
        435                 440                 445

Ser Leu Ala Met Gly Tyr Asn Ile Leu Ala Thr Gly Ile Gln Met Val
    450                 455                 460

Gln Ala Tyr Ala Ile Leu Ala Asn Gly Gly Tyr Ala Val Arg Pro Thr
465                 470                 475                 480

Leu Val Lys Lys Ile Val Ser Ala Ser Gly Glu Glu Tyr His Leu Pro
                485                 490                 495

Thr Lys Glu Lys Thr Arg Leu Phe Ser Glu Glu Ile Thr Arg Glu Val
            500                 505                 510

Val Arg Ala Met Arg Phe Thr Thr Leu Pro Gly Gly Ser Gly Phe Arg
        515                 520                 525

Ala Ser Pro Lys His His Ser Ser Ala Gly Lys Thr Gly Thr Thr Glu
    530                 535                 540

Lys Met Ile His Gly Lys Tyr Asp Lys Arg Arg His Ile Ala Ser Phe
545                 550                 555                 560

Ile Gly Phe Thr Pro Val Glu Ser Ser Glu Gly Asn Phe Pro Pro Leu
                565                 570                 575

Val Met Leu Val Ser Ile Asp Asp Pro Glu Tyr Gly Leu Arg Ala Asp
            580                 585                 590

Gly Thr Lys Asn Tyr Met Gly Gly Arg Cys Ala Ala Pro Ile Phe Ser
        595                 600                 605

Arg Val Ala Asp Arg Thr Leu Leu Tyr Leu Gly Ile Leu Pro Asp Lys
    610                 615                 620

Lys Leu Arg Asn Cys Asp Glu Glu Ala Ala Ala Leu Lys Arg Leu Tyr
625                 630                 635                 640

Glu Glu Trp Asn Arg Ser Pro Lys Gln Gly Gly Thr Arg
                645                 650

<210> 17
<211> 2738
<212> DNA
<213> Chlamydia pneumoniae

<220>
<221> CDS
<222> (101)..(2635)

<400> 17
gaattttacc aaatttgctg gtttagagcg aagagttgca tcattatttt aaatttcgta 60

tatgcttaag gaaagttcta cccctgtctt ttaggttttt atg ttt gag aag ttc    115
                                            Met Phe Glu Lys Phe
                                            1               5

act aat aga gca aaa caa gtc att aaa ctg gcg aaa aag gag gct cag    163
Thr Asn Arg Ala Lys Gln Val Ile Lys Leu Ala Lys Lys Glu Ala Gln
                10                  15                  20

cgt tta aat cat aac tac ctg ggt act gag cac atc ctg ctt ggt ctt    211
Arg Leu Asn His Asn Tyr Leu Gly Thr Glu His Ile Leu Leu Gly Leu
            25                  30                  35

ctc aaa ctt ggt caa ggg gta gct gtt aat gta tta cgc aac ctc ggt 259
Leu Lys Leu Gly Gln Gly Val Ala Val Asn Val Leu Arg Asn Leu Gly
        40                  45                  50

ata gat ttt gat acg gca cgg caa gag gtg gaa cgc ctg att ggt tat 307
Ile Asp Phe Asp Thr Ala Arg Gln Glu Val Glu Arg Leu Ile Gly Tyr
    55                  60                  65

ggt cca gaa att caa gtc tac gga gac cct gcc ctt aca gga aga gta 355
Gly Pro Glu Ile Gln Val Tyr Gly Asp Pro Ala Leu Thr Gly Arg Val
70                  75                  80                  85

aaa aaa tct ttt gaa tca gca aat gaa gag gcc agc ctt tta gag cac 403
Lys Lys Ser Phe Glu Ser Ala Asn Glu Glu Ala Ser Leu Leu Glu His
                90                  95                  100

aat tat gtc ggg acg gag cat tta ctc tta ggg atc cta cat caa tca 451
Asn Tyr Val Gly Thr Glu His Leu Leu Leu Gly Ile Leu His Gln Ser
            105                 110                 115

gat agt gtc gct ctt cag gta tta gaa aac tta cat atc gat cca aga 499
Asp Ser Val Ala Leu Gln Val Leu Glu Asn Leu His Ile Asp Pro Arg
        120                 125                 130

gag gtt cgt aag gaa att ctt aga gaa tta gag acc ttc aat cta caa 547
Glu Val Arg Lys Glu Ile Leu Arg Glu Leu Glu Thr Phe Asn Leu Gln
    135                 140                 145

ctt cct cct tcg tcg tcg tct tct tcc tca tcc tct cga agc aac cct 595
Leu Pro Pro Ser Ser Ser Ser Ser Ser Ser Ser Ser Arg Ser Asn Pro
150                 155                 160                 165

tca tct tca aaa tct cct tta ggt cat agc tta ggt tct gac aaa aac 643
Ser Ser Ser Lys Ser Pro Leu Gly His Ser Leu Gly Ser Asp Lys Asn
                170                 175                 180

gaa aag ctt tct gct ctg aaa gca tat ggt tat gat tta acg gag atg 691
Glu Lys Leu Ser Ala Leu Lys Ala Tyr Gly Tyr Asp Leu Thr Glu Met
            185                 190                 195

gtc cga gag tct aag ctc gat cct gtc att ggt cgt tct tca gaa gtc 739
Val Arg Glu Ser Lys Leu Asp Pro Val Ile Gly Arg Ser Ser Glu Val
        200                 205                 210

gaa cgg ttg att ttg att ctt tgc cga aga aga aaa aac aat cct gta 787
Glu Arg Leu Ile Leu Ile Leu Cys Arg Arg Arg Lys Asn Asn Pro Val
    215                 220                 225

ctt att gga gaa gct gga gtt ggt aag act gca att gtt gag ggt ctg 835
Leu Ile Gly Glu Ala Gly Val Gly Lys Thr Ala Ile Val Glu Gly Leu
230                 235                 240                 245

gct caa aaa atc att ctg aat gag gtt cct gat gcc tta cgg aaa aag 883
Ala Gln Lys Ile Ile Leu Asn Glu Val Pro Asp Ala Leu Arg Lys Lys
                250                 255                 260

cga ctg att act cta gat cta gca tta atg att gct gga aca aaa tat 931
Arg Leu Ile Thr Leu Asp Leu Ala Leu Met Ile Ala Gly Thr Lys Tyr
            265                 270                 275

cga ggg caa ttt gag gaa cgg atc aaa gct gtc atg gat gaa gtt cgc 979
Arg Gly Gln Phe Glu Glu Arg Ile Lys Ala Val Met Asp Glu Val Arg
        280                 285                 290

aag cat gga aac atc ttg ctc ttc att gac gag ctc cac acg att gta 1027
Lys His Gly Asn Ile Leu Leu Phe Ile Asp Glu Leu His Thr Ile Val
    295                 300                 305

gga gca gga gca gct gaa ggt gct atc gat gct tca aac att tta aaa 1075
Gly Ala Gly Ala Ala Glu Gly Ala Ile Asp Ala Ser Asn Ile Leu Lys
310                 315                 320                 325

cct gcg tta gcg cga ggt gaa att cag tgt att gga gca act acg ata 1123
Pro Ala Leu Ala Arg Gly Glu Ile Gln Cys Ile Gly Ala Thr Thr Ile
                330                 335                 340

gat gag tat cgc aag cac ata gaa aaa gac gca gct tta gaa cgt cgt 1171
Asp Glu Tyr Arg Lys His Ile Glu Lys Asp Ala Ala Leu Glu Arg Arg
            345                 350                 355

ttc caa aaa atc gtg gtt cac cct cct agt gta gat gag act att gag 1219
Phe Gln Lys Ile Val Val His Pro Pro Ser Val Asp Glu Thr Ile Glu
        360                 365                 370

att tta cgt ggc ctc aag aaa aag tat gaa gaa cat cac aat gtc ttc 1267
Ile Leu Arg Gly Leu Lys Lys Lys Tyr Glu Glu His His Asn Val Phe
    375                 380                 385

att act gaa gaa gct tta aaa gca gct gcg act ctt tct gat caa tat 1315
Ile Thr Glu Glu Ala Leu Lys Ala Ala Ala Thr Leu Ser Asp Gln Tyr
390                 395                 400                 405

gtt cat gga cgt ttc ctc cct gat aaa gca ata gat ctt tta gat gaa 1363
Val His Gly Arg Phe Leu Pro Asp Lys Ala Ile Asp Leu Leu Asp Glu
                410                 415                 420

gct ggg gct cgt gtc cgt gtg aat aca atg ggt cag cct aca gat tta 1411
Ala Gly Ala Arg Val Arg Val Asn Thr Met Gly Gln Pro Thr Asp Leu
            425                 430                 435

atg aag cta gag gct gaa atc gaa aat aca aaa ttg gcc aaa gag cag 1459
Met Lys Leu Glu Ala Glu Ile Glu Asn Thr Lys Leu Ala Lys Glu Gln
        440                 445                 450

gcc att gga act caa gaa tac gaa aaa gct gca ggt tta cgt gat gaa 1507
Ala Ile Gly Thr Gln Glu Tyr Glu Lys Ala Ala Gly Leu Arg Asp Glu
    455                 460                 465

gag aaa aaa ctt cgc gaa cgt ctg caa agt atg aaa cag gaa tgg gaa 1555
Glu Lys Lys Leu Arg Glu Arg Leu Gln Ser Met Lys Gln Glu Trp Glu
470                 475                 480                 485

aat cat aaa gaa gag cac caa gtt cct gta gat gaa gaa gca gtc gct 1603
Asn His Lys Glu Glu His Gln Val Pro Val Asp Glu Glu Ala Val Ala
                490                 495                 500

cag gta gtt tct cta caa aca gga att ccc tca gca agg ctc aca gaa 1651
Gln Val Val Ser Leu Gln Thr Gly Ile Pro Ser Ala Arg Leu Thr Glu
            505                 510                 515

gct gaa agt gag aag ctt ctg aag tta gaa gac acg tta aga aga aaa 1699
Ala Glu Ser Glu Lys Leu Leu Lys Leu Glu Asp Thr Leu Arg Arg Lys
        520                 525                 530

gtc att ggt caa aat gat gcc gtt acc agc att tgc cgt gcc atc cga 1747
Val Ile Gly Gln Asn Asp Ala Val Thr Ser Ile Cys Arg Ala Ile Arg
    535                 540                 545

cgt tct cga aca ggg atc aaa gat cct aac cga cct acg ggc tcc ttc 1795
Arg Ser Arg Thr Gly Ile Lys Asp Pro Asn Arg Pro Thr Gly Ser Phe
550                 555                 560                 565

cta ttc ctt ggg cct acc ggt gta ggg aaa agc ctg ctc gcc caa caa 1843
Leu Phe Leu Gly Pro Thr Gly Val Gly Lys Ser Leu Leu Ala Gln Gln
                570                 575                 580

att gct ata gag atg ttc ggt ggt gaa gac gct ctg att cag gta gac 1891
Ile Ala Ile Glu Met Phe Gly Gly Glu Asp Ala Leu Ile Gln Val Asp
            585                 590                 595

atg tca gag tac atg gag aaa ttt gct gct acc aag atg atg gga tca 1939
Met Ser Glu Tyr Met Glu Lys Phe Ala Ala Thr Lys Met Met Gly Ser
        600                 605                 610

cct cca gga tat gta ggt cat gaa gaa ggg ggc cac ctt acg gaa cag 1987
Pro Pro Gly Tyr Val Gly His Glu Glu Gly Gly His Leu Thr Glu Gln
    615                 620                 625

gta cgt cgc cgt cct tac tgc gtt gtt ctc ttt gat gag ata gaa aag 2035
Val Arg Arg Arg Pro Tyr Cys Val Val Leu Phe Asp Glu Ile Glu Lys
630                 635                 640                 645

gca cac cca gac att atg gac ctg atg ttg caa att tta gag caa gga 2083
Ala His Pro Asp Ile Met Asp Leu Met Leu Gln Ile Leu Glu Gln Gly
                650                 655                 660

cgt ctt act gat tct ttt ggt cgc aaa gtg gat ttc cgt cat gcc att 2131
Arg Leu Thr Asp Ser Phe Gly Arg Lys Val Asp Phe Arg His Ala Ile
            665                 670                 675

att atc atg acc tcc aat ttg gga gct gat ctc att cgt aaa agc gga 2179
Ile Ile Met Thr Ser Asn Leu Gly Ala Asp Leu Ile Arg Lys Ser Gly
        680                 685                 690

gaa att ggt ttt ggc ttg aag tcc cat atg gac tat aag gtc atc caa 2227
Glu Ile Gly Phe Gly Leu Lys Ser His Met Asp Tyr Lys Val Ile Gln
    695                 700                 705

gag aaa atc gaa cat gct atg aag aaa cac tta aag cct gag ttc att 2275
Glu Lys Ile Glu His Ala Met Lys Lys His Leu Lys Pro Glu Phe Ile
710                 715                 720                 725

aac cgt ttg gat gaa agt gtg att ttc cgt ccc ctc gag aaa gaa tct 2323
Asn Arg Leu Asp Glu Ser Val Ile Phe Arg Pro Leu Glu Lys Glu Ser
                730                 735                 740

cta tcg gag atc atc cat tta gag atc aac aaa ctg gac tcg aga ctg 2371
Leu Ser Glu Ile Ile His Leu Glu Ile Asn Lys Leu Asp Ser Arg Leu
            745                 750                 755

aaa aac tac caa atg gct ttg aac atc cca gac tct gtg att tcc ttc 2419
Lys Asn Tyr Gln Met Ala Leu Asn Ile Pro Asp Ser Val Ile Ser Phe
        760                 765                 770

cta gta acg aag ggg cat tct cca gaa atg gga gca cgt cct cta cgc 2467
Leu Val Thr Lys Gly His Ser Pro Glu Met Gly Ala Arg Pro Leu Arg
    775                 780                 785

cgt gtc att gag cag tac ctt gaa gat cct cta gcg gag ctc ttg ctt 2515
Arg Val Ile Glu Gln Tyr Leu Glu Asp Pro Leu Ala Glu Leu Leu Leu
790                 795                 800                 805

aaa gag tcc tgc cgt caa gaa gct cgc aag cta cga gca acc ttg gtt 2563
Lys Glu Ser Cys Arg Gln Glu Ala Arg Lys Leu Arg Ala Thr Leu Val
                810                 815                 820

gaa aat cgc gtt gcc ttt gaa agg gaa gaa gag gag cag gaa gct gct 2611
Glu Asn Arg Val Ala Phe Glu Arg Glu Glu Glu Glu Gln Glu Ala Ala
            825                 830                 835

ctc cct agc cct cac ttg gaa tca taggaacgtc gataactcca ctaccaaggc 2665
Leu Pro Ser Pro His Leu Glu Ser
        840                 845

aggtatctcc ttgataaaac gctattgttt gtcctggagt taccgccttg acgggttgtg 2725

aaaatcgcac ctt 2738

<210> 18
<211> 845
<212> PRT
<213> Chlamydia pneumoniae

<220>
<221> SITE
<222> (467)...(492)
<223> B-cell epitope

<220>
<221> SITE
<222> (548)...(563)
<223> B-cell epitope

<220>
<221> SITE
<222> (565)...(574)
<223> T-cell epitope

<220>
<221> SITE
<222> (410)...(418)
<223> T-cell epitope

<400> 18
Met Phe Glu Lys Phe Thr Asn Arg Ala Lys Gln Val Ile Lys Leu Ala
1               5                   10                  15

Lys Lys Glu Ala Gln Arg Leu Asn His Asn Tyr Leu Gly Thr Glu His
            20                  25                  30

Ile Leu Leu Gly Leu Leu Lys Leu Gly Gln Gly Val Ala Val Asn Val
        35                  40                  45

Leu Arg Asn Leu Gly Ile Asp Phe Asp Thr Ala Arg Gln Glu Val Glu
    50                  55                  60

Arg Leu Ile Gly Tyr Gly Pro Glu Ile Gln Val Tyr Gly Asp Pro Ala
65                  70                  75                  80

Leu Thr Gly Arg Val Lys Lys Ser Phe Glu Ser Ala Asn Glu Glu Ala
                85                  90                  95

Ser Leu Leu Glu His Asn Tyr Val Gly Thr Glu His Leu Leu Leu Gly
            100                 105                 110

Ile Leu His Gln Ser Asp Ser Val Ala Leu Gln Val Leu Glu Asn Leu
        115                 120                 125

His Ile Asp Pro Arg Glu Val Arg Lys Glu Ile Leu Arg Glu Leu Glu
    130                 135                 140

Thr Phe Asn Leu Gln Leu Pro Pro Ser Ser Ser Ser Ser Ser Ser Ser
145                 150                 155                 160

Ser Arg Ser Asn Pro Ser Ser Ser Lys Ser Pro Leu Gly His Ser Leu
                165                 170                 175

Gly Ser Asp Lys Asn Glu Lys Leu Ser Ala Leu Lys Ala Tyr Gly Tyr
            180                 185                 190

Asp Leu Thr Glu Met Val Arg Glu Ser Lys Leu Asp Pro Val Ile Gly

        195                 200                 205

Arg Ser Ser Glu Val Glu Arg Leu Ile Leu Ile Leu Cys Arg Arg Arg
    210                 215                 220

Lys Asn Asn Pro Val Leu Ile Gly Glu Ala Gly Val Gly Lys Thr Ala
225                 230                 235                 240

Ile Val Glu Gly Leu Ala Gln Lys Ile Ile Leu Asn Glu Val Pro Asp
                245                 250                 255

Ala Leu Arg Lys Lys Arg Leu Ile Thr Leu Asp Leu Ala Leu Met Ile
            260                 265                 270

Ala Gly Thr Lys Tyr Arg Gly Gln Phe Glu Glu Arg Ile Lys Ala Val
        275                 280                 285

Met Asp Glu Val Arg Lys His Gly Asn Ile Leu Leu Phe Ile Asp Glu
    290                 295                 300

Leu His Thr Ile Val Gly Ala Gly Ala Ala Glu Gly Ala Ile Asp Ala
305                 310                 315                 320

Ser Asn Ile Leu Lys Pro Ala Leu Ala Arg Gly Glu Ile Gln Cys Ile
                325                 330                 335

Gly Ala Thr Thr Ile Asp Glu Tyr Arg Lys His Ile Glu Lys Asp Ala
            340                 345                 350

Ala Leu Glu Arg Arg Phe Gln Lys Ile Val Val His Pro Pro Ser Val
        355                 360                 365

Asp Glu Thr Ile Glu Ile Leu Arg Gly Leu Lys Lys Lys Tyr Glu Glu
    370                 375                 380

His His Asn Val Phe Ile Thr Glu Glu Ala Leu Lys Ala Ala Ala Thr
385                 390                 395                 400

Leu Ser Asp Gln Tyr Val His Gly Arg Phe Leu Pro Asp Lys Ala Ile
                405                 410                 415

Asp Leu Leu Asp Glu Ala Gly Ala Arg Val Arg Val Asn Thr Met Gly
            420                 425                 430

Gln Pro Thr Asp Leu Met Lys Leu Glu Ala Glu Ile Glu Asn Thr Lys
        435                 440                 445

Leu Ala Lys Glu Gln Ala Ile Gly Thr Gln Glu Tyr Glu Lys Ala Ala
    450                 455                 460

Gly Leu Arg Asp Glu Glu Lys Lys Leu Arg Glu Arg Leu Gln Ser Met
465                 470                 475                 480

Lys Gln Glu Trp Glu Asn His Lys Glu Glu His Gln Val Pro Val Asp
                485                 490                 495

Glu Glu Ala Val Ala Gln Val Val Ser Leu Gln Thr Gly Ile Pro Ser
            500                 505                 510

Ala Arg Leu Thr Glu Ala Glu Ser Glu Lys Leu Leu Lys Leu Glu Asp
        515                 520                 525

Thr Leu Arg Arg Lys Val Ile Gly Gln Asn Asp Ala Val Thr Ser Ile
    530                 535                 540

Cys Arg Ala Ile Arg Arg Ser Arg Thr Gly Ile Lys Asp Pro Asn Arg
545                 550                 555                 560

Pro Thr Gly Ser Phe Leu Phe Leu Gly Pro Thr Gly Val Gly Lys Ser
                565                 570                 575

Leu Leu Ala Gln Gln Ile Ala Ile Glu Met Phe Gly Gly Glu Asp Ala
            580                 585                 590

Leu Ile Gln Val Asp Met Ser Glu Tyr Met Glu Lys Phe Ala Ala Thr
        595                 600                 605

Lys Met Met Gly Ser Pro Pro Gly Tyr Val Gly His Glu Glu Gly Gly
    610                 615                 620

His Leu Thr Glu Gln Val Arg Arg Arg Pro Tyr Cys Val Val Leu Phe
625                 630                 635                 640

Asp Glu Ile Glu Lys Ala His Pro Asp Ile Met Asp Leu Met Leu Gln
                645                 650                 655

Ile Leu Glu Gln Gly Arg Leu Thr Asp Ser Phe Gly Arg Lys Val Asp
            660                 665                 670

Phe Arg His Ala Ile Ile Ile Met Thr Ser Asn Leu Gly Ala Asp Leu
        675                 680                 685

Ile Arg Lys Ser Gly Glu Ile Gly Phe Gly Leu Lys Ser His Met Asp
    690                 695                 700

Tyr Lys Val Ile Gln Glu Lys Ile Glu His Ala Met Lys Lys His Leu
705                 710                 715                 720

Lys Pro Glu Phe Ile Asn Arg Leu Asp Glu Ser Val Ile Phe Arg Pro
                725                 730                 735

Leu Glu Lys Glu Ser Leu Ser Glu Ile Ile His Leu Glu Ile Asn Lys
            740                 745                 750

Leu Asp Ser Arg Leu Lys Asn Tyr Gln Met Ala Leu Asn Ile Pro Asp
        755                 760                 765

Ser Val Ile Ser Phe Leu Val Thr Lys Gly His Ser Pro Glu Met Gly
    770                 775                 780

Ala Arg Pro Leu Arg Arg Val Ile Glu Gln Tyr Leu Glu Asp Pro Leu
785                 790                 795                 800

Ala Glu Leu Leu Leu Lys Glu Ser Cys Arg Gln Glu Ala Arg Lys Leu
                805                 810                 815

Arg Ala Thr Leu Val Glu Asn Arg Val Ala Phe Glu Arg Glu Glu Glu
            820                 825                 830

Glu Gln Glu Ala Ala Leu Pro Ser Pro His Leu Glu Ser
        835                 840                 845

<210> 19
<211> 509
<212> DNA
<213> Chlamydia pneumoniae

<220>
<221> CDS
<222> (101)..(406)

<400> 19
gattcaggtt ctagtgagct tatgctcatg gaagttcaag tcttcttagc tgcaagaaaa 60

taacagggac agtaattcga tttttcgaga agggaaactt atg gta aag atc ata    115
                                           Met Val Lys Ile Ile

                                                           5

tca agt gaa aat ttt gac tct ttt att gca tcg ggg ctc gtt ctc gtt     163
Ser Ser Glu Asn Phe Asp Ser Phe Ile Ala Ser Gly Leu Val Leu Val
                10                  15                  20

gat ttc ttt gca gaa tgg tgt ggc ccc tgt cgg atg ctc act cct atc     211
Asp Phe Phe Ala Glu Trp Cys Gly Pro Cys Arg Met Leu Thr Pro Ile
            25                  30                  35

tta gaa aat ctt gct gcg gaa ctt cct cat gtc act att gga aaa atc     259
Leu Glu Asn Leu Ala Ala Glu Leu Pro His Val Thr Ile Gly Lys Ile
        40                  45                  50

aat ata gat gag aac agc aag cct gca gaa acg tac gaa gtc agc tct     307
Asn Ile Asp Glu Asn Ser Lys Pro Ala Glu Thr Tyr Glu Val Ser Ser
    55                  60                  65

att cct acg ctt att ctt ttt aag gat ggg aac gag gtg gct cgg gtc     355
Ile Pro Thr Leu Ile Leu Phe Lys Asp Gly Asn Glu Val Ala Arg Val
70                  75                  80                  85

gta ggt ctt aag gat aaa gaa ttc cta acc aat ctt atc aat aag cac     403
Val Gly Leu Lys Asp Lys Glu Phe Leu Thr Asn Leu Ile Asn Lys His
                90                  95                  100

gct taaaaagacg ctgcaatatt aaaccgtagg attcttttgc aatgctacgg          456
Ala

ttttctgcct taccacttca tataaaacga tccctacact ggtagctaaa ttt          509


<210> 20
<211> 102
<212> PRT
<213> Chlamydia pneumoniae

<220>
<221> SITE
<222> (543)...(66)
<223> B-cell epitope

<220>
<221> SITE
<222> (40)...(48)
<223> T-cell epitope

<220>
<221> SITE
<222> (74)...(83)
<223> T-cell epitope

<400> 20
Met Val Lys Ile Ile Ser Ser Glu Asn Phe Asp Ser Phe Ile Ala Ser
 1               5                  10                  15

Gly Leu Val Leu Val Asp Phe Phe Ala Glu Trp Cys Gly Pro Cys Arg
            20                  25                  30

Met Leu Thr Pro Ile Leu Glu Asn Leu Ala Ala Glu Leu Pro His Val
        35                  40                  45

Thr Ile Gly Lys Ile Asn Ile Asp Glu Asn Ser Lys Pro Ala Glu Thr
    50                  55                  60

Tyr Glu Val Ser Ser Ile Pro Thr Leu Ile Leu Phe Lys Asp Gly Asn
65                  70                  75                  80

Glu Val Ala Arg Val Val Gly Leu Lys Asp Lys Glu Phe Leu Thr Asn
                85                  90                  95

Leu Ile Asn Lys His Ala
            100

<210> 21
<211> 43
<212> DNA
<213> Artificial Sequence

<220>
<223> 5' PCR primer

<400> 21
ataagaatgc ggccgccacc atgaagatgc ataggcttaa acc          43

<210> 22
<211> 36
<212> DNA
<213> Artificial Sequence

<220>
<223> 3' PCR primer

<400> 22
gcgccggatc ccacttaaga tatcgatatt tttgag          36

<210> 23
<211> 45
<212> DNA
<213> Artificial Sequence

<220>
<223> 5' PCR primer

<400> 23
ataagaatgc ggccgccacc atgcggttgg gaaataagcc tatgc          45

<210> 24
<211> 35
<212> DNA
<213> Artificial Sequence

<220>
<223> 3' PCR primer

<400> 24
gcgccggtac cgtaatttaa tactctttga agggc          35

<210> 25
<211> 49
<212> DNA
<213> Artificial Sequence

<220>
<223> 5' PCR primer

<400> 25
ataagaatgc ggccgccacc atgctcaccc taggcttgga aagttcttg          49

<210> 26
<211> 36

<212> DNA
<213> Artificial Sequence

<220>
<223> 3' PCR primer

<400> 26
gctttggagg atccccggag aggctaagga gaatgg                                36

<210> 27
<211> 44
<212> DNA
<213> Artificial Sequence

<220>
<223> 5' PCR primer

<400> 27
ataagaatgc ggccgccacc atgaaaaaag ggaaattagg agcc                        44

<210> 28
<211> 33
<212> DNA
<213> Artificial Sequence

<220>
<223> 3' PCR primer

<400> 28
gcgccggatc cccgaagcag aagtcgttgt ggg                                   33

<210> 29
<211> 48
<212> DNA
<213> Artificial Sequence

<220>
<223> 5' PCR primer

<400> 29
ataagaatgc ggccgccacc atgagaaaac ttattttatg caatccta                   48

<210> 30
<211> 33
<212> DNA
<213> Artificial Sequence

<220>
<223> 3' PCR primer

<400> 30
gcgccggatc ccagaacaac ggagttcttt tgg                                   33

<210> 31
<211> 46
<212> DNA
<213> Artificial Sequence

<220>
<223> 5' PCR primer

<400> 31
ataagaatgc ggccgccacc atgaataaaa aaaatctaac tatttg                     46

<210> 32
<211> 32
<212> DNA
<213> Artificial Sequence

<220>
<223> 3' PCR primer

<400> 32
gcgccggatc ccagcgatag cttctggggt cc                                    32


<210> 33
<211> 42
<212> DNA
<213> Artificial Sequence

<220>
<223> 5' PCR primer

<400> 33
ataagaatgc ggccgccacc atgacactgg taccctatgt tg                         42


<210> 34
<211> 35
<212> DNA
<213> Artificial Sequence

<220>
<223> 3' PCR primer

<400> 34
gcgccggatc ccagtgctac ttgtatcctt attag                                 35

<210> 35
<211> 45
<212> DNA
<213> Artificial Sequence

<220>
<223> 5' PCR primer

<400> 35
ataagaatgc ggccgccacc atgagctacc gtaaacgttc gactc                      45


<210> 36
<211> 35
<212> DNA
<213> Artificial Sequence

<220>
<223> 3' PCR primer

<400> 36
gcgccggatc cccctcgttc ccccttgttt cggag                                 35

<210> 37
<211> 46
<212> DNA
<213> Artificial Sequence

<220>
<223> 5' PCR primer

<400> 37

ataagaatgc ggccgccacc atgtttgaga agttcactaa tagagc 46

<210> 38
<211> 34
<212> DNA
<213> Artificial Sequence

<220>
<223> 3' PCR primer

<400> 38
gcgccggtac cgtgattcca agtgagggct aggg 34

<210> 39
<211> 42
<212> DNA
<213> Artificial Sequence

<220>
<223> 5' PCR primer

<400> 39
ataagaatgc ggccgccacc atggtaaaga tcatatcaag tg 42

<210> 40
<211> 30
<212> DNA
<213> Artificial Sequence

<220>
<223> 3' PCR primer

<400> 40
gcgccggatc ccagcgtgct tattgataag 30

<210> 41
<211> 17
<212> PRT
<213> Artificial Sequence

<220>
<223> B-cell epitope of ATP-binding cassette protein

<400> 41
Val His His Thr Leu Arg Glu Ser Tyr Lys Lys Gly Thr Pro Pro
1               5                   10                  15

Ser Thr

<210> 42
<211> 16

<212> PRT
<213> Artificial Sequence

<220>
<223> B-cell epitope of ATP-binding cassette protein

<400> 42
Asn Leu Gln Lys Glu Ile Ser Thr Glu Glu Arg Gln Thr Lys Ala
1               5                   10                  15

Arg

<210> 43
<211> 9
<212> PRT
<213> Artificial Sequence

<220>
<223> T-cell epitope of ATP-binding cassette protein

<400> 43
Trp Ile Ala Glu Tyr Val Ser Pro Val
1               5

<210> 44
<211> 12
<212> PRT
<213> Artificial Sequence

<220>
<223> B-cell epitope of endopeptidase protein

<400> 44
Lys Gly Asn Asn Ser Ser Pro Arg Ser Pro Ala Pro
1               5                   10

<210> 45
<211> 9
<212> PRT
<213> Artificial Sequence

<220>
<223> B-cell epitope of endopeptidase protein

<400> 45
Gly Glu Asn Phe Gln Lys Asn Ser Ser
1               5

<210> 46
<211> 9
<212> PRT
<213> Artificial Sequence

<220>
<223> T-cell epitope of endopeptidase protein

<400> 46
Leu Leu Ile Glu Asp Met Asp Leu Ile
1               5

<210> 47
<211> 9
<212> PRT
<213> Artificial Sequence

<220>
<223> T-cell epitope of endopeptidase protein

<400> 47
Asn Leu Leu Ile Glu Asp Met Asp Leu
1               5

<210> 48
<211> 16
<212> PRT
<213> Artificial Sequence

<220>
<223> B-cell epitope of protease protein

<400> 48
Thr Asp Leu Glu Gly Leu Glu Glu Asp His Lys Asp Ser Pro Trp
1               5                   10                  15

Glu


<210> 49
<211> 18
<212> PRT
<213> Artificial Sequence

<220>
<223> B-cell epitope of protease protein

<400> 49
Ser Glu Asn Ala Lys Lys Ser Glu Glu Gln Thr Ser Pro Gln Glu
1               5                   10                  15

Thr Pro Glu


<210> 50
<211> 9
<212> PRT
<213> Artificial Sequence

<220>
<223> T-cell epitope of protease protein

<400> 50
Tyr Leu Gly Asp Glu Ile Leu Glu Val
1               5

<210> 51
<211> 9
<212> PRT
<213> Artificial Sequence

<220>
<223> T-cell epitope of protease protein

<400> 51
Tyr Leu Tyr Ser Leu Leu Ser Met Leu
1               5

<210> 52
<211> 8
<212> PRT
<213> Artificial Sequence

<220>
<223> B-cell epitope of metalloprotease protein

<400> 52
Thr Thr Asn Arg Gln Lys Ala Leu
1               5

<210> 53
<211> 11
<212> PRT
<213> Artificial Sequence

<220>
<223> B-cell epitope of metalloprotease protein

<400> 53
Val Asn Ser Ser Asn Ser Asn Arg Leu Arg Glu

1                   5                        10

<210> 54
<211> 9
<212> PRT
<213> Artificial Sequence

<220>
<223> T-cell epitope of metalloprotease protein

<400> 54
Ser Val Leu Ser Arg Val Asn Tyr Val
1               5

<210> 55
<211> 9
<212> PRT
<213> Artificial Sequence

<220>
<223> T-cell epitope of metalloprotease protein

<400> 55
Lys Leu Ser Ser Leu Ile Pro Gly Leu
1               5

<210> 56
<211> 9
<212> PRT
<213> Artificial Sequence

<220>
<223> T-cell epitope of metalloprotease protein

<400> 56
Ile Leu Ile Gly His Lys Lys His Val
1               5

<210> 57
<211> 14
<212> PRT
<213> Artificial Sequence

<220>
<223> B-cell epitope of CLP protease ATPase protein

<400> 57
Pro Pro Lys Gly Gly Arg Lys His Pro Asn Gln Glu Tyr Ile
                5                   10

<210> 58
<211> 14
<212> PRT
<213> Artificial Sequence

<220>
<223> B-cell epitope of CLP protease ATPase protein

<400> 58
Ser Asp Asp Gln Ala Asp Leu Ser Gln Lys Thr Arg Asp His
1               5                   10

<210> 59
<211> 9
<212> PRT
<213> Artificial Sequence

<220>

<223> T-cell epitope of CLP protease ATPase protein

<400> 59
Lys Ile Leu Asp Val Pro Phe Thr Ile
1               5

<210> 60
<211> 9
<212> PRT
<213> Artificial Sequence

<220>
<223> T-cell epitope of CLP protease ATPase protein

<400> 60
Leu Leu Gln Ala Ala Asp Tyr Asp Val
1               5

<210> 61
<211> 9
<212> PRT
<213> Artificial Sequence

<220>
<223> B-cell epitope of CLP protease subunit protein

<400> 61
Gly Thr Lys Gly Lys Arg His Ala Leu
1               5

<210> 62
<211> 11
<212> PRT
<213> Artificial Sequence

<220>
<223> B-cell epitope of CLP protease subunit protein

<400> 62
Ala Lys Glu Thr Asn Lys Asp Thr Ser Ser Thr
1               5                   10

<210> 63
<211> 9
<212> PRT
<213> Artificial Sequence

<220>
<223> T-cell epitope of CLP protease subunit protein

<400> 63
Ala Ile Tyr Asp Thr Ile Arg Phe Leu
1               5

<210> 64
<211> 19
<212> PRT
<213> Artificial Sequence

<220>
<223> B-cell epitope of translycolase/transpeptidase protein

<400> 64
Asp Pro Thr Asn Tyr Lys Glu Tyr Phe Asn Asn Lys Glu Arg Ile
1               5                   10                  15

Glu His Thr Lys

<210> 65
<211> 17
<212> PRT
<213> Artificial Sequence

<220>
<223> B-cell epitope of translycolase/transpeptidase protein

<400> 65
Lys Arg Leu Tyr Glu Glu Trp Asn Arg Ser Pro Lys Gln Gly Gly
1               5                   10                  15

Thr Arg


<210> 66
<211> 9
<212> PRT
<213> Artificial Sequence

<220>
<223> T-cell epitope of translycolase/transpeptidase protein

<400> 66
Ala Leu Gly Gln His Glu Phe Cys Val
1               5

<210> 67
<211> 9
<212> PRT
<213> Artificial Sequence

<220>
<223> T-cell epitope of translycolase/transpeptidase protein

<400> 67
Ile Leu Ala Thr Gly Ile Gln Met Val
1               5

<210> 68
<211> 26
<212> PRT
<213> Artificial Sequence

<220>
<223> B-cell epitope of CLPc protease protein

<400> 68
Arg Asp Glu Glu Lys Lys Leu Arg Glu Arg Leu Gln Ser Met Lys
1               5                   10                  15

Gln Glu Trp Glu Asn His Lys Glu Glu His Gln
                20                  25


<210> 69
<211> 16
<212> PRT
<213> Artificial Sequence

<220>
<223> B-cell epitope of CLPc protease protein

<400> 69
Ile Arg Arg Ser Arg Thr Gly Ile Lys Asp Pro Asn Arg Pro Thr
1               5                   10                  15

Gly

<210> 70
<211> 9
<212> PRT
<213> Artificial Sequence

<220>
<223> T-cell epitope of CLPc protease protein

<400> 70
Phe Leu Phe Leu Gly Pro Thr Gly Val
1               5

<210> 71
<211> 9
<212> PRT
<213> Artificial Sequence

<220>
<223> T-cell epitope of CLPc protease protein

<400> 71
Phe Leu Pro Asp Lys Ala Ile Asp Leu
1               5

<210> 72
<211> 13
<212> PRT
<213> Artificial Sequence

<220>
<223> B-cell epitope of thioredoxin

<400> 72
Asn Ile Asp Glu Asn Ser Lys Pro Ala Glu Thr Tyr Glu
1               5                   10

<210> 73
<211> 9
<212> PRT
<213> Artificial Sequence

<220>
<223> B-cell epitope of thioredoxin

<400> 73
Asn Leu Ala Ala Glu Leu Pro His Val
1               5

<210> 74
<211> 9
<212> PRT
<213> Artificial Sequence

<220>
<223> T-cell epitope of thioredoxin

<400> 74
Ile Leu Phe Lys Asp Gly Asn Glu Val
1               5

## Description

### REFERENCE TO RELATED APPLICATIONS

This application claims the benefit of U.S. Provisional Application Nos. 60/202,672, filed May 8, 2000; 60/207,852 filed May 30, 2000; 60/211,801, 60/212,044, 60/211,797, 60/211,796 and 60/211,798 filed June 16, 2000; and 60/235,335, 60/235,361 and 60/235,398 filed September 26, 2000.

### FIELD OF INVENTION

The present invention relates to a number of *Chlamydia* antigens, including an ATP-binding cassette protein, a secretory locus ORF, an endopeptidase, a protease, a metalloprotease, CLP protease ATPase, a CLP protease subunit, a translycolase / transpeptidase, a CLPc protease and thioredoxin, and their corresponding DNA molecules, for the prevention and treatment of *Chlamydia* infection in mammals.

### BACKGROUND OF THE INVENTION

*Chlamydiae* are prokaryotes. They exhibit morphologic and structural similarities to gram-negative bacteria including a trilaminar outer membrane, which contains lipopolysaccharide and several membrane proteins that are structurally and functionally analogous to proteins found in *E coli.* They are obligate intra-cellular parasites with a unique biphasic life cycle consisting of a metabolically inactive but infectious extracellular stage and a replicating but non-infectious intracellular stage. The replicative stage of the life-cycle takes place within a membrane-bound inclusion which sequesters the bacteria away from the cytoplasm of the infected host cell.

*C. pneumoniae* is a common human pathogen, originally described as the TWAR strain of *Chlamydia psittaci* but subsequently recognised to be a new species. *C. pneumoniae* is antigenically, genetically and morphologically distinct from other *Chlamydia* species *(C. trachomatis, C. pecorum* and *C. psittaci).* It shows 10% or less DNA sequence homology with either of *C. trachomatis* or *C. psittaci.*

In general, all chlamydiae share a common developmental microbiology and appear to share a common immunobiology. Genome analysis shown that over 80% of *C. pneumoniae* and *C. trachomatis* protein-coding genes are orthologs that share a similar genome organization.

*C. pneumoniae* is the third most common cause of community acquired pneumonia, only less frequent than *Streptococcus pneumoniae* and *Mycoplasma pneumoniae* (Grayston et al. (1995) Journal of Infectious Diseases 168:1231; Campos et al. (1995) Investigation of Ophthalmology and Visual Science 36:1477). It can also cause upper respiratory tract symptoms and disease, including bronchitis and sinusitis (Grayston et al. (1995) Journal of Infectious Diseases 168:1231; Grayston et al (1990) Journal of Infectious Diseases 161:618-625; Marrie (1993) Clinical Infectious Diseases. 18:501-513; Wang et al (1986) Chlamydial infections Cambridge University Press, Cambridge. p. 329. The great majority of the adult population (over 60%) has antibodies to *C. pneumoniae* (Wang et al (1986) Chlamydial infections. Cambridge University Press, Cambridge. p. 329), indicating past infection which was unrecognized or asymptomatic.

*C. pneumoniae* infection usually presents as an acute respiratory disease (*i.e.*, cough, sore throat, hoarseness, and fever; abnormal chest sounds on auscultation). For most patients, the cough persists for 2 to 6 weeks, and recovery is slow. In approximately 10% of these cases, upper respiratory tract infection is followed by bronchitis or pneumonia. Furthermore, during a *C. pneumoniae* epidemic, subsequent co-infection with pneumococcus has been noted in about half of these pneumonia patients, particularly in the infirm and the elderly. As noted above, there is increasing evidence that *C. pneumoniae* infection is also linked to diseases other than respiratory infections.

The reservoir for the organism is presumably people. In contrast to *C. psittaci* infections, there is no known bird or animal reservoir. Transmission has not been clearly defined. It may result from direct contact with secretions, from fomites, or from airborne spread. There is a long incubation period, which may last for many months. Based on analysis of epidemics, *C. pneumoniae* appears to spread slowly through a population (case-to-case interval averaging 30 days) because infected persons are inefficient transmitters of the organism. Susceptibility to *C. pneumoniae* is universal. Reinfections occur during adulthood, following the primary infection as a child. *C. pneumoniae* appears to be an endemic disease throughout the world, noteworthy for superimposed intervals of increased incidence (epidemics) that persist for 2 to 3 years. *C. trachomatis* infection does not confer cross-immunity to *C. pneumoniae.* Infections are easily treated with oral antibiotics, tetracycline or erythromycin (2 g/d, for at least 10 to 14 d). A recently developed drug, azithromycin, is highly effective as a single-dose therapy against chlamydial infections.

In most instances, *C. pneumoniae* infection is often mild and without complications, and up to 90% of infections are subacute or unrecognized. Among children in industrialized countries, infections have been thought to be rare up to the age of 5 y, although a recent study (E Normann et al, Chlamydia pneumoniae in children with acute respiratory tract infections, Acta Paediatrica, 1998, Vol 87, Iss 1, pp 23-27) has reported that many children in this age group show PCR evidence of infection despite being seronegative, and estimates a prevalence of 17-19% in 2-4 y olds. In developing countries, the seroprevalence of *C. pneumoniae* antibodies among young children is elevated, and there are suspicions that *C. pneumoniae* may be an important cause of acute lower respiratory tract disease and mortality for infants and children in tropical regions of the world.

From seroprevalence studies and studies of local epidemics, the initial *C. pneumoniae* infection usually happens between the ages of 5 and 20 y. In the USA, for example, there are estimated to be 30,000 cases of childhood pneumonia each year caused by *C. pneumoniae.* Infections may cluster among groups of children or young adults (*e.g.*, school pupils or military conscripts).

*C. pneumoniae* causes 10 to 25% of community-acquired lower respiratory tract infections (as reported from Sweden, Italy, Finland, and the USA). During an epidemic, *C. pneumonia* infection may account for 50 to 60% of the cases of pneumonia. During these periods, also, more episodes of mixed infections with *S. pneumoniae* have been reported.

Reinfection during adulthood is common; the clinical presentation tends to be milder. Based on population seroprevalence studies, there tends to be increased exposure with age, which is particularly evident among men. Some investigators have speculated that a persistent, asymptomatic *C. pneumoniae* infection state is common.

In adults of middle age or older, *C. pneumoniae* infection may progress to chronic bronchitis and sinusitis. A study in the USA revealed that the incidence of pneumonia caused by *C. pneumoniae* in persons younger than 60 years is 1 case per 1,000 persons per year; but in the elderly, the disease incidence rose three-fold. *C. pneumoniae* infection rarely leads to hospitalization, except in patients with an underlying illness.

Of considerable importance is the association of atherosclerosis and *C. pneumoniae* infection. There are several epidemiological studies showing a correlation of previous infections with *C. pneumoniae* and heart attacks, coronary artery and carotid artery disease (Saikku et al.(1988) Lancet;ii:983-986; Thom et al. (1992) JAMA 268:68-72; Linnanmaki et al. (1993), Circulation 87:1030; Saikku et al. (1992)Annals Internal Medicine 116:273-287; Melnick et al (1993) American Journal of Medicine 95:499). Moreover, the organisms has been detected in atheromas and fatty streaks of the coronary, carotid, peripheral arteries and aorta (Shor et al. (1992) South African. Medical Journal 82:158-161; Kuo et al. (1993) Journal of Infectious Diseases 167:841-849; Kuo et al. (1993) Arteriosclerosis and Thrombosis 13:1501-1504; Campbell et al (1995) Journal of Infectious Diseases 172:585; Chiu et al. Circulation, 1997. Circulation. 96:2144-2148). Viable *C. pneumoniae* has been recovered from the coronary and carotid artery (Ramirez et al (1996) Annals of Internal Medicine 125:979-982; Jackson et al. 1997. J. Infect. Dis. 176:292-295). Furthermore, it has been shown that *C. pneumoniae* can induce changes of atherosclerosis in a rabbit model (Fong et al. 1997. Journal of Clinical Microbiolology 35:48 and Laitinen et al. 1997. Infect. Immun. 65:4832-4835). Taken together, these results indicate that it is highly probable that *C. pneumoniae* can cause atherosclerosis in humans, though the epidemiological importance of chlamydial atherosclerosis remains to be demonstrated.

A number of recent studies have also indicated an association between *C. pneumoniae* infection and asthma. Infection has been linked to wheezing, asthmatic bronchitis, adult-onset asthma and acute exacerbations of asthma in adults, and small-scale studies have shown that prolonged antibiotic treatment was effective at greatly reducing the severity of the disease in some individuals (Hahn DL, et al. Evidence for Chlamydia pneumoniae infection in steroid-dependent asthma.Ann Allergy Asthma Immunol. 1998 Jan; 80(1): 45-49.; Hahn DL, et al. Association of Chlamydia pneumoniae IgA antibodies with recently symptomatic asthma. Epidemiol Infect. 1996 Dec; 117(3): 513-517; Bjornsson E, et al. Serology of chlamydia in relation to asthma and bronchial hyperresponsiveness. Scand J Infect Dis. 1996; 28(1): 63-69.; Hahn DL. Treatment of Chlamydia pneumoniae infection in adult asthma: a before-after trial. J Fam Pract. 1995 Oct; 41(4): 345-351.; Allegra L, et al. Acute exacerbations of asthma in adults: role of Chlamydia pneumoniae infection. Eur Respir J. 1994 Dec; 7(12): 2165-2168.; Hahn DL, et al. Association of Chlamydia pneumoniae (strain TWAR) infection with wheezing, asthmatic bronchitis, and adult-onset asthma. JAMA. 1991 Jul 10; 266(2): 225-230).

In light of these results a protective vaccine against *C. pneumoniae* infection would be of considerable importance. There is not yet an effective vaccine for any human chlamydial infection. It is conceivable that an effective vaccine can be developed using physically or chemically inactivated *Chlamydiae.* However, such a vaccine does not have a high margin of safety. In general, safer vaccines are made by genetically manipulating the organism by attenuation or by recombinant means.

A disease associated with *C. trachomatis* infection is trachoma, a sequela of ocular infection. This disease continues to be a major cause of preventable blindness, with an estimated 500 million cases of active trachoma worldwide (seven million include blindness from conjunctival scarring and eyelid deformities). In the last two decades, genital chlamydial infection has been identified as a major public health problem because of the recognition that chlamydial infection is associated with disease syndromes such as non-gonococcal urethritis, mucopurulent cervicitis, pelvic inflammatory disease (PID), ectopic pregnancy, and tubal infertility. The World Health Organization estimated 89 million new cases of genital chlamydial infections worldwide in 1995. In the United States, each year an estimated four million new cases occur and 50,000 women become infertile as a result of infection.

Studies with *C. trachomatis* and *C. psittaci* indicate that safe and effective vaccine against *Chlamydia* is an attainable goal. For example, mice which have recovered from a lung infection with *C. trachomatis* are protected from infertility induced by a subsequent vaginal challenge (Pal et al. (1996) Infection and Immunity.64:5341). Similarly, sheep immunized with inactivated *C. psittaci* were protected from subsequent chlamydial-induced abortions and stillbirths (Jones et al. (1995) Vaccine 13:715). In a mouse model, protection from chlamydial infections has been associated with Th1 immune responses, particularly CD8+ CTL response (Rottenberg et al. 1999. J. Immunol. 162:2829-2836 and Penttila et al. 1999. Immunology. 97:490-496) and it is unlikely that similar responses will need to be induced in humans to confer protection. However, antigens able to elicit a protective immune response against *C. pneumoniae* are largely unknown. The presence of sufficiently high titres of neutralising antibody at mucosal surfaces can also exert a protective effect (Cotter et al. (1995) Infection and Immunity 63:4704).

Antigenic variation within the species *C. pneumoniae* is not well documented due to insufficient genetic information, though variation is expected to exist based on *C. trachomatis.* Serovars of *C. trachomatis* are defined on the basis of antigenic variation in the major outer membrane protein (MOMP), but published *C. pneumoniae* MOMP gene sequences show no variation between several diverse isolates of the organism (Campbell et al. Infection and Immunity (1990) 58:93; McCafferty et al Infection and Immunity (1995) 63:2387-9; Gaydos et al. Infection and Immunity.(1992) 60(12):5319-5323). The gene encoding a 76 kDa antigen has been cloned from a single strain of *C. pneumoniae* and the sequence published (Perez Melgosa et al. Infection and Immunity.(1994) 62:880). An operon encoding the 9 kDa and 60 kDa cyteine-rich outer membrane protein genes has been described (Watson et al., Nucleic Acids Res (1990) 18:5299; Watson et al., Microbiology (1995) 141:2489). Many antigens recognized by immune sera to *C. pneumoniae* are conserved across all *chlamydiae,* but 98 kDa, 76 kDa and several other proteins may be *C. pneumoniae*-specific (Knudsen et al. Infect. Immun. 1999. 67:375-383; Perez Melgosa et al. Infection and Immunity. 1994. 62:880; Melgosa et al., FEMS Microbiol Lett 1993. 112 :199;, Campbell et al., J. Clin. Microbiol. 1990. 28 :1261; Iijima et al., J. Clin. Microbiol. 1994. 32:583). Antisera to 76kDa and 54kDa antigens have been reported to neutralize *C. pneumoniae in vitro* (Perez Melgosa et al. 1994. Infect. Immun. 62:880-886 and Wiedman-Al-Ahmad et al. 1997. Clin. Diagn. Lab. Immunol. 4:700-704). An assessment of the number and relative frequency of any *C. pneumoniae* serotypes, and the defining antigens, is not yet possible. The entire genome sequence of *C. pneumoniae* strain CWL-029 is now known (http://chlamydia-www.berkeley.edu:4231/) and as further sequences become available a better understanding of antigenic variation may be gained.

Many antigens recognised by immune sera to *C. pneumoniae* are conserved across all *chlamydiae,* but 98kDa, 76 kDa and 54 kDa proteins appear to be *C. pneumoniae-*specific (Campos et al. (1995) Investigation of Ophthalmology and Visual Science 36:1477; Marrie (1993) Clinical Infectious Diseases. 18:501-513; Wiedmann-Al-Ahmad M, et al. Reactions of polyclonal and neutralizing anti-p54 monoclonal antibodies with an isolated, species-specific 54-kilodalton protein of Chlamydia pneumoniae. Clin Diagn Lab Immunol. 1997 Nov; 4(6): 700-704).

Immunoblotting of isolates with sera from patients does show variation of blotting patterns between isolates, indicating that serotypes *C. pneumoniae* may exist (Grayston et al. (1995) Journal of Infectious Diseases 168:1231; Ramirez et al (1996) Annals of Internal Medicine 125:979-982). However, the results are potentially confounded by the infection status of the patients, since immunoblot profiles of a patient's sera change with time post-infection. An assessment of the number and relative frequency of any serotypes, and the defining antigens, is not yet possible.

The use of DNA immunization to elicit a protective immune response in Balb/c mice against pulmonary infection with the mouse pneumonitis (MoPn) strain of *Chlamydia trachomatis* has recently been described (Zhang et al. 1997. J. Infect. Dis. 76:1035-1040 and Zhang et al. 1999. Immunology. 96:314-321). Recently the genome sequence from C. pneumoniae strain CM1 (ATCC #1360-VR) has been disclosed by Griffais in WO99/27105 on June 3, 1999.

Accordingly, a need exists for identifying and isolating polynucleotide sequences of *C. pneumoniae* for use in preventing and treating *Chlamydia* infection.

### SUMMARY OF THE INVENTION

The present invention provides purified and isolated polynucleotide molecules that encode a *Chlamydia* polypeptide selected from: an ATP-binding cassette protein, a secretory locus ORF, an endopeptidase, a protease, a metalloprotease, CLP protease ATPase, a CLP protease subunit, a translycolase / transpeptidase, a CLPc protease and thioredoxin. The polynucleotide molecules can be used in methods to prevent, treat, and diagnose *Chlamydia* infection. In one embodiment of the invention, the polynucleotide molecules is DNA that encode a polypeptide of any one of SEQ ID Nos: 2, 4, 6, 8, 10, 12, 14, 16, 18 and 20.

Another form of the invention provides polypeptides corresponding to an isolated DNA molecule. Amino acid sequences of the corresponding encoded polypeptides are shown in one embodiment as SEQ ID Nos: 2, 4, 6, 8, 10, 12, 14, 16, 18 and 20.

Those skilled in the art will readily understand that the invention, having provided the polynucleotide sequences encoding *Chlamydia* polypeptides, also provides polynucleotides encoding fragments derived from such polypeptides. Moreover, the invention is understood to provide mutants and derivatives of such polypeptides and fragments derived therefrom, which result from the addition, deletion, or substitution of non-essential amino acids as described herein. Those skilled in the art would also readily understand that the invention, having provided the polynucleotide sequences encoding *Chlamydia* polypeptides, further provides monospecific antibodies that specifically bind to such polypeptides.

The present invention has wide application and includes expression cassettes, vectors, and cells transformed or transfected with the polynucleotides of the invention. Accordingly, the present invention further provides (i) a method for producing a polypeptide of the invention in a recombinant host system and related expression cassettes, vectors, and transformed or transfected cells; (ii) a vaccine, or a live vaccine vector such as a pox virus, *Salmonella typhimurium,* or *Vibrio cholerae* vector, containing a polypeptide or a polynucleotide of the invention, such vaccines and vaccine vectors being useful for, *e.g.*, preventing and treating *Chlamydia* infection, in combination with a diluent or carrier, and related pharmaceutical compositions and associated therapeutic and/or prophylactic methods; (iii) a therapeutic and/or prophylactic use of an RNA or DNA molecule of the invention, either in a naked form or formulated with a delivery vehicle, a polypeptide or combination of polypeptides, or a monospecific antibody of the invention, and related pharmaceutical compositions; (iv) a method for diagnosing the presence of *Chlamydia* in a biological sample, which can involve the use of a DNA or RNA molecule, a monospecific antibody, or a polypeptide of the invention; and (v) a method for purifying a polypeptide of the invention by antibody-based affinity chromatography.

One aspect of the invention provides a vaccine comprising a vaccine vector and at least one first nucleic acid selected from any one of:
(i) a nucleic acid sequence set forth in any one of SEQ ID Nos: 1, 3, 5, 7, 9, 11, 13, 15, 17 and 19;
(ii) a nucleic acid sequence which encodes a polypeptide encoded by any one of SEQ ID Nos: 1, 3, 5, 7, 9, 11, 13, 15, 17 and 19;
(iii) a nucleic acid sequence which encodes a polypeptide which is at least 75% identical in amino acid sequence to the polypeptide encoded by any one of SEQ ID Nos: 1, 3, 5, 7, 9, 11, 13, 15, 17 and 19; and
(iv) a nucleic acid sequence which encodes a polypeptide whose sequence is set forth in any one of SEQ ID Nos: 2, 4, 6, 8, 10, 12, 14, 16, 18 and 20;
(v) a nucleic acid sequence as defined in (i), (ii) or (iv), which has been modified to encode a modified polypeptide, wherein the modified polypeptide retains immunogenicity and is at least 75% identical in amino acid sequence to the corresponding polypeptide encoded by the nucleic acid of (i), (ii) or (iv);
wherein each first nucleic acid is capable of being expressed.

Another aspect of the invention provides a vaccine comprising a vaccine vector and at least one first nucleic acid selected from any one of:
(i) a nucleic acid sequence comprising at least 36 consecutive nucleotides from any one of SEQ ID Nos: 1, 3, 5, 7, 9, 11, 13, 15, 17 and 19;
(ii) a nucleic acid sequence which encodes an immunogenic fragment comprising at least 12 consecutive amino acids from any one of SEQ ID Nos: 2, 4, 6, 8, 10, 12, 14, 16, 18 and 20;
(iii) a nucleic acid sequence as defined in (i) or (ii), which has been modified to encode a modified polypeptide,
wherein the modified polypeptide retains immunogenicity and is at least 75% identical in amino acid sequence to the corresponding fragment of (i) or (ii);
wherein each first nucleic acid is capable of being expressed.

### BRIEF DESCRIPTION OF THE DRAWINGS

The present invention will be further understood from the following description with reference to the drawings, in which:

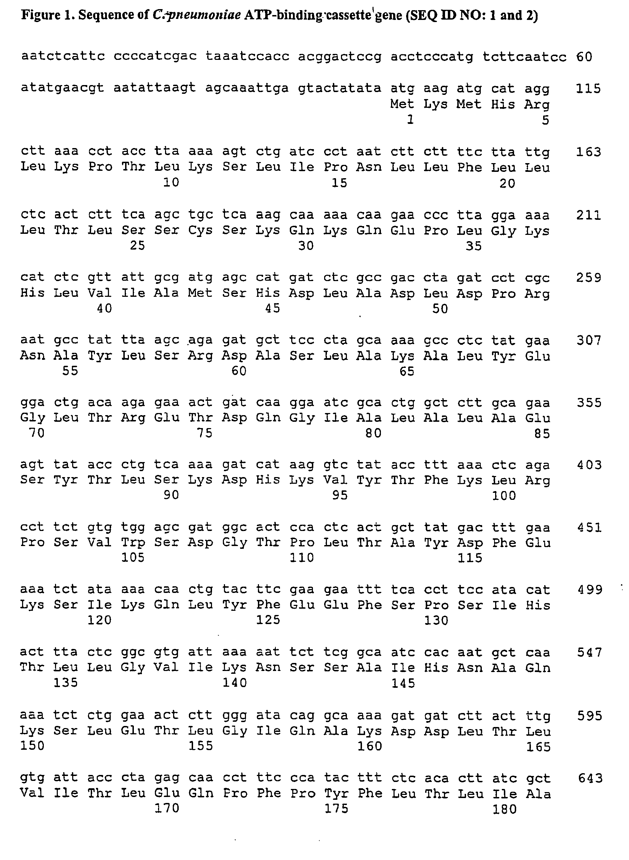

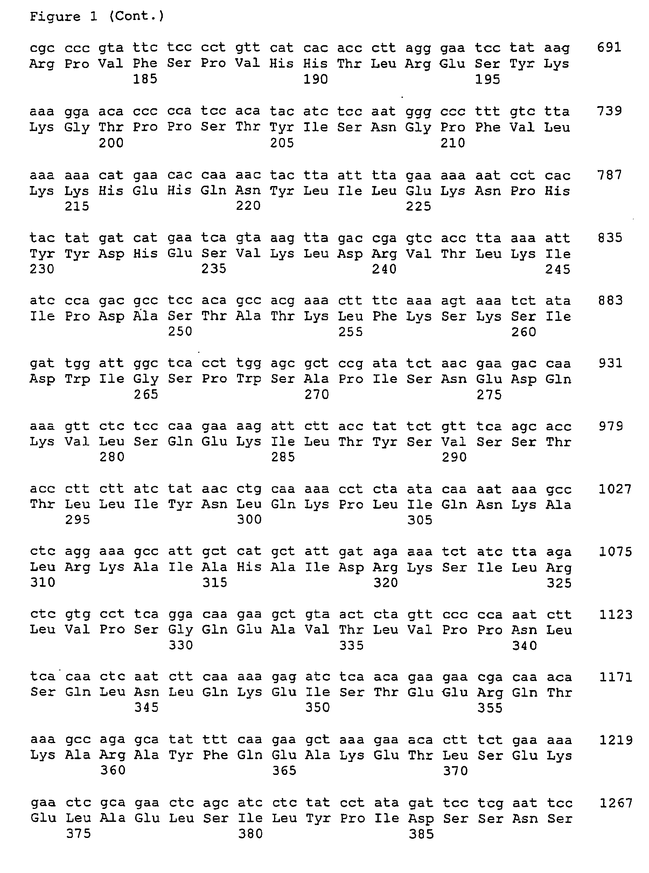

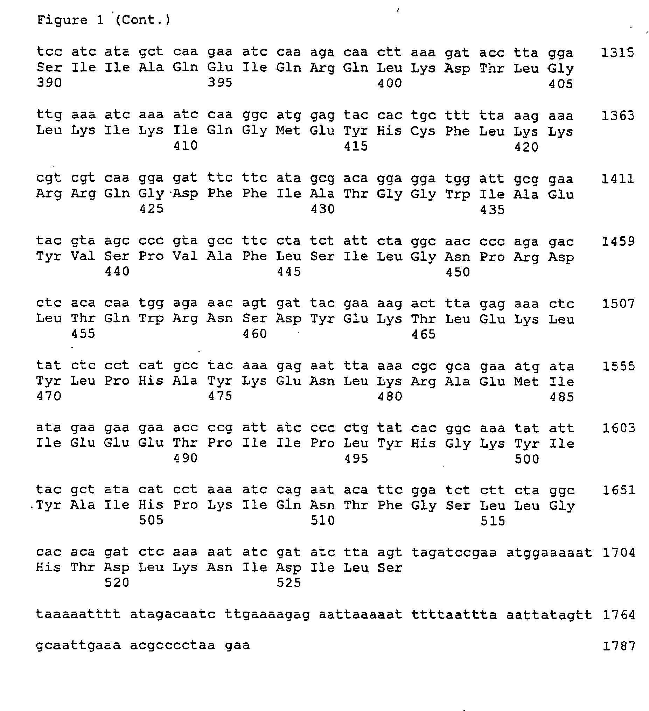

Figure 1 shows the nucleotide sequence of the gene encoding an ATP-binding cassette (SEQ ID No: 1) and the deduced amino acid sequence of the ATP-binding cassette from *Chlamydia pneumoniae* (SEQ ID No: 2).

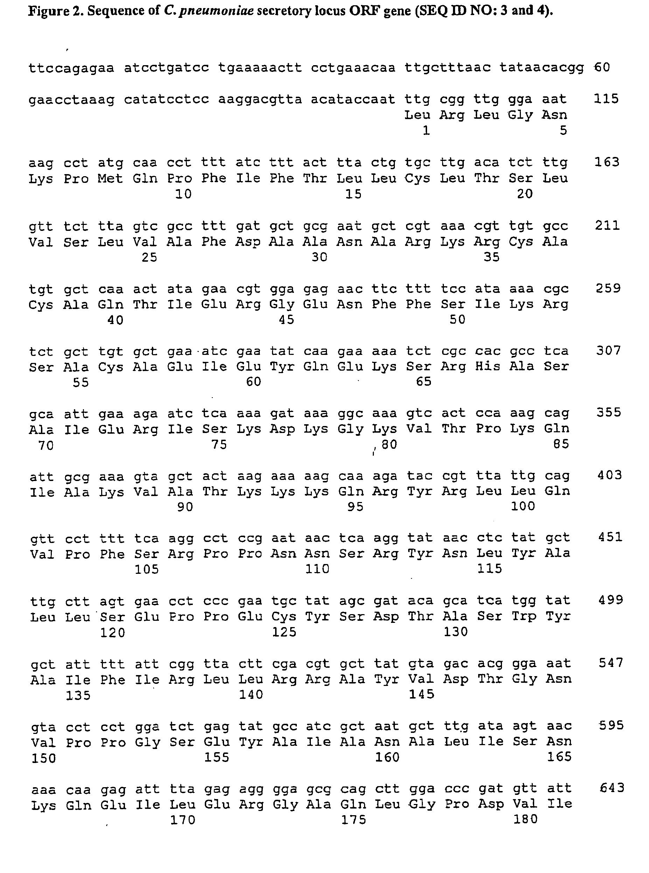

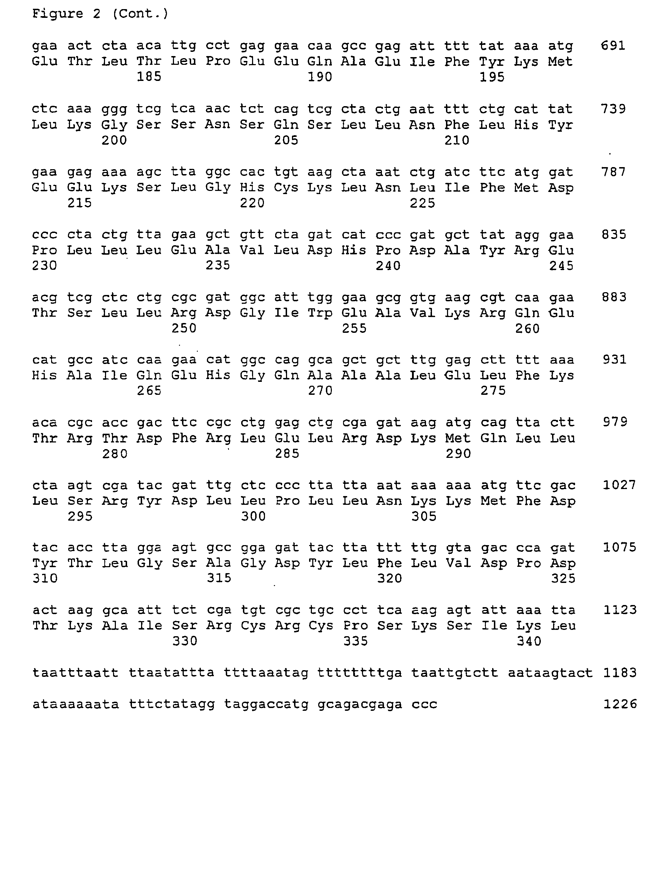

Figure 2 shows the nucleotide sequence of the gene encoding a secretory locus ORF (SEQ ID No: 3) and the deduced amino acid sequence of the secretory locus ORF from *Chlamydia pneumoniae* (SEQ ID No: 4).

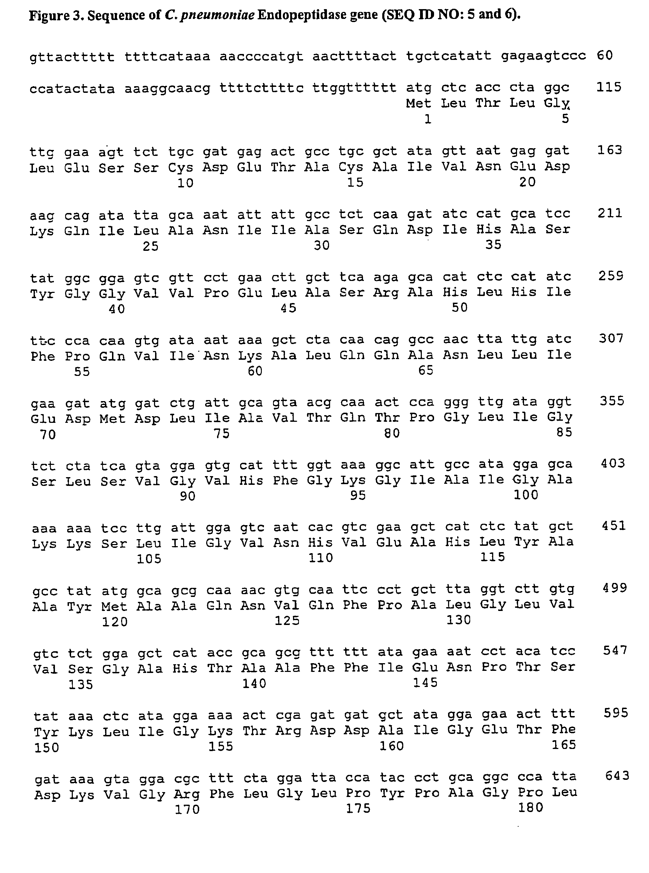

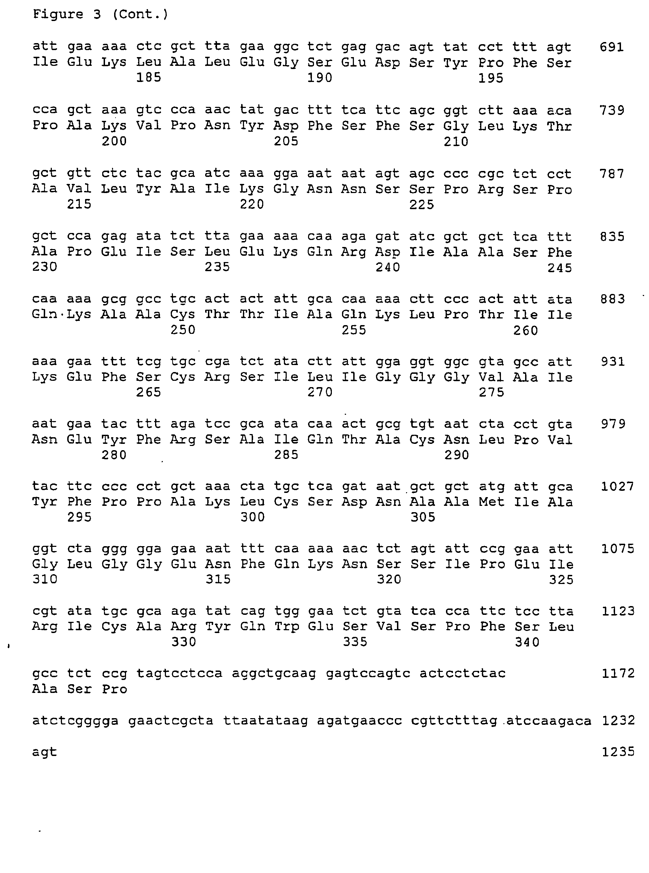

Figure 3 shows the nucleotide sequence of the gene encoding an endopeptidase (SEQ ID No: 5) and the deduced amino acid sequence of the endopeptidase from *Chlamydia pneumoniae* (SEQ ID No: 6).

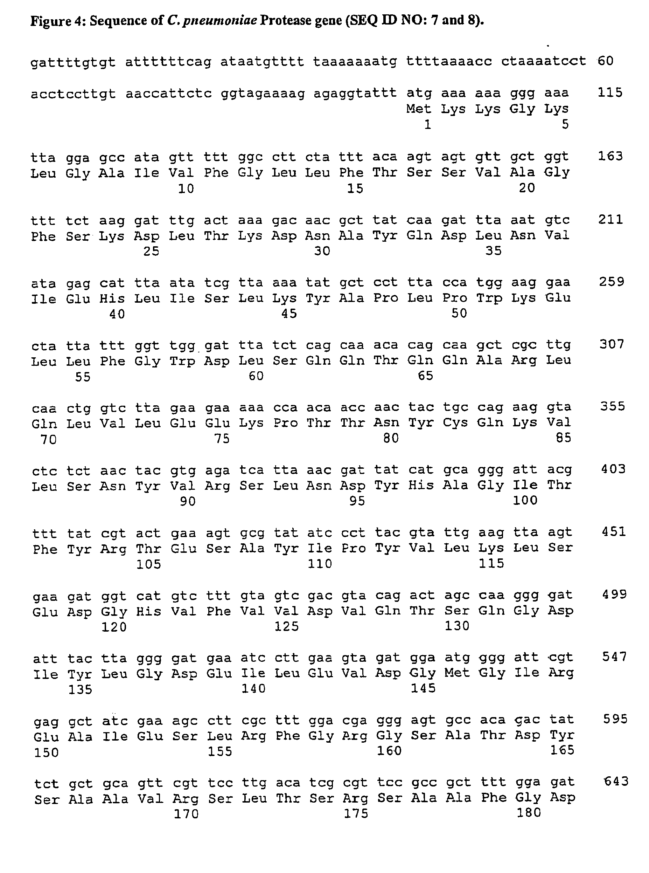

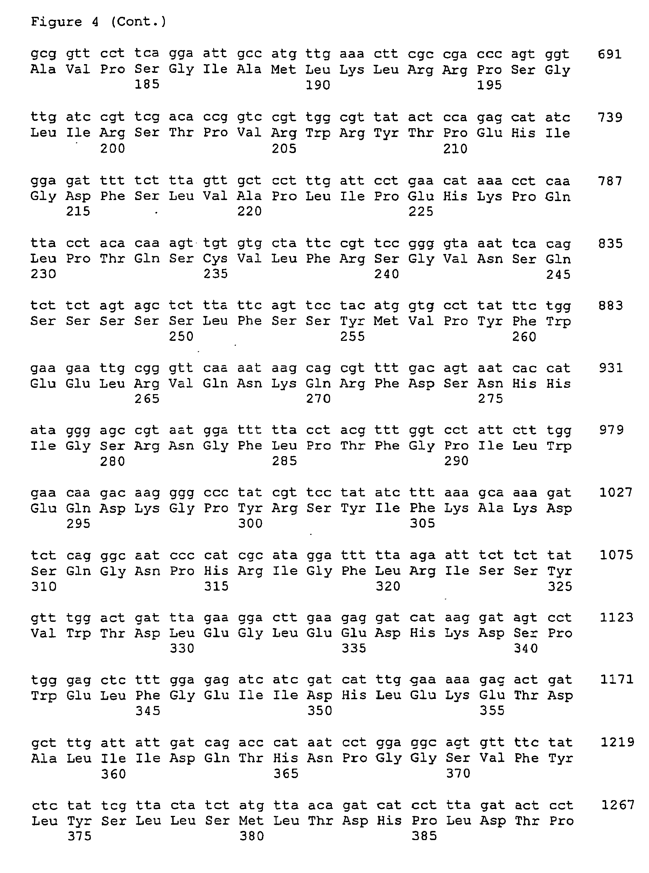

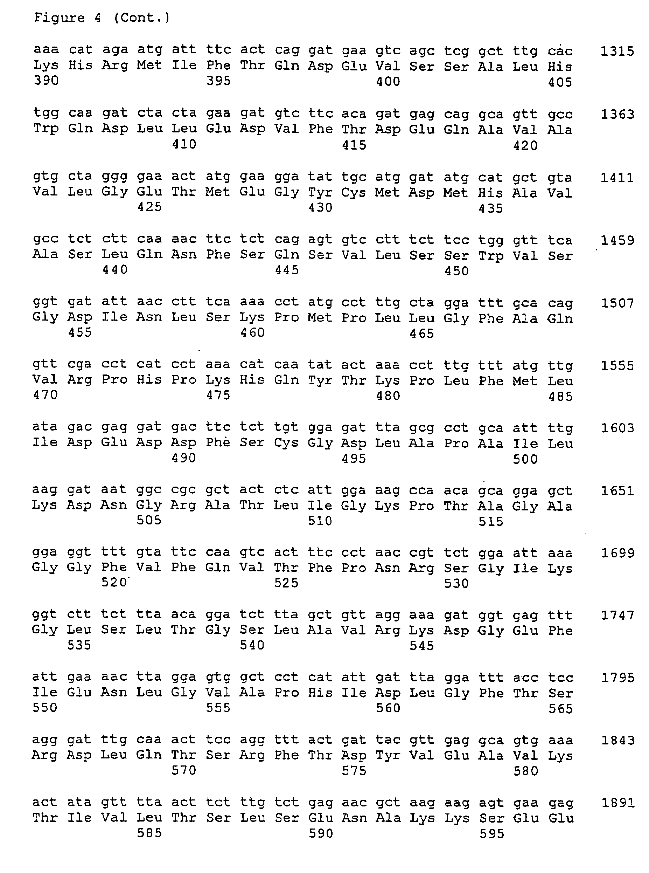

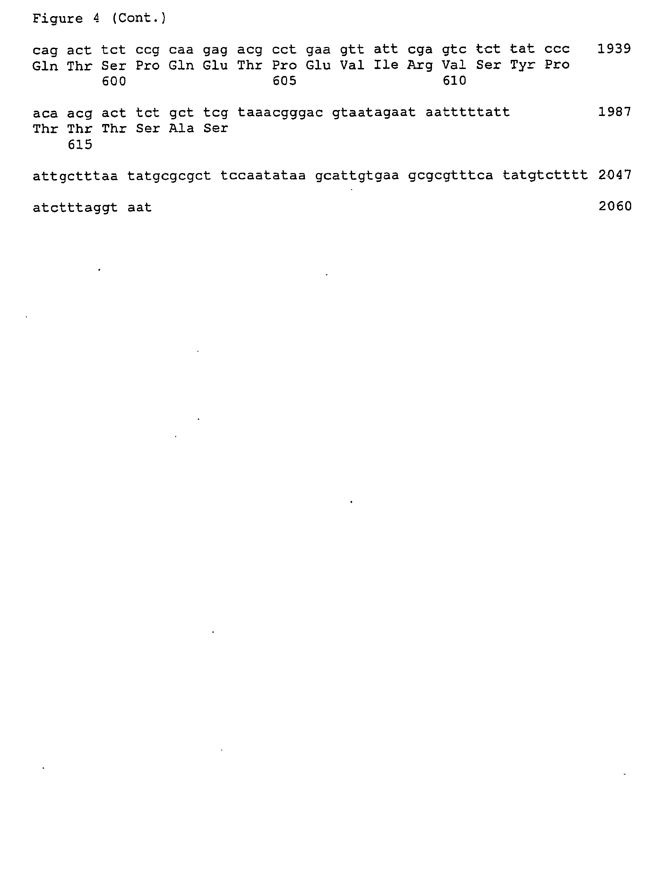

Figure 4 shows the nucleotide sequence of the gene encoding a protease (SEQ ID No: 7) and the deduced amino acid sequence of the protease from *Chlamydia pneumoniae* (SEQ ID No: 8).

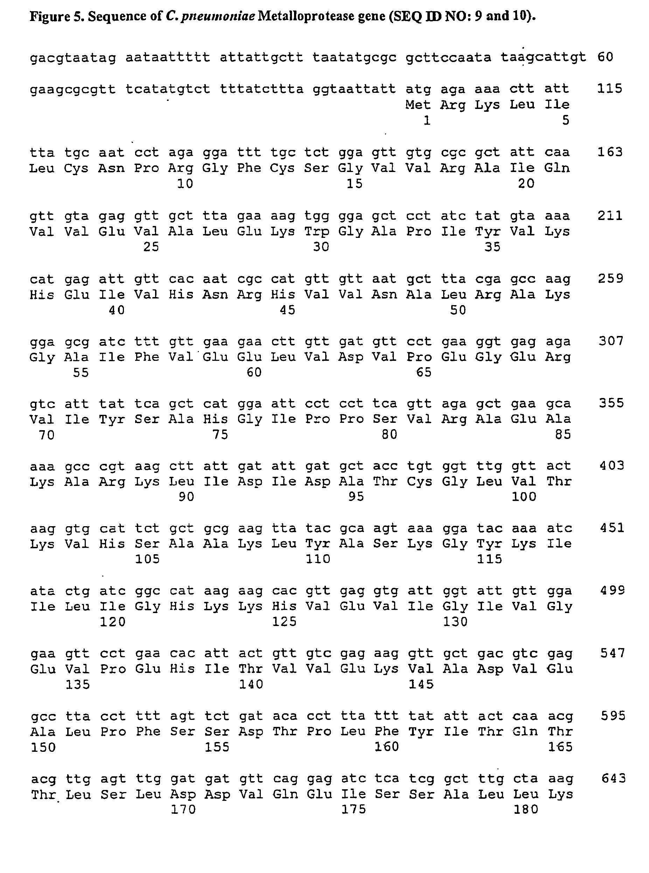

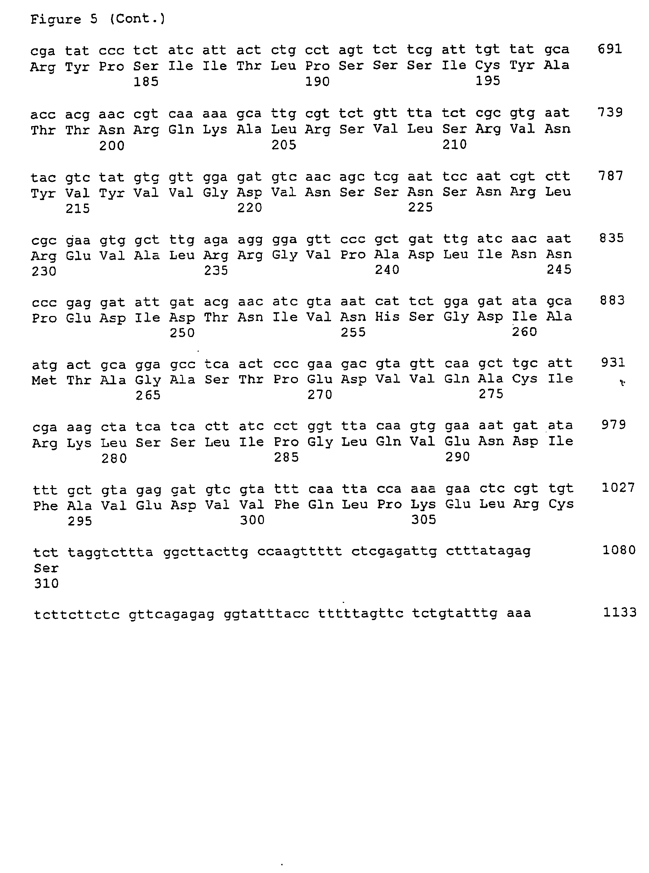

Figure 5 shows the nucleotide sequence of the gene encoding a metalloprotease (SEQ ID No: 9) and the deduced amino acid sequence of the metalloprotease from *Chlamydia pneumoniae* (SEQ ID No: 10).

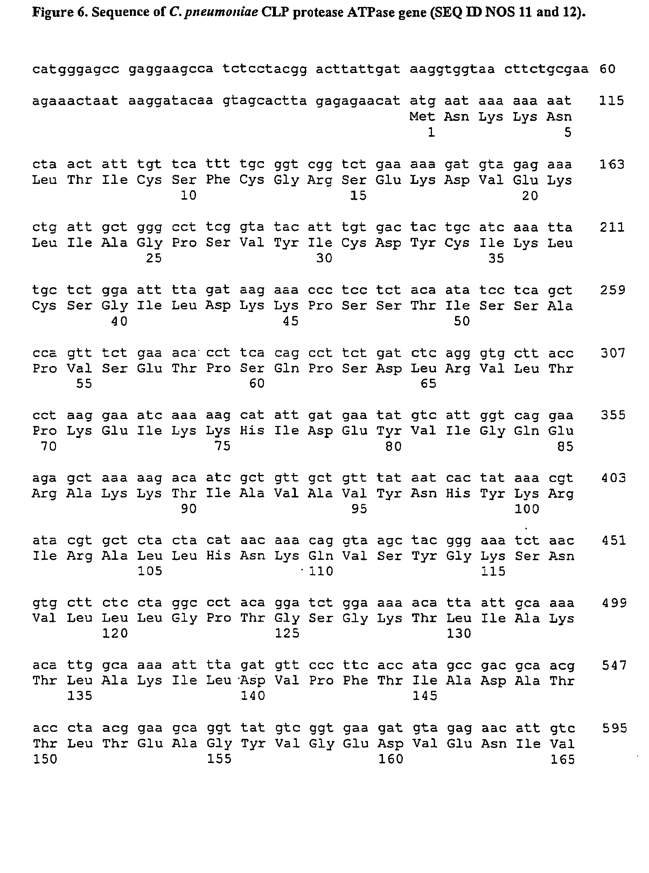

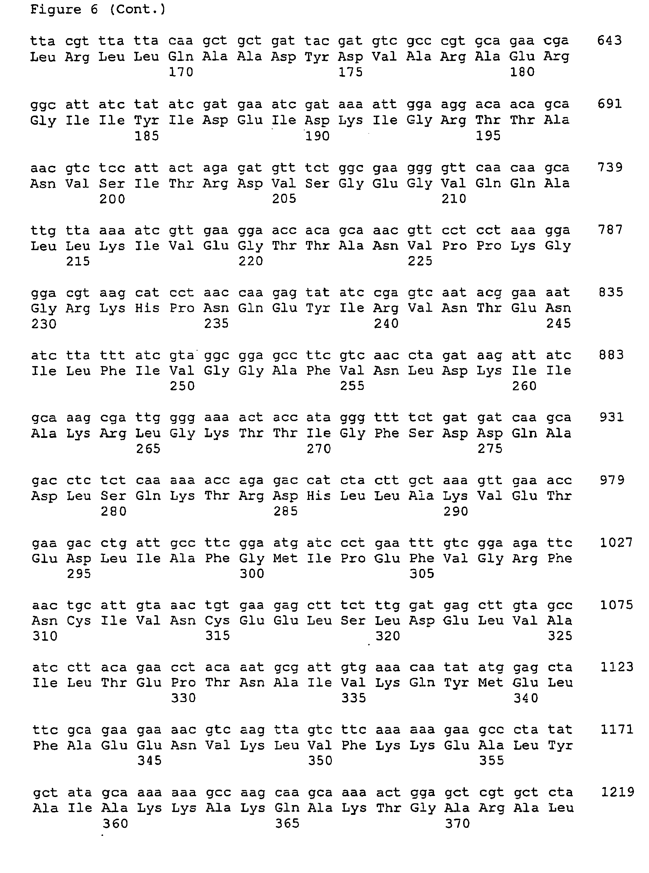

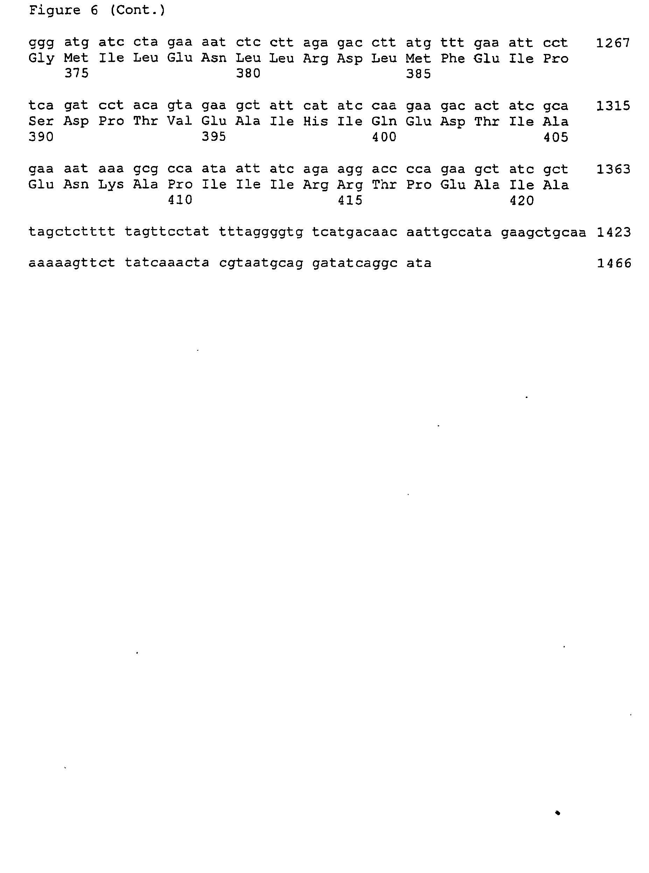

Figure 6 shows the nucleotide sequence of the gene encoding CLP protease ATPase (SEQ ID No: 11) and the deduced amino acid sequence of the CLP protease ATPase from *Chlamydia pneumoniae* (SEQ ID No: 12).

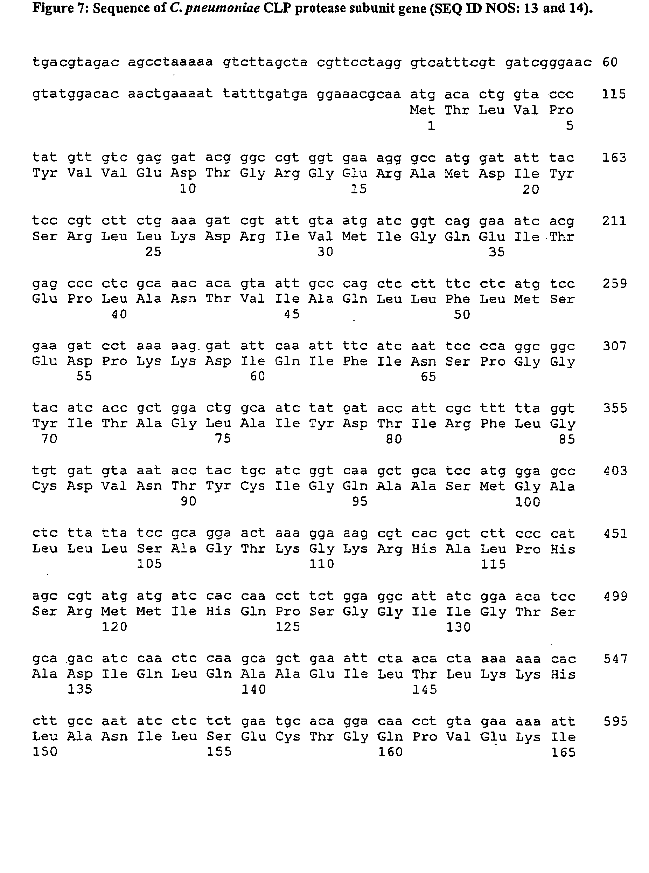

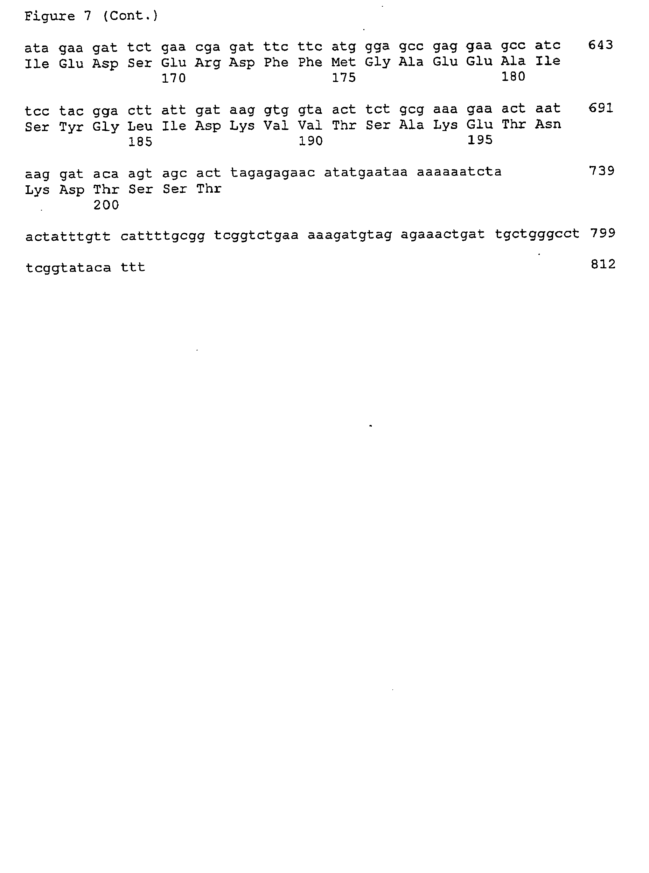

Figure 7 shows the nucleotide sequence of the gene encoding a CLP protease subunit (SEQ ID No: 13) and the deduced amino acid sequence of the CLP protease subunit from *Chlamydia pneumoniae* (SEQ ID No: 14).

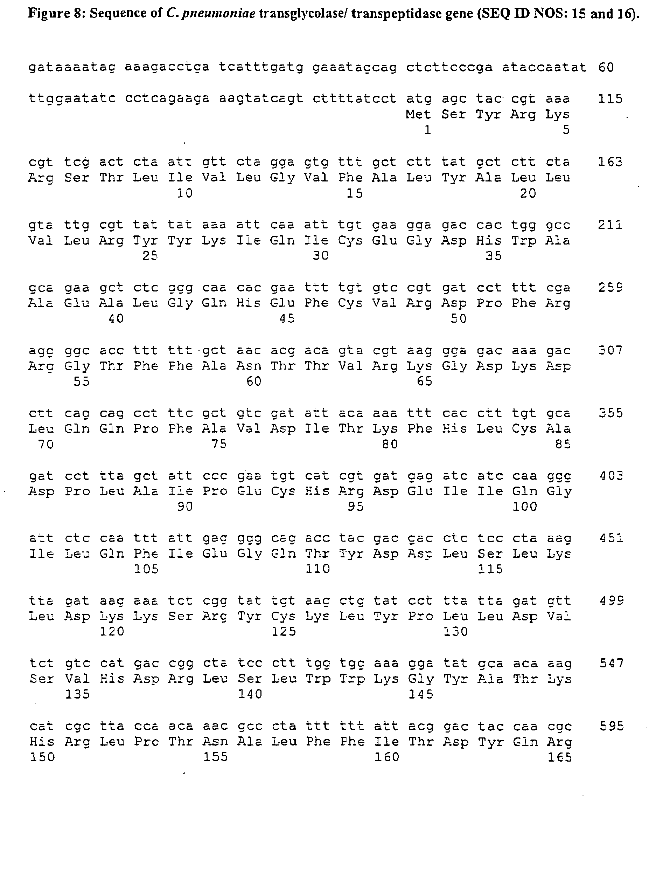

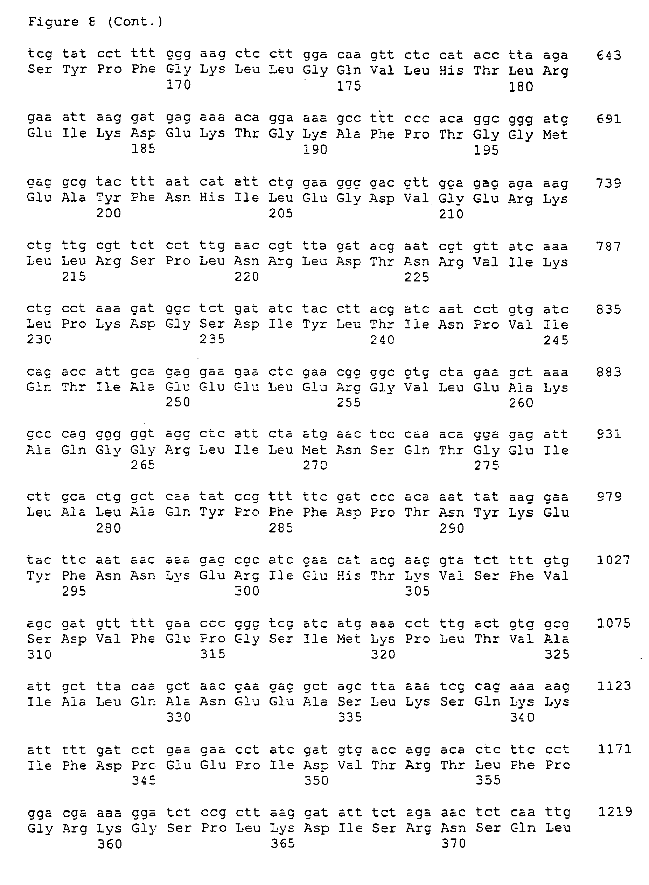

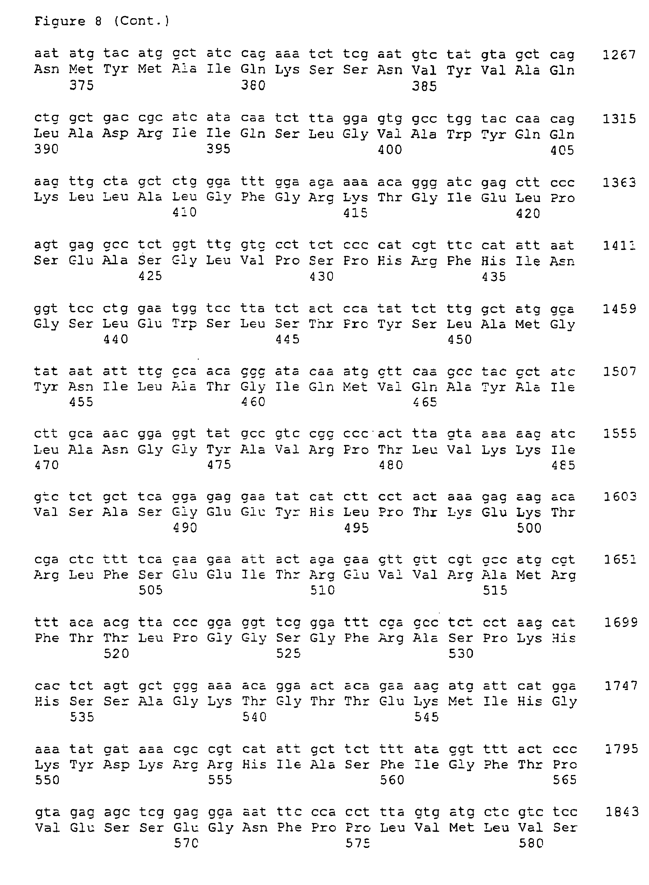

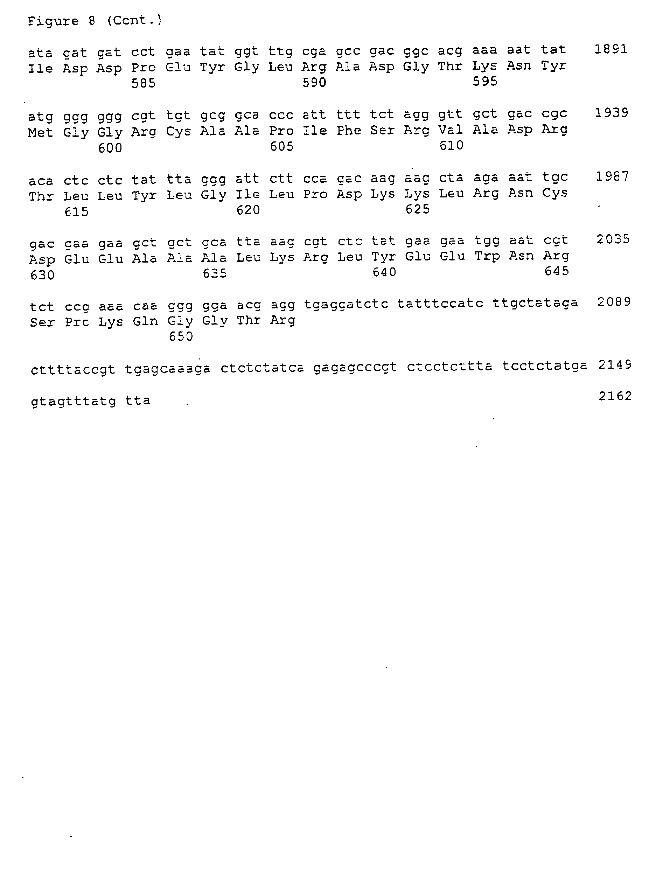

Figure 8 shows the nucleotide sequence of the gene encoding a translycolase / transpeptidase (SEQ ID No: 15) and the deduced amino acid sequence of the transglycolase / transpeptidase from *Chlamydia pneumoniae* (SEQ ID No: 16).

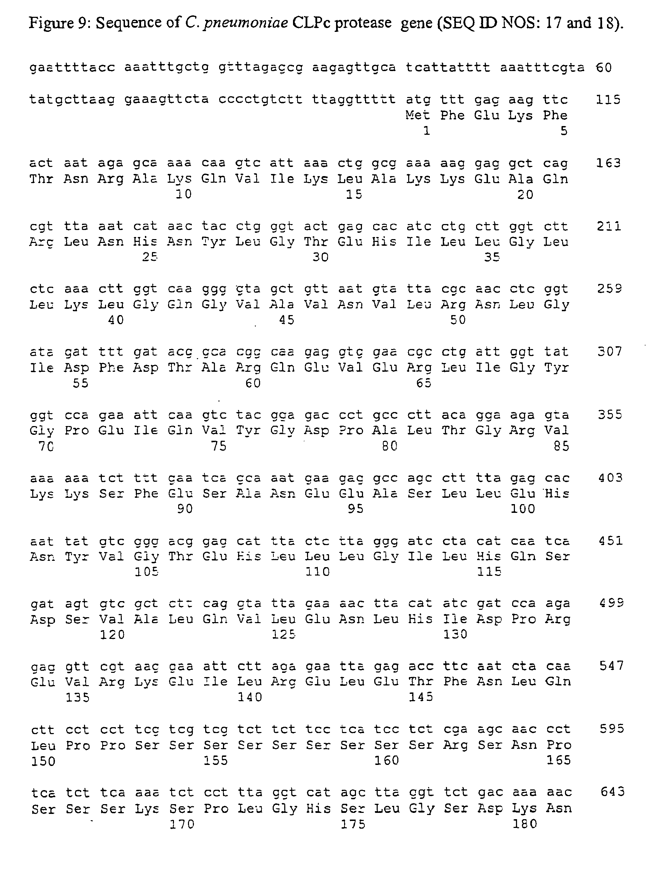

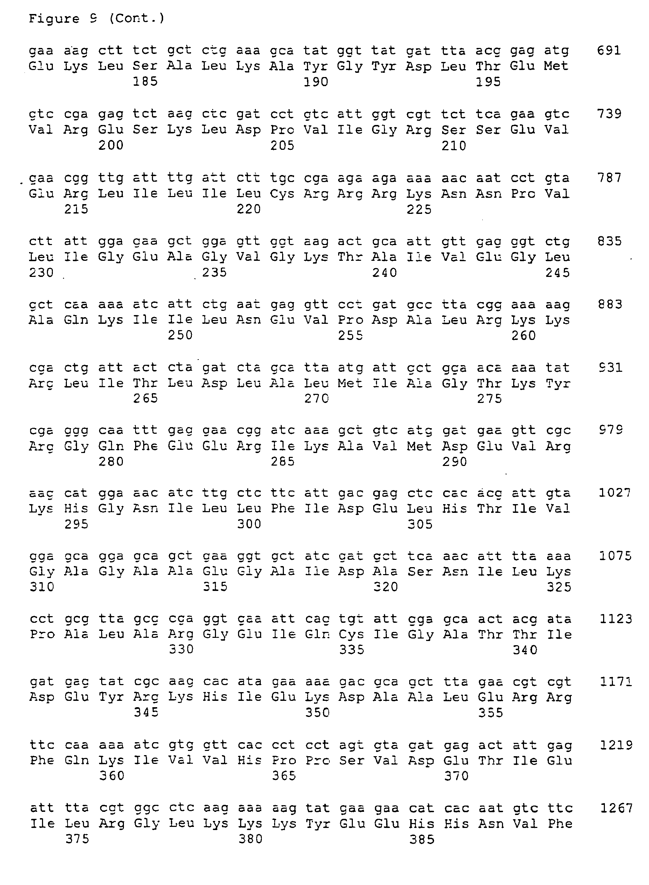

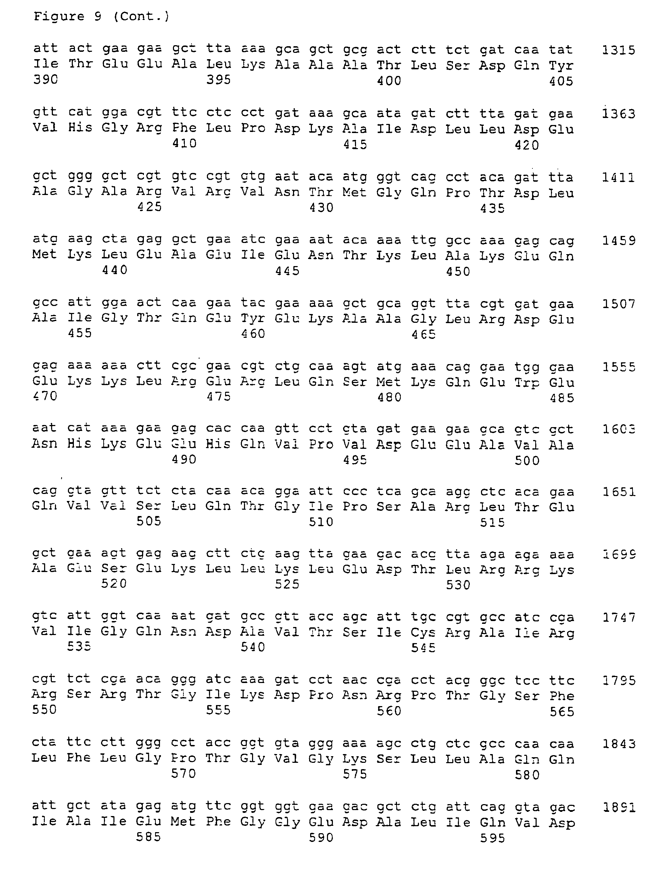

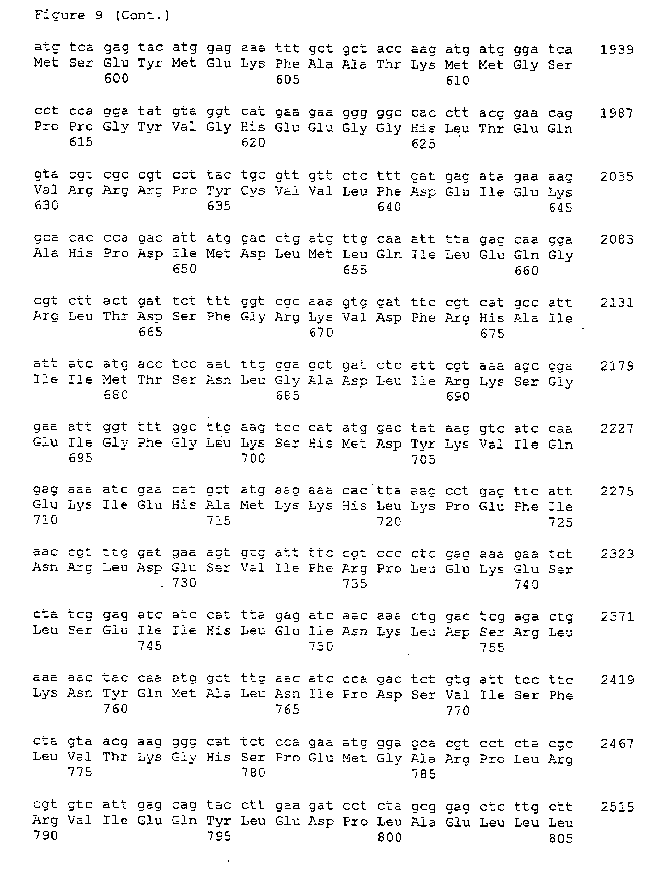

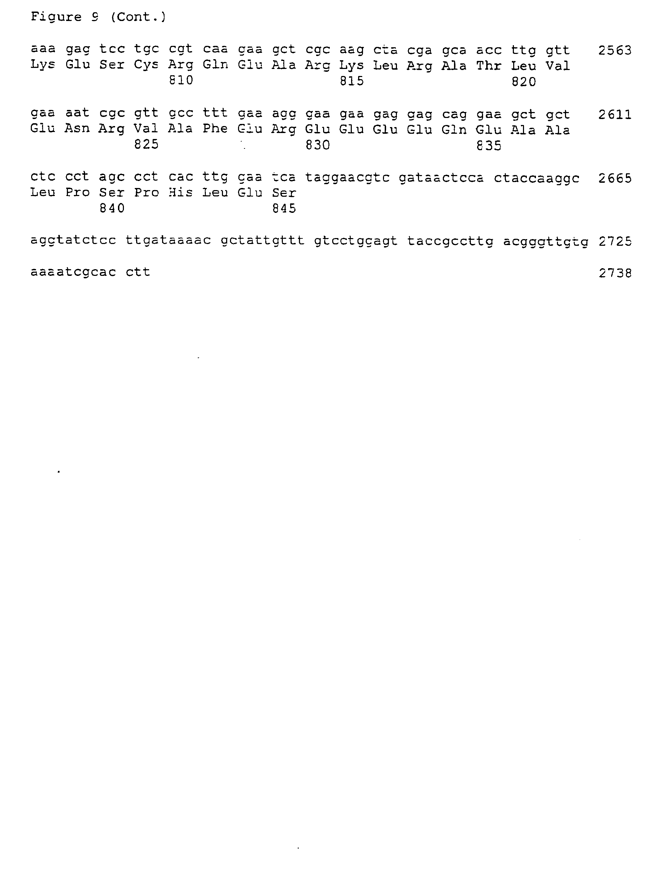

Figure 9 shows the nucleotide sequence of the gene encoding a CLPc protease(SEQ ID No: 17) and the deduced amino acid sequence of the CLPc protease from *Chlamydia pneumoniae* (SEQ ID No: 18).

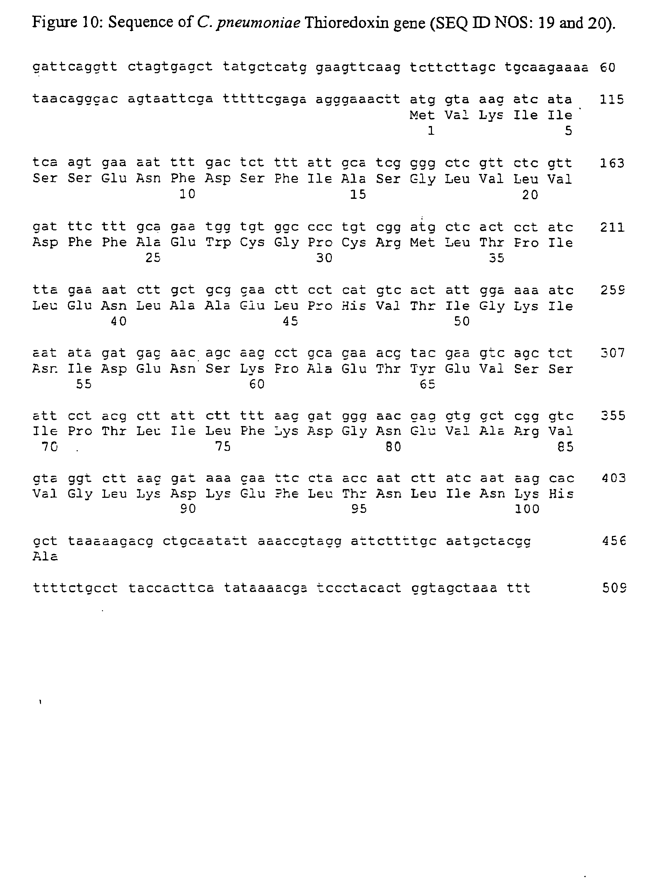

Figure 10 shows the nucleotide sequence of the gene encoding thioredoxin(SEQ ID No: 19) and the deduced amino acid sequence of thioredoxin from *Chlamydia pneumoniae* (SEQ ID No: 20).

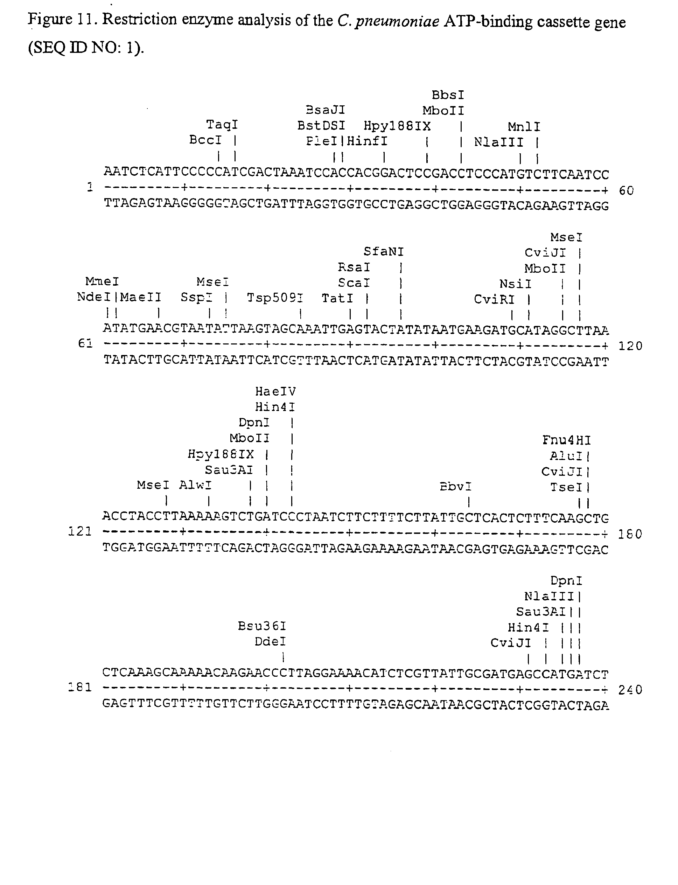

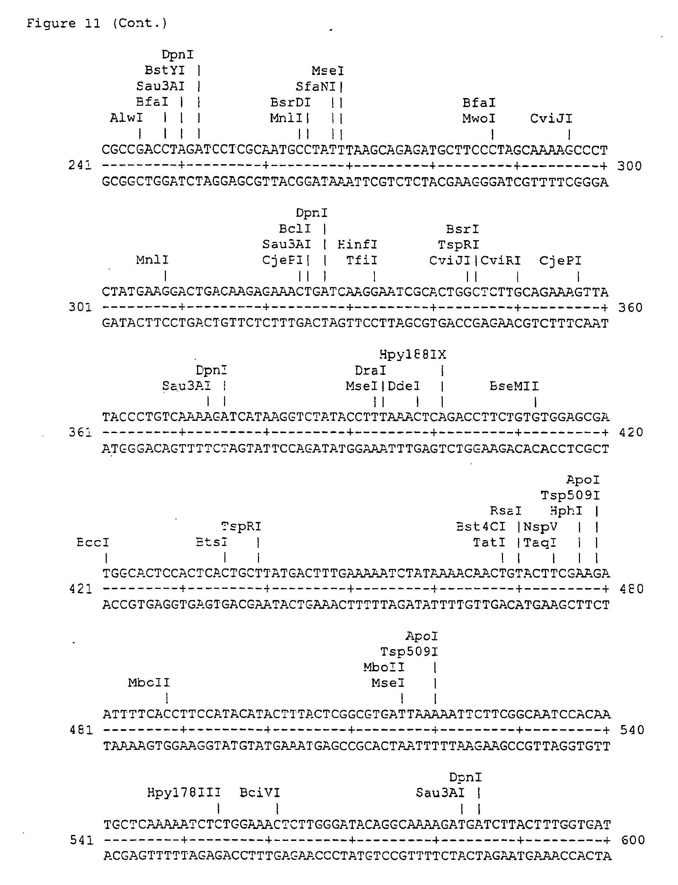

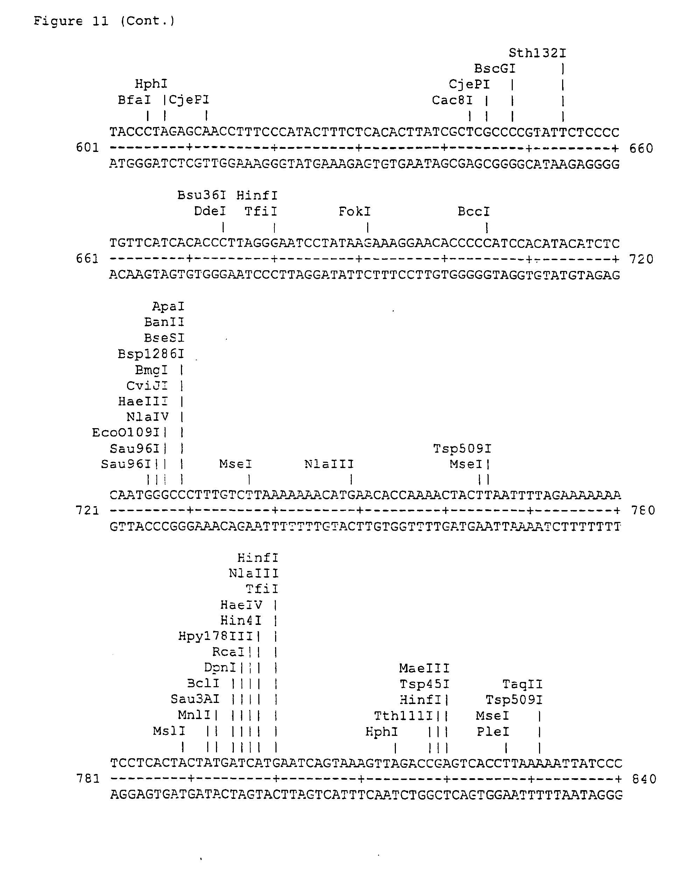

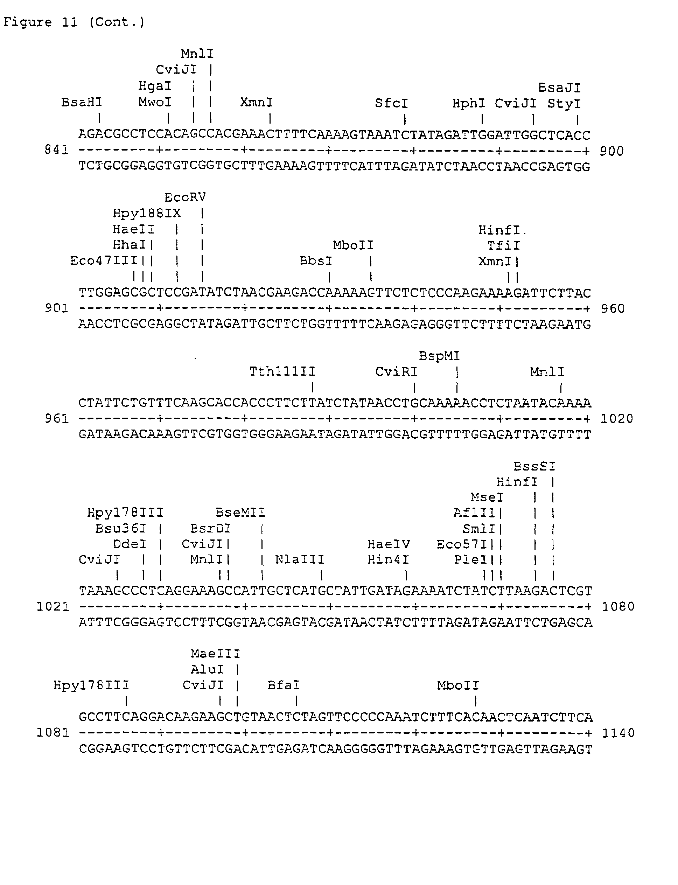

Figure 11 shows the restriction enzyme analysis of the *C. pneumoniae* gene encoding an ATP-binding cassette.

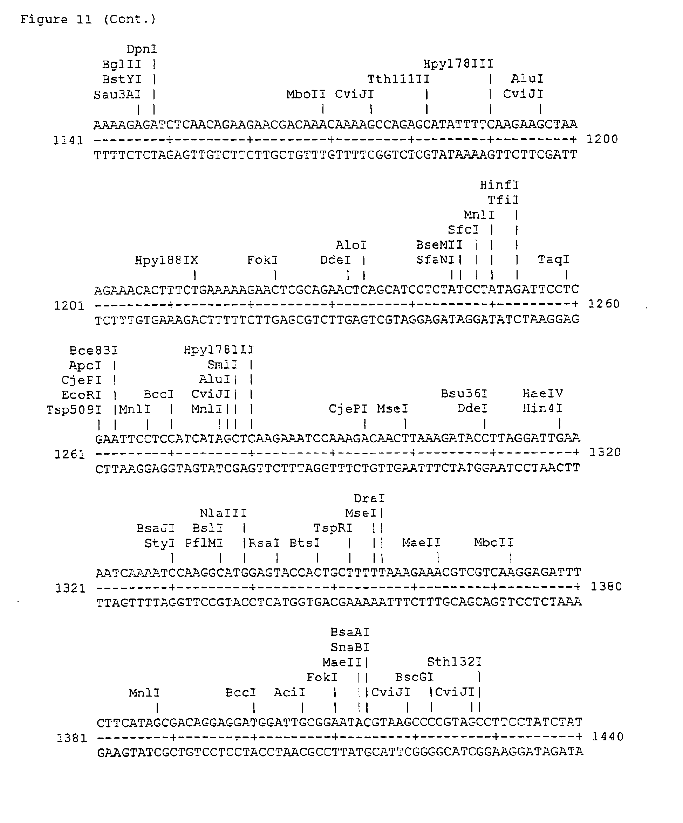

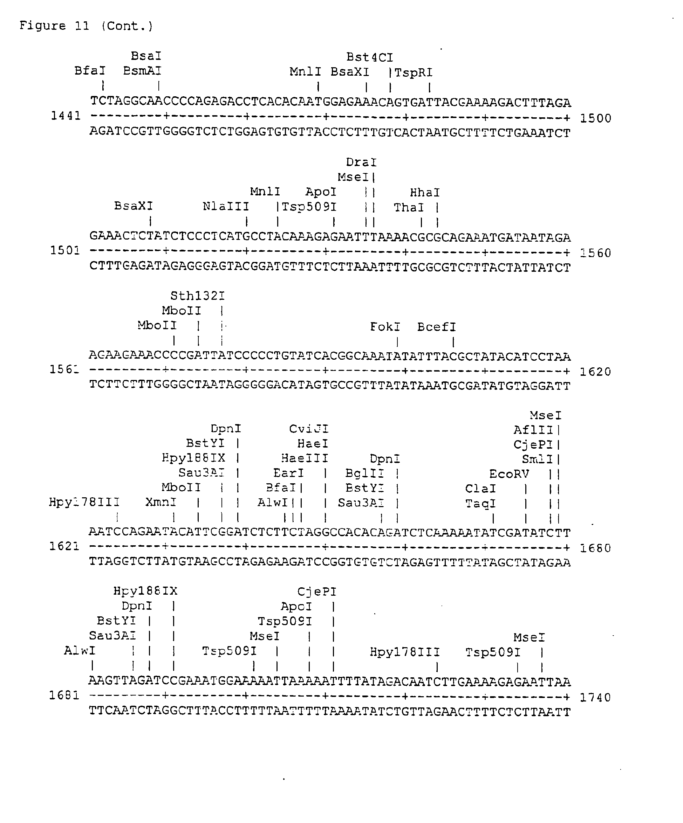

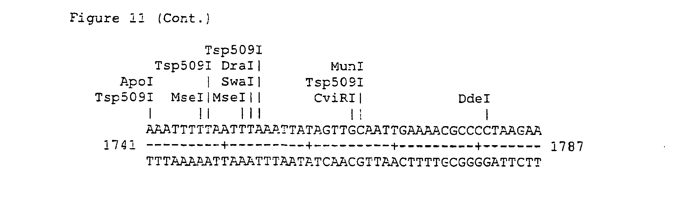

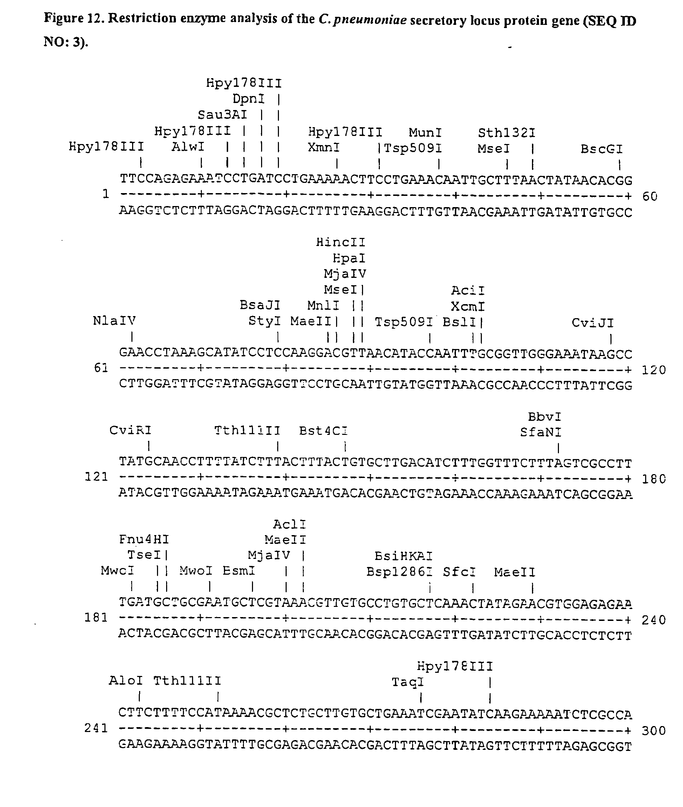

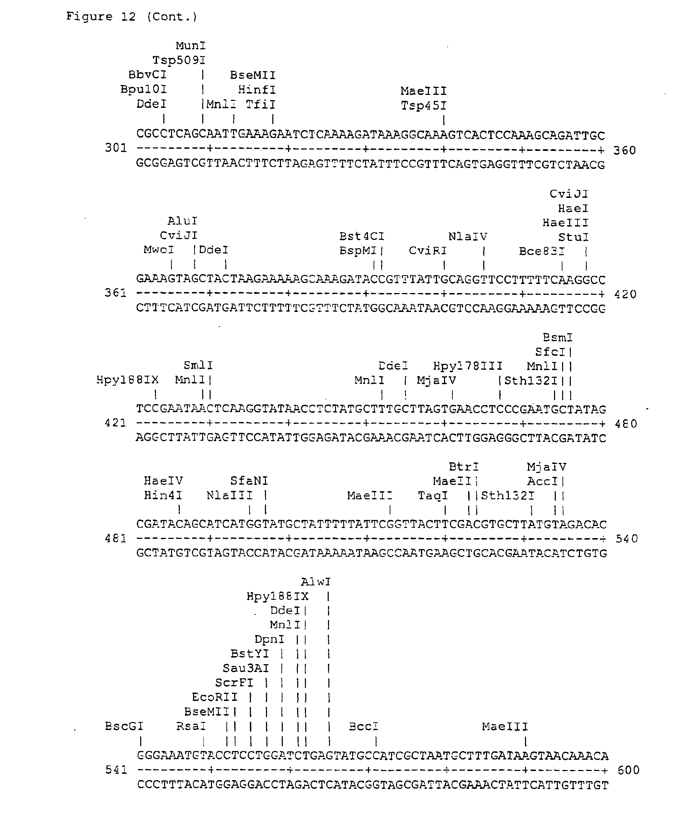

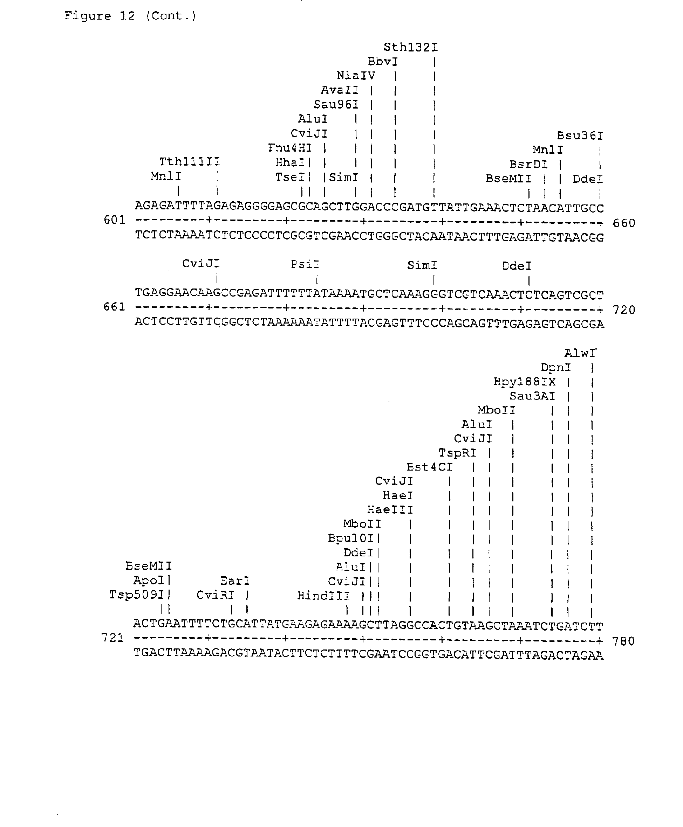

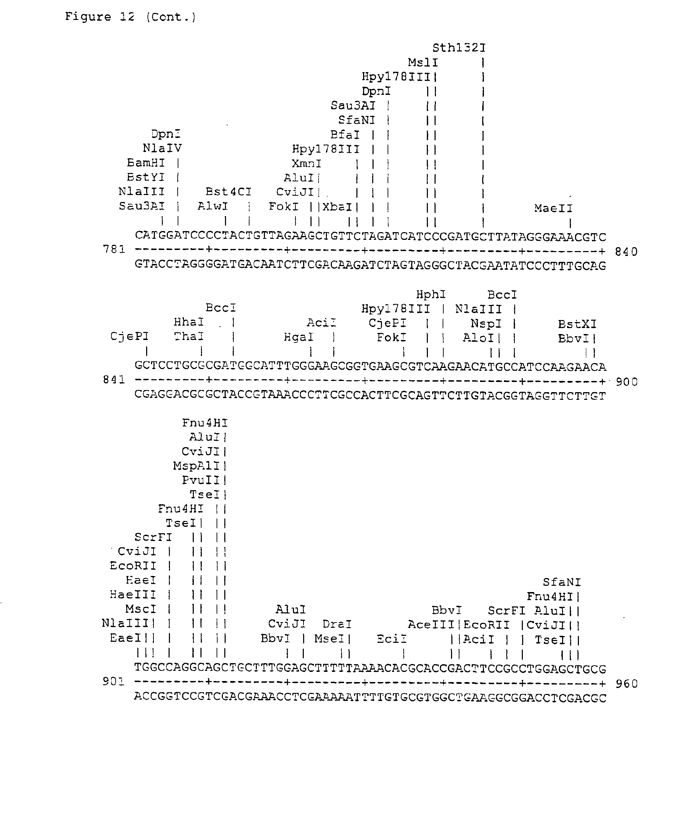

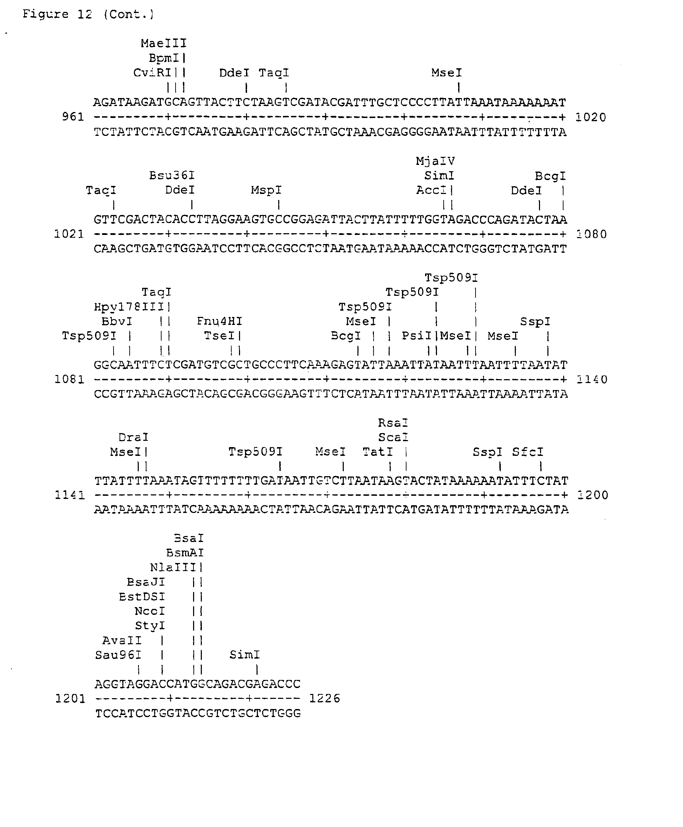

Figure 12 shows shows the restriction enzyme analysis of the *C. pneumoniae* gene encoding a secretory locus ORF.

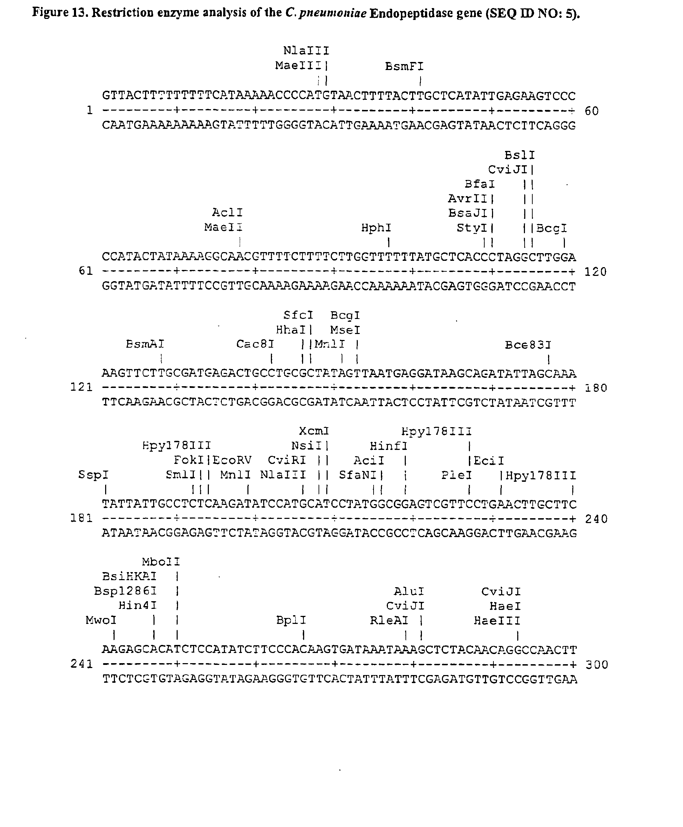

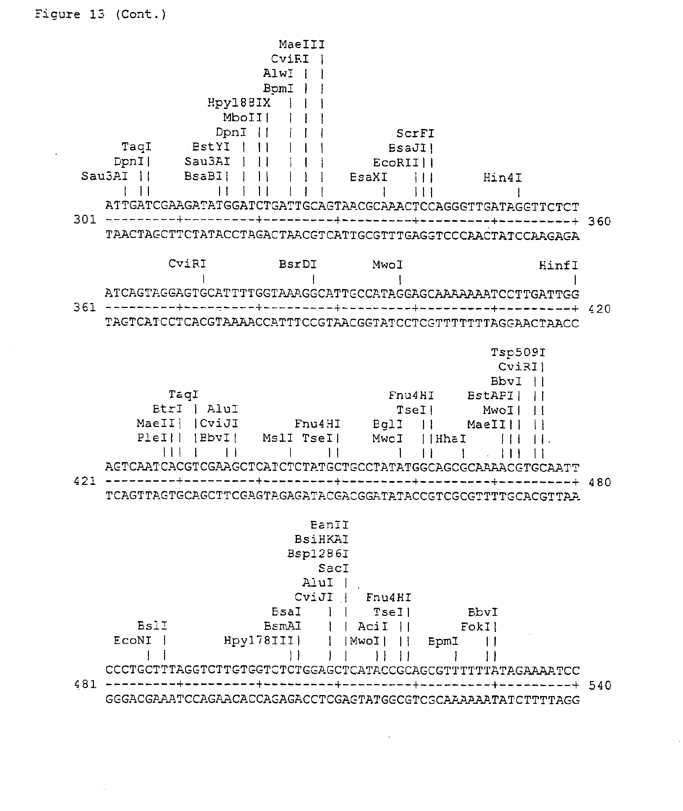

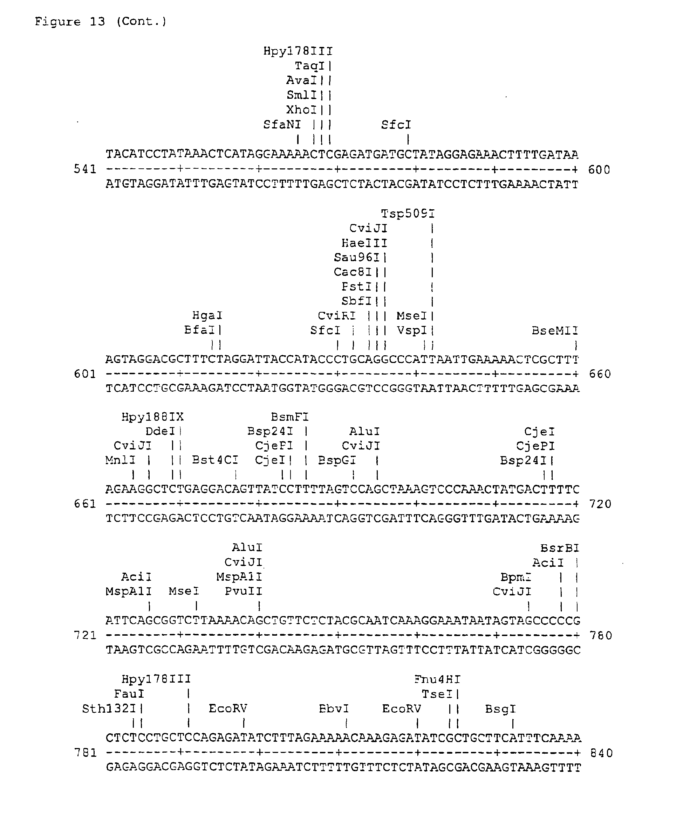

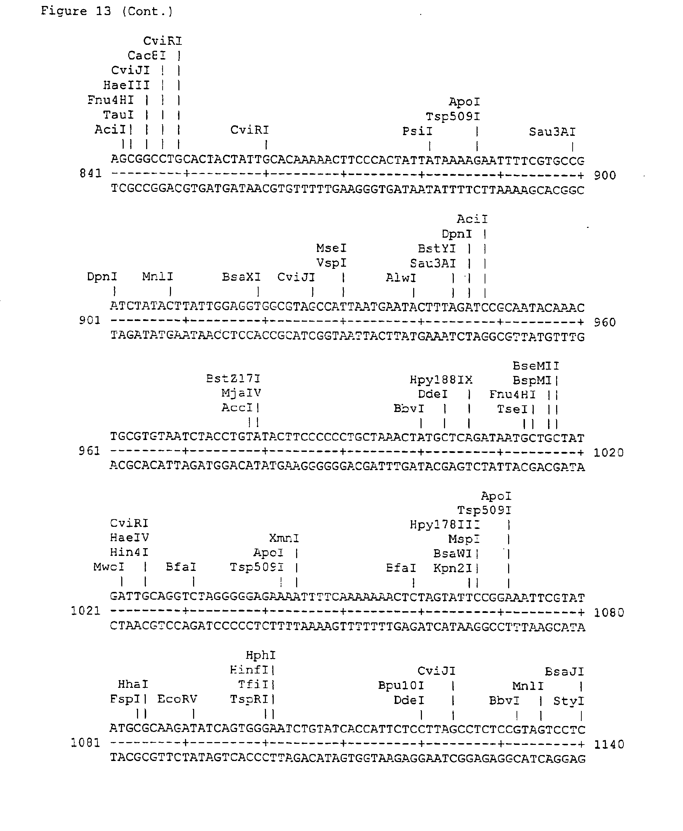

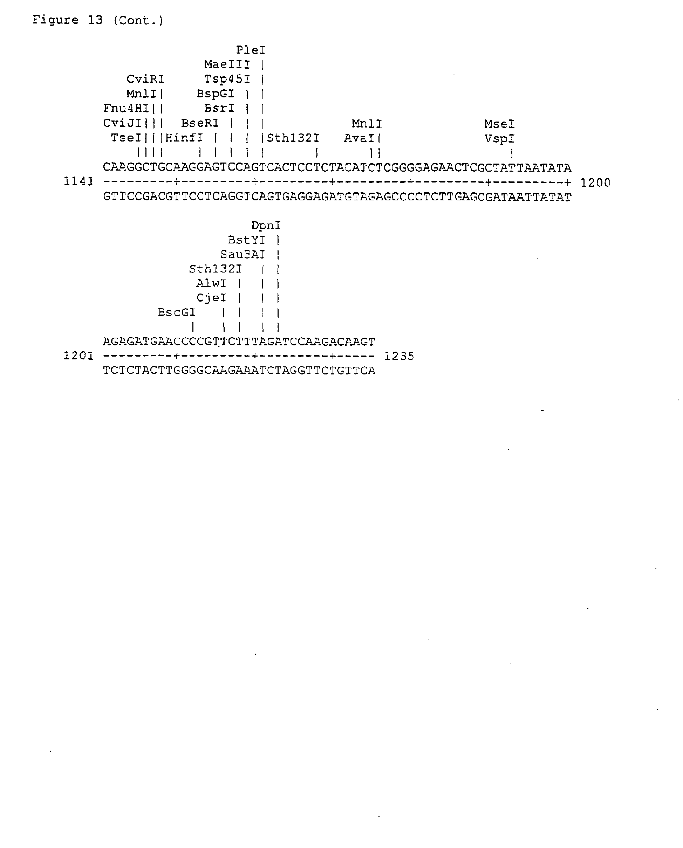

Figure 13 shows the restriction enzyme analysis of the *C. pneumoniae* gene encoding an endopeptidase.

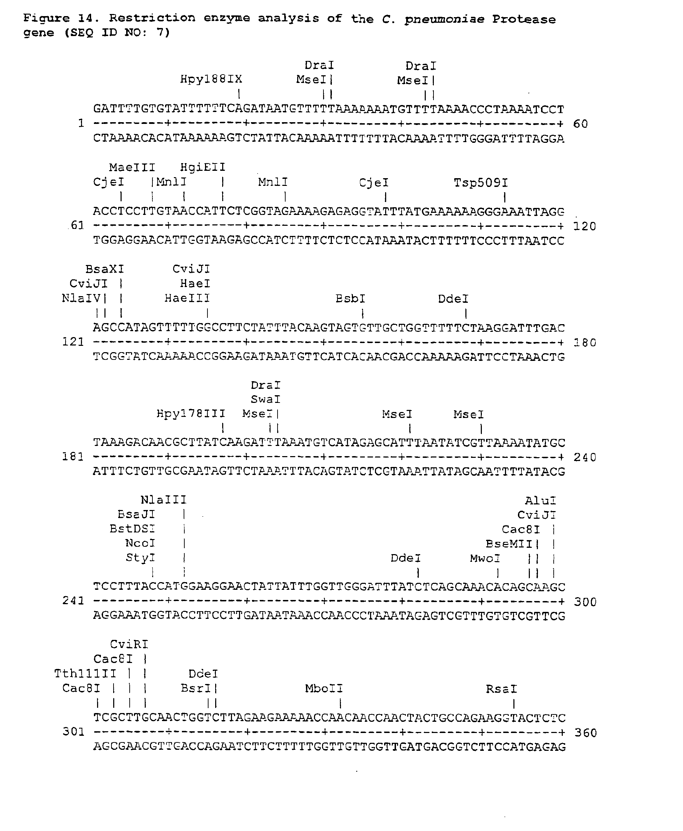

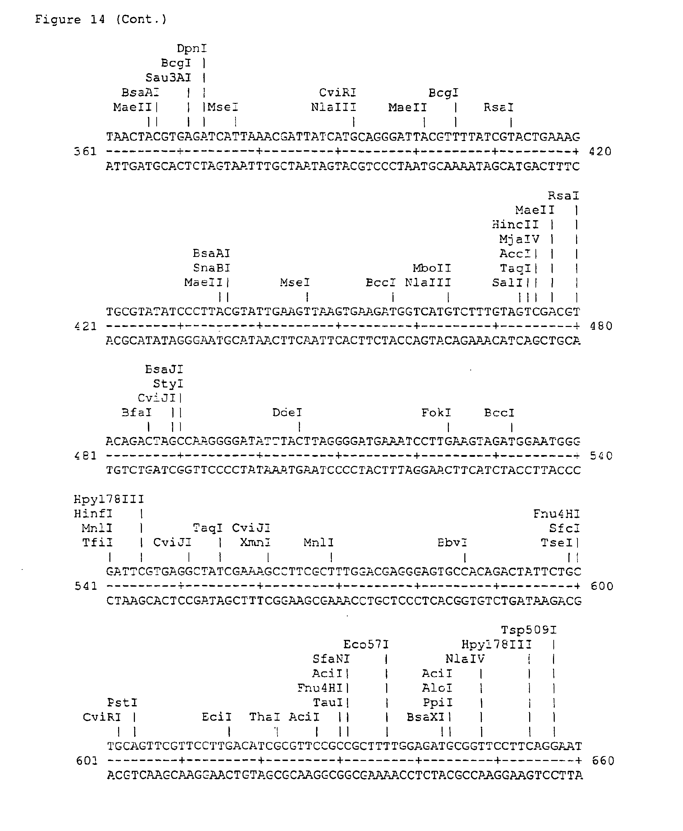

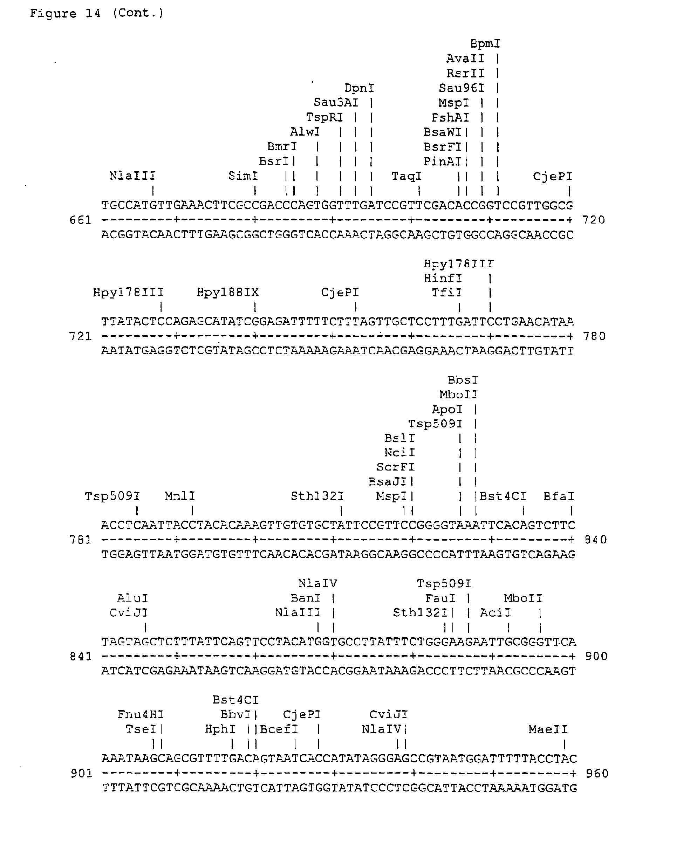

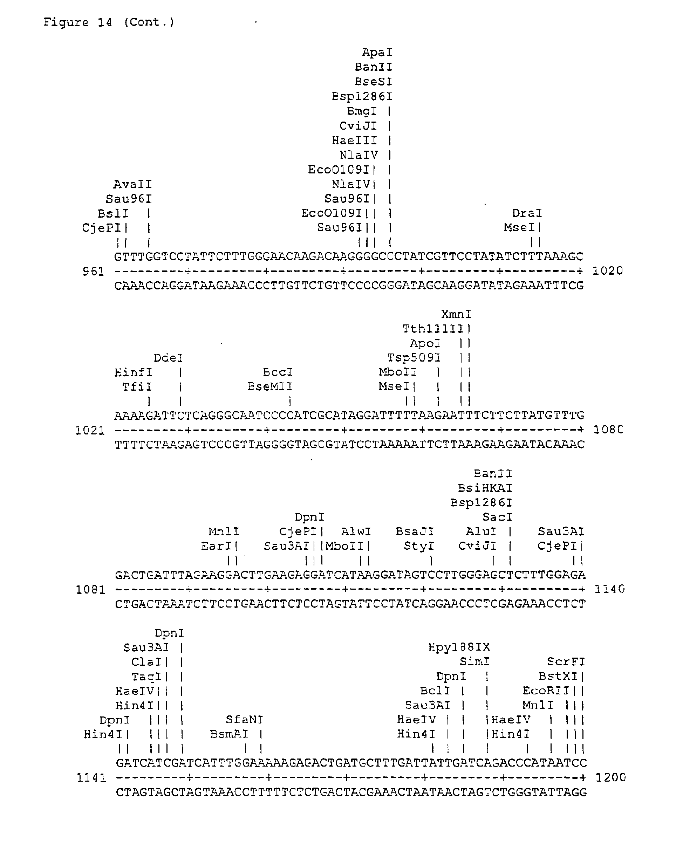

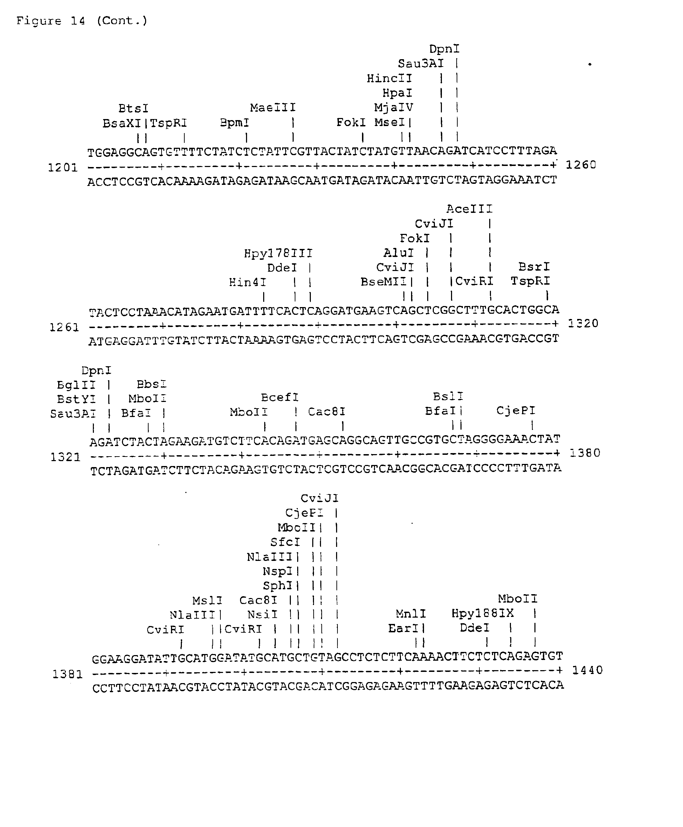

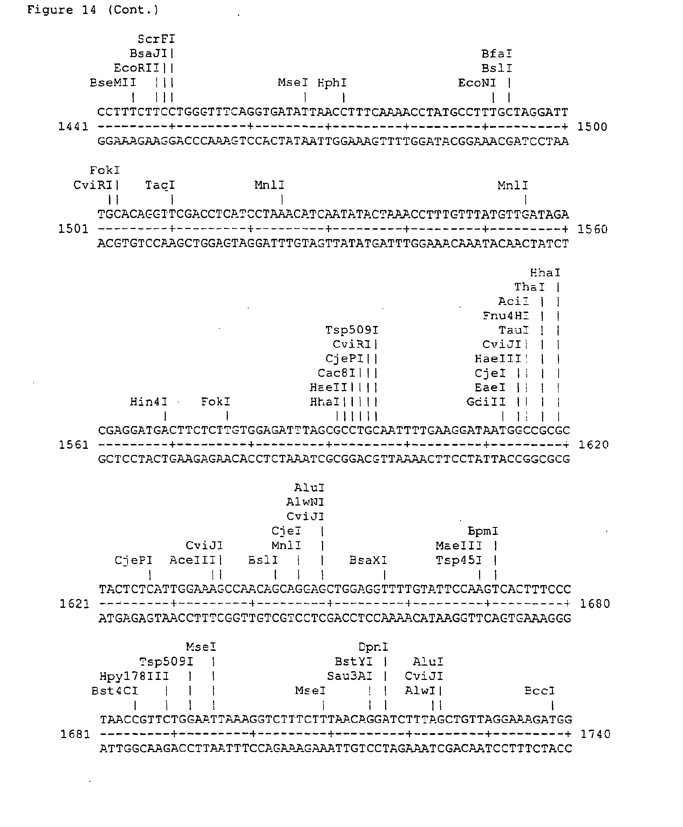

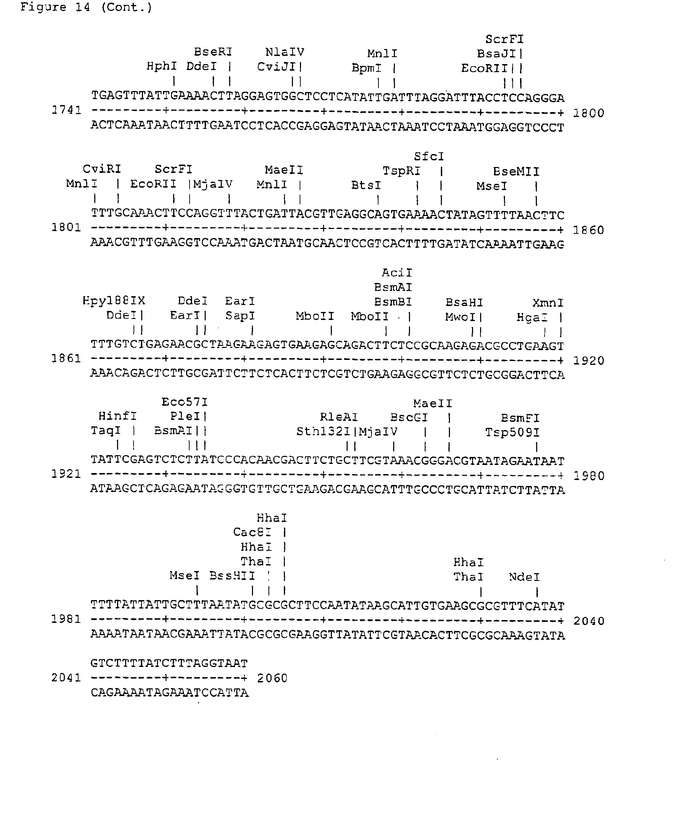

Figure 14 shows the restriction enzyme analysis of the *C. pneumoniae* gene encoding a protease.

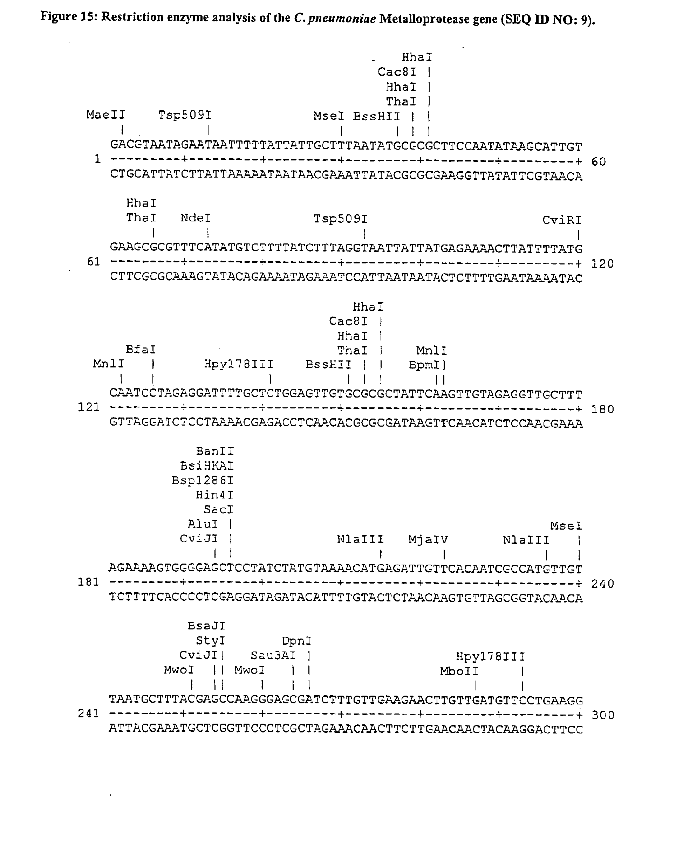

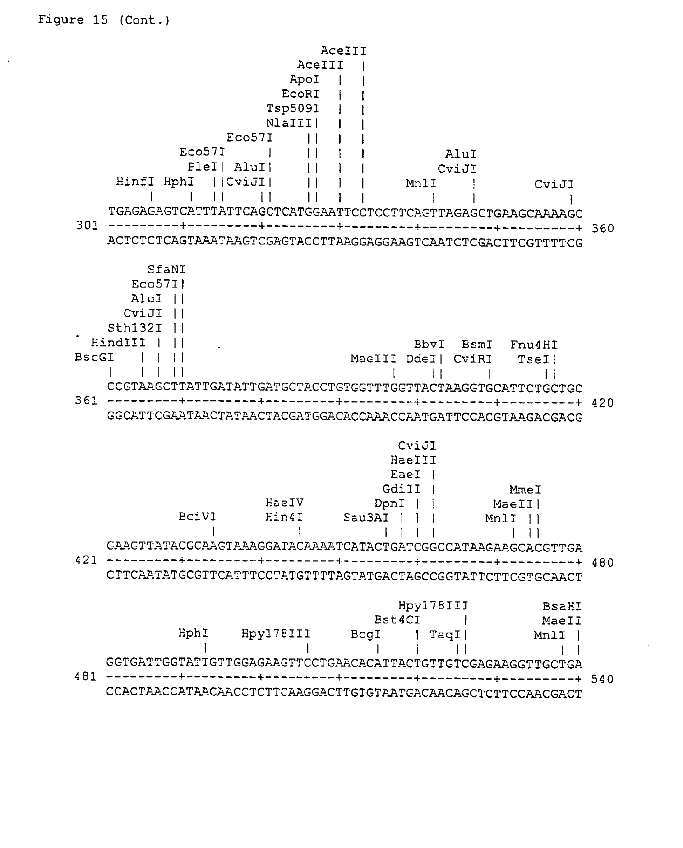

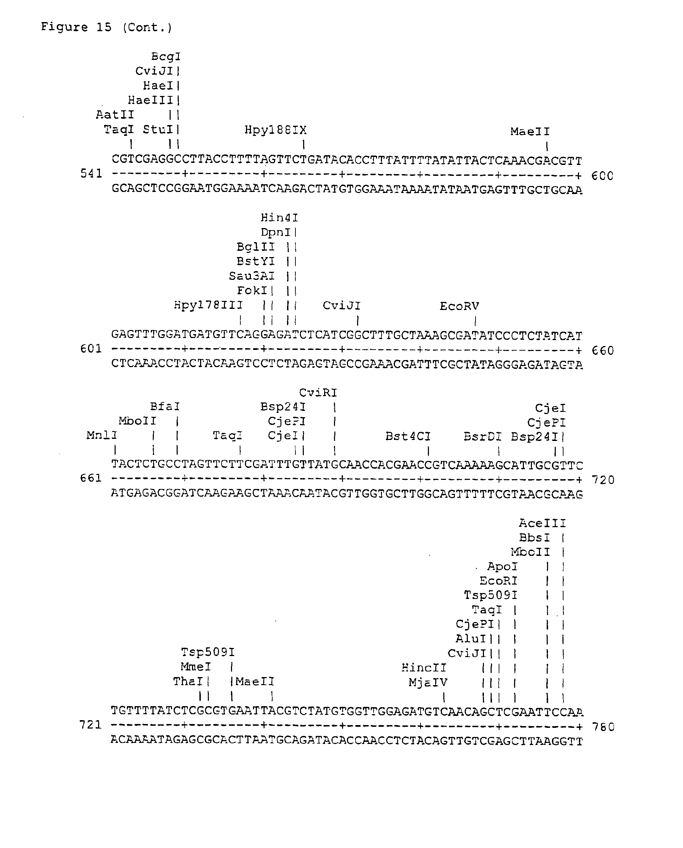

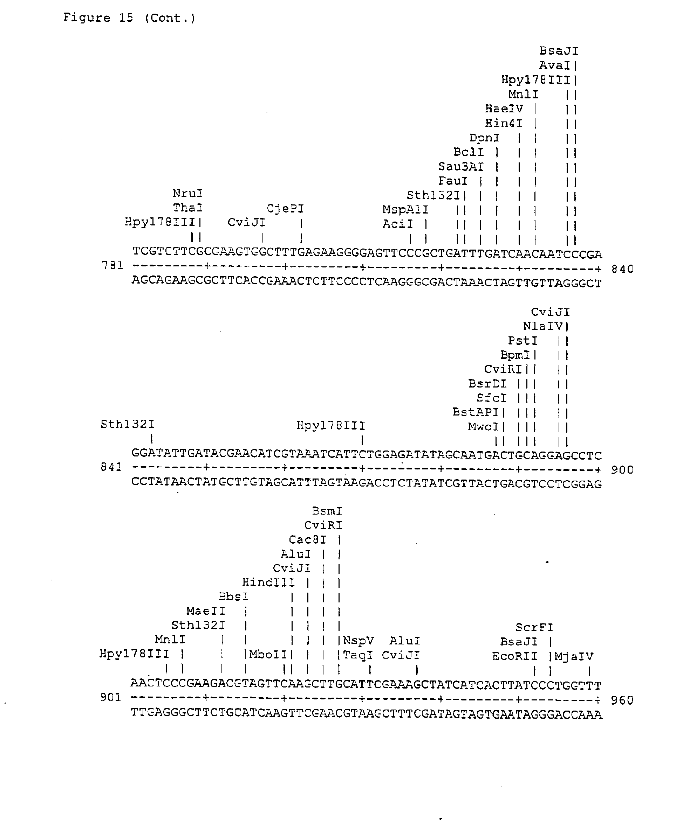

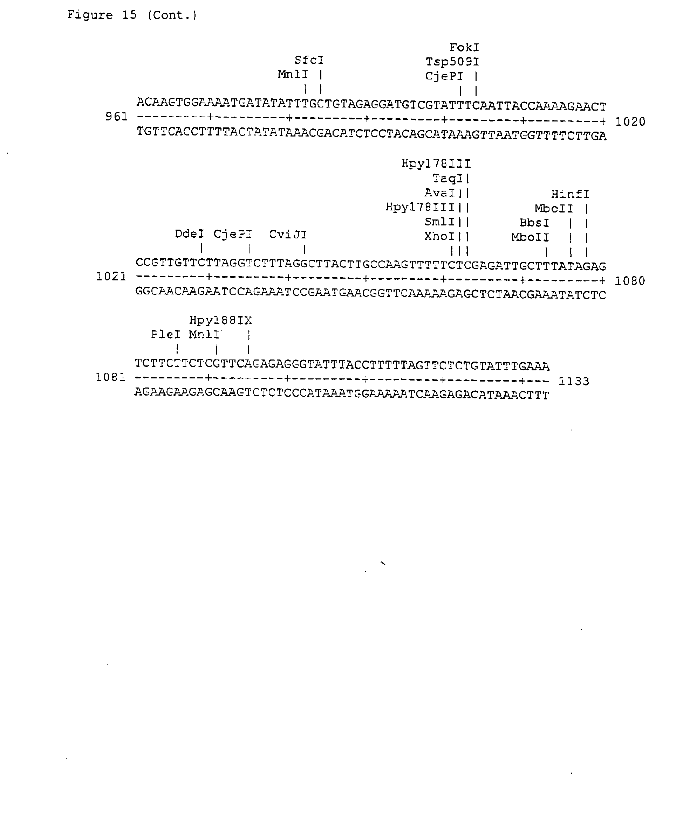

Figure 15 shows the restriction enzyme analysis of the *C. pneumoniae* gene encoding a metalloprotease.

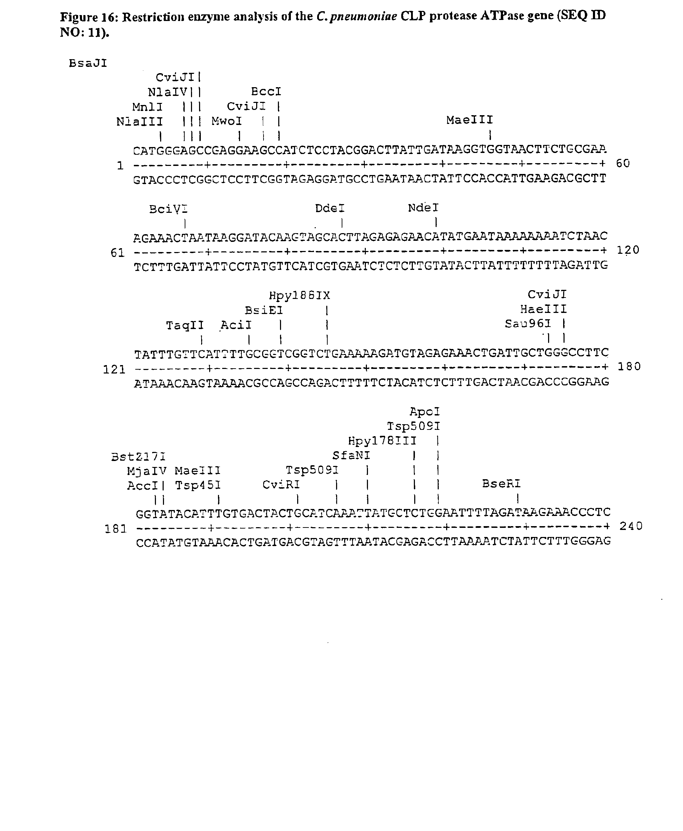

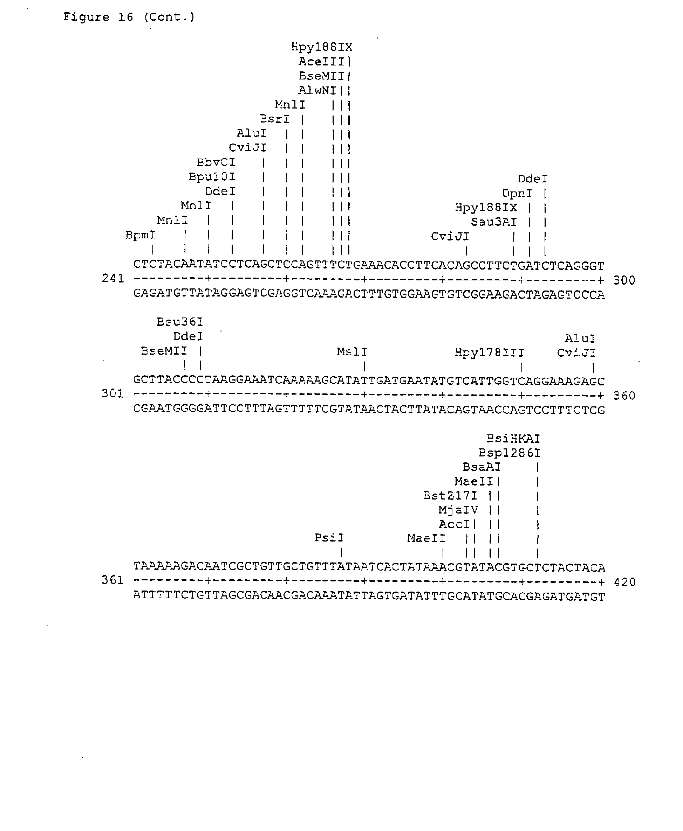

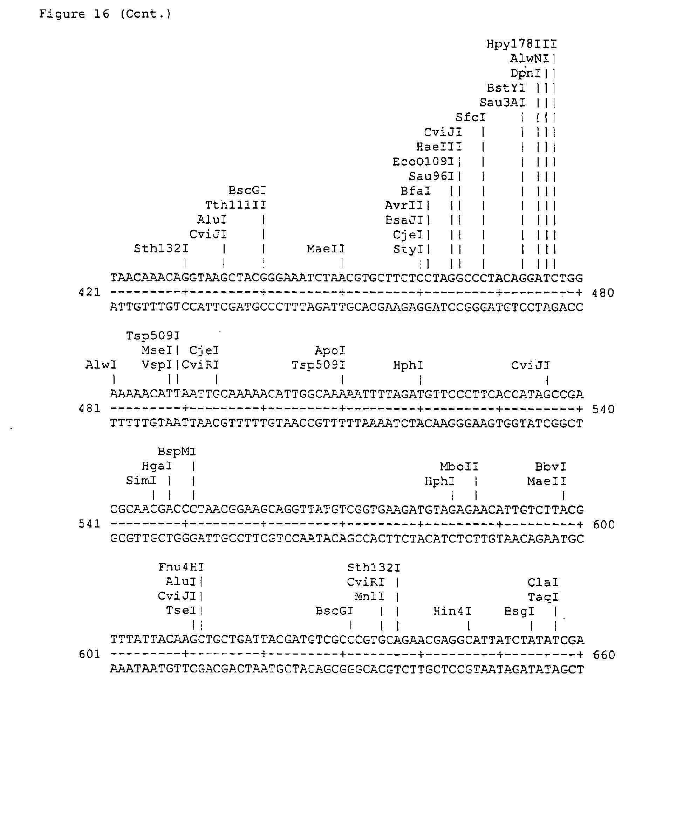

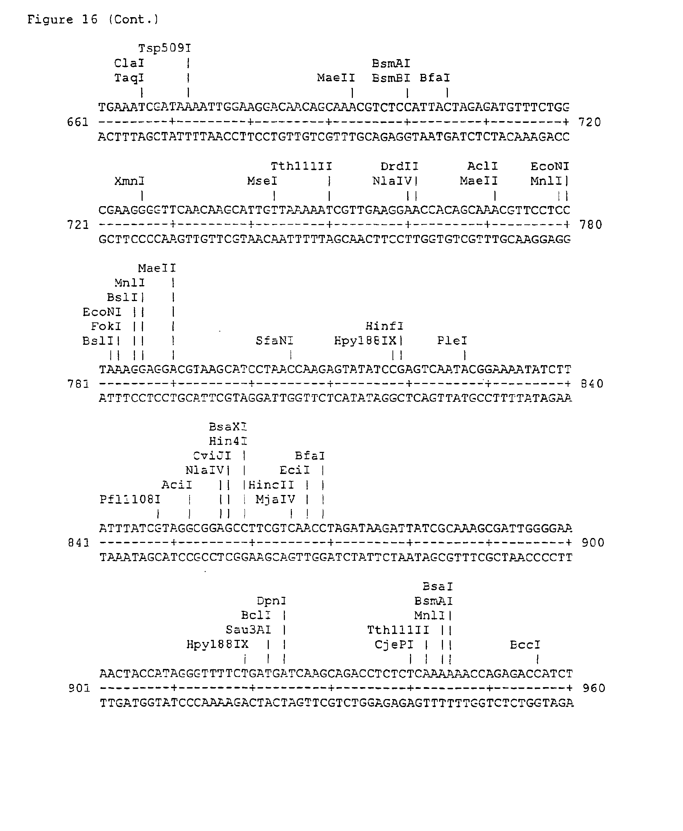

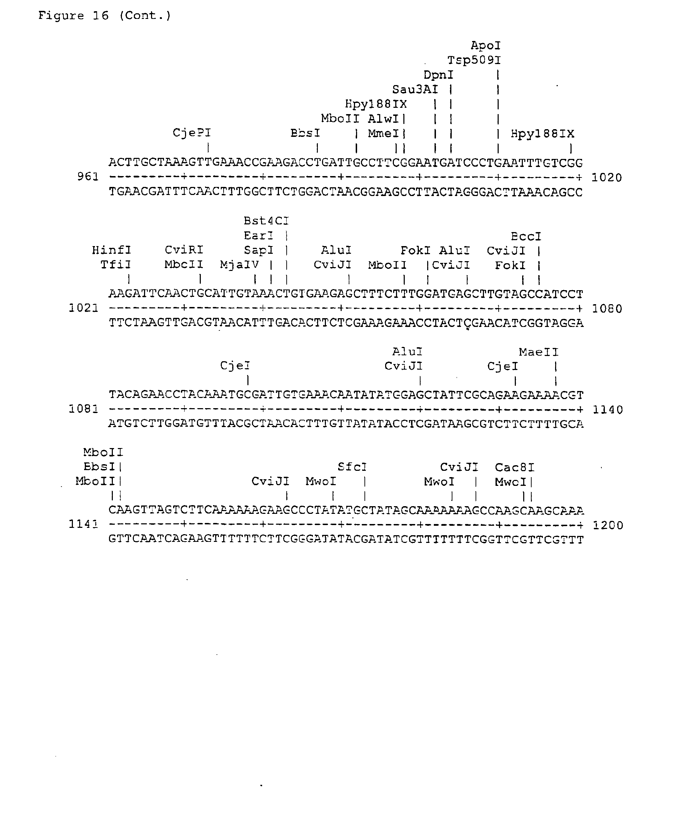

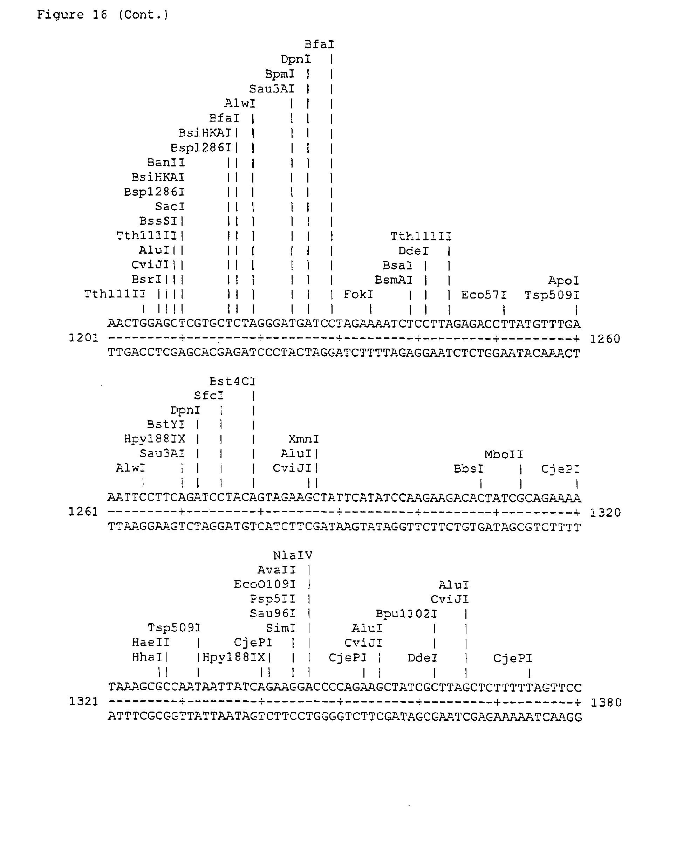

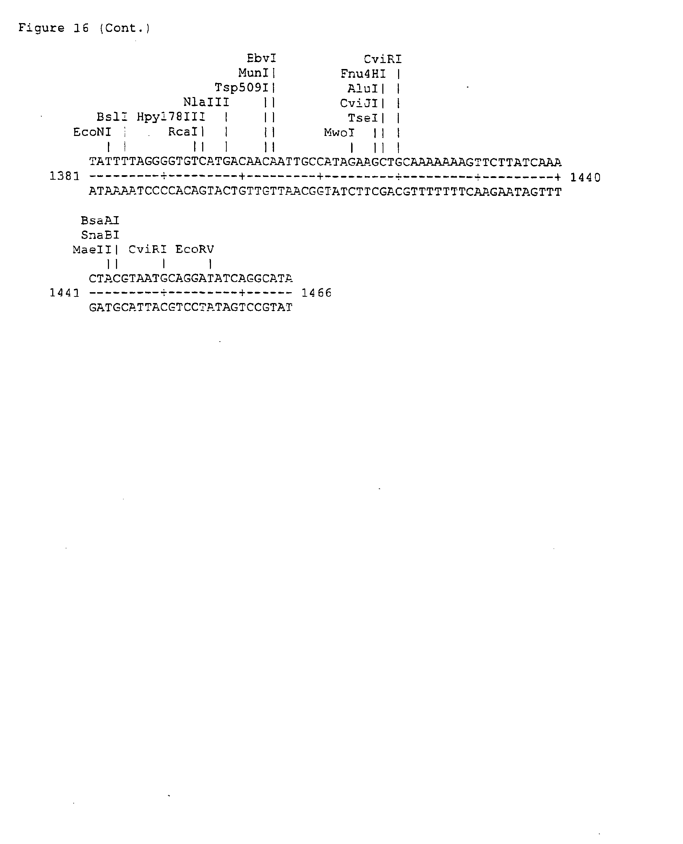

Figure 16 shows the restriction enzyme analysis of the *C. pneumoniae* gene encoding CLP protease ATPase.

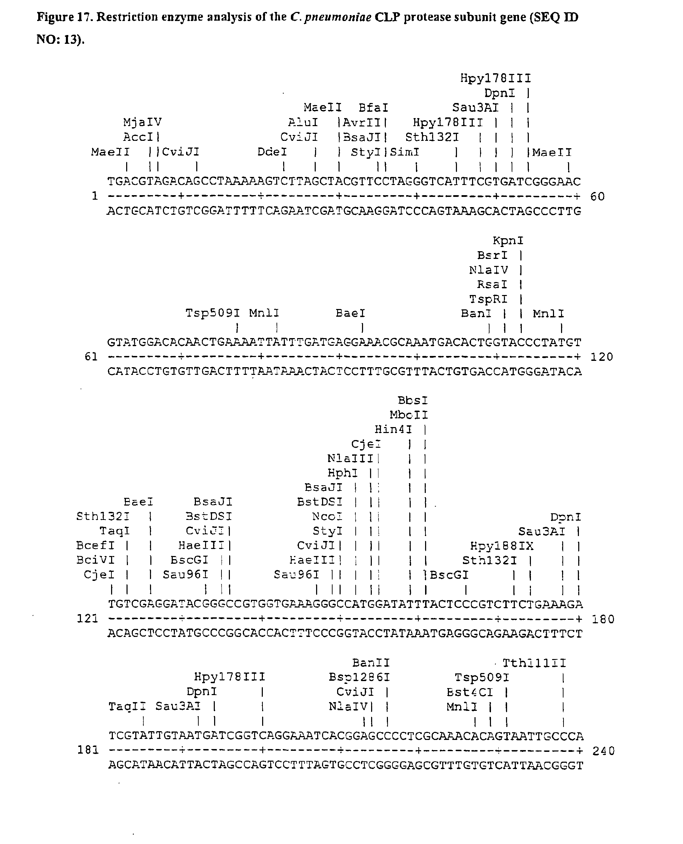

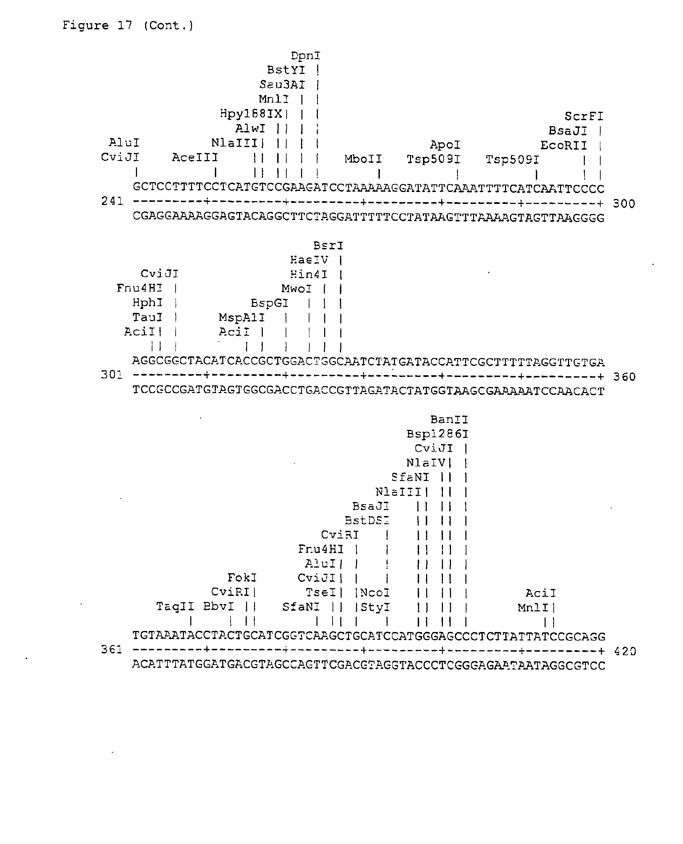

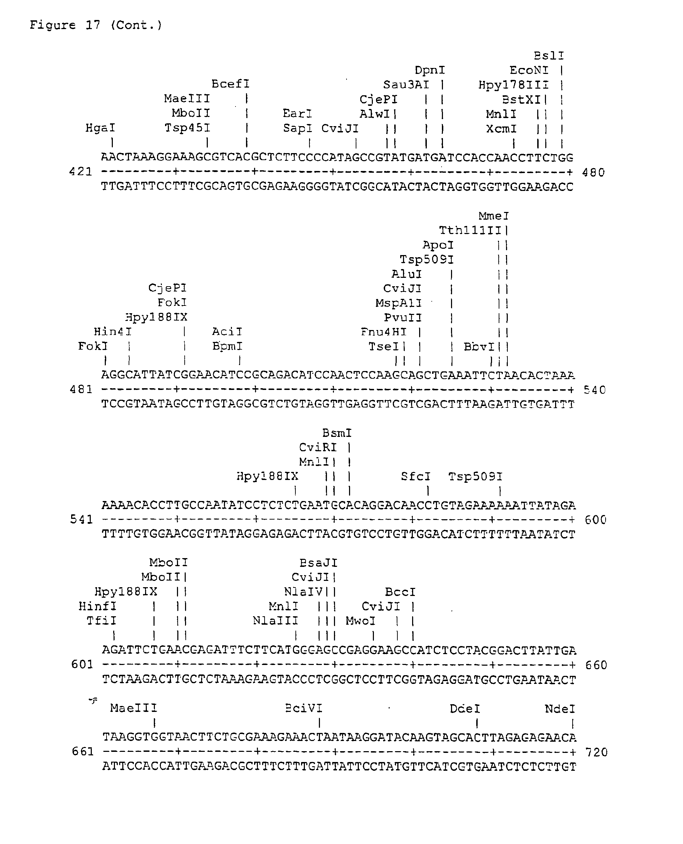

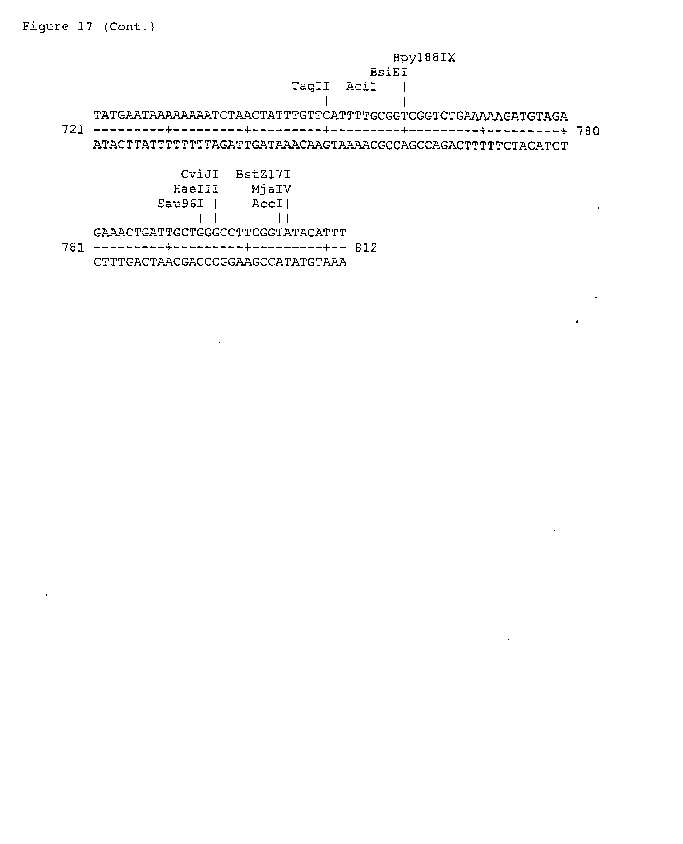

Figure 17 shows the restriction enzyme analysis of the *C. pneumoniae* gene encoding a CLP protease subunit.

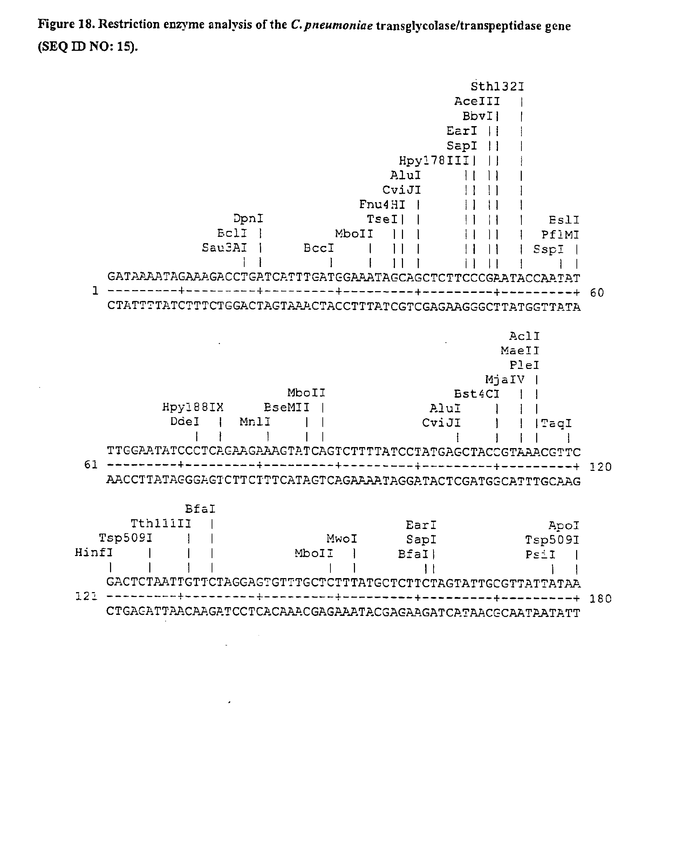

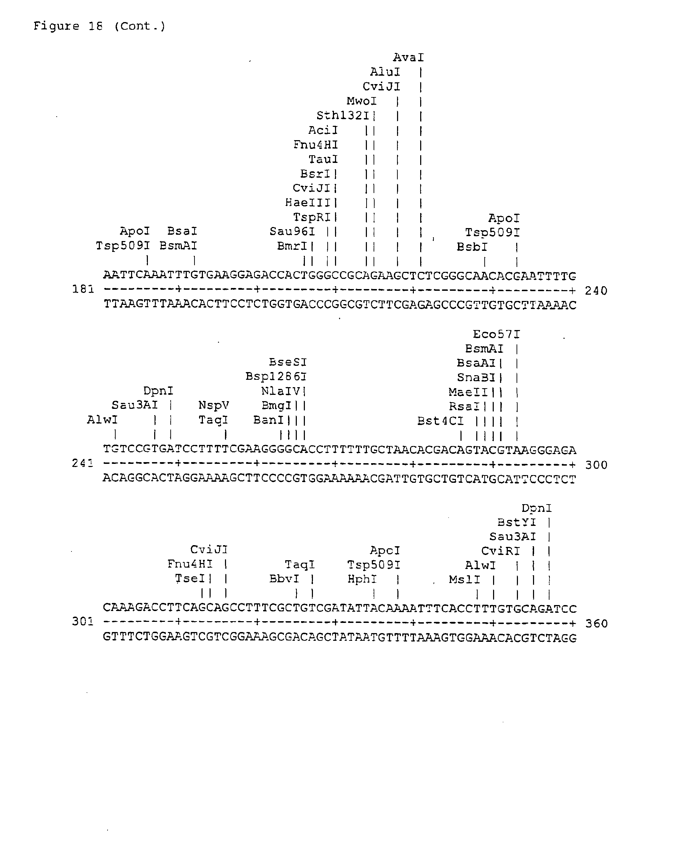

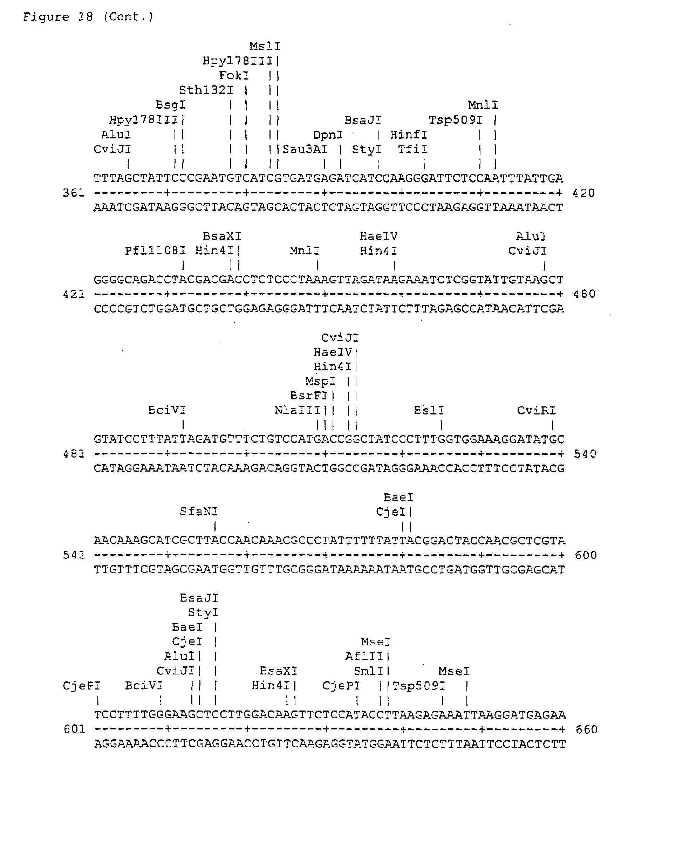

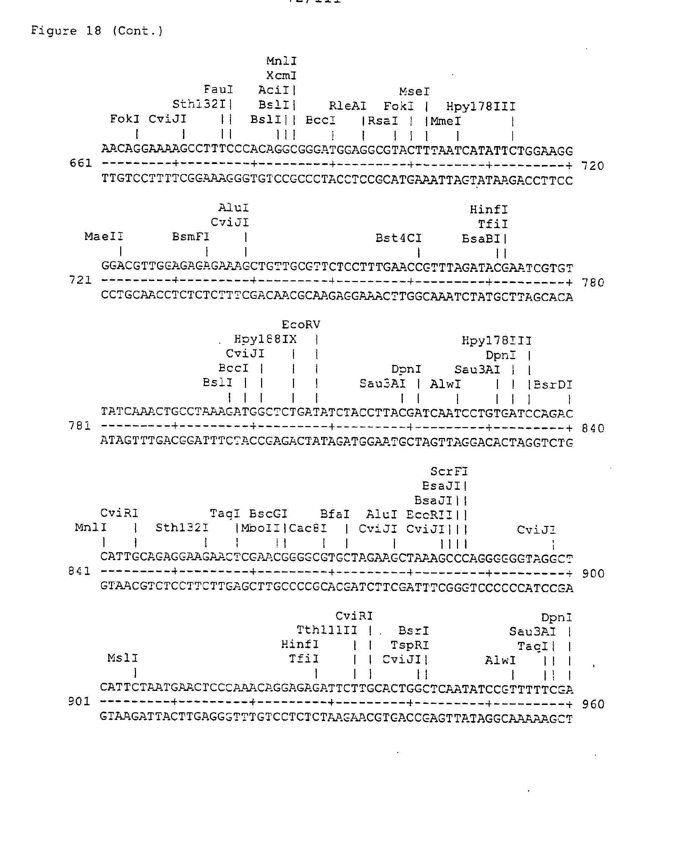

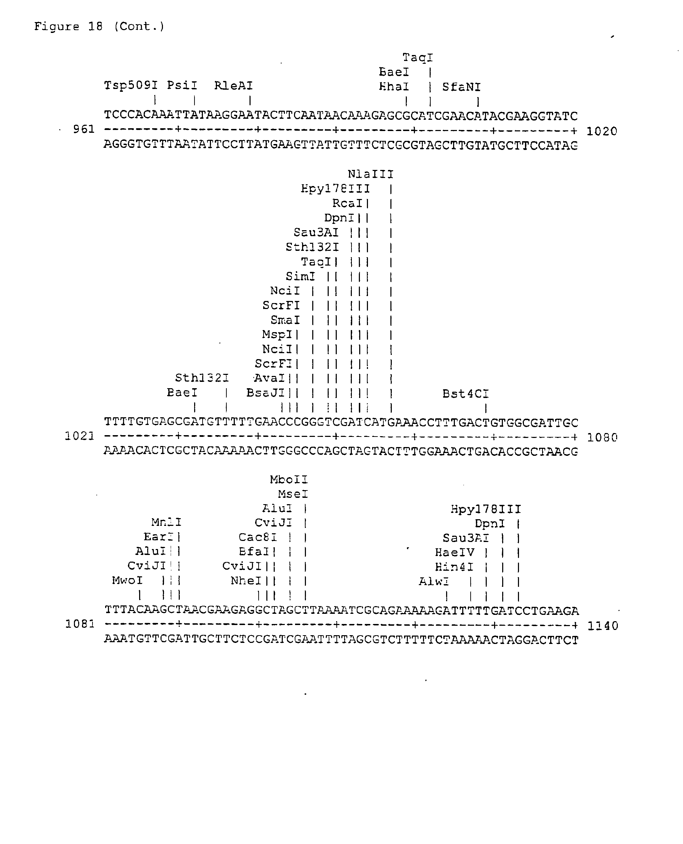

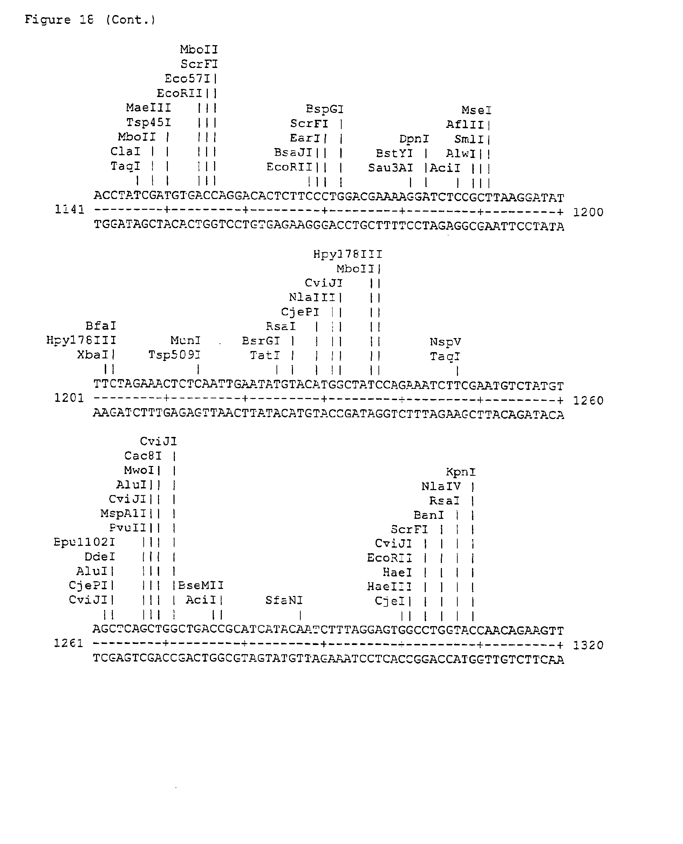

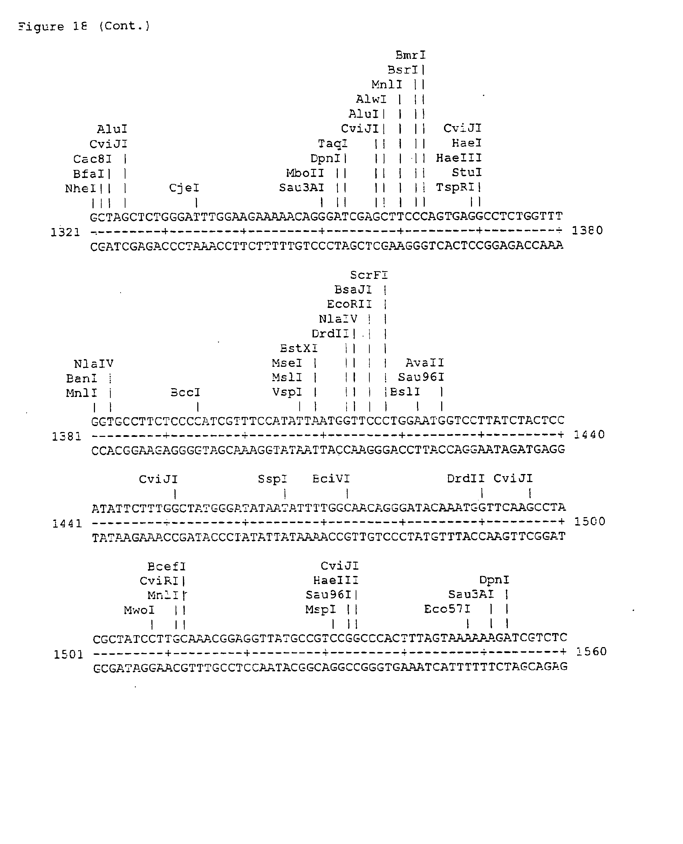

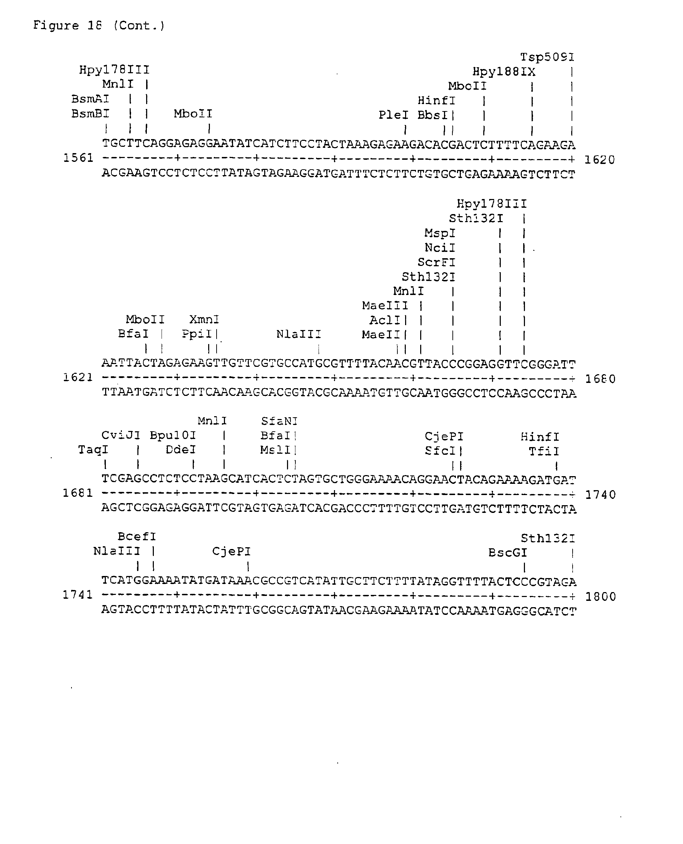

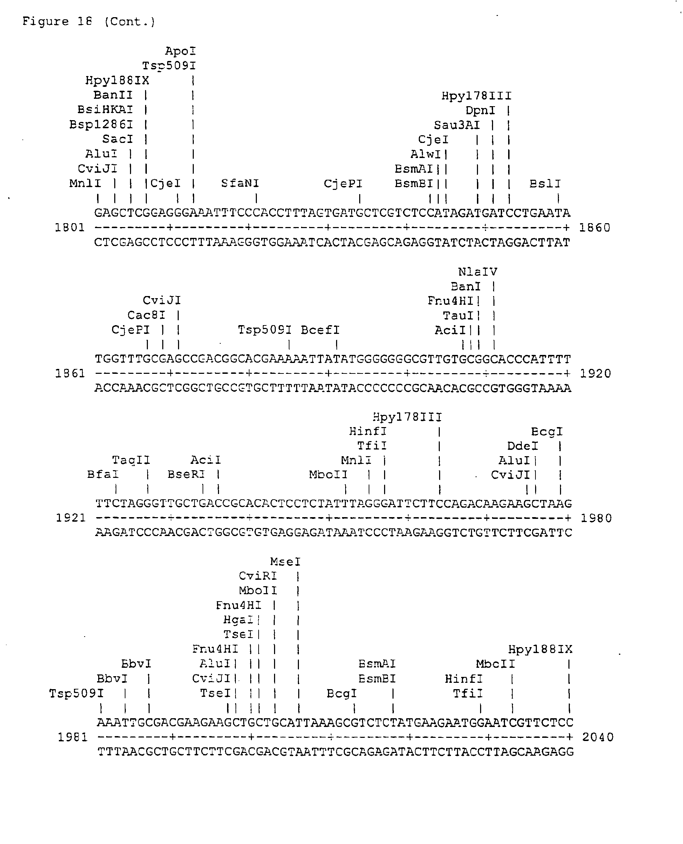

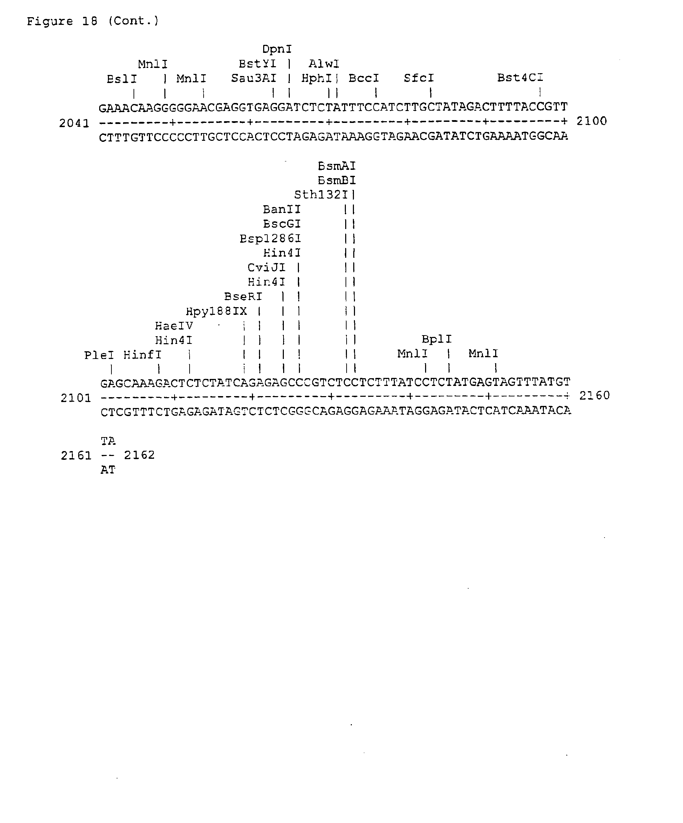

Figure 18 shows the restriction enzyme analysis of the *C. pneumoniae* gene encoding a translycolase / transpeptidase.

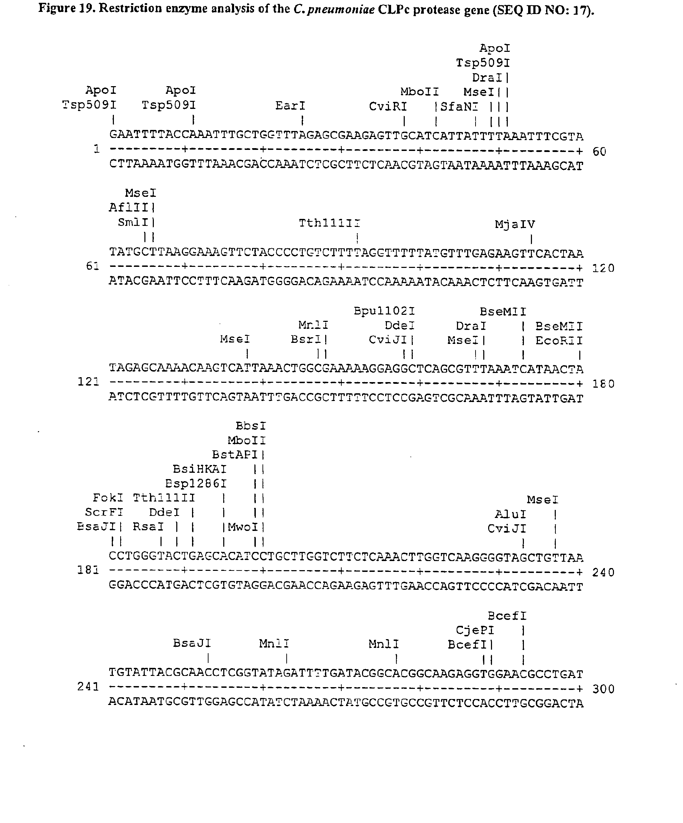

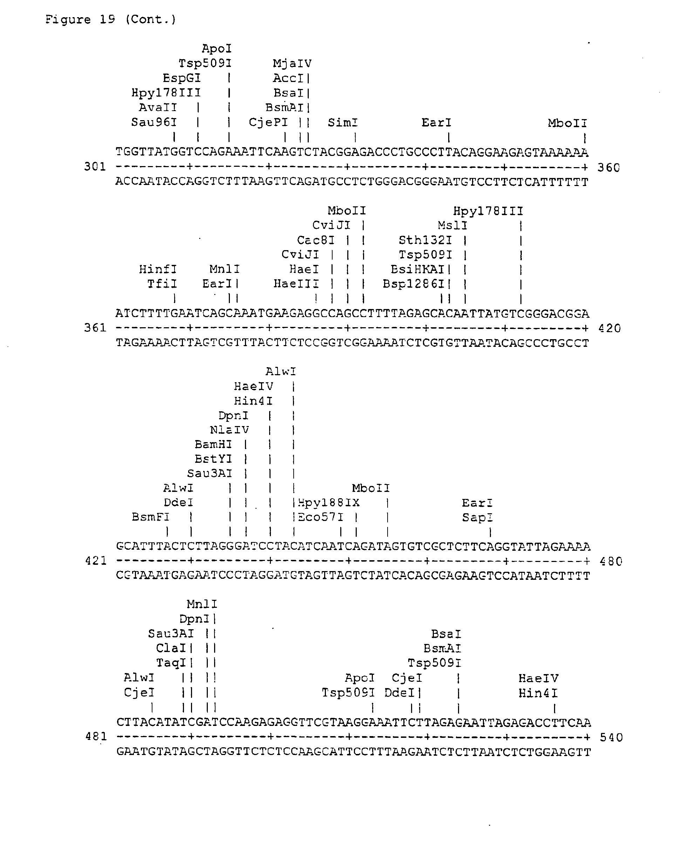

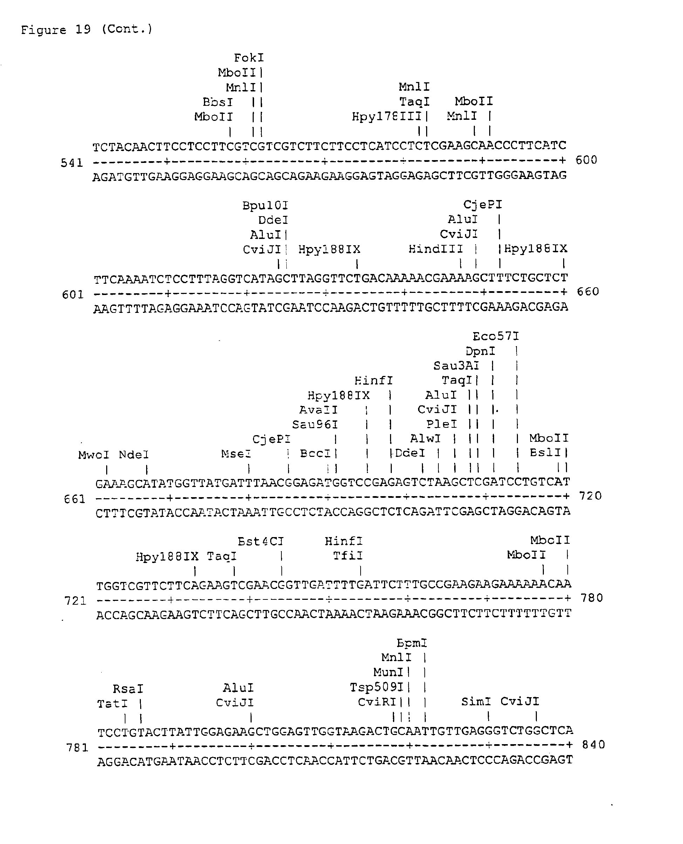

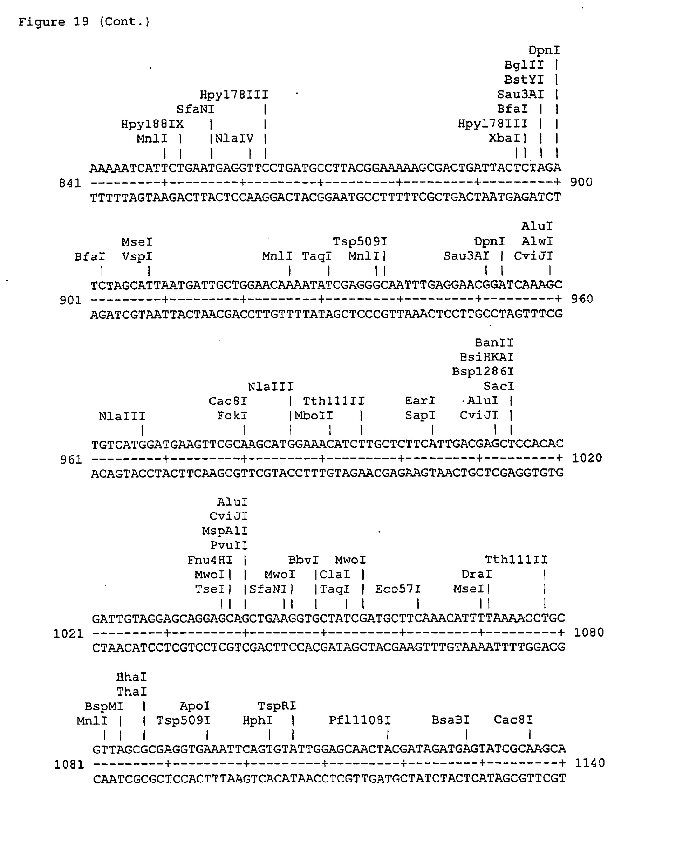

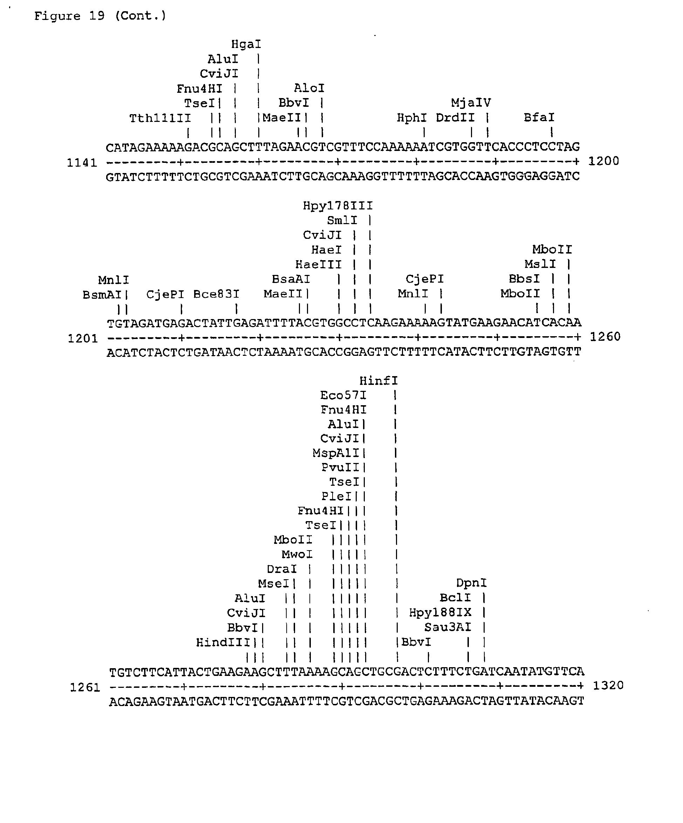

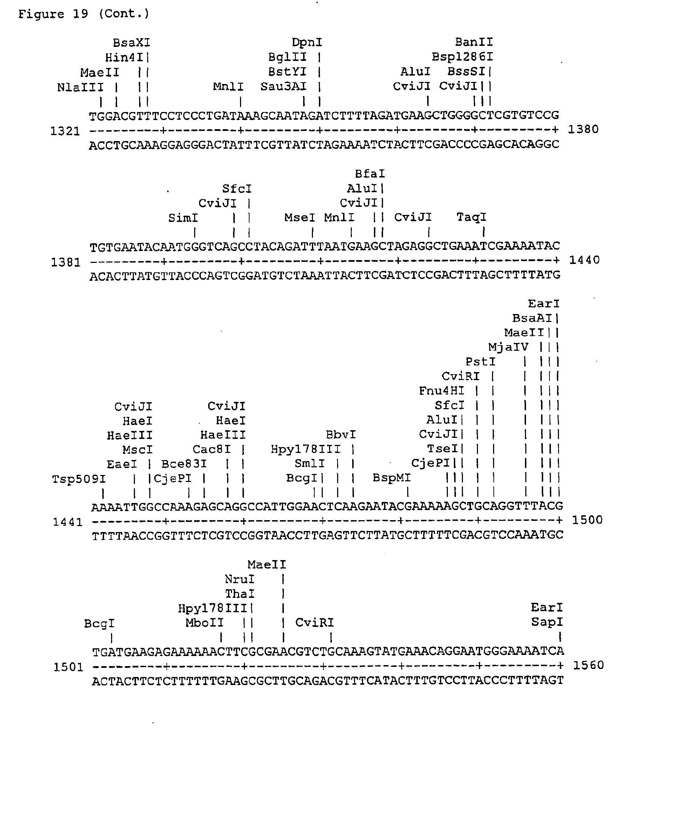

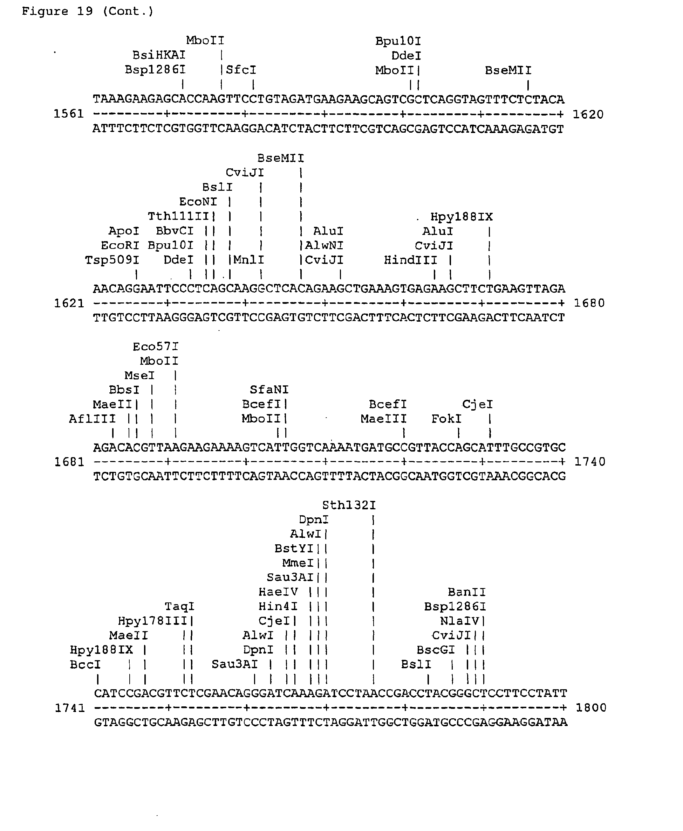

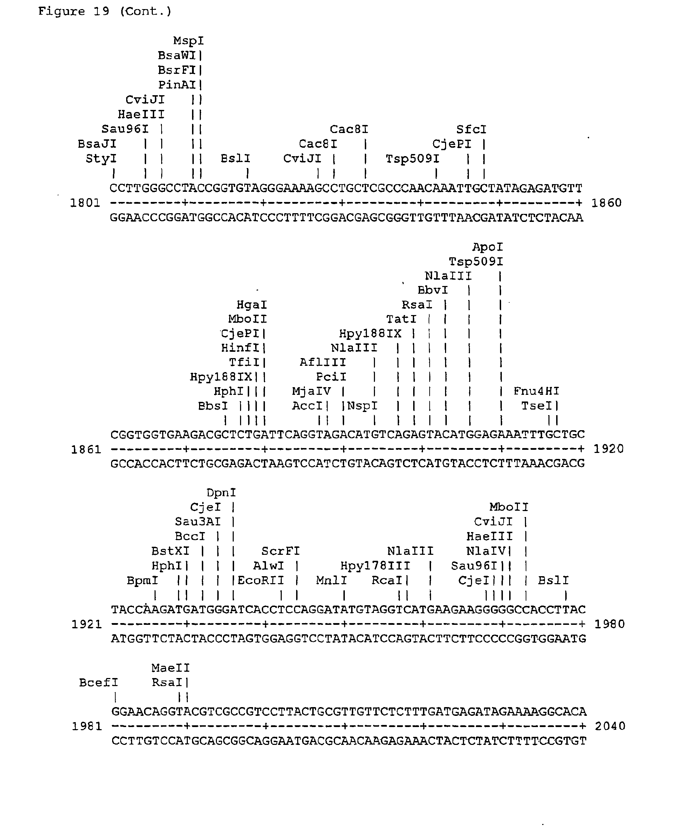

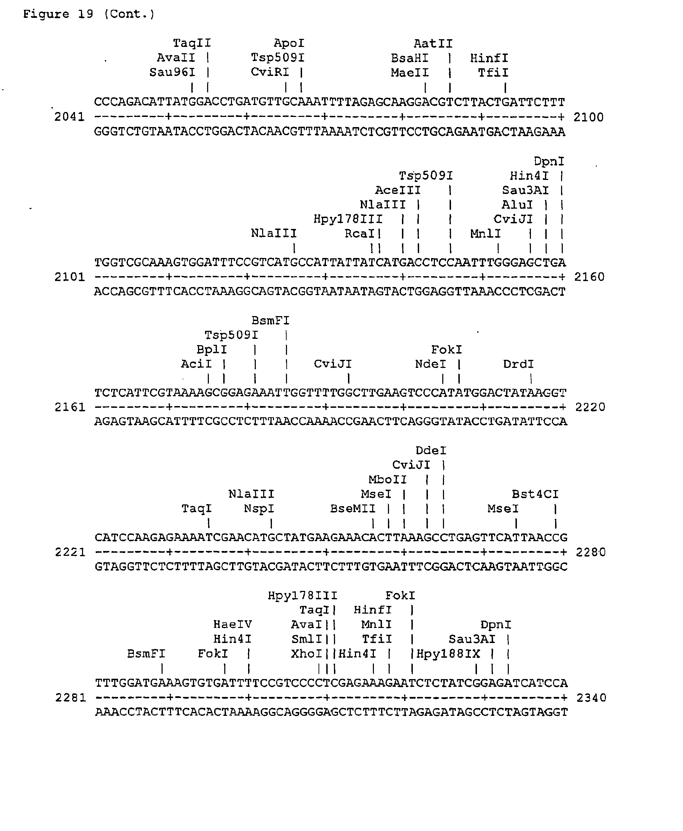

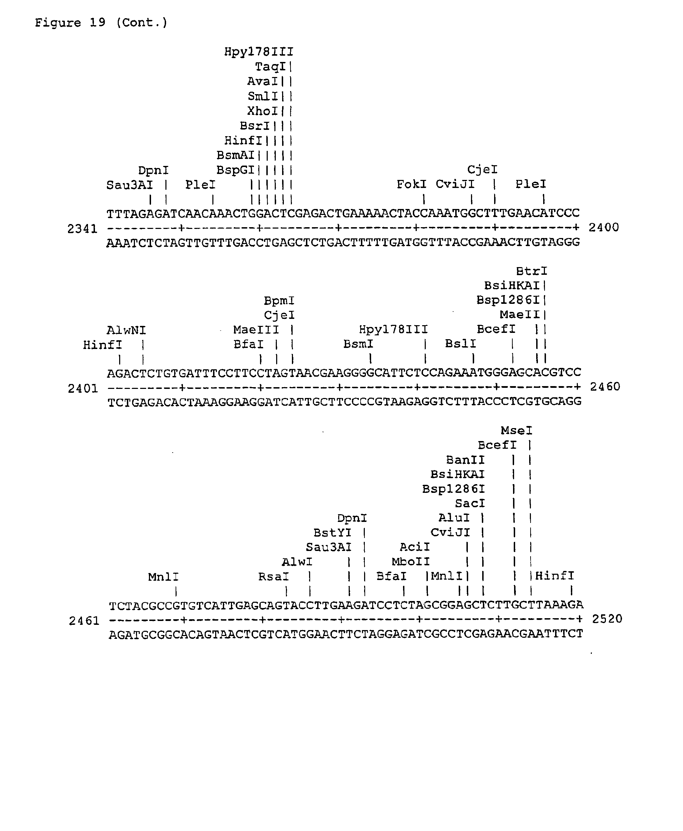

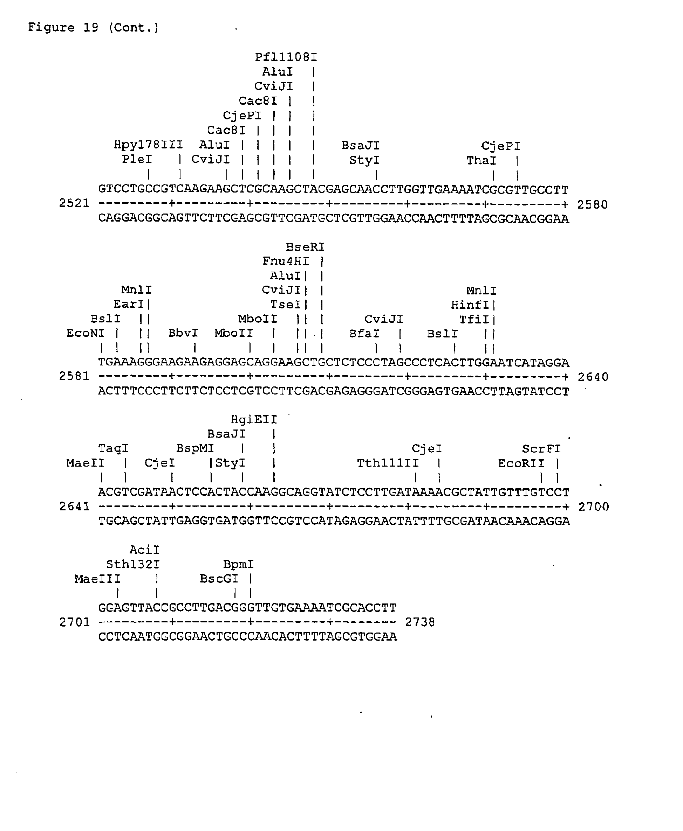

Figure 19 shows the restriction enzyme analysis of the *C. pneumoniae* gene encoding a CLPc protease.

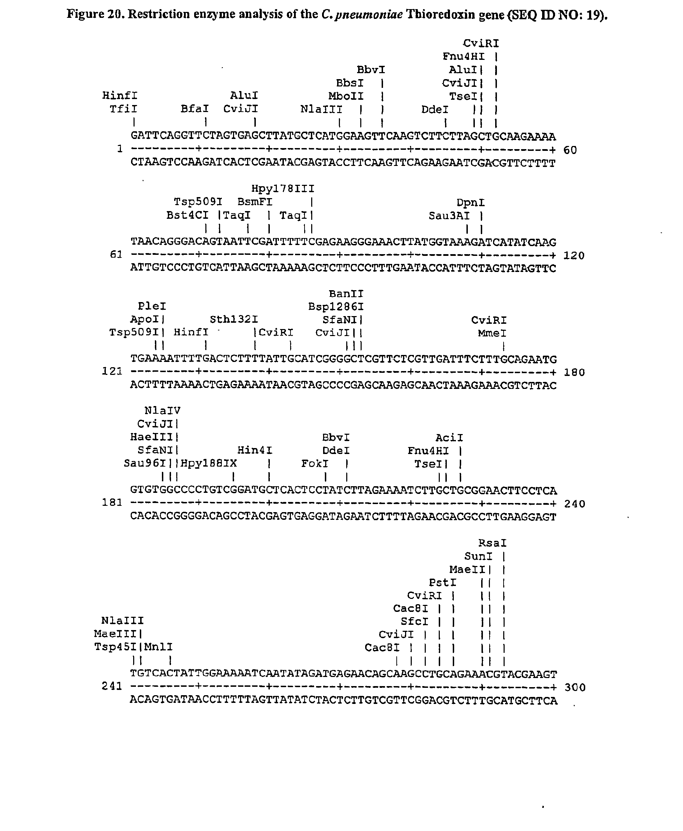

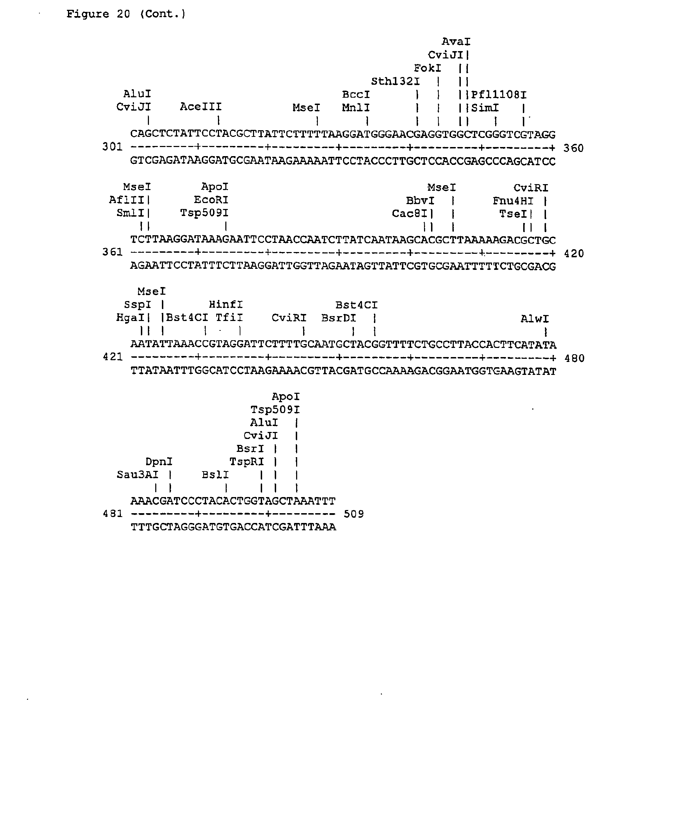

Figure 20 shows the restriction enzyme analysis of the *C. pneumoniae* gene encoding thioredoxin.
Figure 21 shows the construction and elements of plasmid pCACPNM213.
Figure 22 shows the construction and elements of plasmid pCACPNM882.
Figure 23 shows the construction and elements of plasmid pCACPNM208.
Figure 24 shows the construction and elements of plasmid pCACPNM1096.
Figure 25 shows the construction and elements of plasmid pCACPNM1097.
Figure 26 shows the construction and elements of plasmid pCACPNM908.
Figure 27 shows the construction and elements of plasmid pCACPNM909.
Figure 28 shows the construction and elements of plasmid pCACPNM440.
Figure 29 shows the construction and elements of plasmid pCACPNM459.
Figure 30 shows the construction and elements of plasmid pCACPNM708.
Figure 31 illustrates protection against *C. pneumoniae* infection by pCACPNM213 following DNA immunization.
Figure 32 illustrates protection against *C. pneumoniae* infection by pCACPNM882 following DNA immunization.
Figure 33 illustrates protection against *C. pneumoniae* infection by pCACPNM208 following DNA immunization.
Figure 34 illustrates protection against *C. pneumoniae* infection by pCACPNM1096 following DNA immunization.
Figure 35 illustrates protection against *C. pneumoniae* infection by pCACPNM1097 following DNA immunization.
Figure 36 illustrates protection against *C. pneumoniae* infection by pCACPNM908 following DNA immunization.
Figure 37 illustrates protection against *C. pneumoniae* infection by pCACPNM909 following DNA immunization.
Figure 38 illustrates protection against *C. pneumoniae* infection by pCACPNM2440 following DNA immunization.
Figure 39 illustrates protection against *C. pneumoniae* infection by pCACPNM459 following DNA immunization.
Figure 40 illustrates protection against *C. pneumoniae* infection by pCACPNM708 following DNA immunization.

### DETAILED DESCRIPTION OF INVENTION

Open reading frames (ORFs) encoding a number of Chlamydial proteins have been identified from the *C. pneumoniae* genome. These proteins include an ATP-binding cassette protein, a secretory locus ORF, an endopeptidase, a protease, a metalloprotease, CLP protease ATPase, a CLP protease subunit, a translycolase / transpeptidase, a CLPc protease and thioredoxin. The gene encoding each of these polypeptides has been inserted into an expression plasmid and shown to confer immune protection against chlamydial infection. Accordingly, any one of these and related polypeptides can be used to prevent and treat *Chlamydia* infection.

According to a first aspect of the invention, isolated polynucleotides are provided which encode *Chlamydia* polypeptides, whose amino acid sequences are shown in SEQ ID No: 2, 4, 6 ,8, 10, 12, 14, 16, 18 and 20.

The term "isolated polynucleotide" is defined as a polynucleotide removed from the environment in which it naturally occurs. For example, a naturally-occurring DNA molecule present in the genome of a living bacteria or as part of a gene bank is not isolated, but the same molecule separated from the remaining part of the bacterial genome, as a result of, *e.g.*, a cloning event (amplification), is isolated. Typically, an isolated DNA molecule is free from DNA regions (*e.g.*, coding regions) with which it is immediately contiguous at the 5' or 3' end, in the naturally occurring genome. Such isolated polynucleotides may be part of a vector or a composition and still be defined as isolated in that such a vector or composition is not part of the natural environment of such polynucleotide.

The polynucleotide of the invention is either RNA or DNA (cDNA, genomic DNA, or synthetic DNA), or modifications, variants, homologs or fragments thereof. The DNA is either double-stranded or single-stranded, and, if single-stranded, is either the coding strand or the non-coding (anti-sense) strand. Any one of the sequences that encode the polypeptides of the invention as shown in any one of SEQ ID Nos: 2, 4, 6 ,8, 10, 12, 14, 16, 18 and 20 is (a) a coding sequence, (b) a ribonucleotide sequence derived from transcription of (a), or (c) a coding sequence which uses the redundancy or degeneracy of the genetic code to encode the same polypeptides. By "polypeptide" or "protein" is meant any chain of amino acids, regardless of length or post-translational modification (*e.g.*, glycosylation or phosphorylation). Both terms are used interchangeably in the present application.

Consistent with the first aspect of the invention, amino acid sequences are provided which are homologous to any one of SEQ ID Nos: 2, 4, 6 ,8, 10, 12, 14, 16, 18 and 20. As used herein, "homologous amino acid sequence" is any polypeptide which is encoded, in whole or in part, by a nucleic acid sequence which hybridizes at 25-35°C below critical melting temperature (Tm), to any portion of the nucleic acid sequence of any one of SEQ ID Nos: 1, 3, 5, 7, 9, 11, 13, 15, 17 and 19. A homologous amino acid sequence is one that differs from an amino acid sequence shown in any one of SEQ ID Nos: 2, 4, 6 ,8, 10, 12, 14, 16, 18 and 20 by one or more conservative amino acid substitutions. Such a sequence also encompass serotypic variants (defined below) as well as sequences containing deletions or insertions which retain inherent characteristics of the polypeptide such as immunogenicity. Preferably, such a sequence is at least 75%, preferably at least 78%, more preferably at least 80%, even more preferably at least 85%, 88% or 90%, and most preferably at least 93%, 95% or 98% identical to any one of SEQ ID Nos: 2, 4, 6 ,8, 10, 12, 14, 16, 18 and 20.

Homologous amino acid sequences include sequences that are identical or substantially identical to any one of SEQ ID Nos: 2, 4, 6 ,8, 10, 12, 14, 16, 18 and 20. By "amino acid sequence substantially identical" is meant a sequence that is at least 90%, preferably 95%, more preferably 97%, and most preferably 99% identical to an amino acid sequence of reference and that preferably differs from the sequence of reference by a majority of conservative amino acid substitutions.

Conservative amino acid substitutions are substitutions among amino acids of the same class. These classes include, for example, amino acids having uncharged polar side chains, such as asparagine, glutamine, serine, threonine, and tyrosine; amino acids having basic side chains, such as lysine, arginine, and histidine; amino acids having acidic side chains, such as aspartic acid and glutamic acid; and amino acids having nonpolar side chains, such as glycine, alanine, valine, leucine, isoleucine, proline, phenylalanine, methionine, tryptophan, and cysteine.

Homology is measured using sequence analysis software such as Sequence Analysis Software Package of the Genetics Computer Group, University of Wisconsin Biotechnology Center, 1710 University Avenue, Madison, WI 53705. Amino acid sequences are aligned to maximize identity. Gaps may be artificially introduced into the sequence to attain proper alignment. Once the optimal alignment has been set up, the degree of homology is established by recording all of the positions in which the amino acids of both sequences are identical, relative to the total number of positions.

Homologous polynucleotide sequences are defined in a similar way. Preferably, a homologous sequence is one that is at least 45%, more preferably 50%, 55%, 60%, 65%, 70%, 75%, 80%, and even more preferably 85%, 87%, 90%, 93%, 96% and most preferably 99% identical to the coding sequence of any one of SEQ ID Nos: 1, 3, 5, 7, 9, 11, 13, 15, 17 and 19.

Consistent with the first aspect of the invention, polypeptides having a sequence homologous to any one of SEQ ID Nos: 2, 4, 6, 8, 10, 12, 14, 16, 18 and 20 include naturally-occurring allelic variants, as well as mutants or any other non-naturally occurring variants that retain the inherent characteristics of the polypeptide of any one of SEQ ID Nos: 2, 4, 6, 8, 10, 12, 14, 16, 18 and 20.

As is known in the art, an allelic variant is an alternate form of a polypeptide that is characterized as having a substitution, deletion, or addition of one or more amino acids that does not alter the biological function of the polypeptide. By "biological function" is meant the function of the polypeptide in the cells in which it naturally occurs, even if the function is not necessary for the growth or survival of the cells. For example, the biological function of a porin is to allow the entry into cells of compounds present in the extracellular medium. Biological function is distinct from antigenic property. A polypeptide can have more than one biological function.

Allelic variants are very common in nature. For example, a bacterial species such as *C. pneumoniae,* is usually represented by a variety of strains that differ from each other by minor allelic variations. Indeed, a polypeptide that fulfills the same biological function in different strains can have an amino acid sequence (and polynucleotide sequence) that is not identical in each of the strains. Despite this variation, an immune response directed generally against many allelic variants has been demonstrated. In studies of the *Chlamydial* MOMP antigen, cross-strain antibody binding plus neutralization of infectivity occurs despite amino acid sequence variation of MOMP from strain to strain, indicating that the MOMP, when used as an immunogen, is tolerant of amino acid variations.

Polynucleotides encoding homologous polypeptides or allelic variants are retrieved by polymerase chain reaction (PCR) amplification of genomic bacterial DNA extracted by conventional methods. This involves the use of synthetic oligonucleotide primers matching upstream and downstream of the 5' and 3' ends of the encoding domain. Suitable primers are designed according to the nucleotide sequence information provided in any one of SEQ ID Nos: 1, 3, 5, 7, 9, 11, 13, 15, 17 and 19. The procedure is as follows: a primer is selected which consists of 10 to 40, preferably 15 to 25 nucleotides. It is advantageous to select primers containing C and G nucleotides in a proportion sufficient to ensure efficient hybridization; *i.e.*, an amount of C and G nucleotides of at least 40%, preferably 50% of the total nucleotide content. A standard PCR reaction contains typically 0.5 to 5 Units of Taq DNA polymerase per 100 µL, 20 to 200 µM deoxynucleotide each, preferably at equivalent concentrations, 0.5 to 2.5 mM magnesium over the total deoxynucleotide concentration, 10⁵ to 10⁶ target molecules, and about 20 pmol of each primer. About 25 to 50 PCR cycles are performed, with an annealing temperature 15°C to 5°C below the true Tm of the primers. A more stringent annealing temperature improves discrimination against incorrectly annealed primers and reduces incorportion of incorrect nucleotides at the 3' end of primers. A denaturation temperature of 95°C to 97°C is typical, although higher temperatures may be appropriate for dematuration of G+C-rich targets. The number of cycles performed depends on the starting concentration of target molecules, though typically more than 40 cycles is not recommended as non-specific background products tend to accumulate.

An alternative method for retrieving polynucleotides encoding homologous polypeptides or allelic variants is by hybridization screening of a DNA or RNA library. Hybridization procedures are well-known in the art and are described in Ausubel *et al.,* (Ausubel et al., Current Protocols in Molecular Biology, John Wiley & Sons Inc., 1994), Silhavy et al. (Silhavy et al. Experiments with Gene Fusions, Cold Spring Harbor Laboratory Press, 1984), and Davis et al. (Davis et al. A Manual for Genetic Engineering: Advanced Bacterial Genetics, Cold Spring Harbor Laboratory Press, 1980)). Important parameters for optimizing hybridization conditions are reflected in a formula used to obtain the critical melting temperature above which two complementary DNA strands separate from each other (Casey & Davidson, Nucl. Acid Res. (1977) 4:1539). For polynucleotides of about 600 nucleotides or larger, this formula is as follows: Tm = 81.5 + 0.41 x (% G+C) + 16.6 log (cation ion concentration) - 0.63 x (% formamide) - 600/base number. Under appropriate stringency conditions, hybridization temperature (Th) is approximately 20 to 40°C, 20 to 25°C, or, preferably 30 to 40°C below the calculated Tm. Those skilled in the art will understand that optimal temperature and salt conditions can be readily determined.

For the polynucleotides of the invention, stringent conditions are achieved for both pre-hybridizing and hybridizing incubations (i) within 4-16 hours at 42°C, in 6 x SSC containing 50% formamide, or (ii) within 4-16 hours at 65°C in an aqueous 6 x SSC solution (1 M NaCl, 0.1 M sodium citrate (pH 7.0)). Typically, hybridization experiments are performed at a temperature from 60 to 68°C, e.g. 65°C. At such a temperature, stringent hybridization conditions can be achieved in 6xSSC, preferably in 2xSSC or 1xSSC, more preferably in 0.5xSSc, 0.3xSSC or 0.1xSSC (in the absence of formamide). 1xSSC contains 0.15 M NaCl and 0.015 M sodium citrate.

Useful homologs and fragments thereof that do not occur naturally are designed using known methods for identifying regions of an antigen that are likely to tolerate amino acid sequence changes and/or deletions. As an example, homologous polypeptides from different species are compared; conserved sequences are identified. The more divergent sequences are the most likely to tolerate sequence changes. Homology among sequences may be analyzed using, as an example, the BLAST homology searching algorithm of Altschul et al., Nucleic Acids Res.; 25:3389-3402 (1997). Alternatively, sequences are modified such that they become more reactive to T- and/or B-cells, based on computer-assisted analysis of probable T- or B-cell epitopes Yet another alternative is to mutate a particular amino acid residue or sequence within the polypeptide in vitro, then screen the mutant polypeptides for their ability to prevent or treat *Chlamydia* infection according to the method outlined below.

A person skilled in the art will readily understand that by following the screening process of this invention, it will be determined without undue experimentation whether a particular homolog of any one of SEQ ID Nos: 2, 4, 6, 8, 10, 12, 14, 16, 18 and 20 may be useful in the prevention or treatment of *Chlamydia* infection. The screening procedure comprises the steps:
(i) immunizing an animal, preferably mouse, with the test homolog or fragment;
(ii) inoculating the immunized animal with *Chlamydia:*
   and
(iii) selecting those homologs or fragments which confer protection against *Chlamydia.*

By "conferring protection" is meant that there is a reduction in severity of any of the effects of *Chlamydia* infection, in comparison with a control animal which was not immunized with the test homolog or fragment.

Consistent with the first aspect of the invention, polypeptide derivatives are provided that are partial sequences of any one of SEQ ID Nos: 2, 4, 6, 8, 10, 12, 14, 16, 18 and 20, partial sequences of polypeptide sequences homologous to any one of SEQ ID Nos: 2, 4, 6, 8, 10, 12, 14, 16, 18 and 20, polypeptides derived from full-length polypeptides by internal deletion, and fusion proteins.

It is an accepted practice in the field of immunology to use fragments and variants of protein immunogens as vaccines, as all that is required to induce an immune response to a protein is a small (*e.g.*, 8 to 10 amino acid) immunogenic region of the protein. Various short synthetic peptides corresponding to surface-exposed antigens of pathogens other than *Chlamydia* have been shown to be effective vaccine antigens against their respective pathogens, *e.g.* an 11 residue peptide of murine mammary tumor virus (Casey & Davidson, Nucl. Acid Res. (1977) 4:1539), a 16-residue peptide of Semliki Forest virus (Snijders et al., 1991. J. Gen. Virol. 72:557-565), and two overlapping peptides of 15 residues each from canine parvovirus (Langeveld et al., Vaccine 12(15):1473-1480, 1994)

Accordingly, it will be readily apparent to one skilled in the art, having read the present description, that partial sequences of any one of SEQ ID Nos: 2, 4, 6, 8, 10, 12, 14, 16, 18 and 20 or their homologous amino acid sequences are inherent to the full-length sequences and are taught by the present invention. Such polypeptide fragments preferably are at least 12 amino acids in length. Advantageously, they are at least 15 amino acids, preferably at least 20, 25, 30, 35, 40, 45, 50 amino acids, more preferably at least 55, 60, 65, 70, 75 amino acids, and most preferably at least 80, 85, 90, 95, 100 amino acids in length.

Polynucleotides of 30 to 600 nucleotides encoding partial sequences of sequences homologous to any one of SEQ ID Nos: 2, 4, 6 ,8, 10, 12, 14, 16, 18 and 20 are retrieved by PCR amplification using the parameters outlined above and using primers matching the sequences upstream and downstream of the 5' and 3' ends of the fragment to be amplified. The template polynucleotide for such amplification is either the full length polynucleotide homologous to any one of SEQ ID Nos: 1, 3, 5, 7, 9, 11, 13, 15, 17 and 19, or a polynucleotide contained in a mixture of polynucleotides such as a DNA or RNA library. As an alternative method for retrieving the partial sequences, screening hybridization is carried out under conditions described above and using the formula for calculating Tm. Where fragments of 30 to 600 nucleotides are to be retrieved, the calculated Tm is corrected by subtracting (600/polynucleotide size in base pairs) and the stringency conditions are defined by a hybridization temperature that is 5 to 10°C below Tm. Where oligonucleotides shorter than 20-30 bases are to be obtained, the formula for calculating the Tm is as follows: Tm = 4 x (G+C) + 2 (A+T). For example, an 18 nucleotide fragment of 50% G+C would have an approximate Tm of 54°C. Short peptides that are fragments of any one of SEQ ID Nos: 2, 4, 6, 8, 10, 12, 14, 16, 18 and 20 or its homologous sequences, are obtained directly by chemical synthesis (E. Gross and H. J. Meinhofer, 4 The Peptides: Analysis, Synthesis, Biology; Modern Techniques of Peptide Synthesis, John Wiley & Sons (1981), and M. Bodanzki, Principles of Peptide Synthesis, Springer -Verlag (1984)).

Useful polypeptide derivatives, *e.g.*, polypeptide fragments, are designed using computer-assisted analysis of amino acid sequences. This would identify probable surface-exposed, antigenic regions (Hughes et al., 1992. Infect. Immun. 60(9):3497). Analysis of 6 amino acid sequences contained in any one of SEQ ID Nos: 2, 4, 6, 8, 10, 12, 14, 16, 18 and 20, based on the product of flexibility and hydrophobicity propensities using the program SEQSEE (Wishart DS, et al. "SEQSEE: a comprehensive program suite for protein sequence analysis." Comput Appl Biosci. 1994 Apr;10 (2):121-32), reveal potential B- and T-cell epitopes which may be used as a basis for selecting useful immunogenic fragments and variants. This analysis uses a reasonable combination of external surface features that is likely to be recognized by antibodies. Probable T-cell epitopes for HLA-A0201 MHC subclass may be revealed by an algorithms that emulate an approach developed at the NIH (Parker KC, et al. "Peptide binding to MHC class I molecules: implications for antigenic peptide prediction." Immunol Res 1995;14(1):34-57). The potential B-cell and T-cell epitopes are shown in Tables 2, 5, 7, 9, 11, 13, 15, 17 and 19 and SEQ ID NOs: 41 to 74. Sequences which are substantially identical to SEQ ID NOS: 41 to 74, or which are conservatively substituted variants of SEQ ID NOs: 41 to 74, are expected to be functional epitopes and are within the scope of the invention.

Epitopes which induce a protective T cell-dependent immune response are present throughout the length of the polypeptide. However, some epitopes may be masked by secondary and tertiary structures of the polypeptide. To reveal such masked epitopes large internal deletions are created which remove much of the original protein structure and exposes the masked epitopes. Such internal deletions sometimes effect the additional advantage of removing immunodominant regions of high variability among strains.

Polynucleotides encoding polypeptide fragments and polypeptides having large internal deletions are constructed using standard methods (Ausubel et al., Current Protocols in Molecular Biology, John Wiley & Sons Inc., 1994). Such methods include standard PCR, inverse PCR, restriction enzyme treatment of cloned DNA molecules, or the method of Kunkel *et al.* (Kunkel et al. Proc. Natl. Acad. Sci. USA (1985) 82:448). Components for these methods and instructions for their use are readily available from various commercial sources such as Stratagene. Once the deletion mutants have been constructed, they are tested for their ability to prevent or treat *Chlamydia* infection as described above.

As used herein, a fusion polypeptide is one that contains a polypeptide or a polypeptide derivative of the invention fused at the N- or C-terminal end to any other polypeptide (hereinafter referred to as a peptide tail). A simple way to obtain such a fusion polypeptide is by translation of an in-frame fusion of the polynucleotide sequences, *i.e.*, a hybrid gene. The hybrid gene encoding the fusion polypeptide is inserted into an expression vector which is used to transform or transfect a host cell. Alternatively, the polynucleotide sequence encoding the polypeptide or polypeptide derivative is inserted into an expression vector in which the polynucleotide encoding the peptide tail is already present. Such vectors and instructions for their use are commercially available, *e.g.* the pMal-c2 or pMal-p2 system from New England Biolabs, in which the peptide tail is a maltose binding protein, the glutathione-S-transferase system of Pharmacia, or the His-Tag system available from Novagen. These and other expression systems provide convenient means for further purification of polypeptides and derivatives of the invention.

An advantageous example of a fusion polypeptide is one where the polypeptide or homolog or fragment of the invention is fused to a polypeptide having adjuvant activity, such as subunit B of either cholera toxin or E. *coli* heat-labile toxin. Another advantageous fusion is one where the polypeptide, homolog or fragment is fused to a strong T-cell epitope or B-cell epitope. Such an epitope may be one known in the art (*e.g.* the Hepatitis B virus core antigen, D.R. Millich et al., "Antibody production to the nucleocapsid and envelope of the Hepatitis B virus primed by a single synthetic T cell site", Nature. 1987. 329:547-549), or one which has been identified in another polypeptide of the invention based on computer-assisted analysis of probable T- or B-cell epitopes. Consistent with this aspect of the invention is a fusion polypeptide comprising T- or B-cell epitopes from any one of SEQ ID Nos: 2, 4, 6, 8, 10, 12, 14, 16, 18 and 20 or its homolog or fragment, wherein the epitopes are derived from multiple variants of said polypeptide or homolog or fragment, each variant differing from another in the location and sequence of its epitope within the polypeptide. Such a fusion is effective in the prevention and treatment of *Chlamydia* infection since it optimizes the T- and B-cell response to the overall polypeptide, homolog or fragment.

To effect fusion, the polypeptide of the invention is fused to the N-, or preferably, to the C-terminal end of the polypeptide having adjuvant activity or T- or B-cell epitope. Alternatively, a polypeptide fragment of the invention is inserted internally within the amino acid sequence of the polypeptide having adjuvant activity. The T- or B-cell epitope may also be inserted internally within the amino acid sequence of the polypeptide of the invention.

Consistent with the first aspect, the polynucleotides of the invention also encode hybrid precursor polypeptides containing heterologous signal peptides, which mature into polypeptides of the invention. By "heterologous signal peptide" is meant a signal peptide that is not found in naturally-occurring precursors of polypeptides of the invention.

Polynucleotide molecules according to the invention, including RNA, DNA, or modifications or combinations thereof, have various applications. A DNA molecule is used, for example, (i) in a process for producing the encoded polypeptide in a recombinant host system, (ii) in the construction of vaccine vectors such as poxviruses, which are further used in methods and compositions for preventing and/or treating *Chlamydia* infection, (iii) as a vaccine agent (as well as an RNA molecule), in a naked form or formulated with a delivery vehicle and, (iv) in the construction of attenuated *Chlamydia* strains that can over-express a polynucleotide of the invention or express it in a non-toxic, mutated form.

Selected genes from pathogenic micro-organisms within an eukaryotic expression plasmid are useful as vaccines. Expression plasmids contain methylated CpG motifs that elicit innate cytokine responses that promote the canalization of CD4 T cell responses to a Th1 cytokine secretion pattern. The intracellular synthesis of the microbial protein, especially within transfected professional antigen-presenting cells, facilitates the presentation of antigen on class I and class II molecules and the induction of cell-mediated immunity. The use of one or a number of microbial protein-coding genes allows the presentation of protective antigens to the immune system to occur in the absence of microbe-directed immune evasion mechanisms and in the absence of competing or pathologic antigens. Immune responses primed by DNA vaccines are also readily amplified by protein-antigen immunization. Thus, immunization with DNA vaccines is particularly relevant to chlamydial vaccine design.

Accordingly, a second aspect of the invention encompasses (i) an expression cassette containing a DNA molecule of the invention placed under the control of the elements required for expression, in particular under the control of an appropriate promoter; (ii) an expression vector containing an expression cassette of the invention; (iii) a procaryotic or eucaryotic cell transformed or transfected with an expression cassette and/or vector of the invention, as well as (iv) a process for producing a polypeptide or polypeptide derivative encoded by a polynucleotide of the invention, which involves culturing a procaryotic or eucaryotic cell transformed or transfected with an expression cassette and/or vector of the invention, under conditions that allow expression of the DNA molecule of the invention and, recovering the encoded polypeptide or polypeptide derivative from the cell culture.

A recombinant expression system is selected from procaryotic and eucaryotic hosts. Eucaryotic hosts include yeast cells *(e.g., Saccharomyces cerevisiae* or *Pichia pastoris),* mammalian cells (*e.g.,* COS1, NIH3T3, or JEG3 cells), arthropods cells *(e.g., Spodoptera frugiperda* (SF9) cells), and plant cells. A preferred expression system is a procaryotic host such as *E. coli.* Bacterial and eucaryotic cells are available from a number of different sources including commercial sources to those skilled in the art, *e.g.*, the American Type Culture Collection (ATCC; Rockville, Maryland). Commercial sources of cells used for recombinant protein expression also provide instructions for usage of the cells.

The choice of the expression system depends on the features desired for the expressed polypeptide. For example, it may be useful to produce a polypeptide of the invention in a particular lipidated form or any other form.

One skilled in the art would redily understand that not all vectors and expression control sequences and hosts would be expected to express equally well the polynucleotides of this invention. With the guidelines described below, however, a selection of vectors, expression control sequences and hosts may be made without undue experimentation and without departing from the scope of this invention.

In selecting a vector, the host must be chosen that is compatible with the vector which is to exist and possibly replicate in it. Considerations are made with respect to the vector copy number, the ability to control the copy number, expression of other proteins such as antibiotic resistance. In selecting an expression control sequence, a number of variables are considered. Among the important variable are the relative strength of the sequence (*e.g.* the ability to drive expression under various conditions), the ability to control the sequence's function, compatibility between the polynucleotide to be expressed and the control sequence (*e.g.* secondary structures are considered to avoid hairpin structures which prevent efficient transcription). In selecting the host, unicellular hosts are selected which are compatible with the selected vector, tolerant of any possible toxic effects of the expressed product, able to secrete the expressed product efficiently if such is desired, to be able to express the product in the desired conformation, to be easily scaled up, and to which ease of purification of the final product.

The choice of the expression cassette depends on the host system selected as well as the features desired for the expressed polypeptide. Typically, an expression cassette includes a promoter that is functional in the selected host system and can be constitutive or inducible; a ribosome binding site; a start codon (ATG) if necessary; a region encoding a signal peptide, *e.g.*, a lipidation signal peptide; a DNA molecule of the invention; a stop codon; and optionally a 3' terminal region (translation and/or transcription terminator). The signal peptide encoding region is adjacent to the polynucleotide of the invention and placed in proper reading frame. The signal peptide-encoding region is homologous or heterologous to the DNA molecule encoding the mature polypeptide and is compatible with the secretion apparatus of the host used for expression. The open reading frame constituted by the DNA molecule of the invention, solely or together with the signal peptide, is placed under the control of the promoter so that transcription and translation occur in the host system. Promoters and signal peptide encoding regions are widely known and available to those skilled in the art and include, for example, the promoter of *Salmonella typhimurium* (and derivatives) that is inducible by arabinose (promoter araB) and is functional in Gram-negative bacteria such as *E. coli* (as described in U.S. Patent No. 5,028,530 and in Cagnon *et al.*, (Cagnon et al., Protein Engineering (1991) 4(7):843)); the promoter of the gene of bacteriophage T7 encoding RNA polymerase, that is functional in a number of *E. coli* strains expressing T7 polymerase (described in U.S. Patent No. 4,952,496); OspA lipidation signal peptide ; and RlpB lipidation signal peptide (Takase et al., J. Bact. (1987) 169:5692).

The expression cassette is typically part of an expression vector, which is selected for its ability to replicate in the chosen expression system. Expression vectors *(e.g.*, plasmids or viral vectors) can be chosen, for example, from those described in Pouwels et al. (Cloning Vectors: A Laboratory Manual 1985, Supp. 1987). Suitable expression vectors can be purchased from various commercial sources.

Methods for transforming/transfecting host cells with expression vectors are well-known in the art and depend on the host system selected as described in Ausubel *et al.*, (Ausubel et al., Current Protocols in Molecular Biology, John Wiley & Sons Inc., 1994).

Upon expression, a recombinant polypeptide of the invention (or a polypeptide derivative) is produced and remains in the intracellular compartment, is secreted/excreted in the extracellular medium or in the periplasmic space, or is embedded in the cellular membrane. The polypeptide is recovered in a substantially purified form from the cell extract or from the supernatant after centrifugation of the recombinant cell culture. Typically, the recombinant polypeptide is purified by antibody-based affinity purification or by other well-known methods that can be readily adapted by a person skilled in the art, such as fusion of the polynucleotide encoding the polypeptide or its derivative to a small affinity binding domain. Antibodies useful for purifying by immunoaffinity the polypeptides of the invention are obtained as described below.

A polynucleotide of the invention can also be useful as a vaccine. There are two major routes, either using a viral or bacterial host as gene delivery vehicle (live vaccine vector) or administering the gene in a free form, *e.g.*, inserted into a plasmid. Therapeutic or prophylactic efficacy of a polynucleotide of the invention is evaluated as described below.

Accordingly, a third aspect of the invention provides (i) a vaccine vector such as a poxvirus, containing a DNA molecule of the invention, placed under the control of elements required for expression; (ii) a composition of matter comprising a vaccine vector of the invention, together with a diluent or carrier; specifically (iii) a pharmaceutical composition containing a therapeutically or prophylactically effective amount of a vaccine vector of the invention; (iv) a method for inducing an immune response against *Chlamydia* in a mammal (*e.g.*, a human; alternatively, the method can be used in veterinary applications for treating or preventing *Chlamydia* infection of animals, *e.g.*, cats or birds), which involves administering to the mammal an immunogenically effective amount of a vaccine vector of the invention to elicit a protective or therapeutic immune response to *Chlamydia ;* and particularly, (v) a method for preventing and/or treating a *Chlamydia (e.g., C. trachomatis, C. psittaci, C. pneumonia, C. pecorum)* infection, which involves administering a prophylactic or therapeutic amount of a vaccine vector of the invention to an infected individual. Additionally, the third aspect of the invention encompasses the use of a vaccine vector of the invention in the preparation of a medicament for preventing and/or treating *Chlamydia* infection.

As used herein, a vaccine vector expresses one or several polypeptides or derivatives of the invention. The vaccine vector may express additionally a cytokine, such as interleukin-2 (IL-2) or interleukin-12 (IL-12), that enhances the immune response (adjuvant effect). It is understood that each of the components to be expressed is placed under the control of elements required for expression in a mammalian cell.

Consistent with the third aspect of the invention is a composition comprising several vaccine vectors, each of them capable of expressing a polypeptide or derivative of the invention. A composition may also comprise a vaccine vector capable of expressing an additional *Chlamydia* antigen, or a subunit, fragment, homolog, mutant, or derivative thereof; optionally together with or a cytokine such as IL-2 or IL-12.

A general principle is that recognition of a particular antigen is not in itself sufficient to produce an effective immune response. In some cases, a cell-mediated response is appropriate; in others, antibody.

Antigens of microorganisms vary considerably in their accessibility to cells of the immune system. Antigens which normally occur inside a pathogen may become accessible only when the pathogen or an infected cell is killed. Even antigens expressed at the cell surface may present only a limited range of their potential epitopes for antibody binding, depending on their orientation in the membrane. Protective structures, such as bacterial capsules, further limit the effective recognition of epitopes.

A distinction should be drawn between the overall composition of the immune response, those components of it which are important in the resolution of infection and the components which are responsible for the prevention of reinfection. In many cases, particular elements of the immune response are critically important; for example, cell-mediated immunity in leprosy. Even when considering a particular effector system, the response directed against some antigens is often much more effective than the responses to others. Immune responses to particular microbial antigens have different degrees of relevance to anti-microbial immunity, depending on the nature of the organism, it pathogenicity and the nature of the immune response it initiates.

The primary effectors against extracellular pathogens are antibody and complement. Binding of antibody to receptors on the pathogen can prevent it from attaching to its target cell. Antibody alone, or more effectively in association with complement, opsonizes pathogens for uptake by phagocytes expressing Fc receptors and complement receptors CR1 and CR3. Usually this will lead to intracellular destruction of the pathogen but if the phagocyte is unable to destroy it and is a facultative host cell, then antibody may actually promote the spread of infection. Such an eventuality, however, depends on the dynamic balance between the actions of the humoral and cell-mediated immune responses.

Sometimes effective antibodies must be of the right class to activate appropriate effectors. The important antigens are those involved in evasion of immune effector mechanisms; that is, pili, fimbriae and capsular antigens which constitute the major antigens of the outer layer of bacteria. Often epitope specificity is important, since it determines whether complement is deposited in a position to damage the outer membrane. There are also numerous protein antigens which can induce an antibody response; however, although the antibody response is partly species-specific and may be diagnostically useful, it is largely irrelevant to immunity. This is most obvious in lepromatous leprosy, where the patients have weak cell-mediated immunity, high levels of specific antibody and tissues heavily infected with bacteria.

In some cases, a particular type of antibody response is mandatory for clearance of the pathogen. This is true of many bacterial infections, where specific antibodies to surface antigens are necessary to neutralize the bacterial defences and opsonize the bacteria for phagocytes.

There are also cases where responses to individual antigens are essential for host immunity. The simplest examples are the toxins produced by the causative agents of diphtheria, tetanus and clostridial enteritis. The damage produced directly by the infectious agent in these diseases is slight by comparison with that produced by the secreted toxins. Consequently, protection against these conditions involves immunization to toxoids. Nevertheless, the immune system must still eradicate the primary site of the bacterial infection if the disease is to be resolved. The target antigens for bactericidal antibodies are extremely diverse and include LPS, capsular polysaccharides and other outer membrane proteins. Virulence factors can also provide good immunogens in a vaccine.

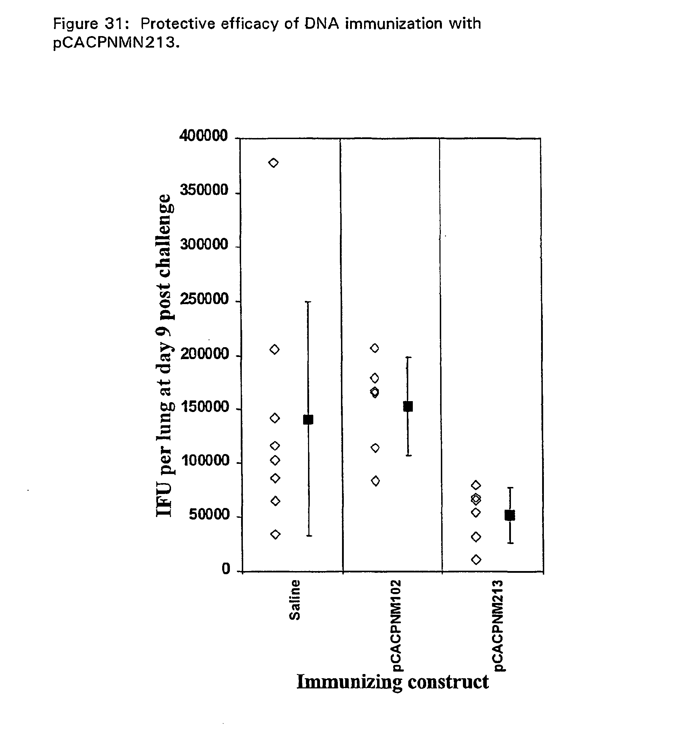

Tables 1, 3, 4, 6, 8, 10, 12, 14, 16 and 18, as well as corresponding Figures 31 to 40, demonstrate that the polypeptides disclosed herein are immunogenic. Furthermore, these Figures demonstrate that the polypeptides disclosed herein confer immunoprotection from *Chlamydia* infection, as evidenced by accelerated clearance of pulmonary infection. Such reduction in the severity of effects of Chlamydia infection is evidence that the polypeptides have generated an active functional immune response against the pathogen, rather than a mere antibody response against the antigen.

Animal models have been used to define the immunobiologic feature of *C. trachomatis* infection. The mouse model is particularly informative, largely because of the ready availability of immune reagents for murine studies and the development of transgenic and knockout (KO) mice. *C. trachomatis* mouse pneumonitis (MoPn) is the most widely tested biovar among the three *C. trachomatis* biovars (trachoma, lymphogranuloma venereum, and MoPn). Although human biovars have also been used in animal models, they normally require high inocula or pretreatment with progesterone. MoPn, which was originally isolated from mouse tissues, is thought to be a natural murine pathogen and thus offers an evolutionarily adapted pathogen for analysis of host-pathogen interactions.

The significant progress in chlamydial immunobiology based on murine models of MoPn infection has extended and clarified recent immunoepidemiologic studies in humans (Yang and Brunham (1998) Can J Infect Dis; 9:99-108). In particular, since the discovery of T helper (Th) 1 and 2 subsets, cytokine patterns have been shown to be critical in the regulation of immune responses to a variety of infectious agents including chlamydiae. Clinical investigation has shown that trachoma patients with severe conjunctival scarring have impaired cell-mediated immune responses to *C. trachomatis* and high IgG antibody titers (Yang and Brunham (1999) Curr Opin Infect Dis; 12:47-52). Cytokine analysis shows increased interleukin (IL)-4 and reduced interferon (IFN)-γ production in subjects with scarring disease due to *C. trachomatis* infection compared with controls without scarring disease.

Vaccination methods for treating or preventing infection in a mammal comprises use of a vaccine vector of the invention to be administered by any conventional route, particularly to a mucosal (*e.g.*, ocular, intranasal, oral, gastric, pulmonary, intestinal, rectal, vaginal, or urinary tract) surface or *via* the parenteral (*e.g.*, subcutaneous, intradermal, intramuscular, intravenous, or intraperitoneal) route. Preferred routes depend upon the choice of the vaccine vector. Treatment may be effected in a single dose or repeated at intervals. The appropriate dosage depends on various parameters understood by skilled artisans such as the vaccine vector itself, the route of administration or the condition of the mammal to be vaccinated (weight, age and the like).

Live vaccine vectors available in the art include viral vectors such as adenoviruses and poxviruses as well as bacterial vectors, *e.g., Shigella, Salmonella, Vibrio cholerae, Lactobacillus,* Bacille bilié de Calmette-Guérin (BCG), and *Streptococcus.*

An example of an adenovirus vector, as well as a method for constructing an adenovirus vector capable of expressing a DNA molecule of the invention, are described in U.S. Patent No. 4,920,209. Poxvirus vectors include vaccinia and canary pox virus, described in U.S. Patent No. 4, 722, 848 and U.S. Patent No. 5,364,773, respectively. (Also see, *e.g.*, Tartaglia et al., Virology (1992) 188:217) for a description of a vaccinia virus vector and Taylor et al, Vaccine (1995) 13:539 for a reference of a canary pox.) Poxvirus vectors capable of expressing a polynucleotide of the invention are obtained by homologous recombination as described in Kieny et al., Nature (1984) 312:163 so that the polynucleotide of the invention is inserted in the viral genome under appropriate conditions for expression in mammalian cells. Generally, the dose of vaccine viral vector, for therapeutic or prophylactic use, can be of from about 1x10⁴ to about 1x10¹¹, advantageously from about 1x10⁷ to about 1x10¹⁰, preferably of from about 1x10⁷ to about 1x10⁹ plaque-forming units per kilogram. Preferably, viral vectors are administered parenterally; for example, in 3 doses, 4 weeks apart. It is preferable to avoid adding a chemical adjuvant to a composition containing a viral vector of the invention and thereby minimizing the immune response to the viral vector itself.

Non-toxicogenic *Vibrio cholerae* mutant strains that are useful as a live oral vaccine are known. Mekalanos et al., Nature (1983) 306:551 and U.S. Patent No. 4,882,278 describe strains which have a substantial amount of the coding sequence of each of the two *ctxA* alleles deleted so that no functional *cholerae* toxin is produced. WO 92/11354 describes a strain in which the *irgA* locus is inactivated by mutation; this mutation can be combined in a single strain with ctxA mutations. WO 94/01533 describes a deletion mutant lacking functional ctxA and *attRS1* DNA sequences. These mutant strains are genetically engineered to express heterologous antigens, as described in WO 94/19482. An effective vaccine dose of a *Vibrio cholerae* strain capable of expressing a polypeptide or polypeptide derivative encoded by a DNA molecule of the invention contains about 1x10⁵ to about 1x10⁹, preferably about 1x10⁶ to about 1x10⁸, viable bacteria in a volume appropriate for the selected route of administration. Preferred routes of administration include all mucosal routes; most preferably, these vectors are administered intranasally or orally.

Attenuated *Salmonella typhimurium* strains, genetically engineered for recombinant expression of heterologous antigens or not, and their use as oral vaccines are described in Nakayama et al. (Bio/Technology (1988) 6:693) and WO 92/11361. Preferred routes of administration include all mucosal routes; most preferably, these vectors are administered intranasally or orally.

Other bacterial strains used as vaccine vectors in the context of the present invention are described for *Shigella flexneri* in High et al., EMBO (1992) 11:1991 and Sizemore et al., Science (1995) 270:299; for *Streptococcus gordonii* in Medaglini et al., Proc. Natl. Acad. Sci. USA (1995) 92:6868; and for Bacille Calmette Guerin in Flynn J.L., Cell. Mol. Biol. (1994) 40 (suppl. I) :31, WO 88/06626, WO 90/00594, WO 91/13157, WO 92/01796, and WO 92/21376.

In bacterial vectors, the polynucleotide of the invention is inserted into the bacterial genome or remains in a free state as part of a plasmid.

The composition comprising a vaccine bacterial vector of the present invention may further contain an adjuvant. A number of adjuvants are known to those skilled in the art. Preferred adjuvants are selected as provided below.

Accordingly, a fourth aspect of the invention provides (i) a composition of matter comprising a polynucleotide of the invention, together with a diluent or carrier; (ii) a pharmaceutical composition comprising a therapeutically or prophylactically effective amount of a polynucleotide of the invention; (iii) a method for inducing an immune response against *Chlamydia* in a mammal by administration of an immunogenically effective amount of a polynucleotide of the invention to elicit a protective immune response to *Chlamydia:* and particularly, (iv) a method for preventing and/or treating a *Chlamydia (e.g., C. trachomatis, C. psittaci, C. pneumoniae, or C. pecorum)* infection, by administering a prophylactic or therapeutic amount of a polynucleotide of the invention to an infected individual. Additionally, the fourth aspect of the invention encompasses the use of a polynucleotide of the invention in the preparation of a medicament for preventing and/or treating *Chlamydia* infection. A preferred use includes the use of a DNA molecule placed under conditions for expression in a mammalian cell, especially in a plasmid that is unable to replicate in mammalian cells and to substantially integrate in a mammalian genome.

Use of the polynucleotides of the invention include their administration to a mammal as a vaccine, for therapeutic or prophylactic purposes. Such polynucleotides are used in the form of DNA as part of a plasmid that is unable to replicate in a mammalian cell and unable to integrate into the mammalian genome. Typically, such a DNA molecule is placed under the control of a promoter suitable for expression in a mammalian cell. The promoter functions either ubiquitously or tissue-specifically. Examples of non-tissue specific promoters include the early Cytomegalovirus (CMV) promoter (described in U.S. Patent No. 4,168,062) and the Rous Sarcoma Virus promoter (described in Norton & Coffin, Molec. Cell Biol. (1985) 5:281). An example of a tissue-specific promoter is the desmin promoter which drives expression in muscle cells (Li et al., Gene (1989) 78:243, Li & Paulin, J. Biol. Chem. (1991) 266:6562 and Li & Paulin, J. Biol. Chem. (1993) 268:10403). Use of promoters is well-known to those skilled in the art. Useful vectors are described in numerous publications, specifically WO 94/21797 and Hartikka et al., Human Gene Therapy (1996) 7:1205.

Polynucleotides of the invention which are used as vaccines encode either a precursor or a mature form of the corresponding polypeptide. In the precursor form, the signal peptide is either homologous or heterologous. In the latter case, a eucaryotic leader sequence such as the leader sequence of the tissue-type plasminogen factor (tPA) is preferred.

As used herein, a composition of the invention contains one or several polynucleotides with optionally at least one additional polynucleotide encoding another *Chlamydia* antigen such as urease subunit A, B, or both, or a fragment, derivative, mutant, or analog thereof. The composition may also contain an additional polynucleotide encoding a cytokine, such as interleukin-2 (IL-2) or interleukin-12 (IL-12) so that the immune response is enhanced. These additional polynucleotides are placed under appropriate control for expression. Advantageously, DNA molecules of the invention and/or additional DNA molecules to be included in the same composition, are present in the same plasmid.

Standard techniques of molecular biology for preparing and purifying polynucleotides are used in the preparation of polynucleotide therapeutics of the invention. For use as a vaccine, a polynucleotide of the invention is formulated according to various methods outlined below.

One method utililizes the polynucleotide in a naked form, free of any delivery vehicles. Such a polynucleotide is simply diluted in a physiologically acceptable solution such as sterile saline or sterile buffered saline, with or without a carrier. When present, the carrier preferably is isotonic, hypotonic, or weakly hypertonic, and has a relatively low ionic strength, such as provided by a sucrose solution, *e.g.*, a solution containing 20% sucrose.

An alternative method utilizes the polynucleotide in association with agents that assist in cellular uptake. Examples of such agents are (i) chemicals that modify cellular permeability, such as bupivacaine (see, *e.g.*, WO 94/16737), (ii) liposomes for encapsulation of the polynucleotide, or (iii) cationic lipids or silica, gold, or tungsten microparticles which associate themselves with the polynucleotides.

Anionic and neutral liposomes are well-known in the art (see, *e.g.*, Liposomes: A Practical Approach, RPC New Ed, IRL press (1990), for a detailed description of methods for making liposomes) and are useful for delivering a large range of products, including polynucleotides.

Cationic lipids are also known in the art and are commonly used for gene delivery. Such lipids include Lipofectin^{™} also known as DOTMA (N-[1-(2,3-dioleyloxy)propyl]-N,N,N-trimethylammonium chloride), DOTAP (1,2-bis(oleyloxy)-3-(trimethylammonio)propane), DDAB (dimethyldioctadecylammonium bromide), DOGS (dioctadecylamidologlycyl spermine) and cholesterol derivatives such as DC-Chol (3 beta-(N-(N',N'-dimethyl aminomethane)-carbamoyl) cholesterol). A description of these cationic lipids can be found in EP 187,702, WO 90/11092, U.S. Patent No. 5,283,185, WO 91/15501, WO 95/26356, and U.S. Patent No. 5,527,928. Cationic lipids for gene delivery are preferably used in association with a neutral lipid such as DOPE (dioleyl phosphatidylethanolamine), as described in WO 90/11092 as an example.

Formulation containing cationic liposomes may optionally contain other transfection-facilitating compounds. A number of them are described in WO 93/18759, WO 93/19768, WO 94/25608, and WO 95/02397. They include spermine derivatives useful for facilitating the transport of DNA through the nuclear membrane (see, for example, WO 93/18759) and membrane-permeabilizing compounds such as GALA, Gramicidine S, and cationic bile salts (see, for example, WO 93/19768).

Gold or tungsten microparticles are used for gene delivery, as described in WO 91/00359, WO 93/17706, and Tang et al. Nature (1992) 356:152. The microparticle-coated polynucleotide is injected *via* intradermal or intraepidermal routes using a needleless injection device ("gene gun"), such as those described in U.S. Patent No. 4,945,050, U.S. Patent No. 5,015,580, and WO 94/24263.

The amount of DNA to be used in a vaccine recipient depends, *e.g.*, on the strength of the promoter used in the DNA construct, the immunogenicity of the expressed gene product, the condition of the mammal intended for administration (*e.g.*, the weight, age, and general health of the mammal), the mode of administration, and the type of formulation. In general, a therapeutically or prophylactically effective dose from about 1 µg to about 1 mg, preferably, from about 10 µg to about 800 µg and, more preferably, from about 25 µg to about 250 µg, can be administered to human adults. The administration can be achieved in a single dose or repeated at intervals.

The route of administration is any conventional route used in the vaccine field. As general guidance, a polynucleotide of the invention is administered *via* a mucosal surface, *e.g.*, an ocular, intranasal, pulmonary, oral, intestinal, rectal, vaginal, and urinary tract surface; or *via* a parenteral route, *e.g.*, by an intravenous, subcutaneous, intraperitoneal, intradermal, intraepidermal, or intramuscular route. The choice of administration route depends on the formulation that is selected. A polynucleotide formulated in association with bupivacaine is advantageously administered into muscles. When a neutral or anionic liposome or a cationic lipid, such as DOTMA or DC-Chol; is used, the formulation can be advantageously injected *via* intravenous, intranasal (aerosolization), intramuscular, intradermal, and subcutaneous routes. A polynucleotide in a naked form can advantageously be administered *via* the intramuscular, intradermal, or sub-cutaneous routes.

Although not absolutely required, such a composition can also contain an adjuvant. If so, a systemic adjuvant that does not require concomitant administration in order to exhibit an adjuvant effect is preferable such as, *e.g.*, QS21, which is described in U.S. Patent No. 5,057,546.

The sequence information provided in the present application enables the design of specific nucleotide probes and primers that are used for diagnostic purposes. Accordingly, a fifth aspect of the invention provides a nucleotide probe or primer having a sequence found in or derived by degeneracy of the genetic code from a sequence shown in any one of SEQ ID Nos: 1, 3, 5, 7, 9, 11, 13, 15, 17 and 19.

The term "probe" as used in the present application refers to DNA (preferably single stranded) or RNA molecules (or modifications or combinations thereof) that hybridize under the stringent conditions, as defined above, to nucleic acid molecules having any one of SEQ ID Nos: 1, 3, 5, 7, 9, 11, 13, 15, 17 and 19 or to a sequence homologous to any one of SEQ ID Nos: 1, 3, 5, 7, 9, 11, 13, 15, 17 and 19, or to its complementary or anti-sense sequence. Generally, probes are significantly shorter than full-length sequences. Such probes contain from about 5 to about 100, preferably from about 10 to about 80, nucleotides. In particular, probes have sequences that are at least 75%, preferably at least 80% or 85%, more preferably 90% or 95% homologous to a portion of any one of SEQ ID Nos: 1, 3, 5, 7, 9, 11, 13, 15, 17 and 19 or that are complementary to such sequences. Probes may contain modified bases such as inosine, methyl-5-deoxycytidine, deoxyuridine, dimethylamino-5-deoxyuridine, or diamino-2, 6-purine. Sugar or phosphate residues may also be modified or substituted. For example, a deoxyribose residue may be replaced by a polyamide (Nielsen et al., Science (1991) 254:1497) and phosphate residues may be replaced by ester groups such as diphosphate, alkyl, arylphosphonate and phosphorothioate esters. In addition, the 2'-hydroxyl group on ribonucleotides may be modified by including such groups as alkyl groups.

Probes of the invention are used in diagnostic tests, as capture or detection probes. Such capture probes are conventionally immobilized on a solid support, directly or indirectly, by covalent means or by passive adsorption. A detection probe is labelled by a detection marker selected from: radioactive isotopes, enzymes such as peroxidase, alkaline phosphatase, and enzymes able to hydrolyze a chromogenic, fluorogenic, or luminescent substrate, compounds that are chromogenic, fluorogenic, or luminescent, nucleotide base analogs, and biotin.

Probes of the invention are used in any conventional hybridization technique, such as dot blot (Maniatis et al., Molecular Cloning: A Laboratory Manual (1982) Cold Spring Harbor Laboratory Press, Cold Spring Harbor, New York), Southern blot (Southern, J. Mol. Biol. (1975) 98:503), northern blot (identical to Southern blot with the exception that RNA is used as a target), or the sandwich technique (Dunn et al., Cell (1977) 12:23). The latter technique involves the use of a specific capture probe and/or a specific detection probe with nucleotide sequences that at least partially differ from each other.

A primer is a probe of usually about 10 to about 40 nucleotides that is used to initiate enzymatic polymerization of DNA in an amplification process (*e.g.*, PCR), in an elongation process, or in a reverse transcription method. Primers used in diagnostic methods involving PCR are labeled by methods known in the art.

As described herein, the invention also encompasses (i) a reagent comprising a probe of the invention for detecting and/or identifying the presence of *Chlamydia* in a biological material; (ii) a method for detecting and/or identifying the presence of *Chlamydia* in a biological material, in which (a) a sample is recovered or derived from the biological material, (b) DNA or RNA is extracted from the material and denatured, and (c) exposed to a probe of the invention, for example, a capture, detection probe or both, under stringent hybridization conditions, such that hybridization is detected; and (iii) a method for detecting and/or identifying the presence of *Chlamydia* in a biological material, in which (a) a sample is recovered or derived from the biological material, (b) DNA is extracted therefrom, (c) the extracted DNA is primed with at least one, and preferably two, primers of the invention and amplified by polymerase chain reaction, and (d) the amplified DNA fragment is produced.

It is apparent that disclosure of a polynucleotide sequence of any one of SEQ ID Nos: 1, 3, 5, 7, 9, 11, 13, 15, 17 and 19, its homologs and partial sequences enable their corresponding amino acid sequences. Accordingly, a sixth aspect of the invention features a substantially purified polypeptide or polypeptide derivative having an amino acid sequence encoded by a polynucleotide of the invention.

A "substantially purified polypeptide" as used herein is defined as a polypeptide that is separated from the environment in which it naturally occurs and/or that is free of the majority of the polypeptides that are present in the environment in which it was synthesized. For example, a substantially purified polypeptide is free from cytoplasmic polypeptides. Those skilled in the art would readily understand that the polypeptides of the invention may be purified from a natural source, *i.e.*, a *Chlamydia* strain, or produced by recombinant means.

Consistent with the sixth aspect of the invention are polypeptides, homologs or fragments which are modified or treated to enhance their immunogenicity in the target animal, in whom the polypeptide, homolog or fragments are intended to confer protection against *Chlamydia.* Such modifications or treatments include: amino acid substitutions with an amino acid derivative such as 3-methyhistidine, 4-hydroxyproline, 5-hydroxylysine etc., modifications or deletions which are carried out after preparation of the polypeptide, homolog or fragment, such as the modification of free amino, carboxyl or hydroxyl side groups of the amino acids.

Identification of homologous polypeptides or polypeptide derivatives encoded by polynucleotides of the invention which have specific antigenicity is achieved by screening for cross-reactivity with an antiserum raised against the polypeptide of reference having an amino acid sequence of any one of SEQ ID Nos: 2, 4, 6, 8, 10, 12, 14, 16, 18 and 20. The procedure is as follows: a monospecific hyperimmune antiserum is raised against a purified reference polypeptide, a fusion polypeptide (for example, an expression product of MBP, GST, or His-tag systems, the description and instructions for use of which are contained in Invitrogen product manuals for pcDNA3.1/Myc-His(+) A, B, and C and for the Xpress^{™} System Protein Purification), or a synthetic peptide predicted to be antigenic. Where an antiserum is raised against a fusion polypeptide, two different fusion systems are employed. Specific antigenicity can be determined according to a number of methods, including Western blot (Towbin et al., Proc. Natl. Acad. Sci. USA (1979) 76:4350), dot blot, and ELISA, as described below.

In a Western blot assay, the product to be screened, either as a purified preparation or a total E. *coli* extract, is submitted to SDS-Page electrophoresis as described by Laemmli (Nature (1970) 227:680). After transfer to a nitrocellulose membrane, the material is further incubated with the monospecific hyperimmune antiserum diluted in the range of dilutions from about 1:5 to about 1:5000, preferably from about 1:100 to about 1:500. Specific antigenicity is shown once a band corresponding to the product exhibits reactivity at any of the dilutions in the above range.

In an ELISA assay, the product to be screened is preferably used as the coating antigen. A purified preparation is preferred, although a whole cell extract can also be used. Briefly, about 100 µl of a preparation at about 10 µg protein/ml are distributed into wells of a 96-well polycarbonate ELISA plate. The plate is incubated for 2 hours at 37°C then overnight at 4°C. The plate is washed with phosphate buffer saline (PBS) containing 0.05% Tween 20 (PBS/Tween buffer). The wells are saturated with 250 µl PBS containing 1% bovine serum albumin (BSA) to prevent non-specific antibody binding. After 1 hour incubation at 37°C, the plate is washed with PBS/Tween buffer. The antiserum is serially diluted in PBS/Tween buffer containing 0.5% BSA. 100 µl of dilutions are added per well. The plate is incubated for 90 minutes at 37°C, washed and evaluated according to standard procedures. For example, a goat anti-rabbit peroxidase conjugate is added to the wells when specific antibodies were raised in rabbits. Incubation is carried out for 90 minutes at 37°C and the plate is washed. The reaction is developed with the appropriate substrate and the reaction is measured by colorimetry (absorbance measured spectrophotometrically). Under the above experimental conditions, a positive reaction is shown by O.D. values greater than a non immune control serum.

In a dot blot assay, a purified product is preferred, although a whole cell extract can also be used. Briefly, a solution of the product at about 100 µg/ml is serially two-fold diluted in 50 mM Tris-HCl (pH 7.5). 100 µl of each dilution are applied to a nitrocellulose membrane 0.45 µm set in a 96-well dot blot apparatus (Biorad). The buffer is removed by applying vacuum to the system. Wells are washed by addition of 50 mM Tris-HCl (pH 7.5) and the membrane is air-dried. The membrane is saturated in blocking buffer (50 mM Tris-HCl (pH 7.5) 0.15 M NaCl, 10 g/L skim milk) and incubated with an antiserum dilution from about 1:50 to about 1:5000, preferably about 1:500. The reaction is revealed according to standard procedures. For example, a goat anti-rabbit peroxidase conjugate is added to the wells when rabbit antibodies are used. Incubation is carried out 90 minutes at 37°C and the blot is washed. The reaction is developed with the appropriate substrate and stopped. The reaction is measured visually by the appearance of a colored spot, *e.g.*, by colorimetry. Under the above experimental conditions, a positive reaction is shown once a colored spot is associated with a dilution of at least about 1:5, preferably of at least about 1:500.

Therapeutic or prophylactic efficacy of a polypeptide or derivative of the invention can be evaluated as described below. A seventh aspect of the invention provides (i) a composition of matter comprising a polypeptide of the invention together with a diluent or carrier; specifically (ii) a pharmaceutical composition containing a therapeutically or prophylactically effective amount of a polypeptide of the invention; (iii) a method for inducing an immune response against *Chlamydia* in a mammal, by administering to the mammal an immunogenically effective amount of a polypeptide of the invention to elicit a protective immune response to *Chlamydia;* and particularly, (iv) a method for preventing and/or treating a *Chlamydia (e.g., C. trachomatis. C. psittaci, C. pneumoniae.* or *C. pecorum)* infection, by administering a prophylactic or therapeutic amount of a polypeptide of the invention to an infected individual. Additionally, the seventh aspect of the invention encompasses the use of a polypeptide of the invention in the preparation of a medicament for preventing and/or treating *Chlamydia* infection.

As used herein, the immunogenic compositions of the invention are administered by conventional routes known the vaccine field, in particular to a mucosal (*e.g.*, ocular, intranasal, pulmonary, oral, gastric, intestinal, rectal, vaginal, or urinary tract) surface or *via* the parenteral (*e.g.*, subcutaneous, intradermal, intramuscular, intravenous, or intraperitoneal) route. The choice of administration route depends upon a number of parameters, such as the adjuvant associated with the polypeptide. If a mucosal adjuvant is used, the intranasal or oral route is preferred. If a lipid formulation or an aluminum compound is used, the parenteral route is preferred with the sub-cutaneous or intramuscular route being most preferred. The choice also depends upon the nature of the vaccine agent. For example, a polypeptide of the invention fused to CTB or LTB is best administered to a mucosal surface.

As used herein, the composition of the invention contains one or several polypeptides or derivatives of the invention. The composition optionally contains at least one additional *Chlamydia* antigen, or a subunit, fragment, homolog, mutant, or derivative thereof.

For use in a composition of the invention, a polypeptide or derivative thereof is formulated into or with liposomes, preferably neutral or anionic liposomes, microspheres, ISCOMS, or virus-like-particles (VLPs) to facilitate delivery and/or enhance the immune response. These compounds are readily available to one skilled in the art; for example, see Liposomes: A Practical Approach, RCP New Ed, IRL press (1990).

Adjuvants other than liposomes and the like are also used and are known in the art. Adjuvants may protect the antigen from rapid dispersal by sequestering it in a local deposit, or they may contain substances that stimulate the host to secrete factors that are chemotactic for macrophages and other components of the immune system. An appropriate selection can conventionally be made by those skilled in the art, for example, from those described below (under the eleventh aspect of the invention).

Treatment is achieved in a single dose or repeated as necessary at intervals, as can be determined readily by one skilled in the art. For example, a priming dose is followed by three booster doses at weekly or monthly intervals. An appropriate dose depends on various parameters including the recipient (*e.g.*, adult or infant), the particular vaccine antigen, the route and frequency of administration, the presence/absence or type of adjuvant, and the desired effect (*e.g.*, protection and/or treatment), as can be determined by one skilled in the art. In general, a vaccine antigen of the invention is administered by a mucosal route in an amount from about 10 µg to about 500 mg, preferably from about 1 mg to about 200 mg. For the parenteral route of administration, the dose usually does not exceed about 1 mg, preferably about 100 µg.

When used as vaccine agents, polynucleotides and polypeptides of the invention may be used sequentially as part of a multistep immunization process. For example, a mammal is initially primed with a vaccine vector of the invention such as a pox virus, *e.g., via* the parenteral route, and then boosted twice with the polypeptide encoded by the vaccine vector, *e.g.*, *via* the mucosal route. In another example, liposomes associated with a polypeptide or derivative of the invention is also used for priming, with boosting being carried out mucosally using a soluble polypeptide or derivative of the invention in combination with a mucosal adjuvant (*e.g.*, LT).

A polypeptide derivative of the invention is also used in accordance with the seventh aspect as a diagnostic reagent for detecting the presence of anti-*Chlamydia* antibodies, *e.g.*, in a blood sample. Such polypeptides are about 5 to about 80, preferably about 10 to about 50 amino acids in length. They are either labeled or unlabeled, depending upon the diagnostic method. Diagnostic methods involving such a reagent are described below.

Upon expression of a DNA molecule of the invention, a polypeptide or polypeptide derivative is produced and purified using known laboratory techniques. As described above, the polypeptide or polypeptide derivative may be produced as a fusion protein containing a fused tail that facilitates purification. The fusion product is used to immunize a small mammal, *e.g.*, a mouse or a rabbit, in order to raise antibodies against the polypeptide or polypeptide derivative (monospecific antibodies). Accordingly, an eighth aspect of the invention provides a monospecific antibody that binds to a polypeptide or polypeptide derivative of the invention.

By "monospecific antibody" is meant an antibody that is capable of reacting with a unique naturally-occurring *Chlamydia* polypeptide. An antibody of the invention is either polyclonal or monoclonal. Monospecific antibodies may be recombinant, *e.g.*, chimeric (*e.g.*, constituted by a variable region of murine origin associated with a human constant region), humanized (a human immunoglobulin constant backbone together with hypervariable region of animal, *e.g.*, murine, origin), and/or single chain. Both polyclonal and monospecific antibodies may also be in the form of immunoglobulin fragments, *e.g.*, F(ab)'2 or Fab fragments. The antibodies of the invention are of any isotype, *e.g.*, IgG or IgA, and polyclonal antibodies are of a single isotype or a mixture of isotypes.

Antibodies against the polypeptides, homologs or fragments of the present invention are generated by immunization of a mammal with a composition comprising said polypeptide, homolog or fragment. Such antibodies may be polyclonal or monoclonal. Methods to produce polyclonal or monoclonal antibodies are well known in the art. For a review, see "Antibodies, A Laboratory Manual, Cold Spring Harbor Laboratory, Eds. E. Harlow and D. Lane (1988), and D.E. Yelton et al., 1981. Ann. Rev. Biochem. 50:657-680. For monoclonal antibodies, see Kohler & Milstein (1975) Nature 256:495-497.

The antibodies of the invention, which are raised to a polypeptide or polypeptide derivative of the invention, are produced and identified using standard immunological assays, *e.g.*, Western blot analysis, dot blot assay, or ELISA (see, *e.g.*, Coligan et al., Current Protocols in Immunology (1994) John Wiley & Sons, Inc., New York, NY). The antibodies are used in diagnostic methods to detect the presence of a *Chlamydia* antigen in a sample, such as a biological sample. The antibodies are also used in affinity chromatography for purifying a polypeptide or polypeptide derivative of the invention. As is discussed further below, such antibodies may be used in prophylactic and therapeutic passive immunization methods.

Accordingly, a ninth aspect of the invention provides (i) a reagent for detecting the presence of *Chlamydia* in a biological sample that contains an antibody, polypeptide, or polypeptide derivative of the invention; and (ii) a diagnostic method for detecting the presence of *Chlamydia* in a biological sample, by contacting the biological sample with an antibody, a polypeptide, or a polypeptide derivative of the invention, such that an immune complex is formed, and by detecting such complex to indicate the presence of *Chlamydia* in the sample or the organism from which the sample is derived.

Those skilled in the art will readily understand that the immune complex is formed between a component of the sample and the antibody, polypeptide, or polypeptide derivative, whichever is used, and that any unbound material is removed prior to detecting the complex. It is understood that a polypeptide reagent is useful for detecting the presence of anti-*Chlamydia* antibodies in a sample, *e.g.*, a blood sample, while an antibody of the invention is used for screening a sample, such as a gastric extract or biopsy, for the presence of *Chlamydia* polypeptides.

For diagnostic applications, the reagent (*i.e.*, the antibody, polypeptide, or polypeptide derivative of the invention) is either in a free state or immobilized on a solid support, such as a tube, a bead, or any other conventional support used in the field. Immobilization is achieved using direct or indirect means. Direct means include passive adsorption (non-covalent binding) or covalent binding between the support and the reagent. By "indirect means" is meant that an anti-reagent compound that interacts with a reagent is first attached to the solid support. For example, if a polypeptide reagent is used, an antibody that binds to it can serve as an anti-reagent, provided that it binds to an epitope that is not involved in the recognition of antibodies in biological samples. Indirect means may also employ a ligand-receptor system, for example, where a molecule such as a vitamin is grafted onto the polypeptide reagent and the corresponding receptor immobilized on the solid phase. This is illustrated by the biotin-streptavidin system. Alternatively, a peptide tail is added chemically or by genetic engineering to the reagent and the grafted or fused product immobilized by passive adsorption or covalent linkage of the peptide tail.

Such diagnostic agents may be included in a kit which also comprises instructions for use. The reagent is labeled with a detection means which allows for the detection of the reagent when it is bound to its target. The detection means may be a fluorescent agent such as fluorescein isocyanate or fluorescein isothiocyanate, or an enzyme such as horse radish peroxidase or luciferase or alkaline phosphatase, or a radioactive element such as ¹²⁵I or ⁵¹Cr.

Accordingly, a tenth aspect of the invention provides a process for purifying, from a biological sample, a polypeptide or polypeptide derivative of the invention, which involves carrying out antibody-based affinity chromatography with the biological sample, wherein the antibody is a monospecific antibody of the invention.

For use in a purification process of the invention, the antibody is either polyclonal or monospecific, and preferably is of the IgG type. Purified IgGs is prepared from an antiserum using standard methods (see, *e.g.,* Coligan et al., Current Protocols in Immunology (1994)John Wiley & Sons, Inc., New York, NY.). Conventional chromatography supports, as well as standard methods for grafting antibodies, are described in, *e.g.*, Antibodies: A Laboratory Manual, D. Lane, E. Harlow, Eds. (1988) and outlined below.

Briefly, a biological.sample, such as an *C. pneumoniae* extract preferably in a buffer solution, is applied to a chromatography material, preferably equilibrated with the buffer used to dilute the biological sample so that the polypeptide or polypeptide derivative of the invention (*i.e.*, the antigen) is allowed to adsorb onto the material. The chromatography material, such as a gel or a resin coupled to an antibody of the invention, is in either a batch form or a column. The unbound components are washed off and the antigen is then eluted with an appropriate elution buffer, such as a glycine buffer or a buffer containing a chaotropic agent, *e.g.*, guanidine HCl, or high salt concentration (*e.g.*, 3 M MgCl₂). Eluted fractions are recovered and the presence of the antigen is detected, *e.g.*, by measuring the absorbance at 280 nm.

An eleventh aspect of the invention provides (i) a composition of matter comprising a monospecific antibody of the invention, together with a diluent or carrier; (ii) a pharmaceutical composition comprising a therapeutically or prophylactically effective amount of a monospecific antibody of the invention, and (iii) a method for treating or preventing a *Chlamydia (e.g., C. trachomatis, C. psittaci, C. pneumoniae* or *C. pecorum)* infection, by administering a therapeutic or prophylactic amount of a monospecific antibody of the invention to an infected individual. Additionally, the eleventh aspect of the invention encompasses the use of a monospecific antibody of the invention in the preparation of a medicament for treating or preventing *Chlamydia* infection.

The monospecific antibody is either polyclonal or monoclonal, preferably of the IgA isotype (predominantly). In passive immunization, the antibody is administered to a mucosal surface of a mammal, *e.g.*, the gastric mucosa, *e.g.*, orally or intragastrically, advantageously, in the presence of a bicarbonate buffer. Alternatively, systemic administration, not requiring a bicarbonate buffer, is carried out. A monospecific antibody of the invention is administered as a single active component or as a mixture with at least one monospecific antibody specific for a different *Chlamydia* polypeptide. The amount of antibody and the particular regimen used are readily determined by one skilled in the art. For example, daily administration of about 100 to 1,000 mg of antibodies over one week, or three doses per day of about 100 to 1,000 mg of antibodies over two or three days, are effective regimens for most purposes.

Therapeutic or prophylactic efficacy are evaluated using standard methods in the art, *e.g.*, by measuring induction of a mucosal immune response or induction of protective and/or therapeutic immunity, using, *e.g.*, the *C. pneumoniae* mouse model. Those skilled in the art will readily recognize that the *C. pneumoniae* strain of the model may be replaced with another *Chlamydia* strain. For example, the efficacy of DNA molecules and polypeptides from *C. pneumoniae* is preferably evaluated in a mouse model using *C. pneumoniae* strain. Protection is determined by comparing the degree of *Chlamydia* infection to that of a control group. Protection is shown when infection is reduced by comparison to the control group. Such an evaluation is made for polynucleotides, vaccine vectors, polypeptides and derivatives thereof, as well as antibodies of the invention.

Adjuvants useful in any of the vaccine compositions described above are as follows.

Adjuvants for parenteral administration include aluminum compounds, such as aluminum hydroxide, aluminum phosphate, and aluminum hydroxy phosphate. The antigen is precipitated with, or adsorbed onto, the aluminum compound according to standard protocols. Other adjuvants, such as RIBI (ImmunoChem, Hamilton, MT), are used in parenteral administration.

Adjuvants for mucosal administration include bacterial toxins, *e.g.*, the cholera toxin (CT), the E. *coli* heat-labile toxin (LT), the *Clostridium difficile* toxin A and the *pertussis* toxin (PT), or combinations, subunits, toxoids, or mutants thereof such as a purified preparation of native cholera toxin subunit B (CTB). Fragments, homologs, derivatives, and fusions to any of these toxins are also suitable, provided that they retain adjuvant activity. Preferably, a mutant having reduced toxicity is used. Suitable mutants are described, *e.g.*, in WO 95/17211 (Arg-7-Lys CT mutant), WO 96/06627 (Arg-192-Gly LT mutant), and WO 95/34323 (Arg-9-Lys and Glu-129-Gly PT mutant). Additional LT mutants that are used in the methods and compositions of the invention include, *e.g.*, Ser-63-Lys, Ala-69Gly, Glu-110-Asp, and Glu-112-Asp mutants. Other adjuvants, such as a bacterial monophosphoryl lipid A (MPLA) of, *e.g., E. coli, Salmonella minnesota, Salmonella typhimurium,* or *Shigella flexneri;* saponins, or polylactide glycolide (PLGA) microspheres, is also be used in mucosal administration.

Adjuvants useful for both mucosal and parenteral administrations include polyphosphazene (WO 95/02415), DC-chol (3 b-(N-(N',N'-dimethyl aminomethane)-carbamoyl) cholesterol; U.S. Patent No. 5,283,185 and WO 96/14831) and QS-21 (WO 88/09336).

Any pharmaceutical composition of the invention containing a polynucleotide, a polypeptide, a polypeptide derivative, or an antibody of the invention, is manufactured in a conventional manner. In particular, it is formulated with a pharmaceutically acceptable diluent or carrier, *e.g.*, water or a saline solution such as phosphate buffer saline. In general, a diluent or carrier is selected on the basis of the mode and route of administration, and standard pharmaceutical practice. Suitable pharmaceutical carriers or diluents, as well as pharmaceutical necessities for their use in pharmaceutical formulations, are described in *Remington's Pharmaceutical Sciences,* a standard reference text in this field and in the USP/NF.

The invention also includes methods in which *Chlamydia* infection are treated by oral administration of a *Chlamydia* polypeptide of the invention and a mucosal adjuvant, in combination with an antibiotic, an antacid, sucralfate, or a combination thereof. Examples of such compounds that can be administered with the vaccine antigen and the adjuvant are antibiotics, including, *e.g.*, macrolides, tetracyclines, and derivatives thereof (specific examples of antibiotics that can be used include azithromycin or doxicyclin or immunomodulators such as cytokines or steroids). In addition, compounds containing more than one of the above-listed components coupled together, are used. The invention also includes compositions for carrying out these methods, *i.e.*, compositions containing a *Chlamydia* antigen (or antigens) of the invention, an adjuvant, and one or more of the above-listed compounds, in a pharmaceutically acceptable carrier or diluent.

It has recently been shown that the 60kDa cysteine rich membrane protein contains a sequence cross-reactive with the murine alpha-myosin heavy chain epitope M7A-alpha, an epitope conserved in humans (Bachmaier et al., Science (1999) 283:1335). This cross-reactivity is proposed to contribute to the development of cardiovascular disease, so it may be beneficial to remove this epitope, and any other epitopes cross-reactive with human antigens, from the protein if it is to be used as a vaccine. Accordingly, a further embodiment of the present invention includes the modification of the coding sequence, for example, by deletion or substitution of the nucleotides encoding the epitope from polynucleotides encoding the protein, as to improve the efficacy and safety of the protein as a vaccine. A similar approach may be appropriate for any protective antigen found to have unwanted homologies or cross-reactivities with human antigens.

Amounts of the above-listed compounds used in the methods and compositions of the invention are readily determined by one skilled in the art. Treatment/immunization schedules are also known and readily designed by one skilled in the art. For example, the non-vaccine components can be administered on days 1-14, and the vaccine antigen + adjuvant can be administered on days 7, 14, 21, and 28.

### EXAMPLES

The above disclosure generally describes the present invention. A more complete understanding can be obtained by reference to the following specific examples. These examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitation.

### Example 1:

These examples illustrate the preparation of plasmid vectors used in immunoprotection studies.

### A. Preparation of plasmid vector pCACPNM213

The ATP-binding cassette gene was amplified from *Chlamydia pneumoniae* genomic DNA strain CWLO29 by polymerase chain reaction (PCR) using a 5' primer (5' ATAAGAATGCGGCCGCCACC**ATGAAGATGCATAGGCTTAAACC** 3'; SEQ ID No:21) and a 3' primer (5' GCGCCGGATCCC**ACTTAAGATATCGATATTTTTGAG** 3'; SEQ ID No:22). The 5' primer contains a NotI restriction site, a ribosome binding site, an initiation codon and a sequence at the 5' end of the ATP-binding cassette protein coding sequence. The 3' primer includes the sequence encoding the C-terminal sequence of the ATP-binding cassette protein gene and a BamHI restriction site. The stop codon was excluded and an additional nucleotide was inserted to obtain an in-frame fusion with the Histidine tag.

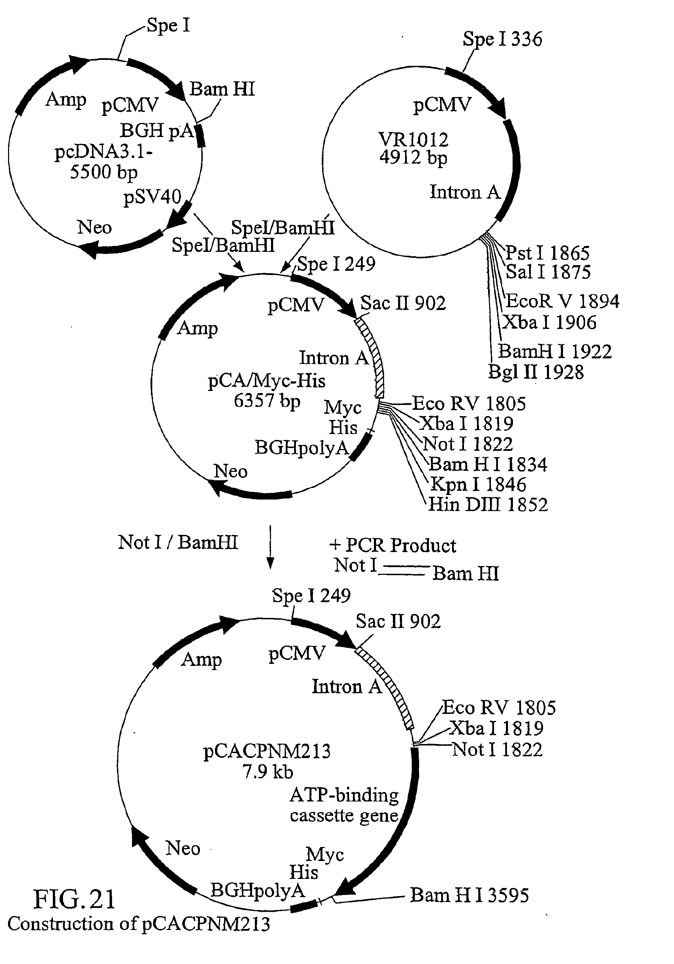

After amplification, the PCR fragment was purified using QIAquick^{™} PCR purification kit (Qiagen), digested with NotI and BamHI and cloned into the pCA-Myc-His eukaryotic expression vector described in Example 2 (Figure 21) with transcription under control of the human CMV promoter.

### B. Preparation of plasmid vector pCACPNM882

The secretory locus ORF gene was amplified from *Chlamydia pneumoniae* genomic DNA strain CWLO29 by polymerase chain reaction (PCR) using a 5' primer (5' ATAAGAATGCGGCCGCCACC**ATGCGGTTGGGAAATAAGCCTATGC** 3'; SEQ ID No:23) and a 3' primer (5' GCGCCGGTACCG**TAATTTAATACTCTTTGAAGGGC** 3'; SEQ ID No:24). The 5' primer contains a NotI restriction site, a ribosome binding site, an initiation codon and a sequence at the 5' end of the secretory locus ORF coding sequence. The 3' primer includes the sequence encoding the C-terminal sequence of the secretory locus ORF protein and a KpnI restriction site. The stop codon was excluded and an additional nucleotide was inserted to obtain an in-frame fusion with the Histidine tag.

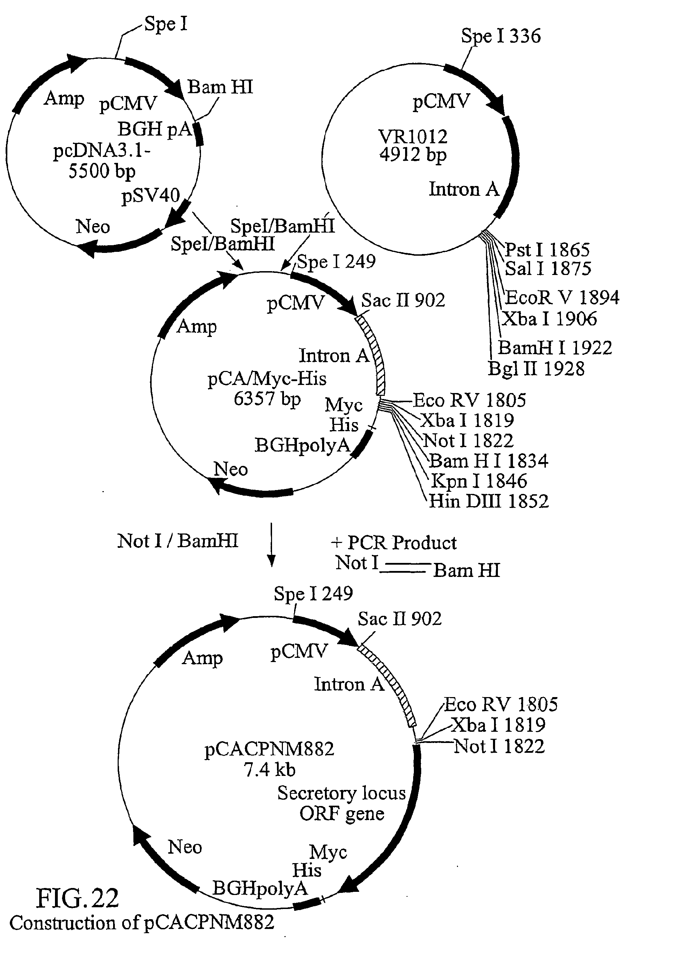

After amplification, the PCR fragment was purified using QIAquick^{™} PCR purification kit (Qiagen), digested with NotI and KpnI and cloned into the pCA-Myc-His eukaryotic expression vector described in Example 2 (Figure 22) with transcription under control of the human CMV promoter.

### C. Preparation of plasmid vector pCACPNM208

The endopeptidase gene was amplified from *Chlamydia pneumoniae* genomic DNA strain CWLO29 by polymerase chain reaction (PCR) using a 5' primer (5' ATAAGAATGCGGCCGCCACC**ATGCTCACCCTAGGCTTGGAAAGTTCTTG** 3'; SEQ ID No:25) and a 3' primer (5' GCTTTGGAGGATCCC**CGGAGAGGCTAAGGAGAATGG** 3'; SEQ ID No:26). The 5' primer contains a NotI restriction site, a ribosome binding site, an initiation codon and a sequence at the 5' end of the endopeptidase protein coding sequence. The 3' primer includes the sequence encoding the C-terminal sequence of the endopeptidase protein gene and a BamHI restriction site. The stop codon was excluded and an additional nucleotide was inserted to obtain an in-frame fusion with the Histidine tag.

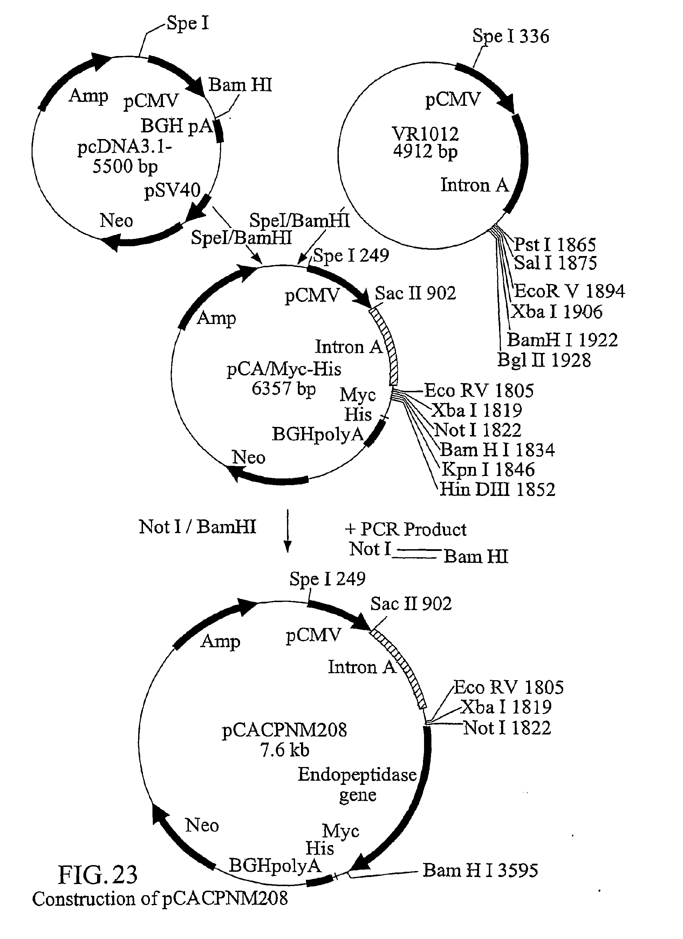

After amplification, the PCR fragment was purified using QIAquick^{™} PCR purification kit (Qiagen), digested with NotI and BamHI and cloned into the pCA-Myc-His eukaryotic expression vector described in Example 2 (Figure 23) with transcription under control of the human CMV promoter.

### D. Preparation of plasmid vector pCACPNM1096

The protease gene was amplified from *Chlamydia pneumoniae* genomic DNA strain CWL029 by polymerase chain reaction (PCR) using a 5' primer (5' ATAAGAATGCGGCCGCCACC**ATGAAAAAAGGGAAATTAGGAGCC** 3'; SEQ ID No:27) and a 3' primer (5' GCGCCGGATCCC**CGAAGCAGAAGTCGTTGTGGG** 3'; SEQ ID No:28). The 5' primer contains a NotI restriction site, a ribosome binding site, an initiation codon and a sequence at the 5' end of the protease protein coding sequence. The 3' primer includes the sequence encoding the C-terminal sequence of the protease protein gene and a BamHI restriction site. The stop codon was excluded and an additional nucleotide was inserted to obtain an in-frame fusion with the Histidine tag.

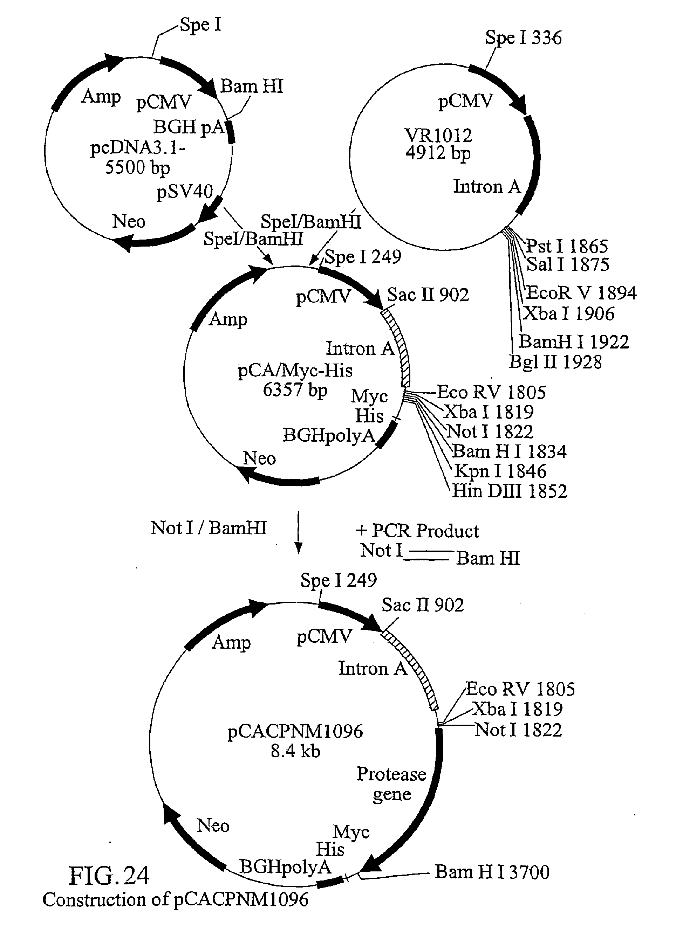

After amplification, the PCR fragment was purified using QIAquick^{™} PCR purification kit (Qiagen), digested with NotI and BamHI and cloned into the pCA-Myc-His eukaryotic expression vector described in Example 2 (Figure 24) with transcription under control of the human CMV promoter.

### E. Preparation of plasmid vector pCACPNM1097

The metalloprotease gene was amplified from *Chlamydia pneumoniae* genomic DNA strain CWLO29 by polymerase chain reaction (PCR) using a 5' primer (5' ATAAGAATGCGGCCGCCACC**ATGAGAAAACTTATTTTATGCAATCCTA** 3'; SEQ ID No:29) and a 3' primer (5' GCGCCGGATCCC**AGAACAACGGAGTTCTTTTGG** 3'; SEQ ID No:30). The 5' primer contains a NotI restriction site, a ribosome binding site, an initiation codon and a sequence at the 5' end of the metalloprotease protein coding sequence. The 3' primer includes the sequence encoding the C-terminal sequence of the metalloprotease protein gene and a BamHI restriction site. The stop codon was excluded and an additional nucleotide was inserted to obtain an in-frame fusion with the Histidine tag.

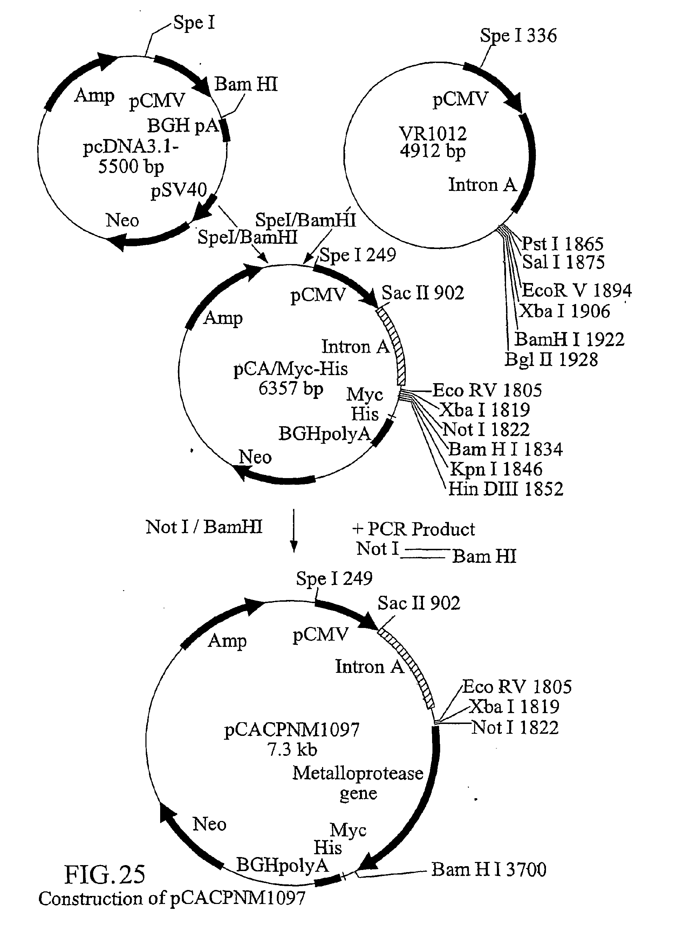

After amplification, the PCR fragment was purified using QIAquick^{™} PCR purification kit (Qiagen), digested with NotI and BamHI and cloned into the pCA-Myc-His eukaryotic expression vector described in Example 2 (Figure 25) with transcription under control of the human CMV promoter.

### F. Preparation of plasmid vector pCACPNM908

The CLP protease ATPase gene was amplified from *Chlamydia pneumoniae* genomic DNA strain CWLO29 by polymerase chain reaction (PCR) using a 5' primer (5' ATAAGAATGCGGCCGCCACC**ATGAATAAAAAAAATCTAACTATTTG** 3'; SEQ ID No:31) and a 3' primer (5' GCGCCGGATCCC**AGCGATAGCTTCTGGGGTCC** 3'; SEQ ID No:32). The 5' primer contains a NotI restriction site, a ribosome binding site, an initiation codon and a sequence at the 5' end of the CLP protease ATPase protein coding sequence. The 3' primer includes the sequence encoding the C-terminal sequence of the CLP protease ATPase gene and a BamHI restriction site. The stop codon was excluded and an additional nucleotide was inserted to obtain an in-frame fusion with the Histidine tag.

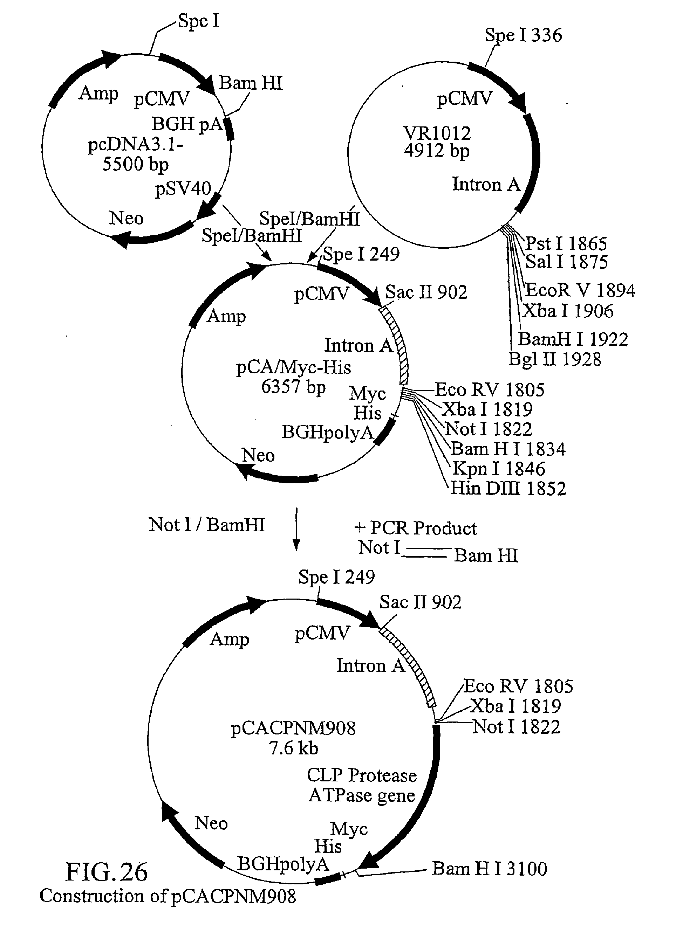

After amplification, the PCR fragment was purified using QIAquick^{™} PCR purification kit (Qiagen), digested with NotI and BamHI and cloned into the pCA-Myc-His eukaryotic expression vector described in Example 2 (Figure 26) with transcription under control of the human CMV promoter.

### G. Preparation of plasmid vector CACPNM909

The gene encoding CLP protease subunit was amplified from *Chlamydia pneumoniae* genomic DNA strain CWLO29 by polymerase chain reaction (PCR) using a 5' primer (5' ATAAGAATGCGGCCGCCACC**ATGACACTGGTACCCTATGTTG** 3'; SEQ ID No:33) and a 3' primer (5' GCGCCGGATCCC**AGTGCTACTTGTATCCTTATTAG** 3'; SEQ ID No:34). The 5' primer contains a NotI restriction site, a ribosome binding site, an initiation codon and a sequence at the 5' end of the CLP protease subunit coding sequence. The 3' primer includes the sequence encoding the C-terminal sequence of the CLP protease subunit gene and a BamHI restriction site. The stop codon was excluded and an additional nucleotide was inserted to obtain an in-frame fusion with the Histidine tag.

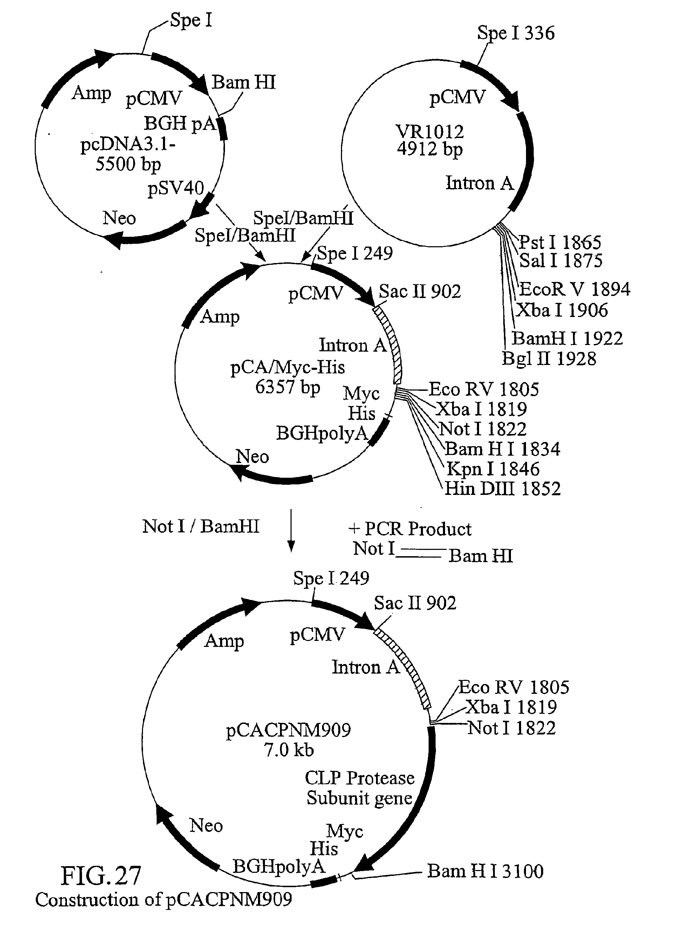

After amplification, the PCR fragment was purified using QIAquick^{™} PCR purification kit (Qiagen), digested with NotI and BamHI and cloned into the pCA-Myc-His eukaryotic expression vector described in Example 2 (Figure 27) with transcription under control of the human CMV promoter.

### H. Preparation of plasmid vector pCACPNM440

The translycolase / transpeptidase gene was amplified from *Chlamydia pneumoniae* genomic DNA strain CWLO29 by polymerase chain reaction (PCR) using a 5' primer (5' ATAAGAATGCGGCCGCCACC**ATGAGCTACCGTAAACGTTCGACTC** 3'; SEQ ID No:35) and a 3' primer (5' GCGCCGGATCCC**CCTCGTTCCCCCTTGTTTCGGAG** 3'; SEQ ID No:36). The 5' primer contains a NotI restriction site, a ribosome binding site, an initiation codon and a sequence at the 5' end of the translycolase / transpeptidase coding sequence. The 3' primer includes the sequence encoding the C-terminal sequence of the translycolase / transpeptidase gene and a BamHI restriction site. The stop codon was excluded and an additional nucleotide was inserted to obtain an in-frame fusion with the Histidine tag.

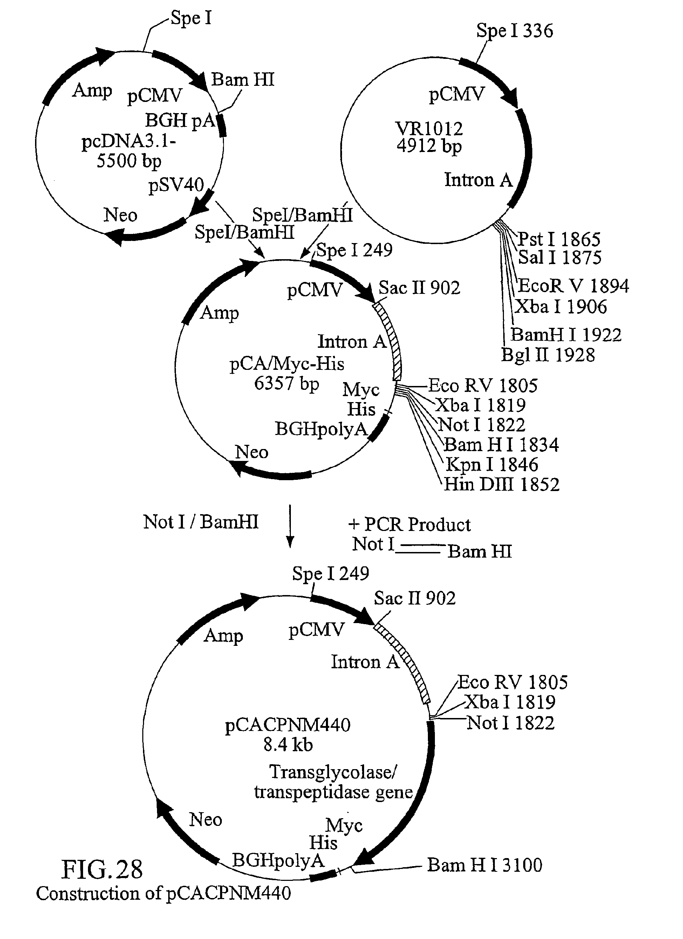

After amplification, the PCR fragment was purified using QIAquick^{™} PCR purification kit (Qiagen), digested with NotI and BamHI and cloned into the pCA-Myc-His eukaryotic expression vector described in Example 2 (Figure 28) with transcription under control of the human CMV promoter.

### I. Preparation of plasmid vector pCACPNM459

The gene encoding CLPc protease was amplified from *Chlamydia pneumoniae* genomic DNA strain CWL029 by polymerase chain reaction (PCR) using a 5' primer (5' ATAAGAATGCGGCCGCCACC**ATGTTTGAGAAGTTCACTAATAGAGC** 3'; SEQ ID No:37) and a 3' primer (5' GCGCCGGTACCG**TGATTCCAAGTGAGGGCTAGGG** 3'; SEQ ID No:38). The 5' primer contains a NotI restriction site, a ribosome binding site, an initiation codon and a sequence at the 5' end of the CLPc protease coding sequence. The 3' primer includes the sequence encoding the C-terminal sequence of the CLPc protease gene and a KpnI restriction site. The stop codon was excluded and an additional nucleotide was inserted to obtain an in-frame fusion with the Histidine tag.

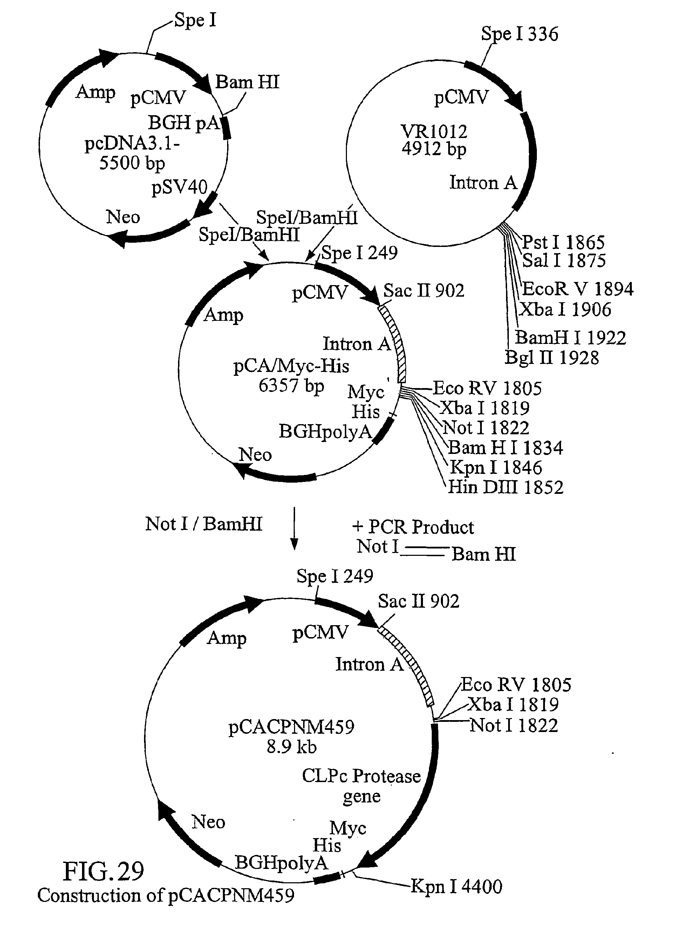

After amplification, the PCR fragment was purified using QIAquick^{™} PCR purification kit (Qiagen), digested with NotI and KpnI and cloned into the pCA-Myc-His eukaryotic expression vector described in Example 2 (Figure 29) with transcription under control of the human CMV promoter.

### J. Preparation of plasmid vector pCACPNM708

The thioredoxin gene was amplified from *Chlamydia pneumoniae* genomic DNA strain CWLO29 by polymerase chain reaction (PCR) using a 5' primer (5' ATAAGAATGCGGCCGCCACC**ATGGTAAAGATCATATCAAGTG** 3'; SEQ ID No:39) and a 3' primer (5' GCGCCGGATCCC**AGCGTGCTTATTGATAAG** 3'; SEQ ID No:40). The 5' primer contains a NotI restriction site, a ribosome binding site, an initiation codon and a sequence at the 5' end of the thioredoxin coding sequence. The 3' primer includes the sequence encoding the C-terminal sequence of the thioredoxin gene and a BamHI restriction site. The stop codon was excluded and an additional nucleotide was inserted to obtain an in-frame fusion with the Histidine tag.

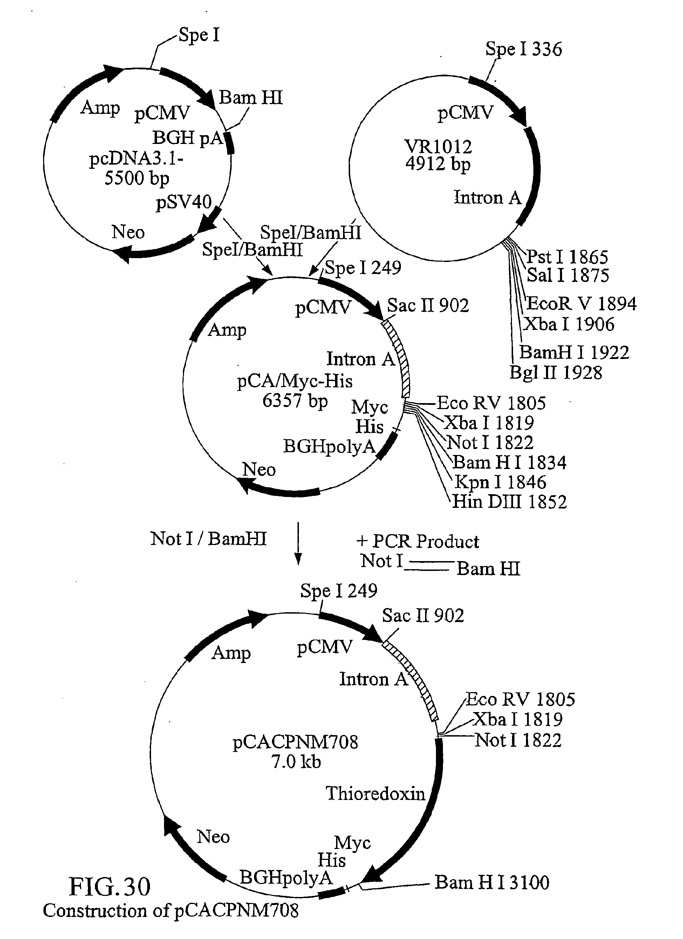

After amplification, the PCR fragment was purified using QIAquick^{™} PCR purification kit (Qiagen), digested with NotI and BamHI and cloned into the pCA-Myc-His eukaryotic expression vector described in Example 2 (Figure 30) with transcription under control of the human CMV promoter.

### Example 2:

Plasmid pcDNA3.1(-)Myc-His C (Invitrogen) was restricted with SpeI and BamHI to remove the CMV promoter and the remaining vector fragment was isolated. The CMV promoter and intron A from plasmid VR-1012 (Vical) was isolated on a SpeI / BamHI fragment. The fragments were ligated together to produce plasmid pCA/Myc-His.

The NotI/BamHI restricted PCR fragment containing the ATP-binding cassette gene was ligated into the NotI and BamHI restricted plasmid pCA/Myc-His to produce plasmid pCACPNM213 (Figure 21).

The NotI/KpnI restricted PCR fragment containing the Secretory locus ORF gene was ligated into the NotI and KpnI restricted plasmid pCA/Myc-His to produce plasmid pCACPNM882 (Figure 22).

The NotI/BamHI restricted PCR fragment containing the endopeptidase gene was ligated into the NotI and BamHI restricted plasmid pCA/Myc-His to produce plasmid pCACPNM208 (Figure 23).

The NotI/BamHI restricted PCR fragment containing the Protease gene was ligated into the NotI and BamHI restricted plasmid pCA/Myc-His to produce plasmid pCACPNM1096 (Figure 24).

The NotI/BamHI restricted PCR fragment containing the Metalloprotease gene was ligated into the NotI and BamHI restricted plasmid pCA/Myc-His to produce plasmid pCACPNM1097 (Figure 25).

The NotI/BamHI restricted PCR fragment containing the CLP protease ATPase gene was ligated into the NotI and BamHI restricted plasmid pCA/Myc-His to produce plasmid pCACPNM908 (Figure 26).

The NotI/BamHI restricted PCR fragment containing the CLP protease subunit gene was ligated into the NotI and BamHI restricted plasmid pCA/Myc-His to produce plasmid pCACPNM909 (Figure 27).

The NotI/BamHI restricted PCR fragment containing the transglycolase/transpeptidase gene was ligated into the NotI and BamHI restricted plasmid pCA/Myc-His to produce plasmid pCACPNM440 (Figure 28).

The NotI/KpnI restricted PCR fragment containing the CLPc protease gene was ligated into the NotI and KpnI restricted plasmid pCA/Myc-His to produce plasmid pCACPNM459 (Figure 29).

The NotI/BamHI restricted PCR fragment containing the Thioredoxin gene was ligated into the NotI and BamHI restricted plasmid pCA/Myc-His to produce plasmid pCACPNM708 (Figure 30).

Each of the resulting plasmids pCACPNM213, pCACPNM882, pCACPNM208, pCACPNM1096, pCACPNM1097, pCACPNM909, pCACPNM440, pCACPNM459 and pCACPNM708, was transferred by electroporation into E. *coli* XL-1 blue (Stratagene) which was grown in LB broth containing 50 µg/ml carbenicillin. The plasmid was isolated by the Endo Free Plasmid Giga Kit^{™} (Qiagen) large scale DNA purification system. DNA concentration was determined by absorbance at 260 nm and the plasmid was verified after gel electrophoresis and ethidium bromide staining by comparison to molecular weight standards. The 5' and 3' ends of the gene were verified by sequencing using a LiCor model 4000 L DNA sequencer and IRD-800 labelled primers.

### Example 3:

This example illustrates the immunization of mice to achieve protection against an intranasal challenge of

### C. pneumoniae.

It has been previously demonstrated (Yang et al. Infect. Immun. May 1993. 61(5):2037-40) that mice are susceptible to intranasal infection with different isolates of *C. pneumoniae.* Strain AR-39 (Grayston et al (1990) Journal of Infectious Diseases 161:618-625) was used in Balb/c mice as a challenge infection model to examine the capacity of *Chlamydia* gene products delivered as naked DNA to elicit a protective response against a sublethal *C. pneumoniae* lung infection. Protective immunity is defined as an accelerated clearance of pulmonary infection.

Groups of 7 to 9 week old male Balb/c mice (8 to 10 per group) were immunized intramuscularly (i.m.) plus intranasally (i.n.) with plasmid DNA containing each of the *C. pneumoniae* protein gene as described in Examples 1 and 2. Saline or the plasmid vector lacking an inserted Chlamydial gene was given to groups of control animals.

For i.m. immunization, alternate left and right quadriceps were injected with 100µg of DNA in 50µl of PBS on three occasions at 0, 3 and 6 weeks. For i.n. immunization, anaesthetized mice were aspirated 50µl of PBS containing 50 µg DNA on three occasions at 0, 3 and 6 weeks. At week 8, immunized mice were inoculated i.n. with 5 x 10⁵ IFU of *C. pneumoniae*, strain AR39 in 100µl of SPG buffer to test their ability to limit the growth of a sublethal *C. pneumoniae* challenge.

Lungs were taken from mice at day 9 post-challenge and immediately homogenised in SPG buffer (7.5% sucrose, 5mM glutamate, 12.5mM phosphate pH7.5). The homogenate was stored frozen at -70°C until assay. Dilutions of the homogenate were assayed for the presence of infectious *Chlamydia* by inoculation onto monolayers of susceptible cells. The inoculum was centrifuged onto the cells at 3000rpm for 1 hour, then the cells were incubated for three days at 35°C in the presence of 1µg/ml cycloheximide. After incubation the monolayers were fixed with formalin and methanol then immunoperoxidase stained for the presence of Chlamydial inclusions using convalescent sera from rabbits infected with *C. pneumoniae* and metal-enhanced DAB as a peroxidase substrate.

### A. Immunization with pCACPNM213

Figure 31 and Table 1 show that mice immunized i.n. and i.m. with pCACPNM213 had chlamydial lung titers less than 60,000 in 3 of 6 cases at day 9 (mean 51,833) whereas the range of values for control mice sham immunized with saline was 34,200-377,800 IFU/lung (mean 141,450) at day 9. DNA immunisation *per se* was not responsible for the observed protective effect since another plasmid DNA construct, pCACPNM102, failed to protect, with lung titers in immunised mice similar to those obtained for saline-immunized control mice (mean 153,283). The construct pCACPNM102 is identical to pCACPNM213 except that the nucleotide sequence encoding the putative ATP-binding cassette is replaced with a *C. pneumoniae* nucleotide sequence encoding an unrelated ATP Synthase Subunit I protein.

### B. Immunization with pCACPNM882

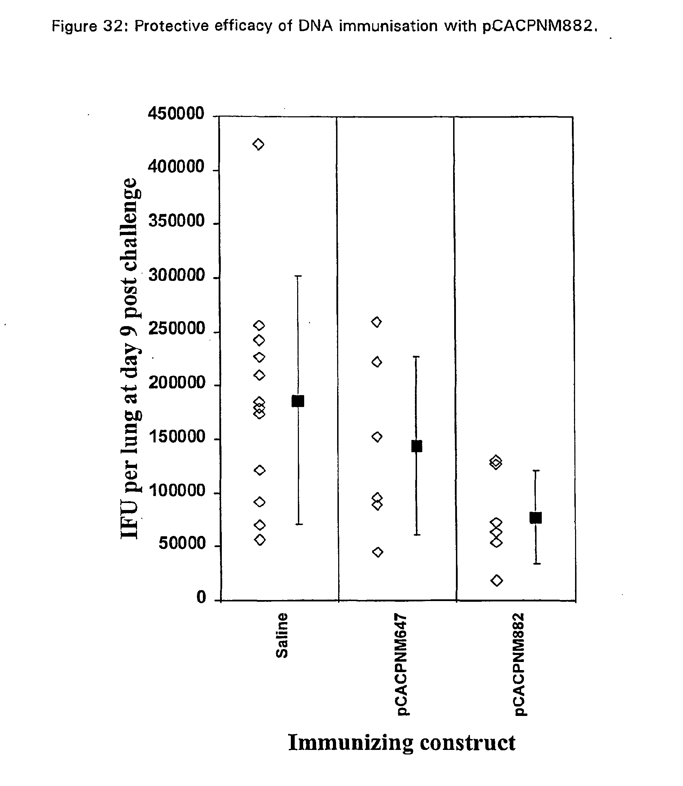

Figure 32 and Table 3 show that mice immunized i.n. and i.m. with pCACPNM882 had chlamydial lung titers less than 73,000 in 4 of 6 cases at day 9 (mean 77,500) whereas the range of values for control mice sham immunized with saline was 56,000-424,000 IFU/lung (mean 186,291) at day 9. DNA immunisation *per se* was not responsible for the observed protective effect since another plasmid DNA construct, pCACPNM647, failed to protect, with lung titers in immunised mice similar to those obtained for saline-immunized control mice (mean 143,883). The construct pCACPNM647 is identical to pCACPNM882 except that the nucleotide sequence encoding the putative Secretory locus ORF is replaced with a *C. pneumoniae* nucleotide sequence encoding an unrelated substrate binding protein.

### C. Immunization with pCACPNM208

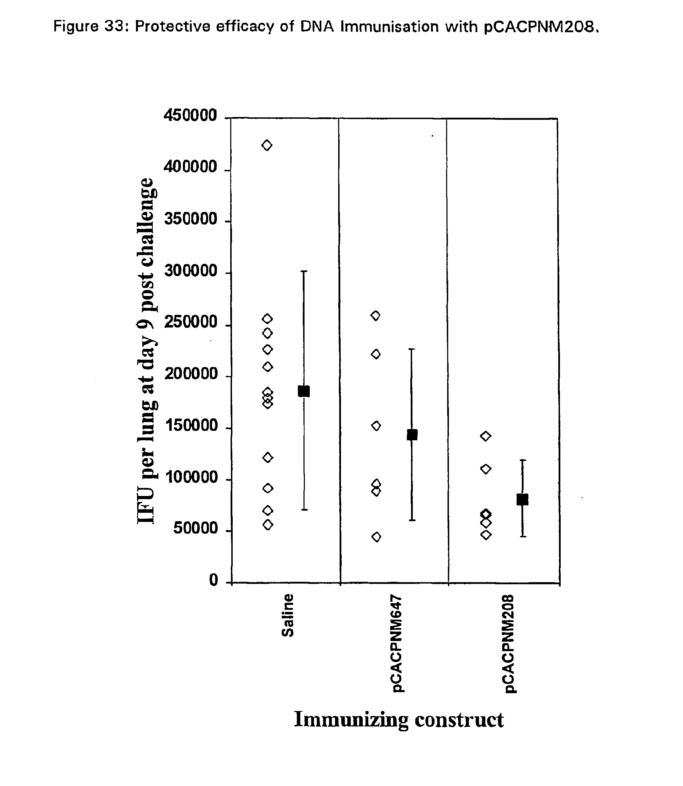

Figure 33 and Table 4 show that mice immunized i.n. and i.m. with pCACPNM208 had chlamydial lung titers less than 67,000 in 4 of 6 cases at day 9 (mean 81,766) whereas the range of values for control mice sham immunized with saline was 56,000-424,100 IFU/lung (mean 186,291) at day 9. DNA immunisation *per se* was not responsible for the observed protective effect since another plasmid DNA construct, pCACPNM647, failed to protect, with lung titers in immunised mice similar to those obtained for saline-immunized control mice (mean 143,883). The construct pCACPNM647 is identical to pCACPNM208 except that the nucleotide sequence encoding the putative Endopeptidase is replaced with a *C. pneumoniae* nucleotide sequence encoding an unrelated protein.

### D. Immunization with pCACPNM1096

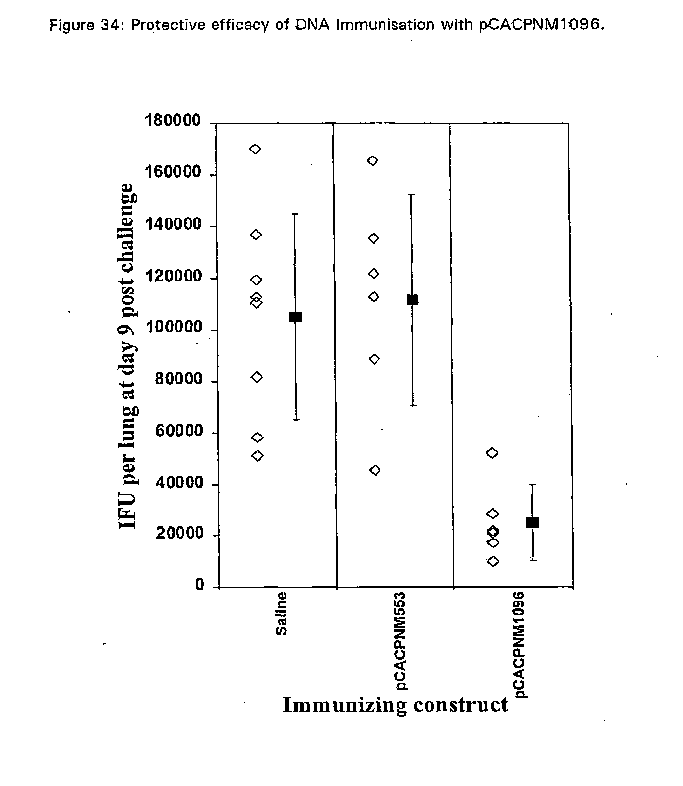

Figure 34 and Table 6 show that mice immunized i.n. and i.m. with pCACPNM1096 had chlamydial lung titers less than 30,000 in 5 of 6 cases at day 9 (mean 25,000) whereas the range of values for control mice sham immunized with saline was 51,300-170,000 IFU/lung (mean 105,150) at day 9. DNA immunisation *per se* was not responsible for the observed protective effect since another plasmid DNA construct, pCACPNM553, failed to protect, with lung titers in immunised mice similar to those obtained for saline-immunized control mice (mean 111,583). The construct pCACPNM553 is identical to pCACPNM1096 except that the nucleotide sequence encoding the putative Protease is replaced with a *C. pneumoniae* nucleotide sequence encoding an unrelated protease.

### E. Immunization with pCACPNMI097

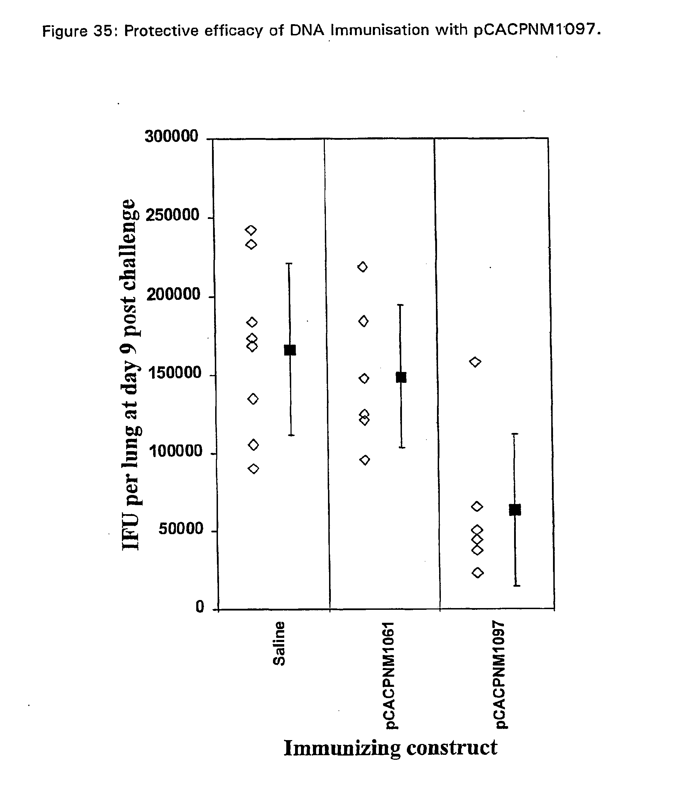

Figure 35 and Table 8 show that mice immunized i.n. and i.m. with pCACPNM1097 had chlamydial lung titers less than 51,000 in 4 of 6 cases at day 9 (mean 62,883) whereas the range of values for control mice sham immunized with saline was 90,000-242,100 IFU/lung (mean 166,287) at day 9. DNA immunisation *per se* was not responsible for the observed protective effect since another plasmid DNA construct, pCACPNM1061, failed to protect, with lung titers in immunised mice similar to those obtained for saline-immunized control mice (mean 148,566). The construct pCACPNM1061 is identical to pCACPNM1097 except that the nucleotide sequence encoding the putative Metalloprotease is replaced with a *C. pneumoniae* nucleotide sequence encoding an unrelated zinc Metalloprotease.

### F. Immunization with pCACPNM908

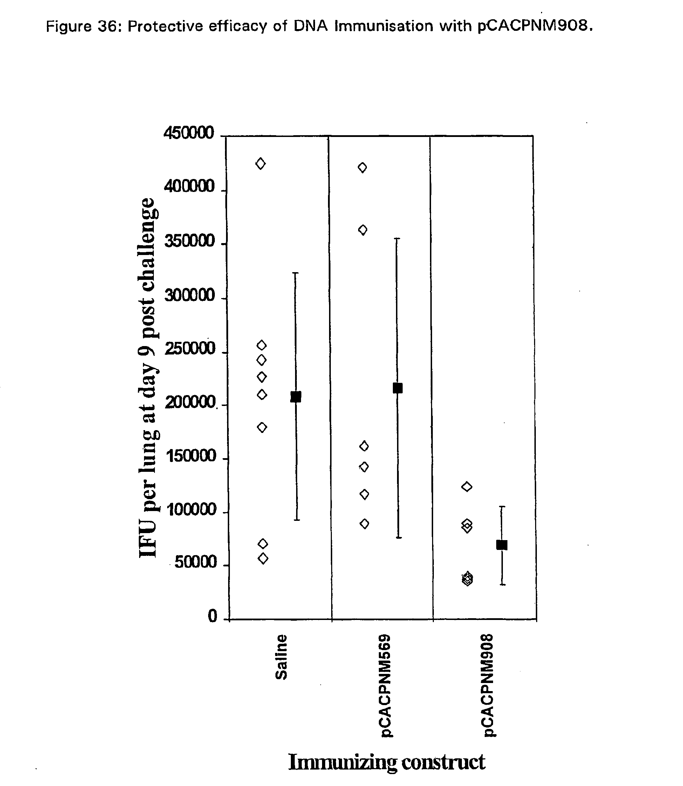

Figure 36 and Table 10 show that mice immunized i.n. and i.m. with pCACPNM908 had chlamydial lung titers less than 40,000 in 3 of 6 cases at day 9 (mean 68,333) whereas the range of values for control mice sham immunized with saline was 56,000-424,100 IFU/lung (mean 207,962) at day 9. DNA immunisation *per se* was not responsible for the observed protective effect since another plasmid DNA construct, pCACPNM569, failed to protect, with lung titers in immunised mice similar to those obtained for saline-immunized control mice (mean 215,600). The construct pCACPNM569 is identical to pCACPNM908 except that the nucleotide sequence encoding the putative CLP protease ATPase is replaced with a *C. pneumoniae* nucleotide sequence encoding an unrelated signal peptidase.

### G. Immunization with pCACPNM909

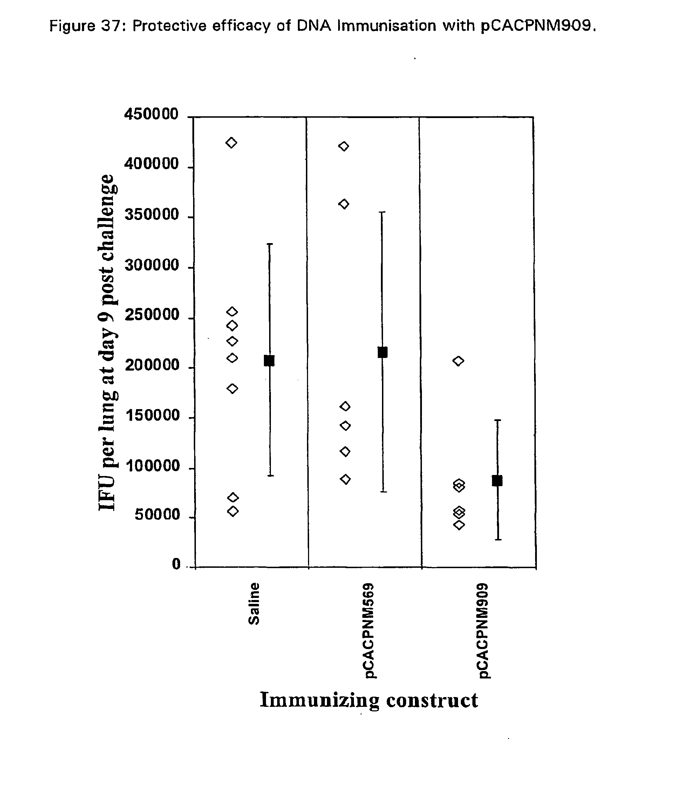

Figure 37 and Table 12 show that mice immunized i.n. and i.m. with pCACPNM909 had chlamydial lung titers less than 85,000 in 5 of 6 cases at day 9 (mean 87,683) whereas the range of values for control mice sham immunized with saline was 56,000-424,100 IFU/lung (mean 207,962) at day 9. DNA immunisation *per se* was not responsible for the observed protective effect since another plasmid DNA construct, pCACPNM569, failed to protect, with lung titers in immunised mice similar to those obtained for saline-immunized control mice (mean 215,600). The construct pCACPNM569 is identical to pCACPNM909 except that the nucleotide sequence encoding the putative CLP protease subunit is replaced with a *C. pneumoniae* nucleotide sequence encoding an unrelated signal peptidase.

### H. Immunization with pCACPNM440

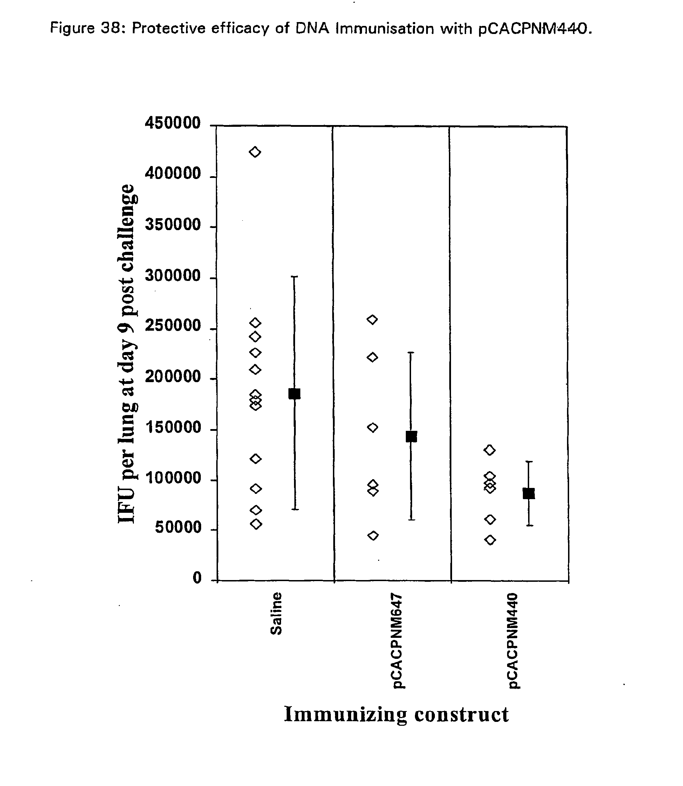

Figure 38 and Table 14 show that mice immunized i.n. and i.m. with pCACPNM440 had chlamydial lung titers less than 98,000 in 4 of 6 cases at day 9 (mean 87,616) whereas the range of values for control mice sham immunized with saline was 56,000-424,100 IFU/lung (mean 186,291) at day 9. DNA immunisation *per se* was not responsible for the observed protective effect since another plasmid DNA construct, pCACPNM647 failed to protect, with lung titers in immunised mice similar to those obtained for saline-immunized control mice (mean 143,883). The construct pCACPNM647 is identical to pCACPNM440 except that the nucleotide sequence encoding the putative transglycolase /transpeptidase gene is replaced with a *C. pneumoniae* nucleotide sequence encoding an unrelated gene.

### I. Immunization with pCACPNM459

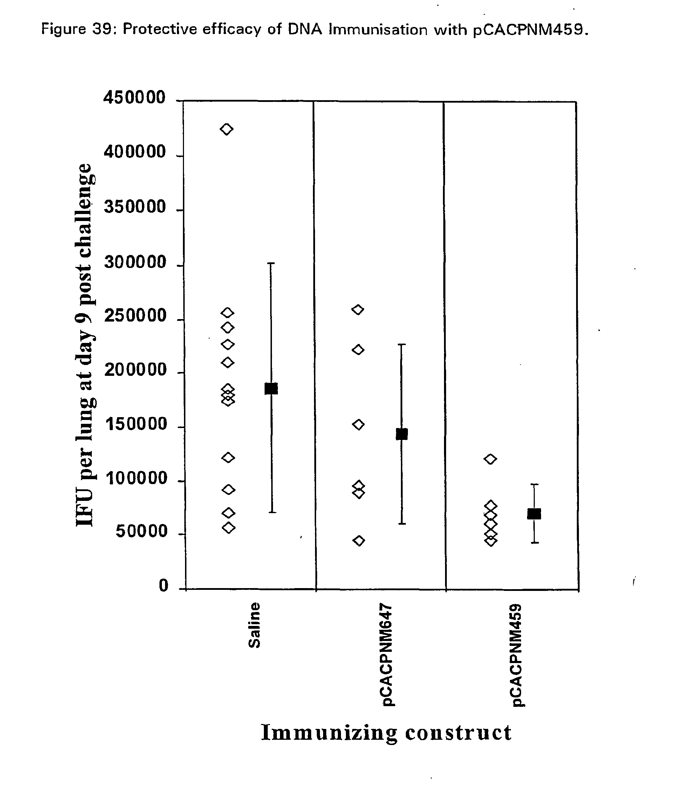

Figure 39 and Table 16 show that mice immunized i.n. and i.m. with pCACPNM459 had chlamydial lung titers less than 70,000 in 4 of 6 cases at day 9 (mean 70,516) whereas the range of values for control mice sham immunized with saline was 56,000-424,100 IFU/lung (mean 186,291) at day 9. DNA immunisation *per se* was not responsible for the observed protective effect since another plasmid DNA construct, pCACPNM647, failed to protect, with lung titers in immunised mice similar to those obtained for saline-immunized control mice (mean 143,883). The construct pCACPNM647 is identical to pCACPNM459 except that the nucleotide sequence encoding the putative CLPc protease is replaced with a *C. pneumoniae* nucleotide sequence encoding an unrelated gene.

### J. Immunization with pCACPNM708

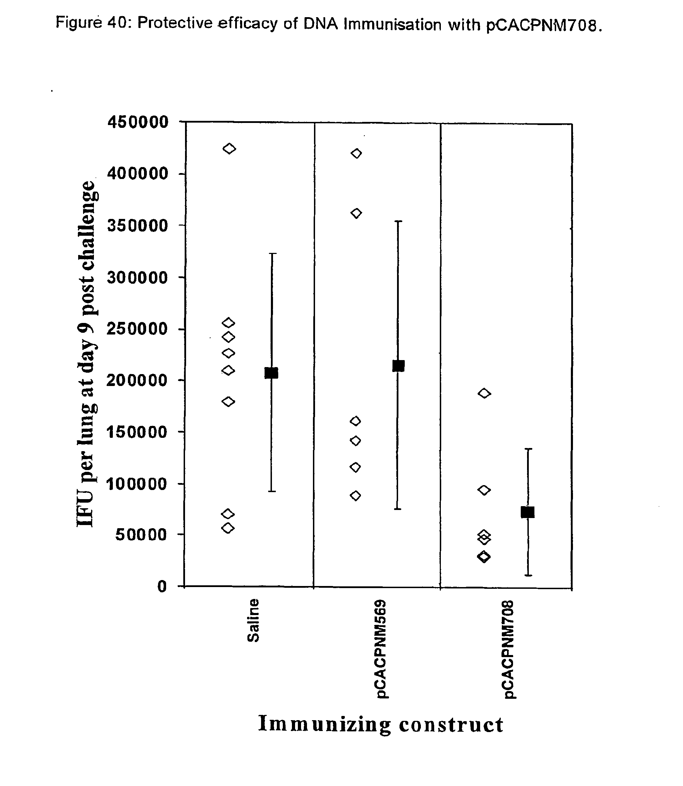

Figure 40 and Table 18 show that mice immunized i.n. and i.m. with pCACPNM708 had chlamydial lung titers less than 52,000 in 4 of 6 cases at day 9 (mean 73,916) whereas the range of values for control mice sham immunized with saline was 56,000-424,100 IFU/lung (mean 207,962) at day 9. DNA immunisation *per se* was not responsible for the observed protective effect since another plasmid DNA construct, pCACPNM569, failed to protect, with lung titers in immunised mice similar to those obtained for saline-immunized control mice (mean 215,600). The construct pCACPNM569 is identical to pCACPNM708 except that the nucleotide sequence encoding the putative thioredoxin is replaced with a *C. pneumoniae* nucleotide sequence encoding an unrelated *C. pneumoniae* gene.

### Example 4:

This example illustrates the identification of B- and T-cell epitopes in proteins as expressed from each of pCACPNM213, pCACPNM882, pCACPNM208, pCACPNM1096, pCACPNM1097, pCACPNM909, pCACPNM440, pCACPNM459 and pCACPNM708.

B-cell epitopes were identified based on the product of flexibilty and hydrophobicity propensities using the program SEQSEE (Wishart DS, et al. "SEQSEE: a comprehensive program suite for protein sequence analysis." Comput Appl Biosci. 1994 Apr;10(2):121-32) to identify external surface features (epitopes). T-cell epitopes for HLA-A0201 MHC subclass were identified based on the algorithm of Parker et al. 1995 (Parker KC, et al. "Peptide binding to MHC class I molecules: implications for antigenic peptide prediction." Immunol Res 1995:14(1):34-57). These epitopes are shown in Tables 2, 5, 7, 9, 11, 13, 15, 17 and 19 and SEQ ID NOs: 41 to 74.

**Table 1**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM102 | pCACPNM213 |
| | Day 9 | Day 9 | Day 9 |
| | | | |
| 1 | 64900 | 207500 | 54200 |
| 2 | 116500 | 166500 | 10600 |
| 3 | 34200 | 114700 | 67400 |
| 4 | 377800 | 167400 | 32000 |
| 5 | 86200 | 179700 | 66900 |
| 6 | 206200 | 83900 | 79900 |
| 7 | 142600 | | |
| 8 | 103200 | | |
| | | | |
| | | | |
| | | | |
| MEAN | 141450 | 153283.333 | 51833.3333 |
| SD | 108598.7 | 45417.99 | 25908.35 |
| | | | |
| Wilcoxon p | | 1.655 | 0.0293 |

**Table 2: Identified B- T-cell epitopes from CPNM213**

| **B cell epitope** | **T cell epitope** |
|---|---|
| 188 VHHTLRESYKKGTPPST (SEQ ID No: 41) | 434 WIAEYVSPV (SEQ ID No: 43) |
| 345 NLQKEISTEERQTKAR (SEQ ID No: 42) | |

**Table 3**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM647 | pCACPNM882 |
| | Day 9 | Day 9 | Day 9 |
| | | | |
| 1 | 209800 | 45100 | 18100 |
| 2 | 70000 | 222000 | 130300 |
| 3 | 226700 | 152500 | 72900 |
| 4 | 178900 | 89000 | 53500 |
| 5 | 424100 | 95500 | 63400 |
| 6 | 242200 | 259200 | 126800 |
| 7 | 256000 | | |
| 8 | 56000 | | |
| 9 | 173600 | | |
| 10 | 185000 | | |
| 11 | 121400 | | |
| 12 | 91800 | | |
| | | | |
| | | | |
| | | | |
| MEAN | 186291.667 | 143883.333 | 77500 |
| SD | 100263.3 | 83169.31 | 43686.75 |
| | | | |
| Wilcoxon p | | 0.4936 | 0.0182 |

**Table 4**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM647 | pCACPNM208 |
| | Day 9 | Day 9 | Day 9 |
| | | | |
| 1 | 209800 | 45100 | 142500 |
| 2 | 70000 | 222000 | 66900 |
| 3 | 226700 | 152500 | 58200 |
| 4 | 178900 | 89000 | 46500 |
| 5 | 424100 | 95500 | 110900 |
| 6 | 242200 | 259200 | 65600 |
| 7 | 256000 | | |
| 8 | 56000 | | |
| 9 | 173600 | | |
| 10 | 185000 | | |
| 11 | 121400 | | |
| 12 | 91800 | | |
| | | | |
| | | | |
| MEAN | 186291.667 | 143883.333 | 81766.6667 |
| SD | 100263.3 | 83169.31 | 36929.10 |
| | | | |
| Wilcoxon p | | 0.4936 | 0.0135 |

**Table 5 Identified B- T-cell epitopes from CPNM208**

| **B cell epitope** | **T cell epitope** |
|---|---|
| 220 KGNNSSPRSPAP (SEQ ID No: 44) | 67 LLIEDMDLI (SEQ ID No: 46) |
| 313 GENFQKNSS (SEQ ID No: 45) | 66 NLLIEDMDL (SEQ ID No:47) |

**Table 6**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | IpCACPNM553 | pCACPNM1096 |
| | Day 9 | Day 9 | Day 9 |
| | | | |
| 1 | 136900 | 135600 | 21000 |
| 2 | 81700 | 112600 | 9700 |
| 3 | 119400 | 88600 | 28500 |
| 4 | 58500 | 121700 | 52000 |
| 5 | 110600 | 165300 | 17200 |
| 6 | 51300 | 45700 | 21600 |
| 7 | 170000 | | |
| 8 | 112800 | | |
| | | | |
| | | | |
| MEAN | 105150 | 111583.333 | 25000 |
| SD | 39876.3 | 41071.91 | 14585.88 |
| | | | |
| Wilcoxon p | | 1.245 | 0.0013 |

**Table 7 Identified B- T-cell epitopes from CPNM1096**

| **B cell epitope** | **T cell epitope** |
|---|---|
| 328 TDLEGLEEDHKDSPWE (SEQ ID No: 48) | 135 YLGDEILEV (SEQ ID No: 50) |
| 589 SENAKKSEEQTSPQETPE (SEQ ID No: 49) | 373 YLYSLLSML (SEQ ID No: 51) |

**Table 8**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM106 1 | pCACPNM1097 |
| | Day 9 | Day 9 | Day 9 |
| | | | |
| 1 | 232900 | 120800 | 50300 |
| 2 | 168100 | 184100 | 43900 |
| 3 | 105500 | 95600 | 65200 |
| 4 | 173100 | 147500 | 157900 |
| 5 | 90000 | 218700 | 22800 |
| 6 | 242100 | 124700 | 37200 |
| 7 | 183700 | | |
| 8 | 134900 | | |
| | | | |
| MEAN | 166287.5 | 148566.667 | 62883.3333 |
| SD | 54821.4 | 45450.00 | 48618.00 |
| | | | |
| Wilcoxon p | | 0.662 | 0.0047 |

**Table 9 Identified B- T-cell epitopes from CPNM1097**

| **B cell epitope** | **T cell epitope** |
|---|---|
| 198 TTNRQKAL (SEQ ID No: 52) | 207 SVLSRVNYV (SEQ ID No: 54) |
| 221 VNSSNSNRLRE (SEQ ID No: 53) | 279 KLSSLIPGL (SEQ ID No: 55) |
| | 118 ILIGHKKHV (SEQ ID No: 56) |

**Table 10**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM569 | pCACPNM908 |
| | Day 9 | Day 9 | Day 9 |
| | | | |
| 1 | 209800 | 142800 | 37300 |
| 2 | 70000 | 420700 | 85000 |
| 3 | 226700 | 116600 | 35700 |
| 4 | 178900 | 161300 | 39700 |
| 5 | 424100 | 89200 | 123400 |
| 6 | 242200 | 363000 | **·** 88900 |
| 7 | 256000 | | |
| 8 | 56000 | | |
| | | | |
| MEAN | 207962.5 | 215600 | 68333.3333 |
| SD | 115585.8 | 139870.70 | 36279.40 |
| | | | |
| Wilcoxon p | | 0.8518 | 0.02 |

**Table 11 Identified B- T-cell epitopes from CPNM908**

| **B cell epitope** | **T cell epitope** |
|---|---|
| 226 PPKGGRKHPNQEYI (SEQ ID No: 57) | 137 KILDVPFTI (SEQ ID No: 59) |
| 273 SDDQADLSQKTRDH (SEQ ID No: 58) | 168 LLQAADYDV (SEQ ID No: 60) |

**Table 12**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM569 | PCACPNM909 |
| | Day 9 | Day 9 | Day 9 |
| | | | |
| 1 | 209800 | 142800 | 206700 |
| 2 | 70000 | 420700 | 84700 |
| 3 | 226700 | 116600 | 81100 |
| 4 | 178900 | 161300 | 56700 |
| 5 | 424100 | 89200 | 53900 |
| 6 | 242200 | 363000 | 43000 |
| 7 | 256000 | | |
| 8 | 56000 | | |
| | | | |
| MEAN | 207962.5 | 215600 | 87683.3333 |
| SD | 115585.8 | 139870.70 | 60522.87 |
| | | | |
| Wilcoxon p | | 0.8518 | 0.0426 |
| | | | |
| | | | |

**Table 13 Identified B- T-cell epitopes from CPNM909**

| **B cell epitope** | **T cell epitope** |
|---|---|
| 107 GTKGKRHAL (SEQ ID No: 61) | 76 AIYDTIRFL (SEQ ID No: 63) |
| 193 AKETNKDTSST (SEQ ID No: 62) | |

**Table 14**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM647 | pCACPNM440 |
| | Day 9 | Day 9 | Day 9 |
| | | | |
| 1 | 209800 | 45100 | 97200 |
| 2 | 70000 | 222000 | 92500 |
| 3 | 226700 | 152500 | 104400 |
| 4 | 178900 | 89000 | 60900 |
| 5 | 424100 | 95500 | 40400 |
| 6 | 242200 | 259200 | 130300 |
| 7 | 256000 | | |
| 8 | 56000 | | |
| 9 | 173600 | | |
| 10 | 185000 | | |
| 11 | 121400 | | |
| 12 | 91800 | | |
| | | | |
| | | | |
| | | | |
| MEAN | 186291.667 | 143883.333 | 87616.6667 |
| SD | 1002.63.3 | 83169.31 | 32132.31 |
| | | | |
| Wilcoxon p | | 0.4936 | 0.0415 |

**Table 15 Identified B- T-cell epitopes from CPNM440**

| **B cell epitope** | **T cell epitope** |
|---|---|
| 287 DPTNYKEYFNNKERIEHTK (SEQ ID No: 64) | 40 ALGQHEFCV (SEQ ID No: 66) |
| 637 KRLYEEWNRSPKQGGTR (SEQ ID No: 65) | 456 ILATGIQMV (SEQ ID No: 67) |

**Table 16**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM647 | pCACPNM459 |
| | Day 9 | Day 9 | Day 9 |
| | | | |
| 1 | 209800 | 45100 | 77400 |
| 2 | 70000 | 222000 | 60700 |
| 3 | 226700 | 152500 | 121000 |
| 4 | 178900 | 89000 | 68500 |
| 5 | 424100 | 95500 | 44800 |
| 6 | 242200 | 259200 | 50700 |
| 7 | 256000 | | |
| 8 | 56000 | | |
| 9 | 173600 | | |
| 10 | 185000 | | |
| 11 | 121400 | | |
| 12 | 91800 | | |
| | | | |
| MEAN | 186291.667 | 143883.333 | 70516.6667 |
| SD | 100263.3 | 83169.31 | 27387.69 |
| | | | |
| Wilcoxon p | | 0.4936 | 0.0047 |

**Table 17 Identified B- T-cell epitopes from CPNM459**

| **B cell epitope** | **T cell epitope** |
|---|---|
| 467 DEEKKLRERLQSMKQEWENHKEEHQ (SEQ ID No: 68) | 565 FLFLGPTGV (SEQ ID No: 70) |
| 548 IRRSRTGIKDPNRPTG (SEQ ID No: 69) | 410 FLPDKAIDL (SEQ ID No: 71) |

**Table 18**

| MOUSE | BACTERIAL LOAD (INCLUSION FORMING UNITS PER LUNG) IN THE LUNGS OF BALB/C MICE IMMUNIZED WITH VARIOUS DNA IMMUNIZATION CONSTRUCTS | | |
|---|---|---|---|
| | IMMUNIZING CONSTRUCT | | |
| | Saline | pCACPNM569 | pCACPNM708 |
| | Day 9 | Day 9 | Day 9 |
| | | | |
| 1 | 209800 | 142800 | 95100 |
| 2 | 70000 | 420700 | 189600 |
| 3 | 226700 | 116600 | 29000 |
| 4 | 178900 | 161300 | 51400 |
| 5 | 424100 | 89200 | 31500 |
| 6 | 242200 | 363000 | 46900 |
| 7 | 256000 | | |
| 8 | 56000 | | |
| | | | |
| MEAN | 207962.5 | 215600 | 73916.6667 |
| SD | 115585.8 | 139870.70 | 61457.22 |
| | | | |
| Wilcoxon p | | 0.8518 | 0.0127 |

**Table 19 Identified B- T-cell epitopes from pCPNM708**

| **B cell epitope** | **T cell epitope** |
|---|---|
| 54 NIDENSKPAETYE (SEQ ID No: 72) | 40 NLAAELPHV (SEQ ID No: 73) |
| | 74 ILFKDGNEV (SEQ ID No: 74) |

## Claims

1. A vaccine comprising a vaccine vector and at least one first nucleic acid selected from any one of:
(i) SEQ ID No: 3;
(ii) a nucleic acid sequence which encodes a polypeptide encoded by SEQ ID No: 3;
(iii) a nucleic acid sequence which encodes a polypeptide which is at least 75% identical in amino acid sequence to the polypeptide encoded by SEQ ID No: 3; and
(iv) a nucleic acid sequence which encodes a polypeptide whose sequence is set forth in SEQ ID No: 4;
(v) a nucleic acid sequence as defined in (i), (ii) or (iv), which has been modified to encode a modified polypeptide, wherein the modified polypeptide retains immunogenicity and is at least 75% identical in amino acid sequence to the corresponding polypeptide encoded by the nucleic acid of (i), (ii) or (iv);
wherein each first nucleic acid is capable of being expressed.

2. A vaccine comprising a vaccine vector and at least one first nucleic acid selected from any one of:
(i) a nucleic acid sequence comprising at least 36 consecutive nucleotides from SEQ ID NO: 3;
(ii) a nucleic acid sequence which encodes an immunogenic fragment comprising at least 12 consecutive amino acids from SEQ ID No: 4;
(iii) a nucleic acid sequence as defined in (i) or (ii), which has been modified to encode a modified polypeptide, wherein the modified polypeptide retains immunogenicity and is at least 75% identical in amino acid sequence to the corresponding fragment of (i) or (ii);
wherein each first nucleic acid is capable of being expressed.

3. A vaccine comprising a vaccine vector and at least one first nucleic acid encoding a fusion protein, wherein the fusion protein comprises:
(a) a first polypeptide encoded by a nucleic acid selected from any one of:
(i) SEQ ID No: 3;
(ii) a nucleic acid sequence which encodes a polypeptide encoded by SEQ ID No: 3;
(iii) a nucleic acid sequence which encodes a polypeptide which is at least 75% identical in amino acid sequence to the polypeptide encoded by SEQ ID No: 3; and
(iv) a nucleic acid sequence which encodes a polypeptide whose sequence is set forth in SEQ ID No: 4;
(v) a nucleic acid sequence as defined in (i), (ii) or (iv), which has been modified to encode a modified polypeptide, wherein the modified polypeptide retains immunogenicity and is at least 75% identical in amino acid sequence to the corresponding polypeptide encoded by the nucleic acid of (i), (ii) or (iv); and
(b) a second polypeptide;
wherein each first nucleic acid is capable of being expressed.

4. A vaccine comprising a vaccine vector and at least one first nucleic acid encoding a fusion protein, wherein the fusion protein comprises:
(a) a first polypeptide encoded by a nucleic acid selected from any one of:
(i) a nucleic acid sequence comprising at least 36 consecutive nucleotides from SEQ ID NO: 3;
(ii) a nucleic acid sequence which encodes an immunogenic fragment comprising at least 12 consecutive amino acids from SEQ ID No: 4;
(iii) a nucleic acid sequence as defined in (i) or (ii), which has been modified to encode a modified polypeptide, wherein the modified polypeptide retains immunogenicity and is at least 75% identical in amino acid sequence to the corresponding fragment of (i) or (ii); and
(b) a second polypeptide;
wherein each first nucleic acid is capable of being expressed.

5. The vaccine of claim 3 or 4 wherein the second polypeptide is a heterologous signal peptide.

6. The vaccine of claim 3 or 4 wherein the second polypeptide has adjuvant activity.

7. The vaccine of any one of claims 1 to 6 wherein wherein each first nucleic acid is operatively linked to one or more expression control sequences.

8. A vaccine according to any one of claims 1 to 7, further comprising a second nucleic acid encoding an additional polypeptide which enhances the immune response to the polypeptide expressed by the first nucleic acid.

9. The vaccine of claim 8 wherein the second nucleic acid encodes an additional *Chlamydia* polypeptide.

10. A pharmaceutical composition comprising a vaccine according to any one of claims 1 to 9 and a pharmaceutically acceptable carrier.

11. A fusion protein comprising a first and a second polypeptide, wherein the first polypeptide is selected from any one of:
(i) a polypeptide encoded by SEQ ID NO: 3;
(ii) a polypeptide which is at least 75% identical in amino acid sequence to SEQ ID NO: 4 or to the polypeptide encoded by SEQ ID NO: 3;
(iii) a polypeptide of SEQ ID NO: 4; and
(iv) a polypeptide as defined in (i), (ii) or (iii) which has been modified without loss of immunogenicity and is at least 75% identical in amino acid sequence to the corresponding polypeptide encoded by the nucleic acid of (i), (ii) or (iii).

12. A fusion protein comprising a first and a second polypeptide, wherein the first polypeptide is selected from any one of:
(i) a polypeptide encoded by a nucleic acid sequence comprising at least 36 consecutive nucleotides from SEQ ID NO: 3;
(ii) a polypeptide which is an immunogenic fragment comprising at least 12 consecutive amino acids from SEQ ID No: 4;
(iii) a polypeptide as defined in (i) or (ii), which has been modified without loss of immunogenicity and is at least 75% identical in amino acid sequence to the corresponding polypeptide of (i) or (ii).

13. The fusion protein of claim 11 or 12 wherein the second polypeptide is a heterologous signal peptide.

14. The fusion protein of claim 11 or 12 wherein the second polypeptide has adjuvant activity.

15. An antibody against the fusion protein of any one of claims 11 to 13.

16. A vaccine comprising at least one first polypeptide selected from any one of:
(i) a polypeptide encoded by SEQ ID NO: 3;
(ii) a polypeptide which is at least 75% identical in amino acid sequence to SEQ ID NO: 4 or to the polypeptide encoded by SEQ ID NO: 3;
(iii) a polypeptide of SEQ ID NO: 4; and
(iv) a polypeptide as defined in (i), (ii) or (iii) which has been modified without loss of immunogenicity and is at least 75% identical in amino acid sequence to the corresponding polypeptide encoded by the nucleic acid of (i), (ii) or (iii).

17. A vaccine comprising at least one first polypeptide selected from any one of:
(i) a polypeptide encoded by a nucleic acid sequence comprising at least 36 consecutive nucleotides from SEQ ID NO: 3;
(ii) a polypeptide which is an immunogenic fragment comprising at least 12 consecutive amino acids from SEQ ID No: 4;
(iii) a polypeptide as defined in (i) or (ii), which has been modified without loss of immunogenicity and is at least 75% identical in amino acid sequence to the corresponding polypeptide of (i) or (ii).

18. A vaccine comprising at least one fusion protein, wherein the fusion protein comprises a first and a second polypeptide, wherein the first polypeptide is selected from any one of:
(i) a polypeptide encoded by SEQ ID NO: 3;
(ii) a polypeptide which is at least 75% identical in amino acid sequence to SEQ ID NO: 4 or to the polypeptide encoded by SEQ ID NO: 3;
(iii) a polypeptide of SEQ ID NO: 4; and
(iv) a polypeptide as defined in (i), (ii) or (iii) which has been modified without loss of immunogenicity and is at least 75% identical in amino acid sequence to the corresponding polypeptide encoded by the nucleic acid of (i), (ii) or (iii).

19. A vaccine comprising at least one fusion protein, wherein the fusion protein comprises a first and a second polypeptide, wherein the first polypeptide is selected from any one of:
(i) a polypeptide encoded by a nucleic acid sequence comprising at least 36 consecutive nucleotides from SEQ ID NO: 3;
(ii) a polypeptide which is an immunogenic fragment comprising at least 12 consecutive amino acids from SEQ ID No: 4;
(iii) a polypeptide as defined in (i) or (ii), which has been modified without loss of immunogenicity and is at least 75% identical in amino acid sequence to the corresponding polypeptide of (i) or (ii).

20. The vaccine of claim 18 or 19 wherein the second polypeptide is a heterologous signal peptide.

21. The vaccine of claim 18 or 19 wherein the second polypeptide has adjuvant activity.

22. A vaccine according to any one of claims 16 or 21, further comprising an additional polypeptide which enhances the immune response to the first polypeptide.

23. The vaccine according to claim 22 wherein the additional polypeptide comprises a *Chlamydia* polypeptide.

24. A pharmaceutical composition comprising a vaccine according to any one of claims 16 to 21 and a pharmaceutically acceptable carrier.

25. A pharmaceutical composition comprising an antibody according to claim 15 and a pharmaceutically acceptable carrier.

26. A method for preventing or treating *Chlamydia* infection comprising administering to a mammal an effective amount of the vaccine of any one of claims 1 to 9 and 16 to 23.

27. A method for preventing or treating *Chlamydia* infection comprising administering to a mammal an effective amount of the composition of any one of claims 10, 24 and 25.

28. A method for preventing or treating *Chlamydia* infection comprising administering to a mammal an effective amount of the fusion protein of any one of claims 11 to 14.

29. A method for preventing or treating *Chlamydia* infection comprising administering to a mammal an effective amount of the antibody of claim 15.

30. A commercial package comprising at least one nucleic acid selected from any one of:
(i) SEQ ID No: 3;
(ii) a nucleic acid sequence which encodes a polypeptide encoded by SEQ ID No: 3;
(iii) a nucleic acid sequence which encodes a polypeptide which is at least 75% identical in amino acid sequence to the polypeptide encoded by SEQ ID No: 3; and
(iv) a nucleic acid sequence which encodes a polypeptide whose sequence is set forth in SEQ ID No: 4;
(v) a nucleic acid sequence as defined in (i), (ii) or (iv), which has been modified to encode a modified polypeptide, wherein the modified polypeptide retains immunogenicity and is at least 75% identical in amino acid sequence to the corresponding polypeptide encoded by the nucleic acid of (i), (ii) or (iv);
wherein each first nucleic acid is capable of being expressed; and
instructions for use in eliciting an immunoprotective response in a mammal.

31. A commercial package comprising at least one nucleic acid selected from any one of:
(i) a nucleic acid sequence comprising at least 36 consecutive nucleotides from SEQ ID NO: 3;
(ii) a nucleic acid sequence which encodes an immunogenic fragment comprising at least 12 consecutive amino acids from SEQ ID No: 4;
(iii) a nucleic acid sequence as defined in (i) or (ii), which has been modified to encode a modified polypeptide, wherein the modified polypeptide retains immunogenicity and is at least 75% identical in amino acid sequence to the corresponding fragment of (i) or (ii);
wherein each first nucleic acid is capable of being expressed; and
instructions for use in eliciting an immunoprotective response in a mammal.

32. A commercial package comprising at least one polypeptide selected from any one of:
(i) a polypeptide encoded by SEQ ID NO: 3;
(ii) a polypeptide which is at least 75% identical in amino acid sequence to SEQ ID NO: 4 or to the polypeptide encoded by SEQ ID NO: 3;
(iii) a polypeptide of SEQ ID NO: 4; and
(iv) a polypeptide as defined in (i), (ii) or (iii) which has been modified without loss of immunogenicity and is at least 75% identical in amino acid sequence to the corresponding polypeptide encoded by the nucleic acid of (i), (ii) or (iii); and
instructions for use in eliciting an immunoprotective response in a mammal.

33. A commercial package comprising at least one polypeptide selected from any one of:
(i) a polypeptide encoded by a nucleic acid sequence comprising at least 36 consecutive nucleotides from SEQ ID NO: 3;
(ii) a polypeptide which is an immunogenic fragment comprising at least 12 consecutive amino acids from SEQ ID No: 4;
(iii) a polypeptide as defined in (i) or (ii), which has been modified without loss of immunogenicity and is at least 75% identical in amino acid sequence to the corresponding polypeptide of (i) or (ii);
and instructions for use in eliciting an immunoprotective response in a mammal.

34. Expression plasmid pCACPNM882 as shown in Figure 22.
